# EUROPEAN PATENT APPLICATION

(11) **EP 4 212 546 A1**
(43) Date of publication of application: **19.07.2023**
(21) Application number: 22209083.9
(22) Date of filing: 10.12.2015
(51) Int. Cl.: C07K 14/61, C07K 14/59, A61K 38/00, C12N 9/64

(54) **METHODS OF PRODUCING LONG ACTING CTP-MODIFIED POLYPEPTIDES**

(30) Priority: 10.12.2014 US 201462090104 P; 10.12.2014 US 201462090116 P; 10.12.2014 US 201462090124 P
(62) Divisional of application: 15867141.2
(71) Applicant: OPKO Biologics Ltd., Kiryat Gat, 8211804 (IL)
(72) Inventor: HERSHKOVITZ, Oren, 7982000 M.P. Shikmim (IL); MOSCHCOVICH, Laura, 5401844 Givat Shmuel (IL)
(74) Representative: Pearl Cohen Zedek Latzer Baratz UK LLP

(57) **Abstract**

A method of manufacturing a highly glycosylated human chorionic gonadotropin carboxy terminal peptide (CTP)-modified polypeptide that is glycosylated at between 13 and 18 O-linked glycosylation sites, the method comprising stably transfecting a predetermined number of cells with an expression vector comprising a coding portion encoding the CTP-modified polypeptide, wherein the polypeptide is a coagulation Factor VII (FVII) or an activated coagulation Factor VII (FVIIa), wherein the CTP-modified polypeptide consists of three CTP units attached to the carboxy terminus of the FVII or the FVIIa, and the transfected cells express and secrete the CTP-modified polypeptide; obtaining cell clones that overexpress the CTP-modified polypeptide; expanding the clones in solution to a predetermined scale and the clones are obtained from a working cell bank (WCB) or master cell bank (MCB) that optimally expresses and secretes the CTP-modified polypeptide; harvesting the solution containing the clones; filtering the solution containing the clones to obtain a clarified harvest solution; and purifying the clarified harvest solution to obtain a purified protein solution of the CTP-modified polypeptide, wherein at least 70% of the purified CTP-modified polypeptide is the highly glycosylated form.

## Description

### FIELD OF INVENTION

The application describes a method for manufacturing a recombinant protein or peptide of interest modified by CTP extensions in a mammalian cell culture system.

### BACKGROUND

Polypeptides are susceptible to denaturation or enzymatic degradation in the blood, liver or kidney. Accordingly, polypeptides typically have short circulatory half-lives of several hours. Because of their low stability, peptide drugs are usually delivered in a sustained frequency so as to maintain an effective plasma concentration of the active peptide. Moreover, since peptide drugs are usually administrated by infusion, frequent injection of peptide drugs cause considerable discomfort to a subject. Thus, there is a need for technologies that will prolong the half-lives of therapeutic polypeptides while maintaining a high pharmacological efficacy thereof. Such desirous peptide drugs should also meet the requirements of enhanced serum stability, high activity and a low probability of inducing an undesired immune response when injected into a subject.

Unfavorable pharmacokinetics, such as a short serum half-life, can prevent the pharmaceutical development of many otherwise promising drug candidates. Serum half-life is an empirical characteristic of a molecule, and must be determined experimentally for each new potential drug. For example, with lower molecular weight polypeptide drugs, physiological clearance mechanisms such as renal filtration can make the maintenance of therapeutic levels of a drug unfeasible because of cost or frequency of the required dosing regimen. Conversely, a long serum half-life is undesirable where a drug or its metabolites have toxic side effects.

Addressed herein is the problem of providing long-lasting therapeutic polypeptides, by providing methods of production of long-acting CTP-modified polypeptides of interest. Use of these long-lasting therapeutic polypeptides of interest may obviate the need for the numerous injections that may be required for a therapeutic, prophylactic or chronic treatment of a subject in need. This technology is based on the use of a natural peptide, the C-terminal peptide (CTP) of the beta chain of human chorionic gonadotropin (hCG), which provides hCG with the required longevity to maintain pregnancy (initial T_{1/2}~10 h, terminal T_{1/2}~37 h). CTP has 28 amino acids and the capacity for glycosylation at four to six O-linked sugar chains, which are all at serine residue. The beta chain of luteinizing hormone (LH), a fertility hormone that triggers ovulation, is almost identical to hCG but does not include the CTP. As a result, LH has a significantly shorter half-life in blood (initial T_{1/2}~1 h, terminal T_{1/2}~10 h).

### SUMMARY OF THE INVENTION

In one aspect, disclosed herein is a method of manufacturing a human chorionic gonadotropin carboxy terminal peptide (CTP)-modified polypeptide of interest, the method comprising the steps of: (a) stably transfecting a predetermined number of cells with an expression vector comprising a coding portion encoding said CTP-modified polypeptide of interest, wherein said transfected cell expresses and secretes said CTP-modified polypeptide of interest; (b) obtaining cell clones that overexpress said CTP-modified polypeptide of interest; (c) expanding said clones in solution to a predetermined scale; (d) harvesting said solution containing said clones; (e) filtering said solution containing said clones to obtain a clarified harvest solution; and, (f) purifying said clarified harvest solution to obtain a purified protein solution having a desired concentration of a CTP-modified polypeptide of interest, thereby manufacturing a human chorionic gonadotropin peptide (CTP)-modified polypeptide of interest.

In a related aspect, said CTP-modified polypeptide of interest is highly glycosylated. In another related aspect, each CTP comprised in a CTP-modified polypeptide comprises glycosylation at more than 4 O-linked glycosylation sites. In another related aspect, the CTP-modified polypeptide of interest is highly sialylated.

In a related aspect, the CTP-modified polypeptide of interest comprises a CTP-polypeptide of interest-CTP-CTP polypeptide. In another related aspect, the CTP-modified polypeptide of interest comprises a polypeptide of interest-CTP₍₃₎ polypeptide. In another related aspect, the CTP-modified polypeptide of interest comprises a CTP₍₁₋₅₎₋polypeptide of interest-CTP₍₁₋₅₎ polypeptide.

Other features and advantages of the polypeptides and methods of manufacturing the same, disclosed herein, will become apparent from the following detailed description examples and figures. It should be understood, however, that the detailed description and the specific examples while indicating embodiments of polypeptide, compositions comprising the same and methods of making the same, are given by way of illustration only, since various changes and modifications within the spirit and scope of the disclosure herein, will become apparent to those skilled in the art from this detailed description.

### BRIEF DESCRIPTION OF THE DRAWINGS

The subject matter disclosed herein for the polypeptides, methods of making the same and method of using the same, is particularly pointed out and distinctly claimed in the concluding portion of the specification. However, both as to the organization and the method of operation, together with objects, features, and advantages thereof, these may best be understood by reference to the following detailed description when read with the accompanying drawings in which:
**Figures 1A-1F** are diagrams illustrating six EPO-CTP constructs. **Figure 1A** is a diagram of the polypeptide of SEQ ID NO: 1. **Figure 1B** is a diagram of the polypeptide of SEQ ID NO: 2. **Figure 1C** is a diagram of the polypeptide of SEQ ID NO: 3. **Figure 1D** is a diagram of the polypeptide of SEQ ID NO: 4. **Figure 1E** is a diagram of the polypeptide of SEQ ID NO: 5. **Figure 1F** is a diagram of the polypeptide of SEQ ID NO: 6.
**Figure 2** is a photograph illustrating the expression of the EPO-CTP variants from transfected DG44 cells. Final test samples from transfected cells were prepared as described under "sample preparation" and run on SDS/PAGE. Proteins were detected by western blot.
**Figure 3** is a Western blot illustrating the molecular weight & identity of commercial interferon-β1a (AVONEX), MOD-9013 (SEQ ID NO: 9), MOD-9016 (SEQ ID NO: 10), MOD-9015 (SEQ ID NO: 11), MOD-9012 (SEQ ID NO: 12), MOD-9011 (SEQ ID NO: 13) and Mock. PAGE SDS gel was blotted and stained using monoclonal anti-IFN-β1A antibodies (B). The photograph indicates that like commercial interferon β1a (AVONEX), MOD-901X variants are recognized by anti IFN-β1A antibodies.
**Figure 4****.** Shows a graph showing the PK profile of purified FIX-CTP-CTP, rhFIX, harvest of FIX-CTP-CTP, and harvest of FIX-CTP.
**Figure 5****.** Shows anti-CTP and anti-gamma carboxylation antibodies Western blots of FIX fused to three, four or five CTPs. FIX-CTPs, FIX-CTP₄, and FIX-CTP₅ harvests were loaded on 12% Tris-Glycine gel using Precision plus dual color protein marker (Bio-Rad). The SDS-PAGE analysis was performed by Western immuno-blot using anti-CTP polyclonal Ab (Adar Biotech Production) or anti-Gla Ab (American Diagnostica).
**Figure 6****.** Shows a Coomassie blue detection of FIX-CTPs, FIX-CTP₄, and FIX-CTP₅. After a purification process utilizing a Jacalin column (immunoaffinity purification of glycosylated proteins), FIX-CTPs, FIX-CTP₄, and FIX-CTP₅ were loaded on 12% Tris-Glycine gel using Precision Plus Dual Color Protein Marker (Bio-Rad). The SDS-PAGE was stained by Coomassie blue dye for sample detection.
**Figure 7****.** Shows FIX Chromogenic activity. A comparative assessment of the *in vitro* potency of fully purified (HA column) FIX-CTP₃ FIX-CTP₄ and FIX-CTP₅ versus human pool normal plasma was performed using a commercially available chromogenic activity test kit, BIOPHEN (Hyphen BioMed 221802). All samples were serially diluted and the potency was assessed by comparing a dose response curve to a reference preparation consisting of normal human plasma.
**Figure 8****.** Shows the comparative pharmacokinetic (PK) profile of FIX-CTP₃ FIX-CTP₄ and FIX-CTP₅. FIX concentration in plasma samples were quantified using human FIX Elisa kits (Affinity Biologicals). Pharmacokinetic profile was calculated and is the mean of 3 animals at each time point. Terminal half-lives were calculated using PK Solutions 2.0 software.
**Figures 9A-9D** show diagrams of rFVII and rFIX-CTP constructs: a diagram illustrating the rFVII-CTP construct **(****Figure 9A****),** rFVII-CTP-CTP construct **(****Figure 9B****),** rFIX-CTP construct **(****Figure 9C****),** and rFIX-CTP-CTP construct **(****Figure 9D****).**

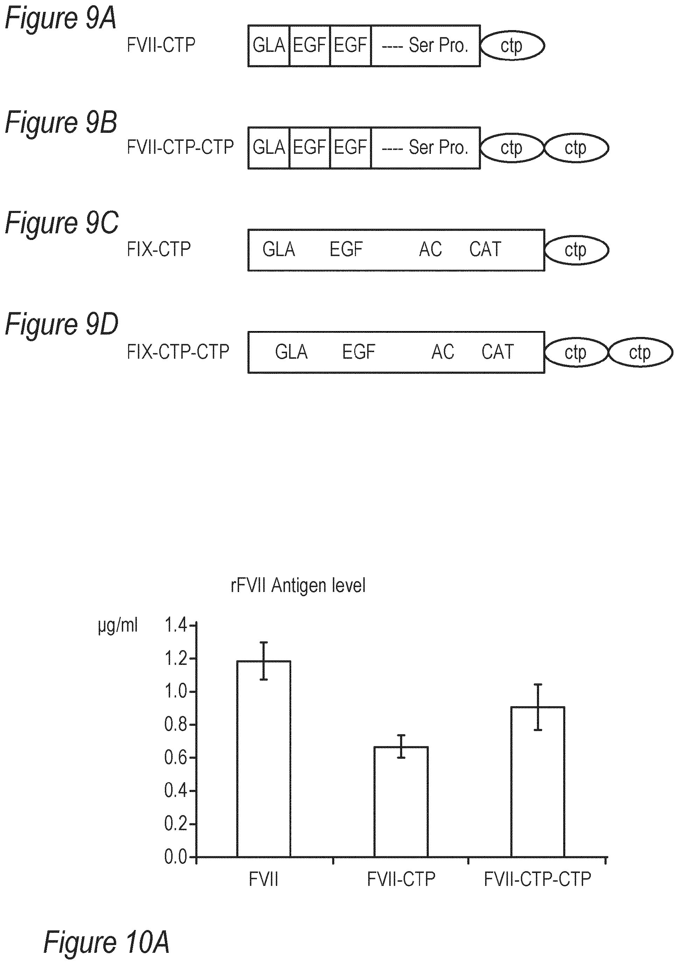

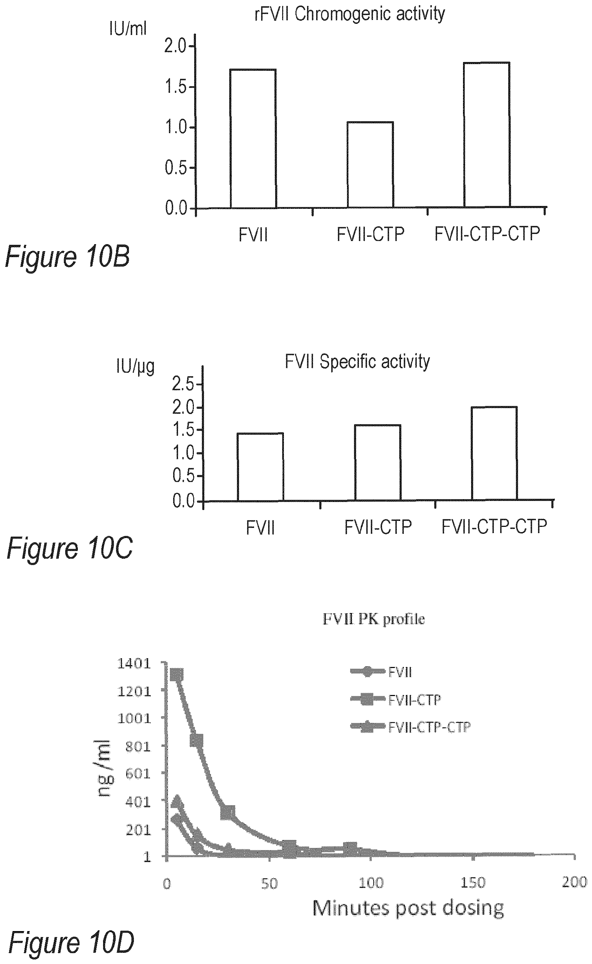

**Figures 10A-10D****.** **Figure 10A** shows a bar graph showing harvests limited diluted clone transfected and selected cells with FVII-CTP variants in the presence of 5µg/ml of Vitamin K3. The level of FVII was quantified using FVII ELISA (AssayPro). **Figure 10B** shows a bar graph showing harvests of limited diluted transfected and selected cells with FVII-CTP variants in the presence of 5µg of Vitamin K3.activity. FVII activity was quantified using FVII chromogenic activity assay (AssayPro). **Figure 10C** shows a bar graph showing harvests of limited diluted transfected and selected cells with FVII-CTP variants in the presence of 5µg of Vitamin K3. The specific activity of FVII was calculated for each version by dividing the activity value by the harvest FVII concentration. **Figure 10D** shows a graph showing PK profile of FVII, FVII-CTP-CTP, and FVII-CTP harvests.
**Figures 11A-11B** show PAGE and Western Blot (WB) analysis of purified OXM-CTP variants: OXM-CTP-CTP-CTP, OXM-CTP 4X, OXM-CTP 5X. **Figure 11A****:** Coomassie staining of OXM samples and OXM-CTP variants (10 and 2 µg protein/lane). **Figure 11B****:** WB analysis of OXM-CTP variants using anti OXM.
**Figure 12** shows PAGE analysis of samples from purification process of OXM-CTP variants: CTP-OXM-CTP, CTP-OXM-CTP-CTP, OXM-CTP-CTP and CTP-CTP-OXM.
**Figure 13** shows PK profiles of three sequential experiments of OXM peptide and OXM-CTP variants in SD-1 rats.
**Figure 14** shows the results of glucose tolerance test of OXM peptide and OXM-CTP variants as measured in C57BL/6 mice.
**Figure 15** Shows upstream process flow production chart of CTP-modified polypeptides.
**Figure 16****.** Presents a flow chart of the purification process of CTP-modified polypeptides.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION

In the following detailed description, numerous specific details are set forth in order to provide a thorough understanding of the long-acting CTP-modified polypeptides disclosed herein and methods of manufacturing the same. However, it will be understood by those skilled in the art that the long-acting CTP-modified polypeptides and methods of manufacturing disclosed herein may be practiced without these specific details. In other instances, well-known methods, procedures, and components have not been described in detail so as not to obscure the long-acting polypeptides and methods of manufacturing disclosed herein.

In one embodiment, disclosed herein are long-acting polypeptides and methods of manufacturing the same. In another embodiment, long-acting polypeptides comprise at least one carboxy terminal peptide (CTP) of human Chorionic Gonadotropin (hCG). In another embodiment, CTP acts as a protectant against degradation of proteins or peptides of interest. In another embodiment, CTP extends circulatory half-lives of proteins or peptides of interest. In some embodiments, CTP enhances the potency of proteins or peptides of interest.

A skilled artisan would recognize that the terms "CTP peptide", "carboxy terminal peptide" and "CTP sequence" may be used interchangeably herein having the same qualities and meanings. In another embodiment, the carboxy terminal peptide is a full-length CTP. In another embodiment, the carboxy terminal peptide is a truncated CTP.

A skilled artisan would recognize that the terms "signal sequence" and "signal peptide" may be used interchangeably herein. A skilled artisan would recognize that the term "sequence" when in reference to a polynucleotide may encompass a coding portion of that sequence.

A skilled artisan would recognize that the terms "polypeptide of interest", "peptide of interest", "peptide", and "polypeptide sequence of interest" may be used interchangeably herein. In another embodiment, the polypeptide of interest is a full-length protein. In another embodiment, the polypeptide of interest is a protein fragment. In one embodiment, a polypeptide of interest is a peptide. In one embodiment, a polypeptide of interest is an amino acid (AA) sequence. In some embodiments, a "polypeptide of interest" is a recombinant polypeptide.

In one embodiment, a polypeptide of interest comprises an erythropoietin (EPO). In another embodiment, a polypeptide of interest comprises an interferon (IFN). In another embodiment, a polypeptide of interest comprises a cytokine. In another embodiment, a polypeptide of interest comprises a coagulation factor. In another embodiment, a coagulation factor comprises Factor VII (FVII), activated Factor VIIa (FVIIa), or Factor IX (FIX). In another embodiment, a polypeptide of interest comprises an oxyntomodulin (OXM) peptide or portion thereof. In another embodiment, a polypeptide of interest comprises a glucagon-like peptide 1 (GLP-1) peptide. In another embodiment, a polypeptide of interest comprises a dual GLP-1/Glucagon receptor agonist. In another embodiment, a polypeptide of interest is not a growth hormone. In another embodiment, a polypeptide of interest is not a human growth hormone. CTP-modified polypeptides of interest are described, for example, in U.S. Patent No. 8,110,376; U.S. Patent No. 8,323,636; U.S. Patent No. 8,426,166; International Application Publication No. WO 2013/121416; International Application Publication No. WO 2013/157002; and International Application Publication WO 2007/094985, each of which are incorporated herein in full.

In one embodiment, disclosed herein is a method of manufacturing a human chorionic gonadotropin carboxy terminal peptide (CTP)-modified polypeptide of interest, the method comprising the steps of: (a) stably transfecting a predetermined number of cells with an expression vector comprising a coding portion encoding said CTP-modified polypeptide of interest, wherein said transfected cell expresses and secretes said CTP-modified polypeptide of interest; (b) obtaining cell clones that overexpress said CTP-modified polypeptide of interest; (c) expanding said clones in solution to a predetermined scale; (d) harvesting said solution containing said clones; (e) filtering said solution containing said clones to obtain a clarified harvest solution; and, (f) purifying said clarified harvest solution to obtain a purified protein solution having a desired concentration of a CTP-modified polypeptide of interest, thereby manufacturing a CTP-modified polypeptide of interest polypeptide. In another embodiment, a method of manufacturing a human chorionic gonadotropin carboxy terminal peptide (CTP)-modified polypeptide of interest comprises the steps of: (a) stably transfecting a predetermined number of cells with an expression vector comprising a coding portion encoding said CTP-modified polypeptide of interest, wherein said transfected cell expresses said CTP-modified polypeptide of interest; (b) obtaining cell clones that overexpress said CTP-modified polypeptide of interest; (c) expanding said clones in solution to a predetermined scale; (d) harvesting said solution containing said clones; (e) filtering said solution containing said clones to obtain a clarified harvest solution; and, (f) purifying said clarified harvest solution to obtain a purified protein solution having a desired concentration of a CTP-modified polypeptide of interest, thereby manufacturing a CTP-modified polypeptide of interest polypeptide. In another embodiment, said expressed CTP-modified polypeptide of interest is isolated from the cell clones directly.

In another embodiment, disclosed herein is a method of manufacturing a CTP-modified polypeptide of interest with increased glycosylation content. In another embodiment, a CTP-modified polypeptide of interest manufactured by the methods disclosed herein, has increased occupancy of O-linked glycosylation sites.

### Human chorionic gonadotropin carboxy terminal peptide (CTP)-modified polypeptides

A skilled artisan would recognize that the phrase "polypeptide" or "polypeptide sequence of interest" may encompass any polypeptide or peptide sequence, such as one comprising a biological activity. In another embodiment, the polypeptide of interest is glycosylated. In another embodiment, the polypeptide of interest is not glycosylated. Examples of polypeptides and peptides of interest, which benefit from an extension in their circulatory half-life include, but are not limited to erythropoietin (EPO), interferon (IFN), glucagon-like peptide-1(GLP-1), cytokine, coagulation factor, FVII, FVIIa, FIX, OXM, and GLP-1/Glucagon receptor agonist. In another embodiment, a polypeptide of interest comprises a cytokine polypeptide, an erythropoietin polypeptide, an interferon polypeptide, a coagulation factor polypeptide, a Factor VII polypeptide, an activated Factor VII polypeptide, a Factor IX polypeptide, a glucagon-like peptide 1 polypeptide, a dual GLP-1/Glucagon receptor agonist polypeptide, or an oxyntomodulin polypeptide. Each possibility represents an embodiment disclosed herein.

In one embodiment, long-acting polypeptides disclosed herein comprise at least one carboxy terminal peptide (CTP) of human Chorionic Gonadotropin (hCG). In another embodiment, an at least one CTP acts as a protectant against degradation of proteins or peptides derived therefrom. In another embodiment, an at least one CTP extends circulatory half-lives of proteins or peptides derived therefrom. In some embodiments, an at least one CTP enhances the potency of proteins or peptides derived therefrom.

In one embodiment, disclosed herein are long-acting erythropoietin (EPO) polypeptides and methods of producing or manufacturing and using same. In another embodiment, disclosed herein are long-acting interferon (IFN) polypeptides and methods of producing or manufacturing and using same. In another embodiment, disclosed herein are long-acting cytokine polypeptides and methods of producing or manufacturing and using same. In another embodiment, disclosed herein are long-acting coagulation factor polypeptides and methods of producing or manufacturing and using same. In another embodiment, disclosed herein are long-acting coagulation Factor VII (FVII) polypeptides and methods of producing or manufacturing and using same. In another embodiment, disclosed herein are long-acting activated coagulation Factor VIIa (FVIIa) polypeptides and methods of producing or manufacturing and using same. In another embodiment, disclosed herein are long-acting oxyntomodulin (OXM) polypeptides and methods of producing or manufacturing and using same. In another embodiment, disclosed herein is a long-acting glucagon-like peptide 1 (GLP-1) polypeptides and methods of producing or manufacturing and using same. In another embodiment, disclosed herein is a long-acting dual GLP-1/Glucagon receptor agonist polypeptides and methods of producing or manufacturing and using same.

In another embodiment, a polypeptide comprising at least two carboxy-terminal peptide (CTP) sequences of chorionic gonadotropin attached to a polypeptide sequence-of-interest, wherein a first CTP sequence of the at least two CTP sequences is attached to an amino terminus of the polypeptide sequence of interest and a second CTP sequence of the at least two CTP sequences is attached to the carboxy terminus of the polypeptide sequence of interest is provided. In another embodiment, the carboxy-terminal peptide (CTP) sequence is of human chorionic gonadotropin.

In another embodiment, the CTP is attached to the polypeptide sequence of interest via a linker. In another embodiment, the linker which connects the CTP sequence to the polypeptide sequence of interest is a covalent bond. In another embodiment, the linker which connects the CTP sequence to the polypeptide sequence of interest is a peptide bond. In another embodiment, the linker which connects the CTP sequence to the polypeptide sequence of interest is a substituted peptide bond.

In another embodiment, the CTP is fused to a protein. In another embodiment, the CTP is fused to a glycoprotein. In another embodiment, the CTP is fused to a glycoprotein hormone. In another embodiment, the CTP is fused to a peptide derived from a glycoprotein hormone. In some embodiments, glycoprotein hormones comprise EPO, FSH, or TSH and peptides derived therefrom.

In some embodiments, a CTP sequences at both the amino terminal end of a polypeptide and at the carboxy terminal end of the polypeptide provide enhanced protection against degradation of a protein. In some embodiments, CTP sequences at both the amino terminal end of a polypeptide and at the carboxy terminal end of the polypeptide provide extended half-life of the attached protein.

In some embodiments, a CTP sequence at the amino terminal end of a polypeptide, a CTP sequence at the carboxy terminal end of the polypeptide, and at least one additional CTP sequence attached in tandem to the CTP sequence at the carboxy terminus provide enhanced protection against degradation of a protein. In some embodiments, a CTP sequence at the amino terminal end of a polypeptide, a CTP sequence at the carboxy terminal end of the polypeptide, and at least one additional CTP sequence attached in tandem to the CTP sequence at the carboxy terminus provide extended half-life of the attached protein. In some embodiments, a CTP sequence at the amino terminal end of a polypeptide, a CTP sequence at the carboxy terminal end of the polypeptide, and at least one additional CTP sequence attached in tandem to the CTP sequence at the carboxy terminus provide enhanced activity of the attached protein.

In some embodiments, a CTP sequence at the amino terminal end of a polypeptide, a CTP sequence at the carboxy terminal end of the polypeptide, and at least one additional CTP sequence attached in tandem to the CTP sequence at the amino terminus provide enhanced protection against degradation of the attached protein. In some embodiments, a CTP sequence at the amino terminal end of a polypeptide, a CTP sequence at the carboxy terminal end of the polypeptide, and at least one additional CTP sequence attached in tandem to the CTP sequence at the amino terminus provide extended half-life of the attached protein. In some embodiments, a CTP sequence at the amino terminal end of a polypeptide, a CTP sequence at the carboxy terminal end of the polypeptide, and at least one additional CTP sequence attached in tandem to the CTP sequence at the amino terminus provide enhanced activity the attached protein.

In one embodiment, a CTP sequence comprises an amino acid sequence selected from the sequences set forth in SEQ ID NO: 14 and SEQ ID NO: 15. In another embodiment, SEQ ID NO: 14 comprise the following amino acid (AA) sequence: DPRFQDSSSSKAPPPSLPSPSRLPGPSDTPIL (SEQ ID NO: 14). In another embodiment, SEQ ID NO: 15 comprise the following amino acid (AA) sequence: SSSSKAPPPSLPSPSRLPGPSDTPILPQ (SEQ ID NO: 15).

In another embodiment, the CTP peptide comprises the amino acid sequence from amino acid 112 to position 145 of human chorionic gonadotropin, as set forth in SEQ ID NO: 14. In another embodiment, the CTP sequence comprises the amino acid sequence from amino acid 118 to position 145 of human chorionic gonadotropin, as set forth in SEQ ID NO: 15. In another embodiment, the CTP sequence also commences from any position between positions 112-118 and terminates at position 145 of human chorionic gonadotropin. In some embodiments, the CTP sequence peptide is 28, 29, 30, 31, 32, 33 or 34 amino acids long and commences at position 112, 113, 114, 115, 116, 117 or 118 of the CTP amino acid sequence.

In another embodiment, the truncated CTP comprises the first 10 amino acids of SEQ ID NO: 15. In another embodiment, the truncated CTP comprises the first 5 amino acids of SEQ ID NO: 15, the first 6 amino acids, the first 7 amino acids, the first 8 amino acids, the first 9 amino acids, the first 11 amino acids, the first 12 amino acids, the first 13 amino acids, or the first 14 amino acids of SEQ ID NO: 15. Each possibility represents an embodiment disclosed herein.

In another embodiment, the CTP peptide is a variant of chorionic gonadotropin CTP which differs from the native CTP by 1-5 conservative amino acid substitutions as described in U.S. Pat. No. 5,712,122. In another embodiment, the CTP peptide is a variant of chorionic gonadotropin CTP which differs from the native CTP by 1 conservative amino acid substitution, by 2 conservative amino acid substitutions, by 3 conservative amino acid substitutions, by 4 conservative amino acid substitutions, or by 5 conservative amino acid substitutions. Each possibility represents an embodiment disclosed herein.

In another embodiment, the CTP peptide amino acid sequence is at least 70% homologous to the native CTP amino acid sequence or a peptide thereof, is at least 80%, is at least 90%, is at least 95%, or is at least 98% homologous to the native CTP amino acid sequence or a peptide thereof. Each possibility represents an embodiment disclosed herein.

In one embodiment, the CTP peptide DNA sequence encodes a peptide that is at least 70% homologous to the native CTP DNA sequence or a peptide thereof. In another embodiment, the CTP peptide DNA sequence encodes a peptide that is at least 80% homologous to the native CTP DNA sequence or a peptide thereof, is at least 90%, is at least 95% homologous, is at least 98% homologous to the native CTP DNA sequence or peptide thereof. Each possibility represents an embodiment disclosed herein.

In one embodiment, at least one of the chorionic gonadotropin CTP amino acid sequences is truncated. In another embodiment, both of the chorionic gonadotropin CTP amino acid sequences are truncated. In another embodiment, 2 of the chorionic gonadotropin CTP amino acid sequences are truncated. In another embodiment, 2 or more of the chorionic gonadotropin CTP amino acid sequences are truncated. In another embodiment, all of the chorionic gonadotropin CTP amino acid sequences are truncated. In one embodiment, the truncated CTP comprises the first 10 amino acids of SEQ ID NO: 16. In one embodiment, the truncated CTP comprises the first 11 amino acids of SEQ ID NO: 16. In one embodiment, the truncated CTP comprises the first 12 amino acids of SEQ ID NO: 16. In another embodiment, SEQ ID NO: 16 comprises the amino acid (AA) sequence: SSSSKAPPPSLP.

In one embodiment, at least one of the CTP amino acid sequences is glycosylated. In another embodiment, both of the CTP amino acid sequences are glycosylated. In another embodiment, 2 of the CTP amino acid sequences are glycosylated. In another embodiment, 3 of the CTP amino acid sequences are glycosylated. In another embodiment, 4 of the CTP amino acid sequences are glycosylated. In another embodiment, 5 of the CTP amino acid sequences are glycosylated. In another embodiment, 2 or more of the CTP amino acid sequences are glycosylated. In another embodiment, all of the CTP amino acid sequences are glycosylated.

In one embodiment, the CTP sequence disclosed herein comprises at least one glycosylation site. In another embodiment, the CTP sequence disclosed herein comprises 2 glycosylation sites, 3 glycosylation sites, 4 glycosylation sites, 5 glycosylation sites, or 6 glycosylation sites. In one embodiment, one or more of the CTP amino acid sequences is fully glycosylated. In another embodiment, one or more of the CTP amino acid sequences is partially glycosylated. In one embodiment, partially glycosylated indicates that one of the CTP glycosylation sites is glycosylated. In another embodiment, two of the CTP glycosylation sites are glycosylated. In another embodiment, three of the CTP glycosylation sites are glycosylated.

In some embodiments, the CTP sequence modification is advantageous in permitting the usage of lower dosages. In some embodiments, the CTP sequences modification is advantageous in permitting fewer dosages. In some embodiments, the CTP sequences modification is advantageous in permitting a safe, long-acting effect.

In another embodiment, disclosed herein is a polypeptide consisting of a cytokine, a single CTP attached to the amino terminus of the cytokine, and two CTPs attached to the carboxy terminus of the cytokine. In another embodiment, disclosed herein is a polypeptide consisting of a cytokine, a single CTP attached to the amino terminus of the cytokine, two CTPs attached to the carboxy terminus of the cytokine, and a signal peptide attached to the amino terminus of one chorionic gonadotropin carboxy terminal peptide.

In another embodiment, a cytokine is a low molecular weight protein. In another embodiment, a cytokine is a protein secreted by a cell. In another embodiment, a cytokine induces and/or regulates an immune response. In another embodiment, a cytokine has a high affinity binding to a specific receptor or receptors. In another embodiment, cytokines as described herein include mimetics of cytokines that can be used to inhibit or potentiate their effects *in vivo.* In another embodiment, a cytokine comprises an autocrine activity. In another embodiment, a cytokine comprises a paracrine activity. In another embodiment, a cytokine comprises an endocrine activity.

In another embodiment, the cytokine is a Hematopoietin cytokine. In another embodiment, the cytokine is an Interferon cytokine. In another embodiment, the cytokine is a chemokine. In another embodiment, the cytokine is a Tumor Necrosis Factor cytokine. In another embodiment, a cytokine as used herein comprises biological activity and clinical efficacy. In another embodiment, a cytokine as used herein is a therapeutic protein .

In another embodiment, a CTP-modified cytokine as described herein has enhanced biological activity *in vivo* compared the same cytokine without CTPs. In another embodiment, a cytokine comprising at least one CTP attached to its amino terminus and at least two CTPs attached to its carboxy terminus has enhanced biological activity *in vivo* compared the same cytokine without CTPs. In another embodiment, a cytokine comprising one CTP attached to its amino terminus and two CTPs attached to its carboxy terminus has enhanced biological in vivo activity compared the same cytokine in without CTPs.

In another embodiment, a CTP-modified cytokine is used to facilitate organ transplantation. In another embodiment, a CTP-modified cytokine is used to reduce inflammation. In another embodiment, a CTP-modified cytokine is used to induce erythropoiesis. In another embodiment, a CTP-modified cytokine is used to induce growth. In another embodiment, a CTP-modified cytokine is used to induce weight gain. In another embodiment, a CTP-modified cytokine is used in cancer therapy as will be readily understood by one of average skill in the art. In another embodiment, a CTP-modified cytokine is used to induce an immune response. In another embodiment, a CTP-modified cytokine is used in infectious disease therapy as will be readily understood by one of average skill in the art. In another embodiment, a CTP-modified cytokine is used in treating allergy as will be readily understood by one of average skill in the art.

In another embodiment, a CTP-cytokine-CTP-CTP as described herein comprises a cytokine or an active fragment thereof connected via a peptide bond to at least one CTP unit. In another embodiment, a CTP-cytokine-CTP-CTP as described herein comprises a cytokine or an active fragment thereof connected via a peptide bond to at least one CTP unit which is connected to an additional CTP unit via a peptide bond. In another embodiment, a polypeptide as described herein comprising a cytokine fragments thereof and CTP units and/or fragments thereof are interconnected via a peptide bond. In another embodiment, one nucleic acid molecule encodes a polypeptide as described herein comprising a cytokine and/or fragments thereof and CTP units and/or fragments thereof.

A skilled artisan would appreciate that a polypeptide of interest disclosed herein may encompass a homologue. In one embodiment, a homologue comprises a deletion, insertion, or substitution variant, including an amino acid substitution, thereof and biologically active polypeptide fragments thereof. In another embodiment, homologues comprise polypeptides which are at least 50%, at least 55%, at least 60%, at least 65%, at least 70%, at least 75%, at least 80%, at least 85%, at least 87%, at least 89%, at least 91%, at least 93%, at least 95%, at least 96%, at least 97%, at least 98%, or at least 99% homologous to a polypeptide of interest as determined using BlastP software of the National Center of Biotechnology Information (NCBI) using default parameters. Each possibility represents an embodiment disclosed herein.

In one embodiment, the cytokine is a homologue.

In another embodiment, disclosed herein is a polypeptide consisting of a cytokine antagonist, a single chorionic gonadotropin carboxy terminal peptide attached to the amino terminus of the cytokine antagonist, and two chorionic gonadotropin carboxy terminal peptides attached to the carboxy terminus of the cytokine antagonist. In another embodiment, disclosed herein is a polypeptide consisting of a cytokine antagonist, a single chorionic gonadotropin carboxy terminal peptide attached to the amino terminus of the cytokine antagonist, two chorionic gonadotropin carboxy terminal peptides attached to the carboxy terminus of the cytokine antagonist, and a signal peptide attached to the amino terminus of one chorionic gonadotropin carboxy terminal peptide.

In another embodiment, a cytokine antagonist modified with CTPs is applied as an anti-cytokine strategy. In another embodiment, a cytokine antagonist modified with CTPs is effective in decrease an inflammatory response. In another embodiment, a cytokine antagonist modified with CTPs is more effective in decreasing an inflammatory response compared to an unmodified cytokine antagonist. In another embodiment, a cytokine antagonist modified with CTPs is more stable than an unmodified cytokine antagonist. In another embodiment, a cytokine antagonist modified with CTPs is more stable in-vivo than an unmodified cytokine antagonist. In another embodiment, a cytokine antagonist modified with CTPs is more bioactive than an unmodified cytokine antagonist.

In another embodiment, a cytokine antagonist is a cytokine homologue. In another embodiment, a cytokine antagonist is a soluble fragment of a cytokine receptor. In another embodiment, a cytokine antagonist is a chemokine receptor homologue.

In another embodiment, a cytokine as described herein is involved in cytokine signaling cascade comprising Ras-MAP kinase pathway. In another embodiment, a cytokine as described herein is involved in induction of JNK. In another embodiment, a cytokine as described herein is involved in induction of p38MAP. In another embodiment, a cytokine as described herein induces cell proliferation. In another embodiment, a cytokine as described herein is involved in cytokine signaling cascade comprising the JAK/STAT pathway. In another embodiment, a cytokine as described herein induces cell growth inhibition. In another embodiment, a cytokine as described herein induces differentiation.

In another embodiment, a cytokine as described herein is a four α-helix bundle cytokine. In another embodiment, a cytokine as described herein is a long-chain 4-helix bundle cytokine. In another embodiment, a cytokine as described herein is a short-chain 4-helix bundle cytokine.

In another embodiment, a cytokine as described herein is a beta-trefoil cytokine. In another embodiment, a cytokine as described herein is a beta-sandwich cytokine. In another embodiment, a cytokine as described herein is an EGF-like cytokine. In another embodiment, a cytokine as described herein comprises a cystine knot dimerization domain. In another embodiment, a cytokine as described herein comprises both alpha and beta chains. In another embodiment, a cytokine as described herein is an alpha superfamily cytokine such as IL-2, IL-4, IL-5, GM-CSF, IL-3, IFN-alpha, or IL-13. In another embodiment, a cytokine as described herein is a dimeric 4-helix bundles cytokine. In another embodiment, a cytokine as described herein is a member of the IL family of cytokines.

In another embodiment, a cytokine as described herein is a long-chain 4-helix bundle superfamily cytokine such as G-CSF, Myelomonocytic growth factor, IL-6, IL-3, IL-7, LIF, Oncostatin M, Ciliary neurotrophic factor (CNTF), or cholinergic differentiation factor (CDF). In another embodiment, a cytokine as described herein is a short-chain 4-helix bundle superfamily cytokine such as IL-2, IL-4, IL-13, IFN-alpha, IL-5, GM-CSF, IL-3, Macrophage colony-stimulating factor (M-CSF). In another embodiment, a cytokine as described herein is a dimeric 4-helix bundles such as IFN-Gamma, IL-10, or IFN-Beta.

In another embodiment, a cytokine as described herein is a Beta-Trefoil cytokine such as IL1-A, IL1-B, or FGF. In another embodiment, a cytokine as described herein is a Beta-sandwich cytokine such as TNF-alpha, or TNF-Beta. In another embodiment, a cytokine as described herein is an EGF-like cytokine such as TGF-Alpha. In another embodiment, a cytokine as described herein comprises cystine knot dimerization domains. In another embodiment, Gonadotropin, Nerve Growth Factor (NGF), Platelet-derived growth factor (PDGF), and TGF-Beta2 comprise cystine knot dimerization domains. In another embodiment, a cytokine as described herein comprises both alpha and beta chains. In another embodiment IL-8, IP10, platelet factor 4 (PF-4), bTG, GRO, 9E3, HLA-A2, macrophage inflammatory protein 1 alpha (MIP-1 alpha), macrophage inflammatory protein 1 beta (MIP-1 beta), and Melanoma growth stimulating activity (MGSA) comprise both alpha and beta chains. Each possibility represents an embodiment disclosed herein.

In another embodiment, a cytokine as described herein binds a hematopoietin-receptor family member (also called the class I cytokine receptor family. In another embodiment, a cytokine as described herein binds a class II cytokine receptor (interferons or interferon-like cytokines). In another embodiment, a cytokine as described herein binds a tumor necrosis factor-receptor (TNFR). In another embodiment, a cytokine as described herein binds a, chemokine-receptor. In another embodiment, a cytokine as described herein binds a G protein-coupled receptor.

In another embodiment, a cytokine as described herein is an IL-2 cytokine, is an interferon, is an IL-10 cytokine, is erythropoietin (EPO), thrombopoietin (THPO), is IL-1, IL-18, or IL-17. In another embodiment, a cytokine as described herein promotes proliferation of T-cells. Each possibility represents an embodiment disclosed herein.

In another embodiment, a cytokine as described herein is close to the cluster formed by ciliary neurotrophic factor and granulocyte colony-stimulating factor (CSF)

In another embodiment, a cytokine as described herein enhances cytokine responses, type 1 (IFN-γ, TGF-β etc.). In another embodiment, a cytokine as described herein enhances antibody responses, type 2 (IL-4, IL-10, IL-13, etc) .

In another embodiment, a cytokine is a peptide. In another embodiment, the cytokine is glycosylated. In another embodiment, a cytokine is a polypeptide. In another embodiment, a cytokine as described herein is a modified cytokine comprising at least one CTP peptide attached to an amino terminus of said cytokine and at least two chorionic gonadotropin carboxy terminal peptides attached to a carboxy terminus of said cytokine. In another embodiment, a cytokine as described herein is a modified cytokine consisting of a cytokine, one CTP peptide attached to an amino terminus of the cytokine, and at least two chorionic gonadotropin carboxy terminal peptides attached to a carboxy terminus of the cytokine. In another embodiment, a cytokine as described herein is a modified cytokine consisting a cytokine, at least one CTP peptide attached to an amino terminus of the cytokine, and two chorionic gonadotropin carboxy terminal peptides attached to a carboxy terminus of the cytokine. In another embodiment, a cytokine as described herein is a modified cytokine consisting a cytokine, one CTP peptide attached to an amino terminus of the cytokine, and two chorionic gonadotropin carboxy terminal peptides attached to a carboxy terminus of the cytokine.

In another embodiment, the carboxy-terminal peptide (CTP) is attached to the cytokine via a linker.

In one embodiment, the polynucleotides disclosed herein further comprise a signal sequence encoding a signal peptide for the secretion of the CTP-modified polypeptide of interest disclosed herein. In another embodiment, the signal sequence is N-terminal to the CTP sequence that is in turn N-terminal to the polypeptide sequence of interest; e.g. the sequence is (a) signal sequence- (b) CTP- (c) sequence-of-interest- (d) optionally 1 or more additional CTP sequences. In another embodiment, 1 or more CTP sequences is inserted between the signal sequence of a polypeptide sequence of interest and the polypeptide sequence of interest itself, thus interrupting the wild-type sequence of interest. In another embodiment, the signal sequence is N-terminal to the polypeptide of interest sequence that is in turn N-terminal to at least one CTP sequence. In another embodiment, following expression and secretion of a CTP-modified polypeptide of interest, the signal peptide is cleaved from the precursor proteins resulting in the mature proteins. In another embodiment, a CTP-modified polypeptide of interest is expressed as a precursor protein including a signal peptide, wherein upon secretion from the cell the signal peptide is cleaved from the precursor protein resulting in secretion of a mature form of the CTP-modified polypeptide of interest.

In some embodiments, signal sequences include, but are not limited to the endogenous signal sequence for EPO as set forth in SEQ ID NO: 19 or the endogenous signal sequence for IFN-β1 as set forth in SEQ ID NO: 108. In another embodiment, the signal sequence is N-terminal to the CTP sequence that is in turn N-terminal to the polypeptide sequence of interest; e.g. the sequence is (a) signal sequence- (b) CTP- (c) sequence-of-interest- (d) optionally 1 or more additional CTP sequences. In another embodiment, 1 or more CTP sequences is inserted between the signal sequence of a polypeptide sequence of interest and the polypeptide sequence of interest itself, thus interrupting the wild-type sequence of interest.

In some embodiments, a CTP sequences at both the amino terminal end of a cytokine and at the carboxy terminal end of the cytokine provide enhanced protection against degradation of a cytokine. In another embodiment, at least one CTP sequence at the amino terminal end of a cytokine and two CTP units in the carboxy terminal end of a cytokine provide enhanced protection against clearance. In another embodiment, at least one CTP sequence at the amino terminal end of a cytokine and two CTP units in the carboxy terminal end of a cytokine provide prolonged clearance time. In another embodiment, at least one CTP sequence at the amino terminal end of a cytokine and two CTP units in the carboxy terminal end of a cytokine enhance Cmax of a cytokine. In another embodiment, at least one CTP sequence at the amino terminal end of a cytokine and two CTP units in the carboxy terminal end of a cytokine enhance Tmax of a cytokine. In another embodiment, at least one CTP sequence at the amino terminal end of a cytokine and two CTP units in the carboxy terminal end of a cytokine enhanced T1/2.

In some embodiments, CTP sequences at both the amino terminal end of a cytokine and at the carboxy terminal end of the cytokine extend the half-life of the attached cytokine. In another embodiment, at least a single CTP sequence at the amino terminal end of a cytokine and at least two CTP sequences at the carboxy terminal end of the cytokine provide extended half-life to the modified cytokine. In another embodiment, a single CTP sequence at the amino terminal end of a cytokine and two CTP sequences at the carboxy terminal end of the cytokine provide extended half-life to the attached cytokine. In another embodiment, a single CTP sequence at the amino terminal end of a cytokine and two CTP sequences in tandem at the carboxy terminal end of the cytokine provide extended half-life to the cytokine.

In some embodiments, a CTP sequence at the amino terminal end of the cytokine a CTP sequence at the carboxy terminal end of the cytokine, and at least one additional CTP sequence attached in tandem to the CTP sequence at the carboxy terminus provide enhanced protection against degradation to a cytokine. In some embodiments, a CTP sequence at the amino terminal end of a cytokine, a CTP sequence at the carboxy terminal end of the cytokine, and at least one additional CTP sequence attached in tandem to the CTP sequence at the carboxy terminus extend the half-life of the cytokine. In some embodiments, a CTP sequence at the amino terminal end of a cytokine, a CTP sequence at the carboxy terminal end of the cytokine, and at least one additional CTP sequence attached in tandem to the CTP sequence at the carboxy terminus enhance the biological activity of the cytokine.

In another embodiment, CTP conjugated cytokines disclosed herein are used in the same manner as unmodified cytokines. In another embodiment, conjugated cytokines disclosed herein have an increased circulating half-life and plasma residence time, decreased clearance, and increased clinical activity *in vivo.* In another embodiment, due to the improved properties of the conjugated cytokines as described herein, these conjugates are administered less frequently than unmodified cytokines. In another embodiment, conjugated cytokines as described herein is administered once a week instead of the three times a week for unmodified cytokines. In another embodiment, decreased frequency of administration will result in improved patient compliance leading to improved treatment outcomes, as well as improved patient quality of life. In another embodiment, compared to conventional conjugates of cytokines linked to poly(ethylene glycol) it has been found that conjugates having the molecular weight and linker structure of the conjugates disclosed herein have an improved potency, improved stability, elevated AUC levels, enhanced circulating half-life. In another embodiment, compared to conventional conjugates of cytokines linked to poly(ethylene glycol) it has been found that EPO having the molecular weight and linker structure of the conjugates disclosed herein have an improved potency, improved stability, elevated AUC levels, enhanced circulating half-life. In another embodiment, a therapeutically effective amount of a conjugated cytokine is the amount of conjugate necessary for the *in vivo* measurable expected biological activity.

In another embodiment, a therapeutically effective amount of a conjugated EPO is the amount of EPO conjugate necessary for the in biological activity of inducing bone marrow cells to increase production of reticulocytes and red blood cells. In another embodiment, a therapeutically effective amount of a conjugated cytokine is determined according to factors as the exact type of condition being treated, the condition of the patient being treated, as well as the other ingredients in the composition. In another embodiment, a therapeutically effective amount of a conjugated cytokine is 0.01 to 10 µg per kg body weight administered once a week. In another embodiment, a therapeutically effective amount of a conjugated cytokine is 0.1 to 1 µg per kg body weight, administered once a week. In another embodiment, a pharmaceutical composition comprising a conjugated cytokine is formulated at a strength effective for administration by various means to a human patient.

In another embodiment, a polypeptide comprising a cytokine and CTP units as described herein is used as an anti-tumor agent. In another embodiment, a polypeptide comprising an IFN α and CTP units as described herein is used as an anti-tumor agent. In another embodiment, a polypeptide comprising an IFN α and CTP units is formulated in a pharmaceutical composition that is administered to a patient afflicted with cancer.

In another embodiment, a polypeptide comprising an IFN protein and CTP units as described herein is used equivalently to a regular or a recombinant interferon as known to one of average skill in the art. In another embodiment, a polypeptide comprising an IFN protein and CTP units is formulated equivalently to a regular or a recombinant interferon as known to one of average skill in the art.

In another embodiment, a polypeptide comprising a cytokine and CTP units as described herein modulates an immune response. In another embodiment, a polypeptide comprising a cytokine and CTP units as described herein modulates a cellular immune response. In another embodiment, a polypeptide comprising a cytokine and CTP units as described herein modulates an antibody immune response. In another embodiment, a polypeptide comprising a cytokine and CTP units as described herein inhibits an immune response as described herein. In another embodiment, a polypeptide comprising a cytokine and CTP units as described herein trigger an immune response as described herein.

In another embodiment, a polypeptide comprising an IFN inhibits the activity of T-cells, while simultaneously reducing the production cytokines that operate in the inflammatory response to infection and injury. In another embodiment, a polypeptide comprising an IFN protein and CTP units as described herein enhances the activity of T-cells, while simultaneously reducing the production cytokines that operate in the inflammatory response to infection and injury. In another embodiment, a polypeptide comprising an IFN protein and CTP units is formulated in a pharmaceutical composition that is administered to a patient in need of T-cells activity enhancement. In another embodiment, a polypeptide comprising an IFN protein and CTP units is formulated in a pharmaceutical composition that is administered to a patient afflicted with multiple sclerosis. In another embodiment, a polypeptide comprising an IFN protein and CTP units is formulated in a pharmaceutical composition that is administered to a patient afflicted with hepatitis C infection.

In another embodiment, a cytokine is an interferon (IFN). In another embodiment, a cytokine is a type I interferon. In another embodiment, the interferon (IFN) is IFN-α2a. In another embodiment, the interferon (IFN) is IFN-α2b In another embodiment, the interferon (IFN) is IFN-β1a. In another embodiment, the interferon (IFN) is IFN-α2b In another embodiment, the interferon (IFN) is IFN-β1b. In another embodiment, a signal peptide of IFN-β1 is set forth in SEQ ID NO: 108: MTNKCLLQIALLLCFSTTALS (SEQ ID NO: 108).

A skilled artisan would appreciate that the term erythropoietin (EPO) may encompass the terms "EPO peptide" and "EPO sequence" and may be used interchangeably herein. In another embodiment, the EPO peptide is an EPO protein.

In some embodiments, erythropoietin (EPO) is utilized according to the teachings disclosed herein. In some embodiments, any EPO encoding amino acid sequence is an EPO sequence. In some embodiments, any EPO encoding nucleic acid sequence is an EPO sequence In some embodiments, the attachment of CTP sequence to both the amino and carboxy termini of the EPO protein results in increased potency at stimulating erythropoiesis, as compared to recombinant EPO and other combinations of EPO and CTP. In some embodiments, an EPO attached to three CTP sequences does not impair binding to its receptor, as evidenced in US Patent No. 8,110,376 incorporated herein in full, which demonstrates that EPO attached to three CTP sequences is equally effective at stimulating proliferation of TF-1 cells as wild-type EPO. In some embodiments CTP-modified EPO polypeptides disclosed herein are set forth in SEQ ID NO: 3 and SEQ ID NO: 6.

A skilled artisan would appreciate that the term "erythropoietin" may encompass mammalian erythropoietin.

A skilled artisan would appreciate that the term "EPO sequence" disclosed herein may also encompass homologues. In one embodiment, the erythropoietin amino acid sequence disclosed herein is at least 50% homologous to an EPO sequence disclosed herein, as determined using BlastP software of the National Center of Biotechnology Information (NCBI) using default parameters). In another embodiment, the EPO amino acid sequence disclosed herein is at least 60%, is at least 70% homologous, is at least 80%, is at least 90%, or is at least 95% homologous to an EPO sequence disclosed herein, as determined using BlastP software of the National Center of Biotechnology Information (NCBI) using default parameters).In one embodiment, an EPO substitution variant comprises a glycine in position 104 of EPO amino acid sequence is substituted by a serine (SEQ ID NO: 7).

In another embodiment, the methods disclosed herein manufacture an EPO peptide having additionally at least one CTP amino acid peptide on the N-terminus and at least one CTP amino acid peptide on the C-terminus for the treatment of anemia. In another embodiment, the methods disclosed herein manufacture an EPO peptide having additionally one CTP amino acid peptide on the N-terminus and two CTP amino acid peptides on the C-terminus for the treatment of anemia. In another embodiment, the methods disclosed herein manufacture an EPO peptide set forth in SEQ ID NO: 1 having additionally at least one CTP amino acid peptide on the N-terminus for the treatment of anemia. In another embodiment, SEQ ID NO: 1 comprises the following amino acid (AA) sequence: MGVHECPAWLWLLLSLLSLPLGLPVLGAPPRLICDSRVLERYLLEAKEAENITTGCAEHC SLNENITVPDTVNFYAWKRMEVGQQAVEVWQGLALLSEAVLRGQALLVNSSQPWEPL QLHVDKAVSGLRSLTTLLRALGAQKEAISPPDAASAAPLRTITADTFRKLFRVYSNFLRG KLKLYTGEACRTGDRSSSSKAPPPSLPSPSRLPGPSDTPILPQ. In another embodiment, the methods disclosed herein manufacture an EPO peptide set forth in SEQ ID NO: 1 having additionally at least one CTP amino acid peptide on the N-terminus and at least one additional CTP amino acid peptide on the C-terminus for the treatment of anemia.

In another embodiment, the methods disclosed herein manufacture an EPO peptide set forth in SEQ ID NO: 2 having additionally at least one CTP amino acid peptide on the N-terminus and at least one CTP amino acid peptide on the C-terminus for the treatment of anemia. In another embodiment, SEQ ID NO: 2 comprises the following amino acid (AA) sequence:

MGVHECPAWLWLLLSLLSLPLGLPVLGAPPRLICDSRVLERYLLEAKEAENITTGCAEHC
SLNENITVPDTVNFYAWKRMEVGQQAVEVWQGLALLSEAVLRGQALLVNSSQPWEPL
QLHVDKAVSGLRSLTTLLRALGAQKEAISPPDAASAAPLRTITADTFRKLFRVYSNFLRG
KLKLYTGEACRTGDRSSSSKAPPPSLPSPSRLPGPSDTPILPQSSSSKAPPPSLPSPSRLPGPS
DTPILPQ.

In another embodiment, the methods disclosed herein manufacture an EPO peptide set forth in SEQ ID NO: 3 having additionally at least one CTP amino acid peptide on the N-terminus and at least one CTP amino acid peptide on the C-terminus for the treatment of anemia. In one embodiment, SEQ ID NO: 3 comprises the following amino acid (AA) sequence:

MGVHECPAWLWLLLSLLSLPLGLPVLGSSSSKAPPPSLPSPSRLPGPSDTPILPQAPPRLIC
DSRVLERYLLEAKEAENITTGCAEHCSLNENITVPDTKVNFYAWKRMEVGQQAVEVWQ
GLALLSEAVLRGQALLVNSSQPWEPLQLHVDKAVSGLRSLTTLLRALGAQKEAISPPDA
ASAAPLRTITADTFRKLFRVYSNFLRGKLKLYTGEACRTGDRSSSSKAPPPSLPSPSRLPG
PSDTPILPQSSSSKAPPPSLPSPSRLPGPSDTPILPQ.

In one embodiment, following expression a mature CTP-modified EPO polypeptide is secreted, the signal peptide having been cleaved from the precursor protein resulting in a mature protein. For example, in the precursor CTP-modified EPO polypeptide set forth in SEQ ID NO: 3, amino acids 1-27: MGVHECPAWLWLLLSLLSLPLGLPVLG (SEQ ID NO: 19) represent the signal peptide of the precursor CTP-modified EPO polypeptide, and amino acids SSSSKAPPPSLPSPSRLPGPSDTPILPQAPPRLICDSRVLERYLLEAKEAENITTGCAEHCSL NENITVPDTKVNFYAWKRMEVGQQAVEVWQGLALLSEAVLRGQALLVNSSQPWEPLQ LHVDKAVSGLRSLTTLLRALGAQKEAISPPDAASAAPLRTITADTFRKLFRVYSNFLRGK LKLYTGEACRTGDRSSSSKAPPPSLPSPSRLPGPSDTPILPQSSSSKAPPPSLPSPSRLPGPSD TPILPQ (SEQ ID NO: 109) represent the mature engineered CTP-modified EPO polypeptide lacking the signal peptide. In one embodiment, the amino acid sequence of CTP-modified EPO without the signal peptide is set forth in SEQ ID NO: 109. In another embodiment, the signal peptide of CTP-modified EPO is set forth in SEQ ID NO: 19.

In another embodiment, the methods disclosed herein manufacture an EPO peptide set forth in SEQ ID NO: 4 having additionally at least one CTP amino acid peptide on the N-terminus and at least one CTP amino acid peptide on the C-terminus for the treatment of anemia. In one embodiment, SEQ ID NO: 4 comprises the following amino acid (AA) sequence

MGVHECPAWLWLLLSLLSLPLGLPVLGAPPRLICDSRVLERYLLEAKEAENITTGCAEHC
SLNENITVPDTKVNFYAWKRMEVGQQAVEVWQGLALLSEAVLRGQALLVNSSQPWEP
LQLHVDKAVSGLRSLTTLLRALGAQKEAISPPDAASAAPLRTITADTFRKLFRVYSNFLR
GKLKLYTGEACRTGDRSSSSKAPPPSLPSPSRLPGPSDTPILPQAPPRLICDSRVLERYLLE
AKEAENITTGCAEHCSLNENITVPDTKVNFYAWKRMEVGQQAVEVWQGLALLSEAVLR
GQALLVNSSQPWEPLQLHVDKAVSGLRSLTTLLRALGAQKEAISPPDAASAAPLRTITAD
TFRKLFRVYSNFLRGKLKLYTGEACRTGDR.

In another embodiment, the methods disclosed herein manufacture an EPO peptide set forth in SEQ ID NO: 5 having additionally at least one CTP amino acid peptide on the N-terminus and at least one CTP amino acid peptide on the C-terminus for the treatment of anemia. In another embodiment, SEQ ID NO: 5 comprises the following amino acid (AA) sequence :

MGVHECPAWLWLLLSLLSLPLGLPVLGSSSKAPPPSLPSPSRLPGPSDTPILPQAPPRLIC
DSRVLERYLLEAKEAENITTGCAEHCSLNENITVPDTKVNFYAWKRMEVGQQAVEVWQ
GLALLSEAVLRGQALLVNSSQPWEPLQLHVDKAVSGLRSLTTLLRALGAQKEAISPPDA
ASAAPLRTITADTFRKLFRVYSNFLRGKLKLYTGEACRTGDR.

In another embodiment, the methods disclosed herein manufacture an EPO peptide set forth in SEQ ID NO: 6 having additionally at least one CTP amino acid peptide on the N-terminus and at least one CTP amino acid peptide on the C-terminus for the treatment of anemia. In one embodiment, SEQ ID NO: 6 comprises the following amino acid (AA) sequence

MGVHECPAWLWLLLSLLSLPLGLPVLGSSSKAPPPSLPSPSRLPGPSDTPILPQAPPRLIC
DSRVLERYLLEAKEAENITTGCAEHCSLNENITVPDTKVNFYAWKRMEVGQQAVEVWQ
GLALLSEAVLRGQALLVNSSQPWEPLQLHVDKAVSGLRSLTTLLRALGAQKEAISPPDA

ASAAPLRTITADTFRKLFRVYSNFLRGKLKLYTGEACRTGDRSSSSKAPPPSLPSPSRLPG
PSDTPILPQ.

In another embodiment, the methods disclosed herein manufacture an EPO peptide set forth in SEQ ID NO: 7 having additionally at least one CTP amino acid peptide on the N-terminus and at least one CTP amino acid peptide on the C-terminus for the treatment of anemia. In one embodiment, SEQ ID NO: 7 comprises the following amino acid (AA) sequence

MGVHECPAWLWLLLSLLSLPLGLPVLGAPPRLICDSRVLERYLLEAKEAENITTGCAEHC
SLNENITVPDTVNFYAWKRMEVGQQAVEVWQGLALLSEAVLRSQALLVNSSQPWEPLQ
LHVDKAVSGLRSLTTLLRALGAQKEAISPPDAASAAPLRTITADTFRKLFRVYSNFLRGK
LKLYTGEACRTGDRSSSSKAPPPSLPSPSRLPGPSDTPILPQ.

In another embodiment, the methods disclosed herein manufacture an EPO peptide set forth in SEQ ID NO: 8 having additionally at least one CTP amino acid peptide on the N-terminus and at least one CTP amino acid peptide on the C-terminus for the treatment of anemia. In one embodiment, SEQ ID NO: 8 comprises the following amino acid (AA) sequence

MGVHECPAWLWLLLSLLSLPLGLPVLGAPPRLICDSRVLERYLLEAKEAENITTGCAEHC
SLNENITVPDTKVNFYAWKRMEVGQQAVEVWQGLALLSEAVLRGQALLVNSSQPWEP
LQLHVDKAVSGLRSLTTLLRALGAQKEAISPPDAASAAPLRTITADTFRKLFRVYSNFLR
GKLKLYTGEACRTGDR.

In another embodiment, the methods disclosed herein utilize a nucleic acid sequence encoding an EPO peptide having additionally at least one CTP amino acid peptide on the N-terminus and at least one CTP amino acid peptide on the C-terminus for the treatment of anemia. In another embodiment, the methods disclosed herein utilize a nucleic acid sequence encoding an EPO peptide having additionally one CTP amino acid peptide on the N-terminus and two CTP amino acid peptides on the C-terminus for the treatment of anemia. In another embodiment, the methods disclosed herein utilize a nucleic acid set forth in SEQ ID NO: 17 encoding an EPO peptide and one CTP amino acid peptide on the N-terminus and at least one CTP amino acid peptide on the C-terminus for the treatment of anemia. In another embodiment, SEQ ID NO: 17 comprises the nucleotide (nt) sequence: tctagaggtc atcatggggg tgcacgaatg tcctgcctgg ctgtggcttc tcctgtccct tctgtcgctc cctctgggcc tcccagtcct gggctcctct tcctcaaagg cccctccccc gagccttcca agtccatccc gactcccggg gccctcggac accccaatat taccacaagc cccaccacgc ctcatctgtg acagccgagt cctggagagg tacctcttgg aggccaagga ggccgagaat atcacgacgg gctgtgctga acactgcagc ttgaatgaga atatcactgt cccagacacc aaagttaatt tctatgcctg gaagaggatg gaggtcgggc agcaggccgt agaagtctgg cagggcctgg ccctgctgtc ggaagctgtc ctgcggggcc aggccctgtt ggtcaactct tcccagccgt gggagcccct gcagctgcat gtggataaag ccgtcagtgg ccttcgcagc ctcaccactc tgcttcgggc tctgggagcc cagaaggaag ccatctcccc tccagatgcg gcctcagctg ctccactccg aacaatcact gctgacactt tccgcaaact cttccgagtc tactccaatt tcctccgggg aaagctgaag ctgtacacag gggaggcctg caggacaggg gacagatcct cttcctcaaa ggcccctccc ccgagccttc caagtccatc ccgactcccg gggccctcgg acaccccgat cctcccacaa taaaggtctt ctggatccgc ggccgc.

In another embodiment, the methods disclosed herein utilize a nucleic acid set forth in SEQ ID NO: 18 encoding an EPO peptide and one CTP amino acid peptide on the N-terminus and two CTP amino acid peptides on the C-terminus for the treatment of anemia. In another embodiment, SEQ ID NO: 18 comprises a nt sequences: tctagaggtc atcatggggg tgcacgaatg tcctgcctgg ctgtggcttc tcctgtccct tctgtcgctc cctctgggcc tcccagtcct gggctcctct tcctcaaagg cccctccccc gagccttcca agtccatccc gactcccggg gccctcggac accccaatat taccacaagc cccaccacgc ctcatctgtg acagccgagt cctggagagg tacctcttgg aggccaagga ggccgagaat atcacgacgg gctgtgctga acactgcagc ttgaatgaga atatcactgt cccagacacc aaagttaatt tctatgcctg gaagaggatg gaggtcgggc agcaggccgt agaagtctgg cagggcctgg ccctgctgtc ggaagctgtc ctgcggggcc aggccctgtt ggtcaactct tcccagccgt gggagcccct gcagctgcat gtggataaag ccgtcagtgg ccttcgcagc ctcaccactc tgcttcgggc tctgggagcc cagaaggaag ccatctcccc tccagatgcg gcctcagctg ctccactccg aacaatcact gctgacactt tccgcaaact cttccgagtc tactccaatt tcctccgggg aaagctgaag ctgtacacag gggaggcctg caggacaggg gacagatcct cttcctcaaa ggcccctccc ccgagccttc caagtccatc ccgactcccg gggccctccg acacaccaat cctgccacag agcagctcct ctaaggcccc tcctccatcc ctgccatccc cctcccggct gcctggcccc tctgacaccc ctatcctgcc tcagtgatga aggtcttctg gatccgcggc cgc.

In another embodiment, the methods disclosed herein manufacture an EPO peptide having additionally at least one CTP amino acid peptide on the N-terminus and at least one CTP amino acid peptide on the C-terminus for inhibiting anemia. In another embodiment, the methods disclosed herein manufacture an EPO peptide having additionally one CTP amino acid peptide on the N-terminus and two CTP amino acid peptides on the C-terminus for inhibiting anemia. In another embodiment, the methods disclosed herein manufacture an EPO peptide set forth in SEQ ID NO: 1 having additionally at least one CTP amino acid peptide on the N-terminus for inhibiting anemia. In another embodiment, the methods disclosed herein manufacture an EPO peptide set forth in SEQ ID NO: 1 having additionally at least one CTP amino acid peptide on the N-terminus and at least additional one CTP amino acid peptide on the C-terminus for inhibiting anemia. In another embodiment, the methods disclosed herein manufacture an EPO peptide set forth in SEQ ID NO: 2 having additionally at least one CTP amino acid peptide on the N-terminus and at least one CTP amino acid peptide on the C-terminus for inhibiting anemia. In another embodiment, the methods disclosed herein manufacture an EPO peptide set forth in SEQ ID NO: 3 having additionally at least one CTP amino acid peptide on the N-terminus and at least one CTP amino acid peptide on the C-terminus for inhibiting anemia. In another embodiment, the methods disclosed herein manufacture an EPO peptide set forth in SEQ ID NO: 4 having additionally at least one CTP amino acid peptide on the N-terminus and at least one CTP amino acid peptide on the C-terminus for inhibiting anemia. In another embodiment, the methods disclosed herein manufacture an EPO peptide set forth in SEQ ID NO: 5 having additionally at least one CTP amino acid peptide on the N-terminus and at least one CTP amino acid peptide on the C-terminus for inhibiting anemia. In another embodiment, the methods disclosed herein manufacture an EPO peptide set forth in SEQ ID NO: 6 having additionally at least one CTP amino acid peptide on the N-terminus and at least one CTP amino acid peptide on the C-terminus for inhibiting anemia. In another embodiment, the methods disclosed herein manufacture an EPO peptide set forth in SEQ ID NO: 8 having additionally at least one CTP amino acid peptide on the N-terminus and at least one CTP amino acid peptide on the C-terminus for inhibiting anemia. In another embodiment, the methods disclosed herein manufacture an EPO peptide set forth in SEQ ID NO: 7 having additionally at least one CTP amino acid peptide on the N-terminus and at least one CTP amino acid peptide on the C-terminus for inhibiting anemia. In another embodiment, the methods disclosed herein manufacture a CTP-modified EPO having the amino acid sequence set forth in SEQ ID NO: 109. In another embodiment, a CTP-modified EPO having the amino acid sequence set forth in SEQ ID NO: 109 is for inhibiting anemia. In another embodiment, a CTP-modified EPO having the amino acid sequence set forth in SEQ ID NO: 109 is for treating anemia.

In another embodiment, the methods disclosed herein utilize a nucleic acid sequence encoding an EPO peptide having additionally at least one CTP amino acid peptide on the N-terminus and at least one CTP amino acid peptide on the C-terminus for inhibiting anemia. In another embodiment, the methods disclosed herein utilize a nucleic acid sequence encoding an EPO peptide having one CTP amino acid peptide on the N-terminus and two CTP amino acid peptides on the C-terminus for inhibiting anemia. In another embodiment, the methods disclosed herein utilize a nucleic acid set forth in SEQ ID NO: 17 encoding an EPO peptide and one CTP amino acid peptide on the N-terminus and at least one CTP amino acid peptide on the C-terminus for inhibiting anemia. In another embodiment, the methods disclosed herein utilize a nucleic acid set forth in SEQ ID NO: 18 encoding an EPO peptide and one CTP amino acid peptide on the N-terminus and two CTP amino acid peptides on the C-terminus for inhibiting anemia.

In another embodiment, the methods disclosed herein manufacture an EPO peptide having additionally at least one CTP amino acid peptide on the N-terminus and at least one CTP amino acid peptide on the C-terminus for the treatment of tumor-associated anemia. In another embodiment, the methods disclosed herein manufacture an EPO peptide having additionally one CTP amino acid peptide on the N-terminus and two CTP amino acid peptides on the C-terminus for the treatment of tumor-associated anemia. In another embodiment, the methods disclosed herein manufacture an EPO peptide set forth in SEQ ID NO: 1 having additionally at least one CTP amino acid peptide on the N-terminus for the treatment of tumor-associated anemia. In another embodiment, the methods disclosed herein manufacture an EPO peptide set forth in SEQ ID NO: 1 having additionally at least one CTP amino acid peptide on the N-terminus and at least additional one CTP amino acid peptide on the C-terminus for the treatment of tumor-associated anemia. In another embodiment, the methods disclosed herein manufacture an EPO peptide set forth in SEQ ID NO: 2 having additionally at least one CTP amino acid peptide on the N-terminus and at least one CTP amino acid peptide on the C-terminus for the treatment of tumor-associated anemia. In another embodiment, the methods disclosed herein manufacture an EPO peptide set forth in SEQ ID NO: 3 having additionally at least one CTP amino acid peptide on the N-terminus and at least one CTP amino acid peptide on the C-terminus for the treatment of tumor-associated anemia. In another embodiment, the methods disclosed herein manufacture an EPO peptide set forth in SEQ ID NO: 4 having additionally at least one CTP amino acid peptide on the N-terminus and at least one CTP amino acid peptide on the C-terminus for the treatment of tumor-associated anemia. In another embodiment, the methods disclosed herein manufacture an EPO peptide set forth in SEQ ID NO: 5 having additionally at least one CTP amino acid peptide on the N-terminus and at least one CTP amino acid peptide on the C-terminus for the treatment of tumor-associated anemia. In another embodiment, the methods disclosed herein manufacture an EPO peptide set forth in SEQ ID NO: 6 having additionally at least one CTP amino acid peptide on the N-terminus and at least one CTP amino acid peptide on the C-terminus for the treatment of tumor-associated anemia. In another embodiment, the methods disclosed herein manufacture an EPO peptide set forth in SEQ ID NO: 8 having additionally at least one CTP amino acid peptide on the N-terminus and at least one CTP amino acid peptide on the C-terminus for the treatment of tumor-associated anemia. In another embodiment, the methods disclosed herein manufacture an EPO peptide set forth in SEQ ID NO: 7 having additionally at least one CTP amino acid peptide on the N-terminus and at least one CTP amino acid peptide on the C-terminus for the treatment of tumor-associated anemia. In another embodiment, a CTP-modified EPO having the amino acid sequence set forth in SEQ ID NO: 109 is for treating tumor associated anemia.

In another embodiment, the methods disclosed herein utilize a nucleic acid sequence encoding an EPO peptide having additionally at least one CTP amino acid peptide on the N-terminus and at least one CTP amino acid peptide on the C-terminus for the treatment of tumor-associated anemia. In another embodiment, the methods disclosed herein utilize a nucleic acid sequence encoding an EPO peptide having additionally one CTP amino acid peptide on the N-terminus and two CTP amino acid peptides on the C-terminus for the treatment of tumor-associated anemia. In another embodiment, the methods disclosed herein utilize a nucleic acid set forth in SEQ ID NO: 17 encoding an EPO peptide having additionally one CTP amino acid peptide on the N-terminus and at least one CTP amino acid peptide on the C-terminus for the treatment of tumor-associated anemia. In another embodiment, the methods disclosed herein utilize a nucleic acid set forth in SEQ ID NO: 18 encoding an EPO peptide having additionally one CTP amino acid peptide on the N-terminus and two CTP amino acid peptides on the C-terminus for the treatment of tumor-associated anemia.

In another embodiment, the methods disclosed herein manufacture an EPO peptide having additionally at least one CTP amino acid peptide on the N-terminus and at least one CTP amino acid peptide on the C-terminus for inhibiting tumor-associated anemia. In another embodiment, the methods disclosed herein manufacture an EPO peptide having additionally one CTP amino acid peptide on the N-terminus and two CTP amino acid peptides on the C-terminus for inhibiting tumor-associated anemia. In another embodiment, the methods disclosed herein manufacture an EPO peptide set forth in SEQ ID NO: 1 having additionally at least one CTP amino acid peptide on the N-terminus for inhibiting tumor-associated anemia. In another embodiment, the methods disclosed herein manufacture an EPO peptide set forth in SEQ ID NO: 1 having additionally at least one CTP amino acid peptide on the N-terminus and at least additional one CTP amino acid peptide on the C-terminus for inhibiting tumor-associated anemia. In another embodiment, the methods disclosed herein manufacture an EPO peptide set forth in SEQ ID NO: 2 having additionally at least one CTP amino acid peptide on the N-terminus and at least one CTP amino acid peptide on the C-terminus for inhibiting tumor-associated anemia. In another embodiment, the methods disclosed herein manufacture an EPO peptide set forth in SEQ ID NO: 3 having additionally at least one CTP amino acid peptide on the N-terminus and at least one CTP amino acid peptide on the C-terminus for inhibiting tumor-associated anemia. In another embodiment, the methods disclosed herein manufacture an EPO peptide set forth in SEQ ID NO: 4 having additionally at least one CTP amino acid peptide on the N-terminus and at least one CTP amino acid peptide on the C-terminus for inhibiting tumor-associated anemia. In another embodiment, the methods disclosed herein manufacture an EPO peptide set forth in SEQ ID NO: 5 having additionally at least one CTP amino acid peptide on the N-terminus and at least one CTP amino acid peptide on the C-terminus for inhibiting tumor-associated anemia. In another embodiment, the methods disclosed herein manufacture an EPO peptide set forth in SEQ ID NO: 6 having additionally at least one CTP amino acid peptide on the N-terminus and at least one CTP amino acid peptide on the C-terminus for inhibiting tumor-associated anemia. In another embodiment, the methods disclosed herein manufacture an EPO peptide set forth in SEQ ID NO: 8 having additionally at least one CTP amino acid peptide on the N-terminus and at least one CTP amino acid peptide on the C-terminus for inhibiting tumor-associated anemia. In another embodiment, the methods disclosed herein manufacture an EPO peptide set forth in SEQ ID NO: 7 having additionally at least one CTP amino acid peptide on the N-terminus and at least one CTP amino acid peptide on the C-terminus for inhibiting tumor-associated anemia. In another embodiment, a CTP-modified EPO having the amino acid sequence set forth in SEQ ID NO: 109 is for inhibiting tumor associated anemia.

In another embodiment, the methods disclosed herein utilize a nucleic acid sequence encoding an EPO peptide having additionally at least one CTP amino acid peptide on the N-terminus and at least one CTP amino acid peptide on the C-terminus for inhibiting tumor-associated anemia. In another embodiment, the methods disclosed herein utilize a nucleic acid sequence encoding an EPO peptide having additionally one CTP amino acid peptide on the N-terminus and two CTP amino acid peptides on the C-terminus for inhibiting tumor-associated anemia. In another embodiment, the methods disclosed herein utilize a nucleic acid set forth in SEQ ID NO: 17 encoding an EPO peptide and one CTP amino acid peptide on the N-terminus and at least one CTP amino acid peptide on the C-terminus for inhibiting tumor-associated anemia. In another embodiment, the methods disclosed herein utilize a nucleic acid set forth in SEQ ID NO: 18 encoding an EPO peptide and one CTP amino acid peptide on the N-terminus and two CTP amino acid peptides on the C-terminus for inhibiting tumor-associated anemia.

In another embodiment, the methods disclosed herein manufacture an EPO peptide having additionally at least one CTP amino acid peptide on the N-terminus and at least one CTP amino acid peptide on the C-terminus for the treatment of tumor hypoxia. In another embodiment, the methods disclosed herein manufacture an EPO peptide having additionally one CTP amino acid peptide on the N-terminus and two CTP amino acid peptides on the C-terminus for the treatment of tumor hypoxia. In another embodiment, the methods disclosed herein manufacture an EPO peptide set forth in SEQ ID NO: 1 having additionally at least one CTP amino acid peptide on the N-terminus for the treatment of tumor hypoxia. In another embodiment, the methods disclosed herein manufacture an EPO peptide set forth in SEQ ID NO: 1 having additionally at least one CTP amino acid peptide on the N-terminus and at least additional one CTP amino acid peptide on the C-terminus for the treatment of tumor hypoxia. In another embodiment, the methods disclosed herein manufacture an EPO peptide set forth in SEQ ID NO: 2 having additionally at least one CTP amino acid peptide on the N-terminus and at least one CTP amino acid peptide on the C-terminus for the treatment of tumor hypoxia. In another embodiment, the methods disclosed herein manufacture an EPO peptide set forth in SEQ ID NO: 3 having additionally at least one CTP amino acid peptide on the N-terminus and at least one CTP amino acid peptide on the C-terminus for the treatment of tumor hypoxia. In another embodiment, the methods disclosed herein manufacture an EPO peptide set forth in SEQ ID NO: 4 having additionally at least one CTP amino acid peptide on the N-terminus and at least one CTP amino acid peptide on the C-terminus for the treatment of tumor hypoxia. In another embodiment, the methods disclosed herein manufacture an EPO peptide set forth in SEQ ID NO: 5 having additionally at least one CTP amino acid peptide on the N-terminus and at least one CTP amino acid peptide on the C-terminus for the treatment of tumor hypoxia. In another embodiment, the methods disclosed herein manufacture an EPO peptide set forth in SEQ ID NO: 6 having additionally at least one CTP amino acid peptide on the N-terminus and at least one CTP amino acid peptide on the C-terminus for the treatment of tumor hypoxia. In another embodiment, the methods disclosed herein manufacture an EPO peptide set forth in SEQ ID NO: 8 having additionally at least one CTP amino acid peptide on the N-terminus and at least one CTP amino acid peptide on the C-terminus for the treatment of tumor hypoxia. In another embodiment, the methods disclosed herein manufacture an EPO peptide set forth in SEQ ID NO: 7 having additionally at least one CTP amino acid peptide on the N-terminus and at least one CTP amino acid peptide on the C-terminus for the treatment of tumor hypoxia. In another embodiment, a CTP-modified EPO having the amino acid sequence set forth in SEQ ID NO: 109 is for treating tumor hypoxia.

In another embodiment, the methods disclosed herein utilize a nucleic acid sequence encoding an EPO peptide having additionally at least one CTP amino acid peptide on the N-terminus and at least one CTP amino acid peptide on the C-terminus for the treatment of tumor hypoxia. In another embodiment, the methods disclosed herein utilize a nucleic acid sequence encoding an EPO peptide having additionally one CTP amino acid peptide on the N-terminus and two CTP amino acid peptides on the C-terminus for the treatment of tumor hypoxia. In another embodiment, the methods disclosed herein utilize a nucleic acid set forth in SEQ ID NO: 17 encoding an EPO peptide and one CTP amino acid peptide on the N-terminus and at least one CTP amino acid peptide on the C-terminus for the treatment of tumor hypoxia. In another embodiment, the methods disclosed herein utilize a nucleic acid set forth in SEQ ID NO: 18 encoding an EPO peptide having additionally one CTP amino acid peptide on the N-terminus and two CTP amino acid peptides on the C-terminus for the treatment of tumor hypoxia.

In another embodiment, the methods disclosed herein manufacture an EPO peptide having additionally at least one CTP amino acid peptide on the N-terminus and at least one CTP amino acid peptide on the C-terminus for the treatment of chronic infections such as HIV, inflammatory bowel disease, or septic episodes. In another embodiment, the methods disclosed herein manufacture an EPO peptide having additionally one CTP amino acid peptide on the N-terminus and two CTP amino acid peptides on the C-terminus for the treatment of chronic infections such as HIV, inflammatory bowel disease, or septic episodes. In another embodiment, the methods disclosed herein manufacture an EPO peptide set forth in SEQ ID NO: 1 having additionally at least one CTP amino acid peptide on the N-terminus for the treatment of chronic infections such as HIV, inflammatory bowel disease, or septic episodes. In another embodiment, the methods disclosed herein manufacture an EPO peptide set forth in SEQ ID NO: 1 having additionally at least one CTP amino acid peptide on the N-terminus and at least additional one CTP amino acid peptide on the C-terminus for the treatment of chronic infections such as HIV, inflammatory bowel disease, or septic episodes. In another embodiment, the methods disclosed herein manufacture an EPO peptide set forth in SEQ ID NO: 2 having additionally at least one CTP amino acid peptide on the N-terminus and at least one CTP amino acid peptide on the C-terminus for the treatment of chronic infections such as HIV, inflammatory bowel disease, or septic episodes. In another embodiment, the methods disclosed herein manufacture an EPO peptide set forth in SEQ ID NO: 3 having additionally at least one CTP amino acid peptide on the N-terminus and at least one CTP amino acid peptide on the C-terminus for the treatment of chronic infections such as HIV, inflammatory bowel disease, or septic episodes. In another embodiment, the methods disclosed herein manufacture an EPO peptide set forth in SEQ ID NO: 4 having additionally at least one CTP amino acid peptide on the N-terminus and at least one CTP amino acid peptide on the C-terminus for the treatment of chronic infections such as HIV, inflammatory bowel disease, or septic episodes. In another embodiment, the methods disclosed herein manufacture an EPO peptide set forth in SEQ ID NO: 5 having additionally at least one CTP amino acid peptide on the N-terminus and at least one CTP amino acid peptide on the C-terminus for the treatment of chronic infections such as HIV, inflammatory bowel disease, or septic episodes. In another embodiment, the methods disclosed herein manufacture an EPO peptide set forth in SEQ ID NO: 6 having additionally at least one CTP amino acid peptide on the N-terminus and at least one CTP amino acid peptide on the C-terminus for the treatment of chronic infections such as HIV, inflammatory bowel disease, or septic episodes. In another embodiment, the methods disclosed herein manufacture an EPO peptide set forth in SEQ ID NO: 8 having additionally at least one CTP amino acid peptide on the N-terminus and at least one CTP amino acid peptide on the C-terminus for the treatment of chronic infections such as HIV, inflammatory bowel disease, or septic episodes. In another embodiment, the methods disclosed herein manufacture an EPO peptide set forth in SEQ ID NO: 7 having additionally at least one CTP amino acid peptide on the N-terminus and at least one CTP amino acid peptide on the C-terminus for the treatment of chronic infections such as HIV, inflammatory bowel disease, or septic episodes. In another embodiment, a CTP-modified EPO having the amino acid sequence set forth in SEQ ID NO: 109 is for the treatment of chronic infections such as HIV, inflammatory bowel disease or septic episodes.

In another embodiment, the methods disclosed herein utilize a nucleic acid sequence encoding an EPO peptide having additionally at least one CTP amino acid peptide on the N-terminus and at least one CTP amino acid peptide on the C-terminus for the treatment of chronic infections such as HIV, inflammatory bowel disease, or septic episodes. In another embodiment, the methods disclosed herein utilize a nucleic acid sequence encoding an EPO peptide having additionally one CTP amino acid peptide on the N-terminus and two CTP amino acid peptides on the C-terminus for the treatment of chronic infections such as HIV, inflammatory bowel disease, or septic episodes. In another embodiment, the methods disclosed herein utilize a nucleic acid set forth in SEQ ID NO: 17 encoding an EPO peptide and one CTP amino acid peptide on the N-terminus and at least one CTP amino acid peptide on the C-terminus for the treatment of chronic infections such as HIV, inflammatory bowel disease, or septic episodes. In another embodiment, the methods disclosed herein utilize a nucleic acid set forth in SEQ ID NO: 18 encoding an EPO peptide and one CTP amino acid peptide on the N-terminus and two CTP amino acid peptides on the C-terminus for the treatment of chronic infections such as HIV, inflammatory bowel disease, or septic episodes.

In another embodiment, the methods disclosed herein manufacture an EPO peptide having additionally at least one CTP amino acid peptide on the N-terminus and at least one CTP amino acid peptide on the C-terminus for inhibiting chronic infections such as HIV, inflammatory bowel disease, or septic episodes. In another embodiment, the methods disclosed herein manufacture an EPO peptide having additionally one CTP amino acid peptide on the N-terminus and two CTP amino acid peptides on the C-terminus for inhibiting chronic infections such as HIV, inflammatory bowel disease, or septic episodes. In another embodiment, the methods disclosed herein manufacture an EPO peptide set forth in SEQ ID NO: 1 having additionally at least one CTP amino acid peptide on the N-terminus for inhibiting chronic infections such as HIV, inflammatory bowel disease, or septic episodes. In another embodiment, the methods disclosed herein manufacture an EPO peptide set forth in SEQ ID NO: 1 having additionally at least one CTP amino acid peptide on the N-terminus and at least additional one CTP amino acid peptide on the C-terminus for inhibiting chronic infections such as HIV, inflammatory bowel disease, or septic episodes. In another embodiment, the methods disclosed herein manufacture an EPO peptide set forth in SEQ ID NO: 2 having additionally at least one CTP amino acid peptide on the N-terminus and at least one CTP amino acid peptide on the C-terminus for inhibiting chronic infections such as HIV, inflammatory bowel disease, or septic episodes. In another embodiment, the methods disclosed herein manufacture an EPO peptide set forth in SEQ ID NO: 3 having additionally at least one CTP amino acid peptide on the N-terminus and at least one CTP amino acid peptide on the C-terminus for inhibiting chronic infections such as HIV, inflammatory bowel disease, or septic episodes. In another embodiment, the methods disclosed herein manufacture an EPO peptide set forth in SEQ ID NO: 4 having additionally at least one CTP amino acid peptide on the N-terminus and at least one CTP amino acid peptide on the C-terminus for inhibiting chronic infections such as HIV, inflammatory bowel disease, or septic episodes. In another embodiment, the methods disclosed herein manufacture an EPO peptide set forth in SEQ ID NO: 5 having additionally at least one CTP amino acid peptide on the N-terminus and at least one CTP amino acid peptide on the C-terminus for inhibiting chronic infections such as HIV, inflammatory bowel disease, or septic episodes. In another embodiment, the methods disclosed herein manufacture an EPO peptide set forth in SEQ ID NO: 6 having additionally at least one CTP amino acid peptide on the N-terminus and at least one CTP amino acid peptide on the C-terminus for inhibiting chronic infections such as HIV, inflammatory bowel disease, or septic episodes. In another embodiment, the methods disclosed herein manufacture an EPO peptide set forth in SEQ ID NO: 8 having additionally at least one CTP amino acid peptide on the N-terminus and at least one CTP amino acid peptide on the C-terminus for inhibiting chronic infections such as HIV, inflammatory bowel disease, or septic episodes. In another embodiment, the methods disclosed herein manufacture an EPO peptide set forth in SEQ ID NO: 7 having additionally at least one CTP amino acid peptide on the N-terminus and at least one CTP amino acid peptide on the C-terminus for inhibiting chronic infections such as HIV, inflammatory bowel disease, or septic episodes. In another embodiment, a CTP-modified EPO having the amino acid sequence set forth in SEQ ID NO: 109 is for the inhibition of chronic infections such as HIV, inflammatory bowel disease or septic episodes.

In another embodiment, the methods disclosed herein utilize a nucleic acid sequence encoding an EPO peptide having additionally at least one CTP amino acid peptide on the N-terminus and at least one CTP amino acid peptide on the C-terminus for inhibiting chronic infections such as HIV, inflammatory bowel disease, or septic episodes. In another embodiment, the methods disclosed herein utilize a nucleic acid sequence encoding an EPO peptide having one CTP amino acid peptide on the N-terminus and two CTP amino acid peptides on the C-terminus for inhibiting chronic infections such as HIV, inflammatory bowel disease, or septic episodes. In another embodiment, the methods disclosed herein utilize a nucleic acid set forth in SEQ ID NO: 17 encoding an EPO peptide and one CTP amino acid peptide on the N-terminus and at least one CTP amino acid peptide on the C-terminus for inhibiting chronic infections such as HIV, inflammatory bowel disease, or septic episodes. In another embodiment, the methods disclosed herein utilize a nucleic acid set forth in SEQ ID NO: 18 encoding an EPO peptide and one CTP amino acid peptide on the N-terminus and two CTP amino acid peptides on the C-terminus for inhibiting chronic infections such as HIV, inflammatory bowel disease, or septic episodes.

In another embodiment, the methods disclosed herein manufacture an EPO peptide having additionally at least one CTP amino acid peptide on the N-terminus and at least one CTP amino acid peptide on the C-terminus for the treatment of fatigue syndrome following cancer chemotherapy. In another embodiment, the methods disclosed herein manufacture an EPO peptide having additionally one CTP amino acid peptide on the N-terminus and two CTP amino acid peptides on the C-terminus for the treatment of fatigue syndrome following cancer chemotherapy. In another embodiment, the methods disclosed herein manufacture an EPO peptide set forth in SEQ ID NO: 1 having additionally at least one CTP amino acid peptide on the N-terminus for the treatment of fatigue syndrome following cancer chemotherapy. In another embodiment, the methods disclosed herein manufacture an EPO peptide set forth in SEQ ID NO: 1 having additionally at least one CTP amino acid peptide on the N-terminus and at least additional one CTP amino acid peptide on the C-terminus for the treatment of fatigue syndrome following cancer chemotherapy. In another embodiment, the methods disclosed herein manufacture an EPO peptide set forth in SEQ ID NO: 2 having additionally at least one CTP amino acid peptide on the N-terminus and at least one CTP amino acid peptide on the C-terminus for the treatment of fatigue syndrome following cancer chemotherapy. In another embodiment, the methods disclosed herein manufacture an EPO peptide set forth in SEQ ID NO: 3 having additionally at least one CTP amino acid peptide on the N-terminus and at least one CTP amino acid peptide on the C-terminus for the treatment of fatigue syndrome following cancer chemotherapy. In another embodiment, the methods disclosed herein manufacture an EPO peptide set forth in SEQ ID NO: 4 having additionally at least one CTP amino acid peptide on the N-terminus and at least one CTP amino acid peptide on the C-terminus for the treatment of fatigue syndrome following cancer chemotherapy. In another embodiment, the methods disclosed herein manufacture an EPO peptide set forth in SEQ ID NO: 5 having additionally at least one CTP amino acid peptide on the N-terminus and at least one CTP amino acid peptide on the C-terminus for the treatment of fatigue syndrome following cancer chemotherapy. In another embodiment, the methods disclosed herein manufacture an EPO peptide set forth in SEQ ID NO: 6 having additionally at least one CTP amino acid peptide on the N-terminus and at least one CTP amino acid peptide on the C-terminus for the treatment of fatigue syndrome following cancer chemotherapy. In another embodiment, the methods disclosed herein manufacture an EPO peptide set forth in SEQ ID NO: 8 having additionally at least one CTP amino acid peptide on the N-terminus and at least one CTP amino acid peptide on the C-terminus for the treatment of fatigue syndrome following cancer chemotherapy. In another embodiment, the methods disclosed herein manufacture an EPO peptide set forth in SEQ ID NO: 7 having additionally at least one CTP amino acid peptide on the N-terminus and at least one CTP amino acid peptide on the C-terminus for the treatment of fatigue syndrome following cancer chemotherapy. In another embodiment, a CTP-modified EPO having the amino acid sequence set forth in SEQ ID NO: 109 is for the treatment of fatigue syndrome following cancer chemotherapy.

In another embodiment, the methods disclosed herein utilize a nucleic acid sequence encoding an EPO peptide having additionally at least one CTP amino acid peptide on the N-terminus and at least one CTP amino acid peptide on the C-terminus for the treatment of fatigue syndrome following cancer chemotherapy. In another embodiment, the methods disclosed herein utilize a nucleic acid sequence encoding an EPO peptide having additionally one CTP amino acid peptide on the N-terminus and two CTP amino acid peptides on the C-terminus for the treatment of fatigue syndrome following cancer chemotherapy. In another embodiment, the methods disclosed herein utilize a nucleic acid set forth in SEQ ID NO: 17 encoding an EPO peptide and one CTP amino acid peptide on the N-terminus and at least one CTP amino acid peptide on the C-terminus for the treatment of fatigue syndrome following cancer chemotherapy. In another embodiment, the methods disclosed herein utilize a nucleic acid set forth in SEQ ID NO: 18 encoding an EPO peptide and one CTP amino acid peptide on the N-terminus and two CTP amino acid peptides on the C-terminus for the treatment of fatigue syndrome following cancer chemotherapy.

In another embodiment, the methods disclosed herein manufacture an EPO peptide having additionally at least one CTP amino acid peptide on the N-terminus and at least one CTP amino acid peptide on the C-terminus for improving stem cell engraftment. In another embodiment, the methods disclosed herein manufacture an EPO peptide having additionally one CTP amino acid peptide on the N-terminus and two CTP amino acid peptides on the C-terminus for improving stem cell engraftment. In another embodiment, the methods disclosed herein manufacture an EPO peptide set forth in SEQ ID NO: 1 having additionally at least one CTP amino acid peptide on the N-terminus for improving stem cell engraftment. In another embodiment, the methods disclosed herein manufacture an EPO peptide set forth in SEQ ID NO: 1 having additionally at least one CTP amino acid peptide on the N-terminus and at least additional one CTP amino acid peptide on the C-terminus for improving stem cell engraftment. In another embodiment, the methods disclosed herein manufacture an EPO peptide set forth in SEQ ID NO: 2 having additionally at least one CTP amino acid peptide on the N-terminus and at least one CTP amino acid peptide on the C-terminus for improving stem cell engraftment. In another embodiment, the methods disclosed herein manufacture an EPO peptide set forth in SEQ ID NO: 3 having additionally at least one CTP amino acid peptide on the N-terminus and at least one CTP amino acid peptide on the C-terminus for improving stem cell engraftment. In another embodiment, the methods disclosed herein manufacture an EPO peptide set forth in SEQ ID NO: 4 having additionally at least one CTP amino acid peptide on the N-terminus and at least one CTP amino acid peptide on the C-terminus for improving stem cell engraftment. In another embodiment, the methods disclosed herein manufacture an EPO peptide set forth in SEQ ID NO: 5 having additionally at least one CTP amino acid peptide on the N-terminus and at least one CTP amino acid peptide on the C-terminus for improving stem cell engraftment. In another embodiment, the methods disclosed herein manufacture an EPO peptide set forth in SEQ ID NO: 6 having additionally at least one CTP amino acid peptide on the N-terminus and at least one CTP amino acid peptide on the C-terminus for improving stem cell engraftment. In another embodiment, the methods disclosed herein manufacture an EPO peptide set forth in SEQ ID NO: 8 having additionally at least one CTP amino acid peptide on the N-terminus and at least one CTP amino acid peptide on the C-terminus for improving stem cell engraftment. In another embodiment, the methods disclosed herein manufacture an EPO peptide set forth in SEQ ID NO: 7 having additionally at least one CTP amino acid peptide on the N-terminus and at least one CTP amino acid peptide on the C-terminus for improving stem cell engraftment. In another embodiment, a CTP-modified EPO having the amino acid sequence set forth in SEQ ID NO: 109 is for improving stem cell engraftment.

In another embodiment, the methods disclosed herein utilize a nucleic acid sequence encoding an EPO peptide having additionally at least one CTP amino acid peptide on the N-terminus and at least one CTP amino acid peptide on the C-terminus for improving stem cell engraftment. In another embodiment, the methods disclosed herein utilize a nucleic acid sequence encoding an EPO peptide having additionally one CTP amino acid peptide on the N-terminus and two CTP amino acid peptides on the C-terminus for improving stem cell engraftment. In another embodiment, the methods disclosed herein utilize a nucleic acid set forth in SEQ ID NO: 17 encoding an EPO peptide and one CTP amino acid peptide on the N-terminus and at least one CTP amino acid peptide on the C-terminus for improving stem cell engraftment. In another embodiment, the methods disclosed herein utilize a nucleic acid set forth in SEQ ID NO: 18 encoding an EPO peptide and one CTP amino acid peptide on the N-terminus and two CTP amino acid peptides on the C-terminus for improving stem cell engraftment.

In another embodiment, the methods disclosed herein manufacture an EPO peptide having additionally at least one CTP amino acid peptide on the N-terminus and at least one CTP amino acid peptide on the C-terminus for increasing the survival rate of a patient with aplastic anemia or myelodysplastic syndrome. In another embodiment, the methods disclosed herein manufacture an EPO peptide having additionally one CTP amino acid peptide on the N-terminus and two CTP amino acid peptides on the C-terminus for increasing the survival rate of a patient with aplastic anemia or myelodysplastic syndrome. In another embodiment, the methods disclosed herein manufacture an EPO peptide set forth in SEQ ID NO: 1 having additionally at least one CTP amino acid peptide on the N-terminus for increasing the survival rate of a patient with aplastic anemia or myelodysplastic syndrome. In another embodiment, the methods disclosed herein manufacture an EPO peptide set forth in SEQ ID NO: 1 having additionally at least one CTP amino acid peptide on the N-terminus and at least additional one CTP amino acid peptide on the C-terminus for increasing the survival rate of a patient with aplastic anemia or myelodysplastic syndrome. In another embodiment, the methods disclosed herein manufacture an EPO peptide set forth in SEQ ID NO: 2 having additionally at least one CTP amino acid peptide on the N-terminus and at least one CTP amino acid peptide on the C-terminus for increasing the survival rate of a patient with aplastic anemia or myelodysplastic syndrome. In another embodiment, the methods disclosed herein manufacture an EPO peptide set forth in SEQ ID NO: 3 having additionally at least one CTP amino acid peptide on the N-terminus and at least one CTP amino acid peptide on the C-terminus for increasing the survival rate of a patient with aplastic anemia or myelodysplastic syndrome. In another embodiment, the methods disclosed herein manufacture an EPO peptide set forth in SEQ ID NO: 4 having additionally at least one CTP amino acid peptide on the N-terminus and at least one CTP amino acid peptide on the C-terminus for increasing the survival rate of a patient with aplastic anemia or myelodysplastic syndrome. In another embodiment, the methods disclosed herein manufacture an EPO peptide set forth in SEQ ID NO: 5 having additionally at least one CTP amino acid peptide on the N-terminus and at least one CTP amino acid peptide on the C-terminus for increasing the survival rate of a patient with aplastic anemia or myelodysplastic syndrome. In another embodiment, the methods disclosed herein manufacture an EPO peptide set forth in SEQ ID NO: 6 having additionally at least one CTP amino acid peptide on the N-terminus and at least one CTP amino acid peptide on the C-terminus for increasing the survival rate of a patient with aplastic anemia or myelodysplastic syndrome. In another embodiment, the methods disclosed herein manufacture an EPO peptide set forth in SEQ ID NO: 8 having additionally at least one CTP amino acid peptide on the N-terminus and at least one CTP amino acid peptide on the C-terminus for increasing the survival rate of a patient with aplastic anemia or myelodysplastic syndrome. In another embodiment, the methods disclosed herein manufacture an EPO peptide set forth in SEQ ID NO: 7 having additionally at least one CTP amino acid peptide on the N-terminus and at least one CTP amino acid peptide on the C-terminus for increasing the survival rate of a patient with aplastic anemia or myelodysplastic syndrome. In another embodiment, a CTP-modified EPO having the amino acid sequence set forth in SEQ ID NO: 109 is for increasing th survival rate of a patient with aplastic anemia or myelodysplastic syndrome.

In another embodiment, the methods disclosed herein utilize a nucleic acid sequence encoding an EPO peptide having additionally at least one CTP amino acid peptide on the N-terminus and at least one CTP amino acid peptide on the C-terminus for increasing the survival rate of a patient with aplastic anemia or myelodysplastic syndrome. In another embodiment, the methods disclosed herein utilize a nucleic acid sequence encoding an EPO peptide having additionally one CTP amino acid peptide on the N-terminus and two CTP amino acid peptides on the C-terminus for increasing the survival rate of a patient with aplastic anemia or myelodysplastic syndrome. In another embodiment, the methods disclosed herein utilize a nucleic acid set forth in SEQ ID NO: 17 encoding an EPO peptide and one CTP amino acid peptide on the N-terminus and at least one CTP amino acid peptide on the C-terminus for increasing the survival rate of a patient with aplastic anemia or myelodysplastic syndrome. In another embodiment, the methods disclosed herein utilize a nucleic acid set forth in SEQ ID NO: 18 encoding an EPO peptide and one CTP amino acid peptide on the N-terminus and two CTP amino acid peptides on the C-terminus for increasing the survival rate of a patient with aplastic anemia or myelodysplastic syndrome.

In another embodiment, the cytokine further comprises a signal peptide for its secretion. In some embodiments, signal sequences include, but are not limited to the endogenous signal sequence for IFN. In some embodiments, signal sequences include, but are not limited to the endogenous signal sequence of any known cytokine. In another embodiment, the polypeptides and methods disclosed herein manufacture a cytokine having additionally a signal peptide of SEQ ID NO: 19 and at least one CTP peptide on the N-terminus and at least one CTP peptide on the C-terminus. In another embodiment, the polypeptides and methods disclosed herein manufacture a cytokine having additionally on the N-terminus the signal peptide of SEQ ID NO: 19 and at least one CTP peptide on the N-terminus and at least two CTP peptides on the C-terminus. In another embodiment, the polypeptides and methods disclosed herein manufacture a cytokine having additionally on the N-terminus the signal peptide of SEQ ID NO: 19 and a single CTP peptide on the N-terminus and two CTP peptides on the C-terminus. In another embodiment, SEQ ID NO: 19 comprise the following amino acid (AA) sequence: MTNKCLLQIALLLCFSTTALS (SEQ ID NO: 19).

In another embodiment, the cytokine is an interferon. In some embodiments, interferon is utilized according to the teachings disclosed herein. In some embodiments, the attachment of CTP sequence to both the amino and carboxy termini of the interferon protein results in increased potency. In some embodiments, the attachment of CTP sequence to both the amino and carboxy termini of the interferon protein results in prolonged in-vivo activity.

A skilled artisan would appreciate that the term "interferon" may encompass a mammalian interferon polypeptide Type I. In another embodiment, "interferon" comprises the mammalian interferon polypeptide Type II. In some embodiments, additional suitable interferon polypeptides as known to those of ordinary skill in the art are utilized. In some embodiments, the interferon is alpha-interferon. In some embodiments, the interferon is beta-interferon. In some embodiments, the interferon is gamma-interferon. In some embodiments, the interferon is omega-interferon. In some embodiments, the interferon is a subspecies interferon. In one embodiment, the subspecies interferon (IFN) is IFN-α2a. In one embodiment, the subspecies interferon (IFN) is IFN-α2b. In one embodiment, the subspecies interferon (IFN) is IFN-β2a. In one embodiment, the interferon (IFN) subspecies is IFN-β2b.

In one embodiment, interferon disclosed herein exhibits interferon activity, such as antiviral or antiproliferative activity. In some embodiments, non-limiting examples of interferons are listed in **Table 1** below.

A skilled artisan would appreciate that the term " interferon" may also encompass homologues. In one embodiment, interferon amino acid sequence disclosed herein is at least 50% homologous to interferon sequences listed in **Table 1** as determined using BlastP software of the National Center of Biotechnology Information (NCBI) using default parameters). In another embodiment, interferon amino acid sequence disclosed herein is at least 60% , is at least 70% , is at least 80% , is at least 90%, or is at least 95% homologous interferon sequences listed in Table 1 as determined using BlastP software of the National Center of Biotechnology Information (NCBI) using default parameters). A skilled artisan would appreciate that homology may encompasses deletions, insertions, or substitution variants, including an amino acid substitution, thereof and biologically active polypeptide fragments thereof. In one embodiment the cysteine in position 17 of interferon β is substituted by a Serine (SEQ ID NO: 20). In one embodiment, SEQ ID NO: 20 comprises the amino acid (AA) sequence:

MSYNLLGFLQRSSNFQSQKLLWQLNGRLEYCLKDRMNFDIPEEIKQLQQFQKEDAALTI
YEMLQNIFAIFRQDSSSTGWNETIVENLLANVYHQINHLKTVLEEKLEKEDFTRGKLMSS
LHLKRYYGRILHYLKAKEYSHCAWTIVRVEILRNFYFINRLTGYLRN.

**Table 1** below lists examples of interferons with their respective NCBI sequence numbers.

**Table 1**

| ***Interferon name*** |
|---|
| interferon, α1 |
| interferon, α10 |
| interferon, α13 |
| interferon, α14 |
| interferon, α16 |
| interferon, α17 |
| interferon, α2 |
| interferon, α21 |
| interferon, α4 |
| interferon, α5 |
| interferon, α6 |
| interferon, α7 |
| interferon, α8 |
| interferon, β1 |
| interferon, ε1 |
| interferon, γ |
| interferon, ε |
| interferon, Ω1 |

In another embodiment, a method of treating or reducing a disease treatable or reducible by a cytokine or a pharmaceutical formulation comprising the same, in a subject, comprises the step of administering to a subject a therapeutically effective amount of the polypeptide comprising a cytokine and CTP units as described herein, thereby treating or reducing a disease treatable or reducible by a cytokine in a subject.

In another embodiment, the methods disclosed herein manufacture an interferon beta 1 peptide having additionally at least one CTP amino acid peptide on the N-terminus and at least one CTP amino acid peptide on the C-terminus for treating or inhibiting multiple sclerosis. In another embodiment, the methods disclosed herein manufacture an interferon beta 1 peptide having additionally one CTP amino acid peptide on the N-terminus and two CTP amino acid peptides on the C-terminus for treating or inhibiting multiple sclerosis. In another embodiment, the methods disclosed herein manufacture an interferon beta 1 peptide set forth in SEQ ID NO: 20 having additionally at least one CTP amino acid peptide on the N-terminus and one CTP amino acid peptide on the C-terminus for treating or inhibiting multiple sclerosis. In another embodiment, the methodsdisclosed herein manufacture an interferon beta 1 peptide set forth in SEQ ID NO: 20 having additionally on the N-terminus the signal peptide of SEQ ID NO: 19 and at least one CTP amino acid peptide on the N-terminus of SEQ ID NO: 19 and at least one CTP amino acid peptide on the C-terminus of SEQ ID NO: 20 for treating or inhibiting multiple sclerosis.

In another embodiment, the cytokine as described herein comprises a cytokine and at least three CTP units. In another embodiment, the polypeptide as described herein comprises an interferon (IFN) peptide and three CTP units. In another embodiment, the cytokine as described herein comprises an interferon (IFN) peptide- CTP polypeptide encoded by an amino acid sequence comprising the amino acid sequence set forth in SEQ ID NO: 13. In another embodiment, SEQ ID NO: 13 comprises the following amino acid (AA) sequence:

MTNKCLLQIALLLCFSTTALSMSYNLLGFLQRSSNFQCQKLLWQLNGRLEYCLKDRMNF
DIPEEIKQLQQFQKEDAALTIYEMLQNIFAIFRQDSSSTGWNETIVENLLANVYHQINHLK
TVLEEKLEKEDFTRGKLMSSLHLKRYYGRILHYLKAKEYSHCAWTIVRVEILRNFYFINR
LTGYLRNSSSSKAPPPSLPSPSRLPGPSDTPILPQ (SEQ ID NO: 13, MOD-9011).

In another embodiment, the cytokine as described herein comprising an interferon (IFN) peptide - and CTP is encoded by a nucleic acid molecule set forth in SEQ ID NO: 22. In another embodiment, SEQ ID NO: 22 comprises the following nucleotide acid (NA) sequence: tctagaggacatgaccaacaagtgcctgctgcagatcgccctgctgctgtgcttcagcaccaccgccctgagcatgagctacaacctgctgg gcttcctgcagaggtccagcaacttccagtgccagaagctgctgtggcagctgaacggcaggctggaatactgcctgaaggacaggatgaa cttcgacatcccagaggaaatcaagcagctgcagcagttccagaaggaggacgccgccctgaccatctacgagatgctgcagaacatcttc gccatcttcaggcaggacagcagcagcaccggctggaacgagaccatcgtggagaacctgctggccaacgtgtaccaccagatcaaccac ctgaaaaccgtgctggaagagaagctggaaaaggaggacttcaccaggggcaagctgatgagcagcctgcacctgaagaggtactacgg cagaatcctgcactacctgaaggccaaggagtacagccactgcgcctggaccatcgtgagggtggagatcctgaggaacttctacttcatca acaggctgaccggctacctgaggaacagctccagcagcaaggcccctccaccttccctgcccagtccaagccgactccctgggccctccg atacaccaattctgccacagtgatga (SEQ ID NO: 22, MOD-9011).

In another embodiment, the cytokine as described herein comprises an interferon (IFN) peptide and two CTP units attached to its carboxy terminus. In another embodiment, the polypeptide as described herein comprises an interferon (IFN) peptide - CTP (x2) encoded by an amino acid sequence comprising the amino acid sequence set forth in SEQ ID NO: 12. In another embodiment, SEQ ID NO: 12 comprises the following amino acid (AA) sequence:

MTNKCLLQIALLLCFSTTALSMSYNLLGFLQRSSNFQCQKLLWQLNGRLEYCLKDRMNF
DIPEEIKQLQQFQKEDAALTIYEMLQNIFAIFRQDSSSTGWNETIVENLLANVYHQINHLK
TVLEEKLEKEDFTRGKLMSSLHLKRYYGRILHYLKAKEYSHCAWTIVRVEILRNFYFINR
LTGYLRNSSSSKAPPPSLPSPSRLPGPSDTPILPQSSSSKAPPPSLPSPSRLPGPSDTPILPQ
(SEQ ID NO: 12, MOD-9012).

In another embodiment, the cytokine as described herein comprising an interferon (IFN) peptide - and two CTP units attached to its carboxy terminus is encoded by a nucleic acid molecule set forth in SEQ ID NO: 23. In another embodiment, SEQ ID NO: 23 comprises the following nucleotide acid (NA) sequence:

tctagaggacatgaccaacaagtgcctgctgcagatcgccctgctgctgtgcttcagcaccaccgccctgagcatgagctacaacctgctgg
gcttcctgcagaggtccagcaacttccagtgccagaagctgctgtggcagctgaacggcaggctggaatactgcctgaaggacaggatgaa
cttcgacatcccagaggaaatcaagcagctgcagcagttccagaaggaggacgccgccctgaccatctacgagatgctgcagaacatcttc
gccatcttcaggcaggacagcagcagcaccggctggaacgagaccatcgtggagaacctgctggccaacgtgtaccaccagatcaaccac
ctgaaaaccgtgctggaagagaagctggaaaaggaggacttcaccaggggcaagctgatgagcagcctgcacctgaagaggtactacgg
cagaatcctgcactacctgaaggccaaggagtacagccactgcgcctggaccatcgtgagggtggagatcctgaggaacttctacttcatca
acaggctgaccggctacctgaggaacagctccagcagcaaggcccctccaccttccctgcccagtccaagccgactccctgggccctccg
acacaccaatcctgccacagagcagctcctctaaggcccctcctccatccctgccatccccctcccggctgcctggcccctctgacaccccta
tcctgcctcagtgatgaaggtctggatccgcggccgc (SEQ ID NO: 23, MOD-9012).

In another embodiment, the cytokine as described herein comprises an interferon (IFN) peptide, a single CTP unit attached to the IFN's amino terminus, and two CTP units attached to the IFN's carboxy terminus. In another embodiment, the polypeptide as described herein comprises an interferon (IFN) peptide, a single CTP unit attached to the IFN's amino terminus and two CTP units attached in tandem to the IFN's carboxy terminus. In another embodiment, the polypeptide as described herein comprises (from amino to carboxy termini): CTP (x1)-interferon (IFN) peptide - CTP (x2) comprising an amino acid sequence set forth in SEQ ID NO: 9. In another embodiment, SEQ ID NO: 9 comprises the following amino acid (AA) sequence:

MTNKCLLQIALLLCFSTTALSSSSSKAPPPSLPSPSRLPGPSDTPILPQMSYNLLGFLQRSSN
FQCQKLLWQLNGRLEYCLKDRMNFDIPEEIKQLQQFQKEDAALTIYEMLQNIFAIFRQDS
SSTGWNETIVENLLANVYHQINHLKTVLEEKLEKEDFTRGKLMSSLHLKRYYGRILHYL
KAKEYSHCAWTIVRVEILRNFYFINRLTGYLRNSSSKAPPPSLPSPSRLPGPSDTPILPQSS
SSKAPPPSLPSPSRLPGPSDTPILPQ (SEQ ID NO: 9, MOD-9013).

In another embodiment, the cytokine as described herein comprising an interferon (IFN) peptide, a single CTP unit attached to the IFN's amino terminus and two CTP units attached to the IFN's carboxy terminus is encoded by a nucleic acid molecule set forth in SEQ ID NO: 24. In another embodiment, SEQ ID NO: 24 comprises the following nucleotide acid (NA) sequence:

tctagaggacatgaccaacaagtgcctgctgcagatcgccctgctgctgtgcttcagcaccaccgccctgagcagcagcagctccaaggcc
ccaccccccagcctgcccagccccagcagactgccaggccccagcgacacccccatcctgccccagatgagctacaacctgctgggcttc
ctgcagaggtccagcaacttccagtgccagaagctgctgtggcagctgaacggcaggctggaatactgcctgaaggacaggatgaacttcg
acatcccagaggaaatcaagcagctgcagcagttccagaaggaggacgccgccctgaccatctacgagatgctgcagaacatcttcgccat

cttcaggcaggacagcagcagcaccggctggaacgagaccatcgtggagaacctgctggccaacgtgtaccaccagatcaaccacctgaa
aaccgtgctggaagagaagctggaaaaggaggacttcaccaggggcaagctgatgagcagcctgcacctgaagaggtactacggcagaa
tcctgcactacctgaaggccaaggagtacagccactgcgcctggaccatcgtgagggtggagatcctgaggaacttctacttcatcaacagg
ctgaccggctacctgaggaacagctccagcagcaaggcccctccaccttccctgcccagtccaagccgactccctgggccctccgacacac
caatcctgccacagagcagctcctctaaggcccctcctccatccctgccatccccctcccggctgcctggcccctctgacacccctatcctgcc
tcagtgatgaaggtctggatccgcggccgc (SEQ ID NO: 24, MOD-9013).

In another embodiment, the cytokine as described herein comprises an interferon (IFN) peptide, a single CTP attached to the IFN's amino terminus, and a single CTP located within an IFN coding sequence. In another embodiment, the polypeptide as described herein comprises (from amino to carboxy termini): CTP (x1)-interferon (IFN) peptide (fragment 1) - CTP- interferon (IFN) peptide (fragment 2) comprising an amino acid sequence set forth in SEQ ID NO: 25. In another embodiment, SEQ ID NO: 25 comprises the following amino acid (AA) sequence:

MTNKCLLQIALLLCFSTTALSSSSSKAPPPSLPSPSRLPGPSDTPILPQMSYNLLGFLQRSSN
FQCQKLLWQLNGRLEYCLKDRMNFDIPEEIKQLQQFQKEDAALTIYEMLQNIFAIFRQDS
SSTGWNETIVENLLANVYHQINHLKTVLEEKLEKEDFTRGKLMSSLHLKRYYGRILHYL
KAKEYSHCAWTIVRVEILRNFYFINRLTGYLRNSSSSKAPPPSLPSPSRLPGPSDTPILPQM
SYNLLGFLQRSSNFQCQKLLWQLNGRLEYCLKDRMNFDIPEEIKQLQQFQKEDAALTIY
EMLQNIFAIFRQDSSSTGWNETIVENLLANVYHQINHLKTVLEEKLEKEDFTRGKLMSSL
HLKRYYGRILHYLKAKEYSHCAWTIVRVEILRNFYFINRLTGYLRN (SEQ ID NO: 25,
MOD-9014).

In another embodiment, the cytokine as described herein comprising an interferon (IFN) peptide, a single CTP unit attached to the IFN's amino terminus, and a single CTP unit located within the IFN coding sequence is encoded by a nucleic acid molecule set forth in SEQ ID NO: 26. In another embodiment, SEQ ID NO: 26 comprises the following nucleotide acid (NA) sequence:

tctagaggacatgaccaacaagtgcctgctgcagatcgccctgctgctgtgcttcagcaccaccgccctgagcagcagcagctccaaggcc
ccaccccccagcctgcccagccccagcaggctgccaggccccagcgacacccccatcctgccccagatgagctacaacctgctgggcttc
ctgcagaggtccagcaacttccagtgccagaaactgctgtggcagctgaacggcaggctggaatactgcctgaaggaccggatgaacttcg
acatccccgaagagatcaagcagctgcagcagttccagaaagaggacgccgccctgaccatctacgagatgctgcagaacatcttcgccat
cttcaggcaggacagcagcagcaccggctggaacgagaccatcgtggagaacctgctggccaacgtgtaccaccagatcaaccacctgaa
aaccgtgctggaagagaagctggaaaaagaggacttcaccaggggcaagctgatgagcagcctgcacctgaagaggtactacggcagaa
tcctgcactacctgaaggccaaagagtacagccactgcgcctggaccatcgtgagggtggagatcctgcggaacttctacttcatcaacagg
ctgaccggctacctgaggaacagctccagcagcaaggcccctccaccctccctgccctccccaagcagactgcccggaccctccgacaca
ccaattctgccacagatgtcctacaatctgctcggatttctgcagcgctcctccaactttcagtgtcagaagctcctctggcagctcaatggccgc
ctggaatattgtctgaaagacagaatgaattttgacatcccagaggaaattaaacagctccagcagtttcagaaagaagatgctgctctcacaat

ctatgaaatgctccagaatatctttgcaatctttcgccaggacagctcctccaccgggtggaatgagacaattgtcgagaatctgctcgccaatg
tctatcatcagatcaatcacctcaagacagtcctcgaagaaaaactcgaaaaagaagatttcacacgcggcaaactgatgtcctccctgcatct
gaagcgctactatgggcgcatcctgcattatctgaaagctaaagaatactcccactgtgcttggacaattgtgcgcgtcgagatcctgagaaac
ttttatttcattaaccgcctgacaggatacctgcgcaactgatgaaggtctggatgcggccgc (SEQ ID NO: 26, MOD-9014).

In another embodiment, the cytokine as described herein comprises an interferon (IFN) peptide and a single CTP unit attached to its amino terminus. In another embodiment, the polypeptide as described herein comprises an interferon (IFN) peptide - CTP comprising an amino acid sequence set forth in SEQ ID NO: 11. In another embodiment, SEQ ID NO: 11 comprises the following amino acid (AA) sequence:

MTNKCLLQIALLLCFSTTALSSSSSKAPPPSLPSPSRLPGPSDTPILPQMSYNLLGFLQRSSN
FQCQKLLWQLNGRLEYCLKDRMNFDIPEEIKQLQQFQKEDAALTIYEMLQNIFAIFRQDS
SSTGWNETIVENLLANVYHQINHLKTVLEEKLEKEDFTRGKLMSSLHLKRYYGRILHYL
KAKEYSHCAWTIVRVEILRNFYFINRLTGYLRN* (SEQ ID NO: 11, MOD-9015).

In another embodiment, the polypeptide as described herein comprising an interferon (IFN) peptide - and a single CTP attached to its amino terminus is encoded by a nucleic acid molecule set forth in SEQ ID NO: 27. In another embodiment, SEQ ID NO: 27 comprises the following nucleotide acid (NA) sequence:

tctagaggacatgaccaacaagtgcctgctgcagatcgccctgctgctgtgcttcagcaccaccgccctgagcagcagcagctccaaggcc
ccaccccccagcctgcccagccccagcaggctgccaggccccagcgacacccccatcctgccccagatgagctacaacctgctgggcttc
ctgcagaggtccagcaacttccagtgccagaaactgctgtggcagctgaacggcaggctggaatactgcctgaaggaccggatgaacttcg
acatccccgaagagatcaagcagctgcagcagttccagaaagaggacgccgccctgaccatctacgagatgctgcagaacatcttcgccat
cttcaggcaggacagcagcagcaccggctggaacgagaccatcgtggagaacctgctggccaacgtgtaccaccagatcaaccacctgaa
aaccgtgctggaagagaagctggaaaaagaggacttcaccaggggcaagctgatgagcagcctgcacctgaagaggtactacggcagaa
tcctgcactacctgaaggccaaagagtacagccactgcgcctggaccatcgtgagggtggagatcctgcggaacttctacttcatcaacagg
ctgaccggctacctgaggaactgatgagtccgcggccgc (SEQ ID NO: 27, MOD-9015).

In another embodiment, the polypeptide as described herein comprises an interferon (IFN) peptide, a single CTP unit attached to its amino terminus, and a single CTP unit attached to its carboxy terminus. In another embodiment, the polypeptide as described herein comprises an interferon (IFN) peptide - CTP comprising an amino acid sequence set forth in SEQ ID NO: 10. In another embodiment, SEQ ID NO: 10 comprises the following amino acid (AA) sequence:

MTNKCLLQIALLLCFSTTALSSSSSKAPPPSLPSPSRLPGPSDTPILPQMSYNLLGFLQRSSN
FQCQKLLWQLNGRLEYCLKDRMNFDIPEEIKQLQQFQKEDAALTIYEMLQNIFAIFRQDS
SSTGWNETIVENLLANVYHQINHLKTVLEEKLEKEDFTRGKLMSSLHLKRYYGRILHYL

KAKEYSHCAWTIVRVEILRNFYFINRLTGYLRNSSSSKAPPPSLPSPSRLPGPSDTPILPQ
(SEQ ID NO: 10, MOD-9016).

In another embodiment, the cytokine as described herein comprising an interferon (IFN) peptide, a single CTP unit attached to its amino terminus, and a single CTP unit attached to its carboxy terminus is encoded by a nucleic acid molecule set forth in SEQ ID NO: 28. In another embodiment, SEQ ID NO: 28 comprises the following nucleotide acid (NA) sequence:

tctagaggacatgaccaacaagtgcctgctgcagatcgccctgctgctgtgcttcagcaccaccgccctgagcagcagcagctccaaggcc
ccaccccccagcctgcccagccccagcagactgccaggccccagcgacacccccatcctgccccagatgagctacaacctgctgggcttc
ctgcagaggtccagcaacttccagtgccagaagctgctgtggcagctgaacggcaggctggaatactgcctgaaggacaggatgaacttcg
acatcccagaggaaatcaagcagctgcagcagttccagaaggaggacgccgccctgaccatctacgagatgctgcagaacatcttcgccat
cttcaggcaggacagcagcagcaccggctggaacgagaccatcgtggagaacctgctggccaacgtgtaccaccagatcaaccacctgaa
aaccgtgctggaagagaagctggaaaaggaggacttcaccaggggcaagctgatgagcagcctgcacctgaagaggtactacggcagaa
tcctgcactacctgaaggccaaggagtacagccactgcgcctggaccatcgtgagggtggagatcctgaggaacttctacttcatcaacagg
ctgaccggctacctgaggaacagctccagcagcaaggcccctccaccttccctgcccagtccaagccgactccctgggccctccgatacac
caattctgccacagtgatgaaggtctggatgcggccgc (SEQ ID NO: 28, MOD-9016).

In another embodiment, an interferon β peptide comprises SEQ ID NO: 29 comprising the following amino acid (AA) sequence:

MSYNLLGFLQRSSNFQSQKLLWQLNGRLEYCLKDRMNFDIPEEIKQLQQFQKEDAALTI
YEMLQNIFAIFRQDSSSTGWNETIVENLLANVYHQINHLKTVLEEKLEKEDFTRGKLMSS
LHLKRYYGRILHYLKAKEYSHCAWTIVRVEILRNFYFINRLTGYLRN (SEQ ID NO: 29).

In another embodiment, the polypeptide of interest comprises a cytokine having additionally at least one CTP amino acid peptide on the N-terminus and one CTP amino acid peptide on the C-terminus. In another embodiment, the cytokine having additionally at least one CTP amino acid peptide on the N-terminus and two CTP amino acid peptides on the C-terminus is selected from lymphokines, monokines, chemokine, and interleukin. In another embodiment, the cytokine comprises an autocrine action activity. In another embodiment, the cytokine comprises a paracrine action activity. In another embodiment, the cytokine comprises an endocrine action activity.

In some embodiments, glucagon-like peptide-1 is utilized according to the teachings disclosed herein. In some embodiments, the attachment of CTP sequences to both the amino and carboxy termini of a "glucagon-like peptide-1" results in increased potency. In some embodiments, the attachment of CTP to both the amino and carboxy termini of a peptide results in prolonged in-vivo activity. In some embodiments, the attachment of CTP to both the amino and carboxy termini of the glucagon-like peptide-results in prolonged *in-vivo* activity.

A skilled artisan would appreciate that the term"glucagon-like peptide-1" (GLP-1) may encompass a mammalian polypeptide. Further, the skilled artisan would appreciate that the term "glucagon-like peptide-1" (GLP-1) may encompass a human polypeptide. In some embodiments, GLP-1 is cleaved from a glucagon preproprotein that has the ability to bind to the GLP-1 receptor and initiate a signal transduction pathway resulting in insulinotropic activity. A skilled artisan would appreciate that the term "insulinotropic activity" may encompass the ability to stimulate insulin secretion in response to elevated glucose levels, thereby causing glucose uptake by cells and decreased plasma glucose levels. In some embodiments, GLP-1 polypeptides include, but are not limited to those described in U.S. Pat. No. 5,118,666; which is incorporated by reference herein.

In one embodiment, the amino acid sequence of GLP-1 comprises the sequence set forth in SEQ ID NO: 21: HAEGTFTSDVSSYLEGQAAKEFIAWLVKGR. A skilled artisan would recognize that a GLP-1 disclosed herein may also encompass a GLP-1 homologue. In one embodiment, GLP-1 amino acid sequence disclosed herein is at least 50% homologous to GLP-1 sequences set forth in SEQ ID NO: 21 as determined using BlastP software of the National Center of Biotechnology Information (NCBI) using default parameters). In another embodiment, GLP-1 amino acid sequence disclosed herein is at least 60% homologous to GLP-1 sequences set forth in SEQ ID NO: 21 as determined using BlastP software of the National Center of Biotechnology Information (NCBI) using default parameters), is at least 70% homologous to GLP-1 sequences set forth in SEQ ID NO: 21,is at least 80% homologous to GLP-1 sequences set forth in SEQ ID NO: 21, is at least 90% homologous to GLP-1 sequences set forth in SEQ ID NO: 21,is at least 95% homologous to GLP-1 sequences set forth in SEQ ID NO: 21 as determined using BlastP software of the National Center of Biotechnology Information (NCBI) using default parameters).

In another embodiment, the methods disclosed herein manufacture a GLP-1 peptide having additionally at least one CTP amino acid peptide on the N-terminus and at least one CTP amino acid peptide on the C-terminus for treating or inhibiting type II diabetes. In another embodiment, the methods disclosed herein manufacture a GLP-1 peptide having additionally one CTP amino acid peptide on the N-terminus and two CTP amino acid peptides on the C-terminus for treating or inhibiting type II diabetes. In another embodiment, the methods disclosed herein manufacture a GLP-1 peptide set forth in SEQ ID NO: 21 having additionally at least one CTP amino acid peptide on the N-terminus and at least one CTP amino acid peptide on the C-terminus for treating or inhibiting type II diabetes.

In one embodiment, disclosed herein are long-acting coagulation factors and methods of producing and using same. In another embodiment, long-acting coagulation factors comprise a carboxy terminal peptide (CTP, also referred to as CTP unit). In another embodiment, long-acting polypeptides which comprise a coagulation factor further comprise a carboxy terminal peptide (CTP) of human Chorionic Gonadotropin (hCG). In another embodiment, CTP acts as a protectant against the degradation of a coagulation factor. In another embodiment, CTP extends the Cₘₐₓ of a coagulation factor. In another embodiment, CTP extends the Tₘₐₓ of a coagulation factor. In another embodiment, CTP extends the circulatory half-life of a coagulation factor. In some embodiments, CTP enhances the potency of a coagulation factor.

In another embodiment, disclosed herein is a method of extending the biological half-life of a coagulation factor, comprising the step of attaching one to ten CTPs to the carboxy terminus of the coagulation factor, thereby extending the biological half-life of the coagulation factor. In another embodiment, disclosed herein is a method of extending the biological half-life of a coagulation factor, comprising the step of attaching one to five CTPs to the carboxy terminus of the coagulation factor, thereby extending the biological half-life of the coagulation factor. In another embodiment, disclosed herein is a method for extending the circulatory half-life of a coagulation factor. In another embodiment, disclosed herein is a method for increasing the half-life of a coagulation factor. In another embodiment, disclosed herein is a method for extending the half-life of a coagulation factor.

Coagulation Factor VII (FVII) is a 444 amino acid glycoprotein (50KDa) secreted by hepatocytes into the bloodstream as an inactive pro-enzyme. Upon tissue injury and exposure to circulating blood, FVII forms a complex with Tissue Factor (TF) which is a true receptor protein to FVII and is expressed by various cells localized in the deeper layers of the vessel wall. The formation of this FVII-TF complex leads to activation of FVII. Activated FVII (FVIIa) initiates the extrinsic coagulation pathway by activating Factor IX and Factor X.

FVII belong to a group of Vitamin K-dependent glycoproteins associated with the coagulation system. Besides FVII, this group consists of Factor IX, Factor X, Protein C and prothrombin. These proteins have similar domain organizations and are synthesized as precursors with an N-terminal propeptide followed by a mature amino acid sequence. The propeptide contains a docking site for gamma carboxylase which converts glutamic acids (Glu) into gamma carboxy glutamic acids (Gla). This domain is followed by two epidermal growth factor-like (EGF) domains, a connecting region (CR) and a C-terminal serine protease domain. Prior to secretion, FVII propeptide is cleaved forming a 406 amino acid single chain zymogen FVII glycoprotein. After secretion, the protein can be activated into a disulfide-linked two chain heterodimer, FVIIa, by cleavage in the CR. The plasma concentration of FVII is 10 nM and approximately 1% circulates in the active form in healthy individuals.

Factor IX (FIX) is a 415 Amino acid (55KDa) glycoprotein; it belongs to a group of vitamin K dependent glycoproteins associated with the coagulation system. FIX has a similar domain organization as factor FVII, Factor X, Protein C and prothrombin that are synthesized as precursors with an N-terminal propeptide followed by a mature amino acid sequence.

FIX is secreted as a single chain molecule that undergoes complex post-transcriptional modifications, many of which are critical to its biochemical and pharmacokinetic properties. Among all the post-transcriptional modifications, 12 glutamic acid residues near the amino terminus of FIX that are gamma carboxylated by the vitamin K-dependent gamma carboxylase are the most crucial ones. Carboxylation is required for the interaction of FIX with the phospholipid surfaces and for optimal FIX activity. The amino terminus propeptide serves as a recognition site for the gamma carboxylase and thus, following gamma carboxylation, it is cleaved off by the Golgi apparatus serine protease known as Paired basic Amino acid Cleave Enzyme (PACE/Furin). Four additional post-transcriptional modifications might occur at the Golgi apparatus: sulfation of tyrosine 155, phosphorylation of serine 158, O- glycosylation on Ser 63 and on 61 and finally, N-glycosylation on Asn 157 and 16, but were shown not to be necessary for proper activity of FIX.

FIX circulates in the plasma (average concentration of 5 µg/ml) as a single chain inactive zymogen. Upon proteolytic cleavage at two peptide bonds: Arg 145 and Arg 180 by either one or two physiological activators, FVIIa-TF complex or FIXa, the activation peptide is removed, converting FIX to a fully active enzyme consisting of a light and heavy chain held together by a single disulfide bond. The N-terminal light chain contains the non-catalytic gamma carboxy glutamic acid (Gla) and two epidermal growth factor-like domains, while the C-terminal heavy chain contains the trypsin-like catalytic domain of the molecule. FIXa alone is characterized by poor catalytic activity. However when complexed with FVIII, its proteolytic activity increase by 4-5 orders of magnitude towards its natural substrate FX.

In another embodiment, disclosed herein is a method of extending the biological half-life or a method of improving the area under the curve (AUC) of a coagulation factor, comprising the step of attaching one to ten CTPs to the carboxy terminus of the coagulation factor, thereby extending the biological half-life or improving the AUC of the coagulation factor. In another embodiment, disclosed herein is a method of extending the biological half-life or a method of improving the area under the curve (AUC) of a coagulation factor, comprising the step of attaching one to five CTPs to the carboxy terminus of the coagulation factor, thereby extending the biological half-life or improving the AUC of the coagulation factor. In another embodiment, disclosed herein is a method of extending the biological half-life or a method of improving the area under the curve (AUC) of FIX, comprising the step of attaching one to five CTPs to the carboxy terminus of the FIX, thereby extending the biological half-life or improving the AUC of the FIX. In another embodiment, disclosed herein is a method of extending the biological half-life or a method of improving the area under the curve (AUC) of FVII or FVIIa, comprising the step of attaching one to five CTPs to the carboxy terminus of FVII or FVIIa, thereby extending the biological half-life or improving the AUC of FVII or FVIIa.

In another embodiment, disclosed herein is a method of extending the biological half-life of a Factor IX (FIX) polypeptide, comprising the step of attaching three chorionic gonadotropin carboxy terminal peptides (CTPs) to the carboxy terminus of said FIX polypeptide, thereby extending the biological half-life of said FIX polypeptide. In another embodiment a method of extending the biological half-life of a Factor VIIa (FVIIa) polypeptide, comprises the step of attaching up to five chorionic gonadotropin carboxy terminal peptides (CTPs) to the carboxy terminus of said FVIIa polypeptide, thereby extending the biological half-life of said FVIIa polypeptide. In one embodiment, three chorionic gonadotropin carboxy terminal peptides (CTPs) are attached to the carboxy terminus of said FVIIa polypeptide. In another embodiment, four chorionic gonadotropin carboxy terminal peptides (CTPs) are attached to the carboxy terminus of said FVIIa polypeptide. In another embodiment, five chorionic gonadotropin carboxy terminal peptides (CTPs) are attached to the carboxy terminus of said FVIIa polypeptide.

In another embodiment, disclosed herein is a method of improving the area under the curve (AUC) of a Factor IX (FIX) polypeptide, comprising the step of attaching three chorionic gonadotropin carboxy terminal peptides (CTPs) to the carboxy terminus of said FIX polypeptide, thereby improving the AUC of said FIX polypeptide. In another embodiment, disclosed herein is a method of improving the area under the curve (AUC) of a Factor VIIa (FVIIa) polypeptide, comprising the step of attaching up to five chorionic gonadotropin carboxy terminal peptides (CTPs) to the carboxy terminus of said FVIIa polypeptide, thereby improving the AUC of said FVIIa polypeptide. In one embodiment, three chorionic gonadotropin carboxy terminal peptides (CTPs) are attached to the carboxy terminus of said FVIIa polypeptide. In another embodiment, four chorionic gonadotropin carboxy terminal peptides (CTPs) are attached to the carboxy terminus of said FVIIa polypeptide. In another embodiment, five chorionic gonadotropin carboxy terminal peptides (CTPs) are attached to the carboxy terminus of said FVIIa polypeptide.

In another embodiment, a coagulation factor disclosed herein is a protein. In another embodiment, a coagulation factor disclosed herein is a peptide. In another embodiment, a coagulation factor disclosed herein is a polypeptide. In another embodiment, the coagulation factor is an enzyme. In another embodiment, the coagulation factor is a serine protease. In another embodiment, the coagulation factor is a glycoprotein. In another embodiment, the coagulation factor is a transglutaminase. In another embodiment, the coagulation factor is an inactive zymogen. In another embodiment, the coagulation factor is any coagulation factor known to one of skill in the art.

In another embodiment, the coagulation factor is Factor VIII (FVIII). In another embodiment, the coagulation factor is Factor V (FV). In another embodiment, the coagulation factor is Factor XIII (FXIII). In another embodiment, the coagulation factor is Factor X (FX). In another embodiment, the coagulation factor is fibrin.

In another embodiment, the coagulation factor is Factor VIIa (FVIIa). In another embodiment, the coagulation factor is Factor VII (FVII). In another embodiment, the coagulation factor is Factor IX (FIX). In another embodiment, the coagulation factor is Factor X (FX). In another embodiment, the coagulation factor is Factor XIa (FXIa). In another embodiment, the coagulation factor is Factor XII (FXII). In another embodiment, the coagulation factor is Factor Xa (FXa). In another embodiment, the coagulation factor is Factor Va (FVa). In another embodiment, the coagulation factor is prothrombin. In another embodiment, the coagulation factor is thrombin. In another embodiment, the coagulation factor is Factor XI (FXI). In another embodiment, the coagulation factor is Von Willebrand factor (vWF). In another embodiment, the coagulation factor is activated Factor VIIIa (FVIIIa). In another embodiment, the coagulation factor is B-deleted Domain FVIII (FVIIIBDD). In another embodiment, the coagulation factor is B domain-deleted FVIII (FVIIIBDD). In another embodiment, the coagulation factor is Beta domain-deleted FVIII (FVIIIBDD). In another embodiment, the coagulation factor is Factor IXa (FIXa). In another embodiment, the coagulation factor is prekallikrein. In another embodiment, the coagulation factor is kallikrein. In another embodiment, the coagulation factor is Factor XIIa (FXIIa). In another embodiment, the coagulation factor is Fibrinogen. In another embodiment, the coagulation factor is thrombomodulin. In another embodiment, the coagulation factor is Factor II (FII).

In another embodiment, the coagulation factor is a glycoprotein. In another embodiment, the coagulation factor is a vitamin K-dependent glycoprotein. In another embodiment, the coagulation factor is a vitamin K-independent glycoprotein.

In another embodiment, the coagulation factor is a recombinant protein. In another embodiment, the coagulation factor is a recombinant glycoprotein. In another embodiment, the coagulation factor is a recombinant glycoprotein FV. In another embodiment, the coagulation factor is a recombinant FVI. In another embodiment, the coagulation factor is a recombinant FVII. In another embodiment, the coagulation factor is a recombinant FVIII. In another embodiment, the coagulation factor is a recombinant FIX. In another embodiment, the coagulation factor is a recombinant FX. In another embodiment, the coagulation factor is a recombinant FXI. In another embodiment, the coagulation factor is a recombinant FXII. In another embodiment, the coagulation factor is a recombinant FvW. In another embodiment, the coagulation factor is a recombinant FII. In another embodiment, the coagulation factor is a recombinant FIXa. In another embodiment, the coagulation factor is a recombinant FXIa. In another embodiment, the coagulation factor is a recombinant fibrin. In another embodiment, the coagulation factor is a recombinant FVIIa. In another embodiment, the coagulation factor is a recombinant FXa. In another embodiment, the coagulation factor is a recombinant FVa. In another embodiment, the coagulation factor is a recombinant prothrombin. In another embodiment, the coagulation factor is a recombinant thrombin. In another embodiment, the coagulation factor is a recombinant FVIIIa. In another embodiment, the coagulation factor is a recombinant prekallikrein. In another embodiment, the coagulation factor is a recombinant kallikrein. In another embodiment, the coagulation factor is a recombinant FXIIa. In another embodiment, the coagulation factor is any known recombinant coagulation factor. In another embodiment, the coagulation factor comprising a signal peptide is any known recombinant coagulation factor.

In another embodiment, a coagulation factor comprises 1-10 CTP repeats attached to the C-terminus and no CTPs attached to the N-terminus. In another embodiment, a coagulation factor comprises at least one CTP attached to the C-terminus and no CTPs attached to the N-terminus. In another embodiment, a coagulation factor comprising 1-10 CTP repeats attached to the C-terminus and no CTPs attached to the N-terminus is an engineered coagulation factor. In another embodiment, a coagulation factor comprising at least one CTP attached to the C-terminus and no CTPs attached to the N-terminus is an engineered coagulation factor. In another embodiment, a coagulation factor comprising 1-10 CTP repeats attached to the C-terminus and no CTPs attached to the N-terminus is a conjugated coagulation factor. In another embodiment, a coagulation factor comprising at least one CTP attached to the C-terminus and no CTPs attached to the N-terminus is a conjugated coagulation factor.

In one embodiment, disclosed herein is a CTP-modified Factor IX (FIX) polypeptide consisting of a FIX polypeptide and three gonadotropin carboxy terminal peptides (CTPs) attached to the carboxy terminus of said CTP-modified FIX polypeptide.

In another embodiment, a CTP-modified Factor VIIa (FVIIa) polypeptide disclosed herein consists of a FVIIa polypeptide and five gonadotropin carboxy terminal peptides (CTPs) attached to the carboxy terminus of said FVIIa.

In another embodiment, the coagulation factor is a coagulation factor comprising a domain organization similar or identical to the domain organization of FIX, FVII, Factor X, Protein C, or prothrombin. In another embodiment, the coagulation factor is synthesized as a precursor with an N-terminal propeptide. In another embodiment, the coagulation factor as used herein is in an inactive pro-enzyme form. In another embodiment, the coagulation factor is produced in hepatocytes. In another embodiment, the coagulation factor comprises a docking site for gammacarboxylase which converts glutamic acids (Glu) into gamma carboxy glutamic acids (Gla). In another embodiment, the coagulation factor as used herein is a commercially available coagulation factor.

In one embodiment, the nucleic acid sequence encoding Factor VII comprises the following nucleic acid sequence:

ctcgaggacatggtctcccaggccctcaggctcctctgccttctgcttgggcttcagggctgcctggctgcagtcttcgtaacccaggaggaa
gcccacggcgtcctgcaccggcgccggcgcgccaacgcgttcctggaggagctgcggccgggctccctggagagggagtgcaaggag
gagcagtgctccttcgaggaggcccgggagatcttcaaggacgcggagaggacgaagctgttctggatttcttacagtgatggggaccagt
gtgcctcaagtccatgccagaatgggggctcctgcaaggaccagctccagtcctatatctgcttctgcctccctgccttcgagggccggaact
gtgagacgcacaaggatgaccagctgatctgtgtgaacgagaacggcggctgtgagcagtactgcagtgaccacacgggcaccaagcgct
cctgtcggtgccacgaggggtactctctgctggcagacggggtgtcctgcacacccacagttgaatatccatgtggaaaaatacctattctaga
aaaaagaaatgccagcaaaccccaaggccgaattgtggggggcaaggtgtgccccaaaggggagtgtccatggcaggtcctgttgttggt
gaatggagctcagttgtgtggggggaccctgatcaacaccatctgggtggtctccgcggcccactgtttcgacaaaatcaagaactggagga
acctgatcgcggtgctgggcgagcacgacctcagcgagcacgacggggatgagcagagccggcgggtggcgcaggtcatcatccccag
cacgtacgtcccgggcaccaccaaccacgacatcgcgctgctccgcctgcaccagcccgtggtcctcactgaccatgtggtgcccctctgcc
tgcccgaacggacgttctctgagaggacgctggccttcgtgcgcttctcattggtcagcggctggggccagctgctggaccgtggcgccacg
gccctggagctcatggtcctcaacgtgccccggctgatgacccaggactgcctgcagcagtcacggaaggtgggagactccccaaatatca
cggagtacatgttctgtgccggctactcggatggcagcaaggactcctgcaagggggacagtggaggcccacatgccacccactaccggg
gcacgtggtacctgacgggcatcgtcagctggggccagggctgcgcaaccgtgggccactttggggtgtacaccagggtctcccagtacat
cgagtggctgcaaaagctcatgcgctcagagccacgcccaggagtcctcctgcgagccccatttccctgaggatgcggccgc (SEQ ID
NO: 30).

In another embodiment, the amino acid sequence of Factor VII comprises the following amino acid sequence:

MVSQALRLLCLLLGLQGCLAAVFVTQEEAHGVLHRRRRANAFLEELRPGSLERECKEEQ
CSFEEAREIFKDAERTKLFWISYSDGDQCASSPCQNGGSCKDQLQSYICFCLPAFEGRNCE
THKDDQLICVNENGGCEQYCSDHTGTKRSCRCHEGYSLLADGVSCTPTVEYPCGKIPILE
KRNASKPQGRIVGGKVCPKGECPWQVLLLVNGAQLCGGTLINTIWVVSAAHCFDKIKN
WRNLIAVLGEHDLSEHDGDEQSRRVAQVIIPSTYVPGTTNHDIALLRLHQPVVLTDHVVP
LCLPERTFSERTLAFVRFSLVSGWGQLLDRGATALELMVLNVPRLMTQDCLQQSRKVG
DSPNITEYMFCAGYSDGSKDSCKGDSGGPHATHYRGTWYLTGIVSWGQGCATVGHFGV
YTRVSQYIEWLQKLMRSEPRPGVLLRAPFP (SEQ ID NO: 31).

In another embodiment, the amino acid sequence of Factor VII comprises the following amino acid sequence:

MVSQALRLLCLLLGLQGCLAAVFVTQEEAHGVLHRRRRANAFLEELRPGSLERECKEEQ
CSFEEAREIFKDAERTKLFWISYSDGDQCASSPCQNGGSCKDQLQSYICFCLPAFEGRNCE
THKDDQLICVNENGGCEQYCSDHTGTKRSCRCHEGYSLLADGVSCTPTVEYPCGKIPILE
KRNASKPQGRIVGGKVCPKGECPWQVLLLVNGAQLCGGTLINTIWVVSAAHCFDKIKN
WRNLIAVLGEHDLSEHDGDEQSRRVAQVIIPSTYVPGTTNHDIALLRLHQPVVLTDHVVP
LCLPERTFSERTLAFVRFSLVSGWGQLLDRGATALELMVLNVPRLMTQDCLQQSRKVG
DSPNITEYMFCAGYSDGSKDSCKGDSGGPHATHYRGTWYLTGIVSWGQGCATVGHFGV
YTRVSQYIEWLQKLMRSEPRPGVLLRAPFP*GCGR (SEQ ID NO: 32).

In another embodiment, the nucleic acid sequence encoding Factor VII-CTP (attached to the carboxy terminus) comprises the following nucleic acid sequence:

ctcgaggacatggtctcccaggccctcaggctcctctgccttctgcttgggcttcagggctgcctggctgcagtcttcgtaacccaggaggaa
gcccacggcgtcctgcaccggcgccggcgcgccaacgcgttcctggaggagctgcggccgggctccctggagagggagtgcaaggag
gagcagtgctccttcgaggaggcccgggagatcttcaaggacgcggagaggacgaagctgttctggatttcttacagtgatggggaccagt
gtgcctcaagtccatgccagaatgggggctcctgcaaggaccagctccagtcctatatctgcttctgcctccctgccttcgagggccggaact
gtgagacgcacaaggatgaccagctgatctgtgtgaacgagaacggcggctgtgagcagtactgcagtgaccacacgggcaccaagcgct
cctgtcggtgccacgaggggtactctctgctggcagacggggtgtcctgcacacccacagttgaatatccatgtggaaaaatacctattctaga
aaaaagaaatgccagcaaaccccaaggccgaattgtggggggcaaggtgtgccccaaaggggagtgtccatggcaggtcctgttgttggt
gaatggagctcagttgtgtggggggaccctgatcaacaccatctgggtggtctccgcggcccactgtttcgacaaaatcaagaactggagga
acctgatcgcggtgctgggcgagcacgacctcagcgagcacgacggggatgagcagagccggcgggtggcgcaggtcatcatccccag
cacgtacgtcccgggcaccaccaaccacgacatcgcgctgctccgcctgcaccagcccgtggtcctcactgaccatgtggtgcccctctgcc
tgcccgaacggacgttctctgagaggacgctggccttcgtgcgcttctcattggtcagcggctggggccagctgctggaccgtggcgccacg
gccctggagctcatggtcctcaacgtgccccggctgatgacccaggactgcctgcagcagtcacggaaggtgggagactccccaaatatca
cggagtacatgttctgtgccggctactcggatggcagcaaggactcctgcaagggggacagtggaggcccacatgccacccactaccggg
gcacgtggtacc
tgaccggcatcgtgagctggggccagggctgcgccaccgtgggccacttcggcgtgtacaccagggtgtcccagtacatcgagtggctgc
agaaactgatgagaagcgagcccagacccggcgtgctgctgagagcccccttccccagcagcagctccaaggcccctccccctagcctgc
ccagccctagcagactgcctgggcccagcgacacccccatcctgccccagtgaggatccgcggccgc (SEQ ID NO: 33).

In another embodiment, the amino acid sequence of Factor VII-CTP (attached to the carboxy terminus) comprises the following amino acid sequence:

MVSQALRLLCLLLGLQGCLAAVFVTQEEAHGVLHRRRRANAFLEELRPGSLERECKEEQ
CSFEEAREIFKDAERTKLFWISYSDGDQCASSPCQNGGSCKDQLQSYICFCLPAFEGRNCE
THKDDQLICVNENGGCEQYCSDHTGTKRSCRCHEGYSLLADGVSCTPTVEYPCGKIPILE

KRNASKPQGRIVGGKVCPKGECPWQVLLLVNGAQLCGGTLINTIWVVSAAHCFDKIKN
WRNLIAVLGEHDLSEHDGDEQSRRVAQVIIPSTYVPGTTNHDIALLRLHQPVVLTDHVVP
LCLPERTFSERTLAFVRFSLVSGWGQLLDRGATALELMVLNVPRLMTQDCLQQSRKVG
DSPNITEYMFCAGYSDGSKDSCKGDSGGPHATHYRGTWYLTGIVSWGQGCATVGHFGV
YTRVSQYIEWLQKLMRSEPRPGVLLRAPFPSSSSKAPPPSLPSPSRLPGPSDTPILPQ* (SEQ
ID NO: 34).

In another embodiment, the nucleic acid sequence encoding Factor VII-CTP-CTP (attached to the carboxy terminus) comprises the following nucleic acid sequence:

ctcgaggacatggtctcccaggccctcaggctcctctgccttctgcttgggcttcagggctgcctggctgcagtcttcgtaacccaggaggaa
gcccacggcgtcctgcaccggcgccggcgcgccaacgcgttcctggaggagctgcggccgggctccctggagagggagtgcaaggag
gagcagtgctccttcgaggaggcccgggagatcttcaaggacgcggagaggacgaagctgttctggatttcttacagtgatggggaccagt
gtgcctcaagtccatgccagaatgggggctcctgcaaggaccagctccagtcctatatctgcttctgcctccctgccttcgagggccggaact
gtgagacgcacaaggatgaccagctgatctgtgtgaacgagaacggcggctgtgagcagtactgcagtgaccacacgggcaccaagcgct
cctgtcggtgccacgaggggtactctctgctggcagacggggtgtcctgcacacccacagttgaatatccatgtggaaaaatacctattctaga
aaaaagaaatgccagcaaaccccaaggccgaattgtggggggcaaggtgtgccccaaaggggagtgtccatggcaggtcctgttgttggt
gaatggagctcagttgtgtggggggaccctgatcaacaccatctgggtggtctccgcggcccactgtttcgacaaaatcaagaactggagga
acctgatcgcggtgctgggcgagcacgacctcagcgagcacgacggggatgagcagagccggcgggtggcgcaggtcatcatccccag
cacgtacgtcccgggcaccaccaaccacgacatcgcgctgctccgcctgcaccagcccgtggtcctcactgaccatgtggtgcccctctgcc
tgcccgaacggacgttctctgagaggacgctggccttcgtgcgcttctcattggtcagcggctggggccagctgctggaccgtggcgccacg
gccctggagctcatggtcctcaacgtgccccggctgatgacccaggactgcctgcagcagtcacggaaggtgggagactccccaaatatca
cggagtacatgttctgtgccggctactcggatggcagcaaggactcctgcaagggggacagtggaggcccacatgccacccactaccggg
gcacgtggtacctgaccggcatcgtgagctggggccagggctgcgccaccgtgggccacttcggcgtgtacaccagggtgtcccagtaca
tcgagtggctgcagaaactgatgagaagcgagcccagacccggcgtgctgctgagagcccccttccccagcagcagctccaaggcccctc
cccctagcctgcccagccctagcagactgcctgggccctccgacacaccaatcctgccacagagcagctcctctaaggcccctcctccatcc
ctgccatccccctcccggctgccaggcccctctgacacccctatcctgcctcagtgatgaaggtctggatccgcggccgc (SEQ ID
NO: 35).

In another embodiment, the amino acid sequence of Factor VII-CTP-CTP (attached to the carboxy terminus) comprises the following amino acid sequence:

MVSQALRLLCLLLGLQGCLAAVFVTQEEAHGVLHRRRRANAFLEELRPGSLERECKEEQ
CSFEEAREIFKDAERTKLFWISYSDGDQCASSPCQNGGSCKDQLQSYICFCLPAFEGRNCE
THKDDQLICVNENGGCEQYCSDHTGTKRSCRCHEGYSLLADGVSCTPTVEYPCGKIPILE
KRNASKPQGRIVGGKVCPKGECPWQVLLLVNGAQLCGGTLINTIWVVSAAHCFDKIKN
WRNLIAVLGEHDLSEHDGDEQSRRVAQVIIPSTYVPGTTNHDIALLRLHQPVVLTDHVVP
LCLPERTFSERTLAFVRFSLVSGWGQLLDRGATALELMVLNVPRLMTQDCLQQSRKVG

DSPNITEYMFCAGYSDGSKDSCKGDSGGPHATHYRGTWYLTGIVSWGQGCATVGHFGV
YTRVSQYIEWLQKLMRSEPRPGVLLRAPFPSSSSKAPPPSLPSPSRLPGPSDTPILPQSSSSK
APPPSLPSPSRLPGPSDTPILPQ (SEQ ID NO: 36).

In one embodiment, a Factor VII-CTP-CTP-CTP is an activated Factor VII-CTP-CTP-CTP (FVIIa-CTP-CTP-CTP).

In another embodiment, the nucleic acid sequence encoding Factor VII-CTP-CTP-CTP (attached to the carboxy terminus) comprises the following nucleic acid sequence:

ctcgaggacatggtctcccaggccctcaggctcctctgccttctgcttgggcttcagggctgcctggctgcagtcttcgtaacccaggaggaa
gcccacggcgtcctgcaccggcgccggcgcgccaacgcgttcctggaggagctgcggccgggctccctggagagggagtgcaaggag
gagcagtgctccttcgaggaggcccgggagatcttcaaggacgcggagaggacgaagctgttctggatttcttacagtgatggggaccagt
gtgcctcaagtccatgccagaatgggggctcctgcaaggaccagctccagtcctatatctgcttctgcctccctgccttcgagggccggaact
gtgagacgcacaaggatgaccagctgatctgtgtgaacgagaacggcggctgtgagcagtactgcagtgaccacacgggcaccaagcgct
cctgtcggtgccacgaggggtactctctgctggcagacggggtgtcctgcacacccacagttgaatatccatgtggaaaaatacctattctaga
aaaaagaaatgccagcaaaccccaaggccgaattgtggggggcaaggtgtgccccaaaggggagtgtccatggcaggtcctgttgttggt
gaatggagctcagttgtgtggggggaccctgatcaacaccatctgggtggtctccgcggcccactgtttcgacaaaatcaagaactggagga
acctgatcgcggtgctgggcgagcacgacctcagcgagcacgacggggatgagcagagccggcgggtggcgcaggtcatcatccccag
cacgtacgtcccgggcaccaccaaccacgacatcgcgctgctccgcctgcaccagcccgtggtcctcactgaccatgtggtgcccctctgcc
tgcccgaacggacgttctctgagaggacgctggccttcgtgcgcttctcattggtcagcggctggggccagctgctggaccgtggcgccacg
gccctggagctcatggtcctcaacgtgccccggctgatgacccaggactgcctgcagcagtcacggaaggtgggagactccccaaatatca
cggagtacatgttctgtgccggctactcggatggcagcaaggactcctgcaagggggacagtggaggcccacatgccacccactaccggg
gcacgtggtacctgaccggcatcgtgagctggggccagggctgcgccaccgtgggccacttcggcgtgtacaccagggtgtcccagtaca
tcgagtggctgcagaaactgatgagaagcgagcccagacccggcgtgctgctgagagcccccttccccagcagcagctccaaggcccctc
cccctagcctgcccagccctagcagactgcctgggcccagtgacacccctatcctgcctcagtccagctccagcaaggccccaccccctag
cctgccttctccttctcggctgcctggccccagcgatactccaattctgccccagtcctccagcagtaaggctccccctccatctctgccatccc
ccagcagactgccaggcccttctgatacacccatcctcccacagtgatgaggatccgcggccgcttaattaa (SEQ ID NO: 37).

In another embodiment, the amino acid sequence of Factor VII-CTP-CTP-CTP (attached to the carboxy terminus) comprises the following amino acid sequence:

MVSQALRLLCLLLGLQGCLAAVFVTQEEAHGVLHRRRRANAFLEELRPGSLERECKEEQ
CSFEEAREIFKDAERTKLFWISYSDGDQCASSPCQNGGSCKDQLQSYICFCLPAFEGRNCE
THKDDQLICVNENGGCEQYCSDHTGTKRSCRCHEGYSLLADGVSCTPTVEYPCGKIPILE
KRNASKPQGRIVGGKVCPKGECPWQVLLLVNGAQLCGGTLINTIWVVSAAHCFDKIKN
WRNLIAVLGEHDLSEHDGDEQSRRVAQVIIPSTYVPGTTNHDIALLRLHQPVVLTDHVVP
LCLPERTFSERTLAFVRFSLVSGWGQLLDRGATALELMVLNVPRLMTQDCLQQSRKVG
DSPNITEYMFCAGYSDGSKDSCKGDSGGPHATHYRGTWYLTGIVSWGQGCATVGHFGV

YTRVSQYIEWLQKLMRSEPRPGVLLRAPFPSSSSKAPPPSLPSPSRLPGPSDTPILPQSSSSK
APPPSLPSPSRLPGPSDTPILPQSSSSKAPPPSLPSPSRLPGPSDTPILPQ (SEQ ID NO: 38).

In one embodiment, following expression a mature CTP-modified FVII polypeptide is secreted, the signal peptide having been cleaved from the precursor protein resulting in a mature protein. For example, in the precursor CTP-modified FVII polypeptide set forth in SEQ ID NO: 38, amino acids 1-38: MVSQALRLLCLLLGLQGCLAAVFVTQEEAHGVLHRRRR (SEQ ID NO: 110) represent the signal peptide of the precursor CTP-modified FVII polypeptide, and amino acids: ANAFLEELRPGSLERECKEEQCSFEEAREIFKDAERTKLFWISYSDGDQCASSPCQNGGS CKDQLQSYICFCLPAFEGRNCETHKDDQLICVNENGGCEQYCSDHTGTKRSCRCHEGYS LLADGVSCTPTVEYPCGKIPILEKRNASKPQGRIVGGKVCPKGECPWQVLLLVNGAQLC GGTLINTIWVVSAAHCFDKIKNWRNLIAVLGEHDLSEHDGDEQSRRVAQVIIPSTYVPGT TNHDIALLRLHQPVVLTDHVVPLCLPERTFSERTLAFVRFSLVSGWGQLLDRGATALEL MVLNVPRLMTQDCLQQSRKVGDSPNITEYMFCAGYSDGSKDSCKGDSGGPHATHYRGT WYLTGIVSWGQGCATVGHFGVYTRVSQYIEWLQKLMRSEPRPGVLLRAPFPSSSSKAPP PSLPSPSRLPGPSDTPILPQSSSSKAPPPSLPSPSRLPGPSDTPILPQSSSSKAPPPSLPSPSRLP GPSDTPILPQ (SEQ ID NO: 111) represent the mature engineered CTP-modified FVII polypeptide lacking the signal peptide. In one embodiment, the amino acid sequence of CTP-modified FVII without the signal peptide is set forth in SEQ ID NO: 111. In another embodiment, the amino acid sequence of activated CTP-modified FVII without the signal peptide is set forth in SEQ ID NO: 111. In another embodiment, the signal peptide of CTP-modified FVII is set forth in SEQ ID NO: 110.

In another embodiment, the amino acid sequence of activated Factor VII-CTP-CTP-CTP (attached to the carboxy terminus) (FVIIa-CTP3) lacks a signal peptide and comprises the amino acid sequence as put forth in SEQ ID NO: 111. Similarly, while the term MOD-5014 is interchangeable with the term FVIIa-CTP3, i.e., represents the active form of the CTP-modified coagulation factor, in certain instances the term MOD-5014 may be used to denote an active form of FVII or a nucleotide sequence encoding a FVII-CTP3, which will then be expressed and secreted from a cell, and purified and activated *in vitro,* resulting in the active form of FVIIa being present in a MOD-5014 molecule.

In another embodiment, the nucleic acid sequence encoding Factor VII-(CTP)₄ (attached to the carboxy terminus) comprises the following nucleic acid sequence:

ctcgaggacatggtctcccaggccctcaggctcctctgccttctgcttgggcttcagggctgcctggctgcagtcttcgtaacccaggaggaa
gcccacggcgtcctgcaccggcgccggcgcgccaacgcgttcctggaggagctgcggccgggctccctggagagggagtgcaaggag

gagcagtgctccttcgaggaggcccgggagatcttcaaggacgcggagaggacgaagctgttctggatttcttacagtgatggggaccagt
gtgcctcaagtccatgccagaatgggggctcctgcaaggaccagctccagtcctatatctgcttctgcctccctgccttcgagggccggaact
gtgagacgcacaaggatgaccagctgatctgtgtgaacgagaacggcggctgtgagcagtactgcagtgaccacacgggcaccaagcgct
cctgtcggtgccacgaggggtactctctgctggcagacggggtgtcctgcacacccacagttgaatatccatgtggaaaaatacctattctaga
aaaaagaaatgccagcaaaccccaaggccgaattgtggggggcaaggtgtgccccaaaggggagtgtccatggcaggtcctgttgttggt
gaatggagctcagttgtgtggggggaccctgatcaacaccatctgggtggtctccgcggcccactgtttcgacaaaatcaagaactggagga
acctgatcgcggtgctgggcgagcacgacctcagcgagcacgacggggatgagcagagccggcgggtggcgcaggtcatcatccccag
cacgtacgtcccgggcaccaccaaccacgacatcgcgctgctccgcctgcaccagcccgtggtcctcactgaccatgtggtgcccctctgcc
tgcccgaacggacgttctctgagaggacgctggccttcgtgcgcttctcattggtcagcggctggggccagctgctggaccgtggcgccacg
gccctggagctcatggtcctcaacgtgccccggctgatgacccaggactgcctgcagcagtcacggaaggtgggagactccccaaatatca
cggagtacatgttctgtgccggctactcggatggcagcaaggactcctgcaagggggacagtggaggcccacatgccacccactaccggg
gcacgtggtacctgaccggcatcgtgagctggggccagggctgcgccaccgtgggccacttcggcgtgtacaccagggtgtcccagtaca
tcgagtggctgcagaaactgatgagaagcgagcccagacccggcgtgctgctgagagcccccttccccagcagcagctccaaggcccctc
cccctagcctgcccagccctagcagactgcctgggcccagtgacacccctatcctgcctcagtccagctccagcaaggccccaccccctag
cctgccttctccttctcggctgcctggccccagcgatactccaattctgccccagtcctccagcagtaaggctccccctccatctctgccatccc
ccagcagactgccaggcccttctgatacacccatcctcccacagtgatgaggatccgc (SEQ ID NO: 39).

In another embodiment, the amino acid sequence of Factor VII-(CTP)₄ (attached to the carboxy terminus) comprises the following amino acid sequence:

LEDMVSQALRLLCLLLGLQGCLAAVFVTQEEAHGVLHRRRRANAFLEELRPGSLERECK
EEQCSFEEAREIFKDAERTKLFWISYSDGDQCASSPCQNGGSCKDQLQSYICFCLPAFEGR
NCETHKDDQLICVNENGGCEQYCSDHTGTKRSCRCHEGYSLLADGVSCTPTVEYPCGKI
PILEKRNASKPQGRIVGGKVCPKGECPWQVLLLVNGAQLCGGTLINTIWVVSAAHCFDK
IKNWRNLIAVLGEHDLSEHDGDEQSRRVAQVIIPSTYVPGTTNHDIALLRLHQPVVLTDH
VVPLCLPERTFSERTLAFVRFSLVSGWGQLLDRGATALELMVLNVPRLMTQDCLQQSRK
VGDSPNITEYMFCAGYSDGSKDSCKGDSGGPHATHYRGTWYLTGIVSWGQGCATVGHF
GVYTRVSQYIEWLQKLMRSEPRPGVLLRAPFPSSSSKAPPPSLPSPSRLPGPSDTPILPQSSS
SKAPPPSLPSPSRLPGPSDTPILPQSSSSKAPPPSLPSPSRLPGPSDTPILPQG (SEQ ID NO:
40).

In another embodiment, the nucleic acid sequence encoding Factor VII-(CTP)₅ (attached to the carboxy terminus) comprises the following nucleic acid sequence:

ctcgaggacatggtctcccaggccctcaggctcctctgccttctgcttgggcttcagggctgcctggctgcagtcttcgtaacccaggaggaa
gcccacggcgtcctgcaccggcgccggcgcgccaacgcgttcctggaggagctgcggccgggctccctggagagggagtgcaaggag
gagcagtgctccttcgaggaggcccgggagatcttcaaggacgcggagaggacgaagctgttctggatttcttacagtgatggggaccagt
gtgcctcaagtccatgccagaatgggggctcctgcaaggaccagctccagtcctatatctgcttctgcctccctgccttcgagggccggaact

gtgagacgcacaaggatgaccagctgatctgtgtgaacgagaacggcggctgtgagcagtactgcagtgaccacacgggcaccaagcgct
cctgtcggtgccacgaggggtactctctgctggcagacggggtgtcctgcacacccacagttgaatatccatgtggaaaaatacctattctaga
aaaaagaaatgccagcaaaccccaaggccgaattgtggggggcaaggtgtgccccaaaggggagtgtccatggcaggtcctgttgttggt
gaatggagctcagttgtgtggggggaccctgatcaacaccatctgggtggtctccgcggcccactgtttcgacaaaatcaagaactggagga
acctgatcgcggtgctgggcgagcacgacctcagcgagcacgacggggatgagcagagccggcgggtggcgcaggtcatcatccccag
cacgtacgtcccgggcaccaccaaccacgacatcgcgctgctccgcctgcaccagcccgtggtcctcactgaccatgtggtgcccctctgcc
tgcccgaacggacgttctctgagaggacgctggccttcgtgcgcttctcattggtcagcggctggggccagctgctggaccgtggcgccacg
gccctggagctcatggtcctcaacgtgccccggctgatgacccaggactgcctgcagcagtcacggaaggtgggagactccccaaatatca
cggagtacatgttctgtgccggctactcggatggcagcaaggactcctgcaagggggacagtggaggcccacatgccacccactaccggg
gcacgtggtacctgaccggcatcgtgagctggggccagggctgcgccaccgtgggccacttcggcgtgtacaccagggtgtcccagtaca
tcgagtggctgcagaaactgatgagaagcgagcccagacccggcgtgctgctgagagccccctttcccagcagcagctccaaggcccctc
cccctagcctgcccagccctagcagactgcctgggccctctgacacccctatcctgcctcagtccagctcctctaaggctccaccaccttccct
gcctagcccttcaagactgccaggccctagcgatacaccaattctgccccagtcctccagcagcaaggctcccccacctagcctgccttctcc
atcaaggctgcctggcccatccgataccccaattttgcctcagagcagctctagcaaggcacctccccccagtctgccctctccaagcagact
ccctggcccttcagacactccaatcctcccacagtcctctagctctaaagctccacctcccagcctgcccagccctagtagactccccggacct
tctgatacccccatcttgccccagtgatgaggatccgc (SEQ ID NO: 41).

In another embodiment, the amino acid sequence of Factor VII-(CTP)₅ (attached to the carboxy terminus) comprises the following amino acid sequence:

LEDMVSQALRLLCLLLGLQGCLAAVFVTQEEAHGVLHRRRRANAFLEELRPGSLERECK
EEQCSFEEAREIFKDAERTKLFWISYSDGDQCASSPCQNGGSCKDQLQSYICFCLPAFEGR
NCETHKDDQLICVNENGGCEQYCSDHTGTKRSCRCHEGYSLLADGVSCTPTVEYPCGKI
PILEKRNASKPQGRIVGGKVCPKGECPWQVLLLVNGAQLCGGTLINTIWVVSAAHCFDK
IKNWRNLIAVLGEHDLSEHDGDEQSRRVAQVIIPSTYVPGTTNHDIALLRLHQPVVLTDH
VVPLCLPERTFSERTLAFVRFSLVSGWGQLLDRGATALELMVLNVPRLMTQDCLQQSRK
VGDSPNITEYMFCAGYSDGSKDSCKGDSGGPHATHYRGTWYLTGIVSWGQGCATVGHF
GVYTRVSQYIEWLQKLMRSEPRPGVLLRAPFPSSSSKAPPPSLPSPSRLPGPSDTPILPQSSS
SKAPPPSLPSPSRLPGPSDTPILPQSSSSKAPPPSLPSPSRLPGPSDTPILPQSSSSKAPPPSLPS
PSRLPGPSDTPILPQSSSSKAPPPSLPSPSRLPGPSDTPILPQGS (SEQ ID NO: 42).

In another embodiment, the nucleic acid sequence encoding Factor IX comprises the following nucleic acid sequence:

gcgatcgccatgcagcgcgtgaacatgatcatggcagaatcaccaggcctcatcaccattgccttttaggatatctactcagtgctgaatgtaca
gtttttcttgatcatgaaaacgccaacaaaattctgaatcggccaaagaggtataattcaggtaaattggaagagtttgttcaagggaaccttgag
agagaatgtatggaagaaaagtgtagttttgaagaagcacgagaagtttttgaaaacactgaaagaacaactgaattttggaagcagtatgttg
atggagatcagtgtgagtccaatccatgtttaaatggcggcagttgcaaggatgacattaattcctatgaatgttggtgtccctttggatttgaagg

aaagaactgtgaattagatgtaacatgtaacattaagaatggcagatgcgagcagttttgtaaaaatagtgctgataacaaggtggtttgctcctg
tactgagggatatcgacttgcagaaaaccagaagtcctgtgaaccagcagtgccatttccatgtggaagagtttctgtttcacaaacttctaagct
cacccgtgctgagactgtttttcctgatgtggactatgtaaattctactgaagctgaaaccattttggataacatcactcaaagcacccaatcattta
atgacttcactcgagttgttggtggagaagatgccaaaccaggtcaattcccttggcaggttgttttgaatggtaaagttgatgcattctgtggag
gctctatcgttaatgaaaaatggattgtaactgctgcccactgtgttgaaactggtgttaaaattacagttgtcgcaggtgaacataatattgagga
gacagaacatacagagcaaaagcgaaatgtgattcgaattattcctcaccacaactacaatgcagctattaataagtacaaccatgacattgcc
cttctggaactggacgaacccttagtgctaaacagctacgttacacctatttgcattgctgacaaggaatacacgaacatcttcctcaaatttggat
ctggctatgtaagtggctggggaagagtcttccacaaagggagatcagctttagttctccagtaccttagagttccacttgttgaccgagccaca
tgtcttcgatctacaaagttcaccatctataacaacatgttctgtgctggcttccatgaaggaggtagagattcatgtcaaggagatagtggggga
ccccatgttactgaagtggaagggaccagtttcttaactggaattattagctggggtgaagagtgtgcaatgaaaggcaaatatggaatatatac
caaggtatcccggtatgtcaactggattaaggaaaaaacaaagctcacttgaacgcggccgc (SEQ ID NO: 43).

In another embodiment, the amino acid sequence of Factor IX comprises the following amino acid sequence:








 (SEQ ID NO: 44).

In another embodiment, the nucleic acid sequence encoding Factor IX-CTP (attached to the carboxy terminus) comprises the following nucleic acid sequence:

gcgatcgccatgcagcgcgtgaacatgatcatggcagaatcaccaggcctcatcaccatctgccttttaggatatctactcagtgctgaatgtac
agtttttcttgatcatgaaaacgccaacaaaattctgaatcggccaaagaggtataattcaggtaaattggaagagtttgttcaagggaaccttga
gagagaatgtatggaagaaaagtgtagttttgaagaagcacgagaagtttttgaaaacactgaaagaacaactgaattttggaagcagtatgtt
gatggagatcagtgtgagtccaatccatgtttaaatggcggcagttgcaaggatgacattaattcctatgaatgttggtgtccctttggatttgaag
gaaagaactgtgaattagatgtaacatgtaacattaagaatggcagatgcgagcagttttgtaaaaatagtgctgataacaaggtggtttgctcct
gtactgagggatatcgacttgcagaaaaccagaagtcctgtgaaccagcagtgccatttccatgtggaagagtttctgtttcacaaacttctaag
ctcacccgtgctgagactgtttttcctgatgtggactatgtaaattctactgaagctgaaaccattttggataacatcactcaaagcacccaatcatt
taatgacttcactcgagttgttggtggagaagatgccaaaccaggtcaattcccttggcaggttgttttgaatggtaaagttgatgcattctgtgga
ggctctatcgttaatgaaaaatggattgtaactgctgcccactgtgttgaaactggtgttaaaattacagttgtcgcaggtgaacataatattgagg
agacagaacatacagagcaaaagcgaaatgtgattcgaattattcctcaccacaactacaatgcagctattaataagtacaaccatgacattgc
ccttctggaactggacgaacccttagtgctaaacagctacgttacacctatttgcattgctgacaaggaatacacgaacatcttcctcaaatttgg

atctggctatgtaagtggctggggaagagtcttccacaaagggagatcagctttagttcttcagtaccttagagttccacttgttgaccgagccac
atgtcttcgatctacaaagttcaccatctataacaacatgttctgtgctggcttccatgaaggaggtagagattcatgtcaaggagatagtggggg
accccatgttactgaagtggaagggaccagtttcttaactggaattattagctggggtgaagagtgtgcaatgaaaggcaaatatggaatatata
ccaaggtatcccggtatgtcaactggattaaggaaaaaacaaagctcactagctccagcagcaaggcccctcccccgagcctgccctcccca
agcaggctgcctgggccctccgacacaccaatcctgccacagtgatgaaggtctggatccgcggccgc (SEQ ID NO: 45).

In another embodiment, the amino acid sequence of Factor IX-CTP (attached to the carboxy terminus) comprises the following amino acid sequence:

MQRVNMIMAESPGLITICLLGYLLSAECTVFLDHENANKILNRPKRYNSGKLEEFVQGNL
ERECMEEKCSFEEAREVFENTERTTEFWKQYVDGDQCESNPCLNGGSCKDDINSYECW
CPFGFEGKNCELDVTCNIKNGRCEQFCKNSADNKVVCSCTEGYRLAENQKSCEPAVPFP
CGRVSVSQTSKLTRAETVFPDVDYVNSTEAETILDNITQSTQSFNDFTRVVGGEDAKPGQ
FPWQVVLNGKVDAFCGGSIVNEKWIVTAAHCVETGVKITVVAGEHNIEETEHTEQKRN
VIRIIPHHNYNAAINKYNHDIALLELDEPLVLNSYVTPICIADKEYTNIFLKFGSGYVSGW
GRVFHKGRSALVLQYLRVPLVDRATCLRSTKFTIYNNMFCAGFHEGGRDSCQGDSGGP
HVTEVEGTSFLTGIISWGEECAMKGKYGIYTKVSRYVNWIKEKTKLTSSSSKAPPPSLPSP
SRLPGPSDTPILPQ (SEQ ID NO: 46).

In another embodiment, the nucleic acid sequence encoding Factor IX-CTP-CTP (attached to the carboxy terminus) comprises the following nucleic acid sequence:

gcgatcgccatgcagcgcgtgaacatgatcatggcagaatcaccaggcctcatcaccatctgccttttaggatatctactcagtgctgaatgtac
agtttttcttgatcatgaaaacgccaacaaaattctgaatcggccaaagaggtataattcaggtaaattggaagagtttgttcaagggaaccttga
gagagaatgtatggaagaaaagtgtagttttgaagaagcacgagaagtttttgaaaacactgaaagaacaactgaattttggaagcagtatgtt
gatggagatcagtgtgagtccaatccatgtttaaatggcggcagttgcaaggatgacattaattcctatgaatgttggtgtccctttggatttgaag
gaaagaactgtgaattagatgtaacatgtaacattaagaatggcagatgcgagcagttttgtaaaaatagtgctgataacaaggtggtttgctcct
gtactgagggatatcgacttgcagaaaaccagaagtcctgtgaaccagcagtgccatttccatgtggaagagtttctgtttcacaaacttctaag
ctcacccgtgctgagactgtttttcctgatgtggactatgtaaattctactgaagctgaaaccattttggataacatcactcaaagcacccaatcatt
taatgacttcactcgagttgttggtggagaagatgccaaaccaggtcaattcccttggcaggttgttttgaatggtaaagttgatgcattctgtgga
ggctctatcgttaatgaaaaatggattgtaactgctgcccactgtgttgaaactggtgttaaaattacagttgtcgcaggtgaacataatattgagg
agacagaacatacagagcaaaagcgaaatgtgattcgaattattcctcaccacaactacaatgcagctattaataagtacaaccatgacattgc
ccttctggaactggacgaacccttagtgctaaacagctacgttacacctatttgcattgctacaaggaatacacgaacatcttcctcaaatttggat
ctggctatgtaagtggctggggaagagtcttccacaaagggagatcagctttagttcttcagtaccttagagttccacttgttgaccgagccacat
gtcttcgatctacaaagttcaccatctataacaacatgttctgtgctggcttccatgaaggaggtagagattcatgtcaaggagatagtggggga
ccccatgttactgaagtggaagggaccagtttcttaactggaattattagctggggtgaagagtgtgcaatgaaaggcaaatatggaatatatac
caaggtatcccggtatgtcaactggattaaggaaaaaacaaagctcactagctccagcagcaaggcccctcccccgagcctgccctccccaa

gcaggctgcctgggccctccgacacaccaatcctgccacagagcagctcctctaaggcccctcctccatccctgccatccccctcccggctg
cctggcccctctgacacccctatcctgcctcagtgatgaaggtctggatccgcggccgc (SEQ ID NO: 47).

In another embodiment, the amino acid sequence of Factor IX-CTP-CTP (attached to the carboxy terminus) comprises the following amino acid sequence:

MQRVNMIMAESPGLITICLLGYLLSAECTVFLDHENANKILNRPKRYNSGKLEEFVQGNL
ERECMEEKCSFEEAREVFENTERTTEFWKQYVDGDQCESNPCLNGGSCKDDINSYECW
CPFGFEGKNCELDVTCNIKNGRCEQFCKNSADNKVVCSCTEGYRLAENQKSCEPAVPFP
CGRVSVSQTSKLTRAETVFPDVDYVNSTEAETILDNITQSTQSFNDFTRVVGGEDAKPGQ
FPWQVVLNGKVDAFCGGSIVNEKWIVTAAHCVETGVKITVVAGEHNIEETEHTEQKRN
VIRIIPHHNYNAAINKYNHDIALLELDEPLVLNSYVTPICIADKEYTNIFLKFGSGYVSGW
GRVFHKGRSALVLQYLRVPLVDRATCLRSTKFTIYNNMFCAGFHEGGRDSCQGDSGGP
HVTEVEGTSFLTGIISWGEECAMKGKYGIYTKVSRYVNWIKEKTKLTSSSSKAPPPSLPSP
SRLPGPSDTPILPQSSSSKAPPPSLPSPSRLPGPSDTPILPQ(SEQ ID NO: 48).

In another embodiment, the nucleic acid sequence encoding Factor IX-(CTP)₃ (attached to the carboxy terminus) comprises the following nucleic acid sequence:

tctagagtcgaccccgccatgcagcgcgtgaacatgatcatggcagaatcaccaggcctcatcaccatctgccttttaggatatctactcagtg
ctgaatgtacagtttttcttgatcatgaaaacgccaacaaaattctgaatcggccaaagaggtataattcaggtaaattggaagagtttgttcaagg
gaaccttgagagagaatgtatggaagaaaagtgtagttttgaagaagcacgagaagtttttgaaaacactgaaagaacaactgaattttggaag
cagtatgttgatggagatcagtgtgagtccaatccatgtttaaatggcggcagttgcaaggatgacattaattcctatgaatgttggtgtccctttg
gatttgaaggaaagaactgtgaattagatgtaacatgtaacattaagaatggcagatgcgagcagttttgtaaaaatagtgctgataacaaggtg
gtttgctcctgtactgagggatatcgacttgcagaaaaccagaagtcctgtgaaccagcagtgccatttccatgtggaagagtttctgtttcacaa
acttctaagctcacccgtgctgaggcagtttttcctgatgtggactatgtaaattctactgaagctgaaaccattttggataacatcactcaaagca
cccaatcatttaatgacttcactcgagttgttggtggagaagatgccaaaccaggtcaattcccttggcaggttgttttgaatggtaaagttgatgc
attctgtggaggctctatcgttaatgaaaaatggattgtaactgctgcccactgtgttgaaactggtgttaaaattacagttgtcgcaggtgaacat
aatattgaggagacagaacatacagagcaaaagcgaaatgtgattcgaattattcctcaccacaactacaatgcagctattaataagtacaacc
atgacattgcccttctggaactggacgaacccttagtgctaaacagctacgttacacctatttgcattgctgacaaggaatacacgaacatcttcc
tcaaatttggatctggctatgtaagtggctggggaagagtcttccacaaagggagatcagctttagttcttcagtaccttagagttccacttgttga
ccgagccacatgtcttcgatctacaaagttcaccatctataacaacatgttctgtgctggcttccatgaaggaggtagagattcatgtcaaggag
atagtgggggaccccatgttactgaagtggaagggaccagtttcttaactggaattattagctggggtgaagagtgtgcaatgaaaggcaaat
atggaatatataccaaggtatcccggtatgtcaactggattaaggaaaaaacaaagctcactagctccagcagcaaggcccctcccccgagc
ctgccctccccaagcaggctgcctgggcccagtgacacccctatcctgcctcagtccagctccagcaaggccccaccccctagcctgccttc
tccttctcggctgcctggccccagcgatactccaattctgccccagtcctccagcagtaaggctccccctccatctctgccatcccccagcaga
ctgccaggcccttctgatacacccatcctcccacagtgatgaggatccgcggccgc (SEQ ID NO: 49).

In another embodiment, the amino acid sequence of Factor IX-(CTP)₃ (attached to the carboxy terminus) comprises the following amino acid sequence:

MQRVNMIMAESPGLITICLLGYLLSAECTVFLDHENANKILNRPKRYNSGKLEEFVQGNL
ERECMEEKCSFEEAREVFENTERTTEFWKQYVDGDQCESNPCLNGGSCKDDINSYECW
CPFGFEGKNCELDVTCNIKNGRCEQFCKNSADNKVVCSCTEGYRLAENQKSCEPAVPFP
CGRVSVSQTSKLTRAEAVFPDVDYVNSTEAETILDNITQSTQSFNDFTRVVGGEDAKPGQ
FPWQVVLNGKVDAFCGGSIVNEKWIVTAAHCVETGVKITVVAGEHNIEETEHTEQKRN
VIRIIPHHNYNAAINKYNHDIALLELDEPLVLNSYVTPICIADKEYTNIFLKFGSGYVSGW
GRVFHKGRSALVLQYLRVPLVDRATCLRSTKFTIYNNMFCAGFHEGGRDSCQGDSGGP
HVTEVEGTSFLTGIISWGEECAMKGKYGIYTKVSRYVNWIKEKTKLTSSSSKAPPPSLPSP
SRLPGPSDTPILPQSSSSKAPPPSLPSPSRLPGPSDTPILPQSSSSKAPPPSLPSPSRLPGPSDTP
ILPQ (SEQ ID NO: 50).

In one embodiment, following expression a mature CTP-modified FIX polypeptide is secreted, the signal peptide having been cleaved from the precursor protein resulting in a mature protein. For example, in the precursor CTP-modified FIX polypeptide set forth in SEQ ID NO: 50 amino acids: 1-46: MQRVNMIMAESPGLITICLLGYLLSAECTVFLDHENANKILNRPKR (SEQ ID NO: 112) represent the signal peptide of the precursor CTP-modified FIX polypeptide, and amino acids: YNSGKLEEFVQGNLERECMEEKCSFEEAREVFENTERTTEFWKQYVDGDQCESNPCLN GGSCKDDINSYECWCPFGFEGKNCELDVTCNIKNGRCEQFCKNSADNKVVCSCTEGYRL AENQKSCEPAVPFPCGRVSVSQTSKLTRAEAVFPDVDYVNSTEAETILDNITQSTQSFNDF TRVVGGEDAKPGQFPWQVVLNGKVDAFCGGSIVNEKWIVTAAHCVETGVKITVVAGE HNIEETEHTEQKRNVIRIIPHHNYNAAINKYNHDIALLELDEPLVLNSYVTPICIADKEYTN IFLKFGSGYVSGWGRVFHKGRSALVLQYLRVPLVDRATCLRSTKFTIYNNMFCAGFHEG GRDSCQGDSGGPHVTEVEGTSFLTGIISWGEECAMKGKYGIYTKVSRYVNWIKEKTKLT SSSSKAPPPSLPSPSRLPGPSDTPILPQSSSSKAPPPSLPSPSRLPGPSDTPILPQSSSSKAPPPS LPSPSRLPGPSDTPILPQ (SEQ ID NO: 113), represent the mature engineered CTP-modified FIX polypeptide lacking the signal peptide. In one embodiment, the amino acid sequence of CTP-modified FIX without the signal peptide is set forth in SEQ ID NO: 113. In another embodiment, the signal peptide of CTP-modified FIX is set forth in SEQ ID NO: 112.

In another embodiment, the nucleic acid sequence encoding Factor IX-(CTP)₄ (attached to the carboxy terminus) comprises the following nucleic acid sequence:

tctagagtcgaccccgccatgcagcgcgtgaacatgatcatggcagaatcaccaggcctcatcaccatctgccttttaggatatctactcagtg
ctgaatgtacagtttttcttgatcatgaaaacgccaacaaaattctgaatcggccaaagaggtataattcaggtaaattggaagagtttgttcaagg

gaaccttgagagagaatgtatggaagaaaagtgtagttttgaagaagcacgagaagtttttgaaaacactgaaagaacaactgaattttggaag
cagtatgttgatggagatcagtgtgagtccaatccatgtttaaatggcggcagttgcaaggatgacattaattcctatgaatgttggtgtccctttg
gatttgaaggaaagaactgtgaattagatgtaacatgtaacattaagaatggcagatgcgagcagttttgtaaaaatagtgctgataacaaggtg
gtttgctcctgtactgagggatatcgacttgcagaaaaccagaagtcctgtgaaccagcagtgccatttccatgtggaagagtttctgtttcacaa
acttctaagctcacccgtgctgaggcagtttttcctgatgtggactatgtaaattctactgaagctgaaaccattttggataacatcactcaaagca
cccaatcatttaatgacttcactcgagttgttggtggagaagatgccaaaccaggtcaattcccttggcaggttgttttgaatggtaaagttgatgc
attctgtggaggctctatcgttaatgaaaaatggattgtaactgctgcccactgtgttgaaactggtgttaaaattacagttgtcgcaggtgaacat
aatattgaggagacagaacatacagagcaaaagcgaaatgtgattcgaattattcctcaccacaactacaatgcagctattaataagtacaacc
atgacattgcccttctggaactggacgaacccttagtgctaaacagctacgttacacctatttgcattgctgacaaggaatacacgaacatcttcc
tcaaatttggatctggctatgtaagtggctggggaagagtcttccacaaagggagatcagctttagttcttcagtaccttagagttccacttgttga
ccgagccacatgtcttcgatctacaaagttcaccatctataacaacatgttctgtgctggcttccatgaaggaggtagagattcatgtcaaggag
atagtgggggaccccatgttactgaagtggaagggaccagtttcttaactggaattattagctggggtgaagagtgtgcaatgaaaggcaaat
atggaatatataccaaggtatcccggtatgtcaactggattaaggaaaaaacaaagctcactagctccagcagcaaggcccctcccccgagc
ctgccctccccaagcaggctgcctgggccctctgacacccctatcctgcctcagtccagctcctctaaggccccaccaccttccctgcctagc
ccttcaagactgccaggccctagcgatacaccaattctgccccagtcctccagcagcaaggctcccccacctagcctgccttctccatcaagg
ctgcctggcccatccgataccccaattttgcctcagagcagctctagcaaggcacctccccccagtctgccctctccaagcagactccctggc
ccttcagacactcccattctgccacagtgatgaggatccgcggccgc (SEQ ID NO: 51).

In another embodiment, the amino acid sequence of Factor IX-(CTP)₄ (attached to the carboxy terminus) comprises the following amino acid sequence:

SRVDPAMQRVNMIMAESPGLITICLLGYLLSAECTVFLDHENANKILNRPKRYNSGKLEE
FVQGNLERECMEEKCSFEEAREVFENTERTTEFWKQYVDGDQCESNPCLNGGSCKDDIN
SYECWCPFGFEGKNCELDVTCNIKNGRCEQFCKNSADNKVVCSCTEGYRLAENQKSCEP
AVPFPCGRVSVSQTSKLTRAEAVFPDVDYVNSTEAETILDNITQSTQSFNDFTRVVGGED
AKPGQFPWQVVLNGKVDAFCGGSIVNEKWIVTAAHCVETGVKITVVAGEHNIEETEHTE
QKRNVIRIIPHHNYNAAINKYNHDIALLELDEPLVLNSYVTPICIADKEYTNIFLKFGSGYV
SGWGRVFHKGRSALVLQYLRVPLVDRATCLRSTKFTIYNNMFCAGFHEGGRDSCQGDS
GGPHVTEVEGTSFLTGIISWGEECAMKGKYGIYTKVSRYVNWIKEKTKLTSSSSKAPPPS
LPSPSRLPGPSDTPILPQSSSSKAPPPSLPSPSRLPGPSDTPILPQSSSSKAPPPSLPSPSRLPGP
SDTPILPQSSSSKAPPPSLPSPSRLPGPSDTPILPQGSAA (SEQ ID NO: 52).

In another embodiment, the nucleic acid sequence encoding Factor IX-(CTP)₅ (attached to the carboxy terminus) comprises the following nucleic acid sequence:

ctagagtcgaccccgccatgcagcgcgtgaacatgatcatggcagaatcaccaggcctcatcaccatctgccttttaggatatctactcagtgc
tgaatgtacagtttttcttgatcatgaaaacgccaacaaaattctgaatcggccaaagaggtataattcaggtaaattggaagagtttgttcaagg
gaaccttgagagagaatgtatggaagaaaagtgtagttttgaagaagcacgagaagtttttgaaaacactgaaagaacaactgaattttggaag

cagtatgttgatggagatcagtgtgagtccaatccatgtttaaatggcggcagttgcaaggatgacattaattcctatgaatgttggtgtccctttg
gatttgaaggaaagaactgtgaattagatgtaacatgtaacattaagaatggcagatgcgagcagttttgtaaaaatagtgctgataacaaggtg
gtttgctcctgtactgagggatatcgacttgcagaaaaccagaagtcctgtgaaccagcagtgccatttccatgtggaagagtttctgtttcacaa
acttctaagctcacccgtgctgaggcagtttttcctgatgtggactatgtaaattctactgaagctgaaaccattttggataacatcactcaaagca
cccaatcatttaatgacttcactcgagttgttggtggagaagatgccaaaccaggtcaattcccttggcaggttgttttgaatggtaaagttgatgc
attctgtggaggctctatcgttaatgaaaaatggattgtaactgctgcccactgtgttgaaactggtgttaaaattacagttgtcgcaggtgaacat
aatattgaggagacagaacatacagagcaaaagcgaaatgtgattcgaattattcctcaccacaactacaatgcagctattaataagtacaacc
atgacattgcccttctggaactggacgaacccttagtgctaaacagctacgttacacctatttgcattgctgacaaggaatacacgaacatcttcc
tcaaatttggatctggctatgtaagtggctggggaagagtcttccacaaagggagatcagctttagttcttcagtaccttagagttccacttgttga
ccgagccacatgtcttcgatctacaaagttcaccatctataacaacatgttctgtgctggcttccatgaaggaggtagagattcatgtcaaggag
atagtgggggaccccatgttactgaagtggaagggaccagtttcttaactggaattattagctggggtgaagagtgtgcaatgaaaggcaaat
atggaatatataccaaggtatcccggtatgtcaactggattaaggaaaaaacaaagctcactagctccagcagcaaggcccctcccccgagc
ctgccctccccaagcaggctgcctgggccctctgacacccctatcctgcctcagtccagctcctctaaggctccaccaccttccctgcctagcc
cttcaagactgccaggccctagcgatacaccaattctgccccagtcctccagcagcaaggctcccccacctagcctgccttctccatcaaggc
tgcctggcccatccgataccccaattttgcctcagagcagctctagcaaggcacctccccccagtctgccctctccaagcagactccctggcc
cttcagacactccaatcctcccacagtcctctagctctaaagctccacctcccagcctgcccagccctagtagactccccggaccttctgatacc
cccatcttgccccagtgatgaggatccgcggccgc (SEQ ID NO: 53).

In another embodiment, the amino acid sequence of Factor IX-(CTP)₅ (attached to the carboxy terminus) comprises the following amino acid sequence:











 (SEQ ID NO: 54).

A skilled artisan would appreciate that the term coagulation factor may encompass a homologue of a known coagulation factor. In one embodiment, the homologue has a coagulating activity. In another embodiment, a homologue of a coagulation factor is a variant comprising conservative substitutions, or deletions, insertions, or substitutions that do not significantly alter the three dimensional structure of the coagulation factor. In another embodiment, the deletion, insertion, or substitution does not alter the function of interest of the coagulation factor, which in one embodiment, is binding to a particular binding partner. In another embodiment, disclosed herein is a homologue of a coagulation factor having functional binding.

In another embodiment, furin is added to a cell expressing the coagulation factor-CTP disclosed herein. In another embodiment, furin increases the production efficiency of a coagulation factor-CTP disclosed herein in a cell. In another embodiment, furin is co-transfected with the vector comprising the coding sequence of the coagulation factor-CTP disclosed herein. In another embodiment, furin is encoded by a separate vector. In another embodiment, furin and a coagulation factor-CTP are encoded by one vector. In another embodiment, the coding sequence of furin is inserted into pCI-DHFR. In another embodiment, the coding sequence of furin is engineered in pCI-dhfr/smaI+NotI, Furin/AsisI F.I.+NotI.

In another embodiment, the nucleic acid sequence encoding furin comprises the following nucleic acid sequence: 
tctagagtcgaccccgccatggagctgaggccctggttgctatgggtggtagcagcaacaggaaccttggtcctgctagcagctgatgctca
gggccagaaggtcttcaccaacacgtgggctgtgcgcatccctggaggcccagcggtggccaacagtgtggcacggaagcatgggttcct
caacctgggccagatcttcggggactattaccacttctggcatcgaggagtgacgaagcggtccctgtcgcctcaccgcccgcggcacagc
cggctgcagagggagcctcaagtacagtggctggaacagcaggtggcaaagcgacggactaaacgggacgtgtaccaggagcccacag
accccaagtttcctcagcagtggtacctgtctggtgtcactcagcgggacctgaatgtgaaggcggcctgggcgcagggctacacagggca
cggcattgtggtctccattctggacgatggcatcgagaagaaccacccggacttggcaggcaattatgatcctggggccagttttgatgtcaat
gaccaggaccctgacccccagcctcggtacacacagatgaatgacaacaggcacggcacacggtgtgcgggggaagtggctgcggtgg
ccaacaacggtgtctgtggtgtaggtgtggcctacaacgcccgcattggaggggtgcgcatgctggatggcgaggtgacagatgcagtgga
ggcacgctcgctgggcctgaaccccaaccacatccacatctacagtgccagctggggccccgaggatgacggcaagacagtggatgggc
cagcccgcctcgccgaggaggccttcttccgtggggttagccagggccgaggggggctgggctccatctttgtctgggcctcggggaacgg
gggccgggaacatgacagctgcaactgcgacggctacaccaacagtatctacacgctgtccatcagcagcgccacgcagtttggcaacgt
gccgtggtacagcgaggcctgctcgtccacactggccacgacctacagcagtggcaaccagaatgagaagcagatcgtgacgactgactt
gcggcagaagtgcacggagtctcacacgggcacctcagcctctgcccccttagcagccggcatcattgctctcaccctggaggccaataag
aacctcacatggcgggacatgcaacacctggtggtacagacctcgaagccagcccacctcaatgccaacgactgggccaccaatggtgtg
ggccggaaagtgagccactcatatggctacgggcttttggacgcaggcgccatggtggccctggcccagaattggaccacagtggccccc
cagcggaagtgcatcatcgacatcctcaccgagcccaaagacatcgggaaacggctcgaggtgcggaagaccgtgaccgcgtgcctggg
cgagcccaaccacatcactcggctggagcacgctcaggcgcggctcaccctgtcctataatcgccgtggcgacctggccatccacctggtc
agccccatgggcacccgctccaccctgctggcagccaggccacatgactactccgcagatgggtttaatgactgggccttcatgacaactcat
tcctgggatgaggatccctctggcgagtgggtcctagagattgaaaacaccagcgaagccaacaactatgggacgctgaccaagttcaccct
cgtactctatggcaccgcccctgaggggctgcccgtacctccagaaagcagtggctgcaagaccctcacgtccagtcaggcctgtgtggtgt
 gcgaggaaggcttctccctgcaccagaagagctgtgtccagcactgccctccaggcttcgccccccaagtcctcgatacgcactatagcacc gagaatgacgtggagaccatccgggccagcgtctgcgccccctgccacgcctcatgtgccacatgccaggggccggccctgacagactg cctcagctgccccagccacgcctccttggaccctgtggagcagacttgctcccggcaaagccagagcagccgagagtccccgccacagca gcagccacctcggctgcccccggaggtggaggcggggcaacggctgcgggcagggctgctgccctcacacctgcctgaggtggtggcc ggcctcagctgcgccttcatcgtgctggtcttcgtcactgtcttcctggtcctgcagctgcgctctggctttagttttcggggggtgaaggtgtaca ccatggaccgtggcctcatctcctacaaggggctgccccctgaagcctggcaggaggagtgcccgtctgactcagaagaggacgagggcc ggggcgagaggaccgcctttatcaaagaccagagcgccctctgaacgcggccgc (SEQ ID NO: 55).In another embodiment, the amino acid sequence of furin comprises the following amino acid sequence:

MELRPWLLWVVAATGTLVLLAADAQGQKVFTNTWAVRIPGGPAVANSVARKHGFLNL
GQIFGDYYHFWHRGVTKRSLSPHRPRHSRLQREPQVQWLEQQVAKRRTKRDVYQEPTD
PKFPQQWYLSGVTQRDLNVKAAWAQGYTGHGIVVSILDDGIEKNHPDLAGNYDPGASF
DVNDQDPDPQPRYTQMNDNRHGTRCAGEVAAVANNGVCGVGVAYNARIGGVRMLDG
EVTDAVEARSLGLNPNHIHIYSASWGPEDDGKTVDGPARLAEEAFFRGVSQGRGGLGSIF
VWASGNGGREHDSCNCDGYTNSIYTLSISSATQFGNVPWYSEACSSTLATTYSSGNQNE
KQIVTTDLRQKCTESHTGTSASAPLAAGIIALTLEANKNLTWRDMQHLVVQTSKPAHLN
ANDWATNGVGRKVSHSYGYGLLDAGAMVALAQNWTTVAPQRKCIIDILTEPKDIGKRL
EVRKTVTACLGEPNHITRLEHAQARLTLSYNRRGDLAIHLVSPMGTRSTLLAARPHDYS
ADGFNDWAFMTTHSWDEDPSGEWVLEIENTSEANNYGTLTKFTLVLYGTAPEGLPVPPE
SSGCKTLTSSQACVVCEEGFSLHQKSCVQHCPPGFAPQVLDTHYSTENDVETIRASVCAP
CHASCATCQGPALTDCLSCPSHASLDPVEQTCSRQSQSSRESPPQQQPPRLPPEVEAGQR
LRAGLLPSHLPEVVAGLSCAFIVLVFVTVFLVLQLRSGFSFRGVKVYTMDRGLISYKGLP
PEAWQEECPSDSEEDEGRGERTAFIKDQSAL (SEQ ID NO: 56).

In another embodiment, disclosed herein is homologues of furin. In another embodiment, disclosed herein is homologues of furin maintaining a function of interest, which in one embodiment is cleaving of a precursor protein. In another embodiment, homologues e.g., polypeptides which are at least 50%, at least 55%, at least 60%, at least 65%, at least 70%, at least 75%, at least 80%, at least 85%, at least 87%, at least 89%, at least 91%, at least 93%, at least 95%, at least 96%, at least 97%, at least 98%, or at least 99% homologous to a furin as determined using BlastP software of the National Center of Biotechnology Information (NCBI) using default parameters.

In another embodiment, disclosed herein is a polypeptide comprising a coagulation factor and one to ten CTPs attached to the carboxy terminus of the coagulation factor. In another embodiment, disclosed herein is a polypeptide comprising a coagulation factor and one to three gonadotropin carboxy terminal peptides (CTPs) attached to the carboxy terminus of the coagulation factor. In another embodiment, disclosed herein is a polypeptide comprising a coagulation factor and one to five gonadotropin carboxy terminal peptides (CTPs) attached to the carboxy terminus of the coagulation factor. In another embodiment, disclosed herein is a polypeptide comprising a coagulation factor having at least one CTP on its carboxy terminus.

In another embodiment, disclosed herein is a polypeptide consisting of a coagulation factor and one to five CTPs attached to the carboxy terminus of the coagulation factor. In another embodiment, disclosed herein is a polypeptide consisting essentially of a coagulation factor and one to five CTPs attached to the carboxy terminus of the coagulation factor.

In one embodiment, disclosed herein is a polypeptide comprising a coagulation factor and two CTPs attached to the carboxy terminus of the coagulation factor, two to three CTPs attached to the carboxy terminus of the coagulation factor, two to four CTPs attached to the carboxy terminus of the coagulation , two to five CTPs attached to the carboxy terminus of the coagulation factor, two to six CTPs attached to the carboxy terminus of the coagulation factor, two to seven CTPs attached to the carboxy terminus of the coagulation factor, two to eight CTPs attached to the carboxy terminus of the coagulation factor, two to nine CTPs attached to the carboxy terminus of the coagulation factor, or two to ten CTPs attached to the carboxy terminus of the coagulation factor.

In one embodiment, disclosed herein is a polypeptide comprising a coagulation factor and three CTPs attached to the carboxy terminus of the coagulation factor, three to four CTPs attached to the carboxy terminus of the coagulation factor, three to five CTPs attached to the carboxy terminus of the coagulation factor, three to six CTPs attached to the carboxy terminus of the coagulation factor, three to seven CTPs attached to the carboxy terminus of the coagulation factor, three to eight CTPs attached to the carboxy terminus of the coagulation factor, three to nine CTPs attached to the carboxy terminus of the coagulation factor, or three to ten CTPs attached to the carboxy terminus of the coagulation factor.

In one embodiment, disclosed herein is a polypeptide comprising a coagulation factor and four CTPs attached to the carboxy terminus of the coagulation factor, four to five CTPs attached to the carboxy terminus of the coagulation factor, four to six CTPs attached to the carboxy terminus of the coagulation factor, four to seven CTPs attached to the carboxy terminus of the coagulation factor, four to eight CTPs attached to the carboxy terminus of the coagulation factor, four to nine CTPs attached to the carboxy terminus of the coagulation factor, four to ten CTPs attached to the carboxy terminus of the coagulation factor.

In one embodiment, disclosed herein is a polypeptide comprising a coagulation factor and five CTPs attached to the carboxy terminus of the coagulation factor, five to six CTPs attached to the carboxy terminus of the coagulation factor, five to seven CTPs attached to the carboxy terminus of the coagulation factor, five to eight CTPs attached to the carboxy terminus of the coagulation factor, five to nine CTPs attached to the carboxy terminus of the coagulation factor, five to ten CTPs attached to the carboxy terminus of the coagulation factor.

In one embodiment, disclosed herein is a polypeptide consisting of a coagulation factor and two CTPs attached to the carboxy terminus of the coagulation factor, two to three CTPs attached to the carboxy terminus of the coagulation factor, two to four CTPs attached to the carboxy terminus of the coagulation factor, two to five CTPs attached to the carboxy terminus of the coagulation factor, two to six CTPs attached to the carboxy terminus of the coagulation factor, two to seven CTPs attached to the carboxy terminus of the coagulation factor, two to eight CTPs attached to the carboxy terminus of the coagulation factor, two to nine CTPs attached to the carboxy terminus of the coagulation factor, two to ten CTPs attached to the carboxy terminus of the coagulation factor.

In one embodiment, disclosed herein is a polypeptide consisting of a coagulation factor and three CTPs attached to the carboxy terminus of the coagulation factor, three to four CTPs attached to the carboxy terminus of the coagulation factor, three to five CTPs attached to the carboxy terminus of the coagulation factor, three to six CTPs attached to the carboxy terminus of the coagulation factor, three to seven CTPs attached to the carboxy terminus of the coagulation factor, three to eight CTPs attached to the carboxy terminus of the coagulation factor, three to nine CTPs attached to the carboxy terminus of the coagulation factor, or three to ten CTPs attached to the carboxy terminus of the coagulation factor.

In one embodiment, disclosed herein is a polypeptide consisting of a coagulation factor and four CTPs attached to the carboxy terminus of the coagulation factor, four to five CTPs attached to the carboxy terminus of the coagulation factor, four to six CTPs attached to the carboxy terminus of the coagulation factor, four to seven CTPs attached to the carboxy terminus of the coagulation factor, four to eight CTPs attached to the carboxy terminus of the coagulation factor, four to nine CTPs attached to the carboxy terminus of the coagulation factor, or four to ten CTPs attached to the carboxy terminus of the coagulation factor.

In one embodiment, disclosed herein is a polypeptide consisting of a coagulation factor and five CTPs attached to the carboxy terminus of the coagulation factor, five to six CTPs attached to the carboxy terminus of the coagulation factor, five to seven CTPs attached to the carboxy terminus of the coagulation factor, five to eight CTPs attached to the carboxy terminus of the coagulation factor, five to nine CTPs attached to the carboxy terminus of the coagulation factor, or five to ten CTPs attached to the carboxy terminus of the coagulation factor.

In one embodiment, disclosed herein is a polypeptide consisting essentially of a coagulation factor and two CTPs attached to the carboxy terminus of the coagulation factor, two to three CTPs attached to the carboxy terminus of the coagulation factor, two to four CTPs attached to the carboxy terminus of the coagulation factor, two to five CTPs attached to the carboxy terminus of the coagulation factor, two to six CTPs attached to the carboxy terminus of the coagulation factor, two to seven CTPs attached to the carboxy terminus of the coagulation factor, two to eight CTPs attached to the carboxy terminus of the coagulation factor, two to nine CTPs attached to the carboxy terminus of the coagulation factor, or two to ten CTPs attached to the carboxy terminus of the coagulation factor.

In one embodiment, disclosed herein is a polypeptide consisting essentially of a coagulation factor and three CTPs attached to the carboxy terminus of the coagulation factor, three to four CTPs attached to the carboxy terminus of the coagulation factor, three to five CTPs attached to the carboxy terminus of the coagulation factor, three to six CTPs attached to the carboxy terminus of the coagulation factor, three to seven CTPs attached to the carboxy terminus of the coagulation factor, three to eight CTPs attached to the carboxy terminus of the coagulation factor, three to nine CTPs attached to the carboxy terminus of the coagulation factor, or three to ten CTPs attached to the carboxy terminus of the coagulation factor.

In one embodiment, disclosed herein is a polypeptide consisting essentially of a coagulation factor and four CTPs attached to the carboxy terminus of the coagulation factor, four to five CTPs attached to the carboxy terminus of the coagulation factor, four to six CTPs attached to the carboxy terminus of the coagulation factor, four to seven CTPs attached to the carboxy terminus of the coagulation factor, four to eight CTPs attached to the carboxy terminus of the coagulation factor, four to nine CTPs attached to the carboxy terminus of the coagulation factor, or four to ten CTPs attached to the carboxy terminus of the coagulation factor.

In one embodiment, disclosed herein is a polypeptide consisting essentially of a coagulation factor and five CTPs attached to the carboxy terminus of the coagulation factor, five to six CTPs attached to the carboxy terminus of the coagulation factor, five to seven CTPs attached to the carboxy terminus of the coagulation factor, five to eight CTPs attached to the carboxy terminus of the coagulation factor, five to nine CTPs attached to the carboxy terminus of the coagulation factor, or five to ten CTPs attached to the carboxy terminus of the coagulation factor.

In another embodiment, disclosed herein is a polypeptide comprising, consisting essentially of, or consisting of a coagulation factor having no CTPs on its amino terminus. In another embodiment, disclosed herein is a polypeptide comprising, consisting essentially of, or consisting of a coagulation factor lacking a CTP on its amino terminus. In another embodiment, disclosed herein is a polypeptide comprising, consisting essentially of, or consisting of a coagulation factor having at least one CTP on its carboxy terminus and no CTPs on its amino terminus. In another embodiment, disclosed herein is a polypeptide comprising, consisting essentially of, or consisting of a coagulation factor having the number of CTPs on its carboxy terminus as described herein and no CTPs on its amino terminus.

In another embodiment, disclosed herein is a composition comprising an expression vector comprising a polynucleotide encoding a CTP-modified polypeptide consisting of a Factor IX (FIX) polypeptide and three CTPs attached to the carboxy terminus of said FIX polypeptide.

In another embodiment, disclosed herein is a polynucleotide encoding a CTP-modified polypeptide consisting of an activated Factor VIIa (FVIIa) polypeptide and three CTPs attached to the carboxy terminus of said FVIIa polypeptide.

In one embodiment, disclosed herein is a recombinant coagulation factor as described herein. In one embodiment, disclosed herein is an engineered coagulation factor as described herein. A skilled artisan would appreciate that the term "engineered coagulation factor" may encompass a CTP-modified coagulation factor.

In one embodiment, the CTPs that are attached to the carboxy terminus of the coagulation factor are attached in tandem to the carboxy terminus.

In one embodiment, an engineered coagulation factor as described herein has equivalent or improved biological activity compared to the non-CTP-modified coagulation factor. In another embodiment, an engineered coagulation factor as described herein has equivalent or improved pharmacological measurements compared to the non-CTP-modified coagulation factor. In another embodiment, an engineered coagulation factor as described herein has equivalent or improved pharmacokinetics compared to the non-CTP-modified coagulation factor. In another embodiment, an engineered coagulation factor as described herein has equivalent or improved pharmacodynamics compared to the non-CTP-modified coagulation factor.

In one embodiment, disclosed herein is a method of preventing or treating a clotting or coagulation disorder. In another embodiment, disclosed herein is a method of preventing or treating hemophilia in a subject comprising administering a CTP-modified coagulation factor disclosed herein. In another embodiment, disclosed herein is a method of preventing and treating hemophilia in a subject comprising administering a CTP-modified coagulation factor disclosed herein. In another embodiment, disclosed herein is a method of treating hemophilia in a subject comprising administering a CTP-modified Factor VII disclosed herein.

In another embodiment, disclosed herein is a method of preventing or treating hemophilia in a subject comprising administering a CTP-modified coagulation factor as set forth in SEQ ID NO: 111. In another embodiment, disclosed herein is a method of preventing and treating hemophilia in a subject comprising administering a CTP-modified coagulation factor as set forth in SEQ ID NO: 111. In another embodiment, disclosed herein is a method of preventing or treating hemophilia in a subject comprising administering a CTP-modified coagulation factor as set forth in SEQ ID NO: 113. In another embodiment, disclosed herein is a method of preventing and treating hemophilia in a subject comprising administering a CTP-modified coagulation factor as set forth in SEQ ID NO: 113.

In another embodiment, disclosed herein is a method of treating hemophilia in a subject comprising administering a CTP-modified Factor IX disclosed herein. In one embodiment, hemophilia is hemophilia B. In one embodiment, hemophilia B is known as factor IX deficiency or Christmas disease. In one embodiment, the hemophilia is severe hemophilia, which in one embodiment, describes hemophilia in which the coagulation factor levels are 0-1%. In another embodiment, the hemophilia is moderate hemophilia, which in one embodiment, describes hemophilia in which the coagulation factor levels are 1-5%. In another embodiment, the hemophilia is mild hemophilia, which in one embodiment, describes hemophilia in which the coagulation factor levels are 5-50%.

In another embodiment, disclosed herein is a method of preventing or treating a clotting or coagulation disorder in a subject comprising administering a CTP-modified Factor IX (FIX) polypeptide comprising a FIX polypeptide and three chorionic CTPs attached to the carboxy terminus of said FIX polypeptide to said subject, thereby preventing or treating a clotting or coagulation disorder in said subject. In another embodiment, disclosed herein is a method of preventing or treating a clotting or coagulation disorder in a subject comprising administering an activated CTP-modified Factor VII (FVIIa) polypeptide comprising an FVIIa polypeptide and three chorionic CTPs attached to the carboxy terminus of said FVIIa polypeptide to said subject, thereby preventing or treating a clotting or coagulation disorder in said subject.

In another embodiment, disclosed herein is a method of preventing or treating hemophilia in a subject comprising administering a CTP-modified Factor IX (FIX) polypeptide comprising a FIX polypeptide and three chorionic CTPs attached to the carboxy terminus of said FIX polypeptide to said subject, thereby preventing or treating hemophilia in said subject. In another embodiment, disclosed herein is a method of preventing or treating hemophilia in a subject comprising administering an activated CTP-modified Factor VIIa (FVIIa) polypeptide comprising a FVIIa polypeptide and three chorionic CTPs attached to the carboxy terminus of said FVIIa polypeptide to said subject, thereby preventing or treating hemophilia in said subject.

In another embodiment, disclosed herein is a method of treating hemophilia in a subject comprising administering one or more CTP-modified coagulation factors as described herein to said subject. Thus, in one embodiment, disclosed herein is a method of treating hemophilia in a subject comprising administering a CTP-modified Factor IX (FIX) polypeptide comprising a FIX polypeptide and three chorionic CTPs attached to the carboxy terminus of said FIX polypeptide and an activated CTP-modified Factor VIIa (FVIIa) polypeptide comprising an activated FVII (FVIIa) polypeptide and three chorionic CTPs attached to the carboxy terminus of said FVIIa polypeptide to said subject, thereby treating hemophilia in said subject. In one embodiment, the CTP-modified FIX and the activated CTP-modified FVIIa are administered in the same composition at the same time. In another embodiment, the CTP-modified FIX and the activated CTP-modified FVIIa are administered in separate compositions at the same time. In another embodiment, the CTP-modified FIX and the activated CTP-modified FVIIa are administered in separate compositions at separate times.

In another embodiment, disclosed herein is a method of preventing or treating hemophilia in a subject comprising administering a CTP-modified Factor IX (FIX) or a CTP-modified Factor VII polypeptide comprising a FIX or a FVII polypeptide and three chorionic CTPs attached to the carboxy terminus of said FIX or said FVII polypeptide to said subject, thereby preventing or treating hemophilia in said subject. In another embodiment, disclosed herein is a method of preventing or treating hemophilia in a subject comprising administering a CTP-modified Factor IX (FIX) or a CTP-modified Factor VII polypeptide comprising a FIX or a FVII polypeptide and four chorionic CTPs attached to the carboxy terminus of said FIX or said FVII polypeptide, five chorionic CTPs attached to the carboxy terminus of said FIX or said FVII polypeptide, three to five chorionic CTPs attached to the carboxy terminus of said FIX or said FVII, three chorionic CTPs attached to the carboxy terminus of said FIX and said FVII, or three to five chorionic CTPs attached to the carboxy terminus of said FIX and said FVII polypeptide to said subject, thereby preventing or treating hemophilia in said subject.

In another embodiment, disclosed herein is a method of preventing or treating hemophilia in a subject comprising subcutaneously or intravenously administering a CTP-modified Factor IX (FIX) or a CTP-modified Factor VII polypeptide comprising a FIX or a FVII polypeptide and three chorionic CTPs attached to the carboxy terminus of said FIX or said FVII polypeptide to said subject, thereby preventing or treating hemophilia in said subject. In another embodiment, disclosed herein is a method of preventing or treating hemophilia in a subject comprising subcutaneously or intravenously administering a CTP-modified Factor IX (FIX) or a CTP-modified Factor VII polypeptide comprising a FIX or a FVII polypeptide and four chorionic CTPs attached to the carboxy terminus of said FIX or said FVII , five chorionic CTPs attached to the carboxy terminus of said FIX or said FVII polypeptide, three to five chorionic CTPs attached to the carboxy terminus of said FIX or said FVII polypeptide, three chorionic CTPs attached to the carboxy terminus of said FIX and said FVII polypeptide, or three to five chorionic CTPs attached to the carboxy terminus of said FIX and said FVII polypeptide to said subject, thereby preventing or treating hemophilia in said subject.

In some embodiments, disclosed herein is a method of preventing or treating a hemophilia in a subject, the method comprising the step of administering to the subject a CTP-modified coagulation factor, comprising three to five chorionic CTPs attached to the carboxy terminus of said coagulation factor polypeptide, wherein the sequence of said CTP-modified coagulation factor is selected from the group consisting of SEQ ID NO: 38, 40, 42, 111, or 113 thereby preventing hemophilia in said subject.

In some embodiments, disclosed herein is a method of preventing or treating a hemophilia in a subject, the method comprising the step of subcutaneously administering to the subject a CTP-modified Factor VII, comprising three to five chorionic CTPs attached to the carboxy terminus of said FVII polypeptide, wherein the sequence of said CTP-modified FVII is selected from the group consisting of SEQ ID NO: 38, 40, 42, 111, or 113 thereby preventing hemophilia in said subject.

In other embodiments, the engineered coagulation factor is for the treatment of hemophilia B patients. In one embodiment, coagulation Factor IX comprising 3 CTPs in tandem in its carboxy terminus is for the treatment of hemophilia B patients. In another embodiment, coagulation Factor IX comprising 4 CTPs in tandem in its carboxy terminus,5 CTPs in tandem in its carboxy, 2 CTPs in tandem in its carboxy terminus, or 1 CTP repeat in its carboxy terminus is for the treatment of hemophilia B patients. In other embodiments, the engineered coagulation factor can reduce the number of infusions required for a patient, reduce the required doses for a patient, or a combination thereof.

In one embodiment, coagulation Factor IX comprising 3 CTPs in tandem in its carboxy terminus exhibits an improved PK profile while maintaining its coagulation activity vs. FIX-CTP-CTP harvest, FIX-CTP harvest or rhFIX. In one embodiment, the elimination half-life of rFIX-CTP3 is 2.5- to 4-fold longer than rFIX in rats and in FIX-deficient mice. In one embodiment, the administration of rFIX-CTP3 significantly prolonged the procoagulatory effect in FIX-deficient mice for at least 76 hr after dosing. In one embodiment, the administration of rFIX-CTP3 produced a higher activity peak than rFIX in FIX-deficient mice. In another embodiment, coagulation Factor IX comprising 2 CTPs in tandem in its carboxy terminus exhibits an improved PK profile while maintaining its coagulation activity vs. FIX-CTP harvest or rhFIX. In another embodiment, coagulation Factor IX comprising 2 CTPs in tandem in its carboxy terminus exhibits 3-fold increase in half-life and 4.5-fold higher AUC compared to rhFIX.

In another embodiment, subcutaneous (SC) administration results in higher bioavailability of CTP-modified FVII as compared to recombinant FVII. In another embodiment, half-life is longer and bioavailability (AUC SC/AUC IV) is higher following FVIIa-CTP3and 5 SC administration when compared to SC administration of NovoSeven^{®}. In another embodiment, subcutaneously injected MOD-5014 and MOD-5019 shows improved mice survival in comparison to recombinant FVII (NovoSeven^{®}) (see Example 8 below).

In another embodiment, a signal peptide is attached to the amino terminus of the CTP-modified coagulation factor, as described in US 7,553,940, which is incorporated by reference herein in its entirety. In another embodiment, a signal peptide is cleaved from the amino terminus of the CTP-modified coagulation factor disclosed herein.

A skilled artisan would appreciate that the term engineered coagulation factor may encompass the amino acid sequence of a matured coagulation factor lacking a signal peptide. In one embodiment, the mature form of an engineered coagulation factor does not include a signal peptide. In another embodiment, the mature form of a CTP-modified coagulation factor does not include a signal peptide. In other embodiments, the term engineered coagulation factor comprises the amino acid sequence of the coagulation factor including its signal sequence or signal peptide.

In another embodiment, an engineered coagulation factor comprising at least one CTP as described herein has enhanced *in vivo* biological activity compared the same coagulation factor without at least one CTP. In one embodiment, the enhanced biological activity stems from the longer half-life of the engineered coagulation factor while maintaining at least some biological activity. In another embodiment, the enhanced biological activity stems from enhanced biological activity resulting from the CTP modification. In another embodiment, the enhanced biological activity stems from both a longer half-life and from enhanced functionality of the CTP-modified coagulation factor.

In some embodiments, at least one CTP sequence at the carboxy terminal end of the coagulation factor provides enhanced protection against degradation of a coagulation factor. In some embodiments, at least one CTP sequence at the carboxy terminal end of the coagulation factor provides enhanced protection against clearance. In some embodiments, at least one CTP sequence at the carboxy terminal end of the coagulation factor provides prolonged clearance time. In some embodiments, at least one CTP sequence at the carboxy terminal end of the coagulation factor enhances its Cmax. In some embodiments, at least one CTP sequence at the carboxy terminal end of the coagulation factor enhances its Tmax. In some embodiments, at least one CTP sequence at the carboxy terminal end of the coagulation factor prolongs its T½.

In another embodiment, a conjugated coagulation factor disclosed herein is used in the same manner as an unmodified conjugated coagulation factor. In another embodiment, a conjugated coagulation factor disclosed herein has an increased circulating half-life and plasma residence time, decreased clearance, and increased clinical activity in vivo. In another embodiment, due to the improved properties of the conjugated coagulation factor as described herein, this conjugate is administered less frequently than the unmodified form of the same coagulation factor.

In another embodiment, decreased frequency of administration will result in improved treatment strategy, which in one embodiment, will lead to improved patient compliance leading to improved treatment outcomes, as well as improved patient quality of life. In another embodiment, compared to conventional conjugates of coagulation factors, it has been found that conjugates having the molecular weight and linker structure of the conjugates disclosed herein have an improved potency, improved stability, elevated AUC levels, and enhanced circulating half-life.

In another embodiment, disclosed herein is a pharmaceutical composition comprising a CTP-modified Factor IX (FIX) polypeptide consisting of a FIX polypeptide and three CTPs attached to the carboxy terminus of said CTP-modified FIX polypeptide. In another embodiment, disclosed herein is a pharmaceutical composition comprising a CTP-modified Factor IX (FIX) polypeptide consisting of a FIX polypeptide and three CTPs attached to the carboxy terminus of said CTP-modified FIX polypeptide, as set forth in SEQ ID NO: 113.

In another embodiment, disclosed herein is a pharmaceutical composition comprising an activated CTP-modified Factor VIIa (FVIIa) polypeptide consisting of a FVIIa polypeptide and five CTPs attached to the carboxy terminus of said FVIIa. In another embodiment, disclosed herein is a pharmaceutical composition comprising an activated CTP-modified Factor VIIa (FVIIa) polypeptide consisting of a FVIIa polypeptide and three CTPs attached to the carboxy terminus of said FVIIa. In another embodiment, disclosed herein is a pharmaceutical composition comprising an activated CTP-modified Factor VIIa (FVIIa) polypeptide consisting of a FVIIa polypeptide and three CTPs attached to the carboxy terminus, as set forth in SEQ ID NO: 111.

In another embodiment, disclosed herein is a composition comprising a conjugated coagulation factor as described herein. In another embodiment, disclosed herein is a pharmaceutical composition comprising the conjugated coagulation factor as described herein. In another embodiment, disclosed herein is a pharmaceutical composition comprising a therapeutically effective amount of the conjugated coagulation factor as described herein. In one embodiment, a therapeutically effective amount of a conjugated coagulation factor is determined according to factors such as the specific condition being treated, the condition of the patient being treated, as well as the other ingredients in the composition.

In another embodiment, a conjugated coagulation factor as described herein is useful in the treatment of subjects afflicted with Hemophilia. In another embodiment, a conjugated coagulation factor as described herein is useful in the prophylactic therapy of Hemophilia thus reducing the risk of bleeding and associated complications. In another embodiment, a conjugated coagulation factor as described herein is useful in the treatment of subjects afflicted with Hemophilia while reducing the risk of developing inhibitory antibodies to exogenously administered coagulation factors. In another embodiment, a conjugated coagulation factor as described herein is useful in the treatment of subjects afflicted with Hemophilia thus inducing homeostasis.

In one embodiment, a CTP-modified coagulation factor disclosed herein has therapeutic uses. In another embodiment, a CTP-modified coagulation factor disclosed herein has prophylactic uses.

In another embodiment, a conjugated coagulation factor as described herein is useful in the treatment of subjects experiencing excessive bleeding or bruising or having a prolonged Prothrombin Time (PT) or Partial Thromboplastin Time (PTT). In another embodiment, a conjugated coagulation factor as described herein is useful in the treatment of subjects having an acquired condition that is causing bleeding, such as vitamin K deficiency or liver disease. In another embodiment, a conjugated coagulation factor as described herein is useful in the treatment of subjects having deficiencies in coagulation factors that are acquired (due to other diseases) or inherited, mild or severe, permanent or temporary. In another embodiment, a conjugated coagulation factor as described herein is useful in the treatment of subjects afflicted with hemophilia A. In another embodiment, a conjugated coagulation factor as described herein is useful in the treatment of subjects afflicted with hemophilia B. In another embodiment, a conjugated coagulation factor as described herein is useful in the treatment of subjects having acquired deficiencies due to chronic diseases, such as liver disease or cancer; to an acute condition such as disseminated intravascular coagulation (DIC), which uses up clotting factors at a rapid rate; or to a deficiency in vitamin K or treatment with a vitamin K antagonist like warfarin (the production of factors II, VII, IX, and X require vitamin K). In another embodiment, a conjugated coagulation factor as described herein is useful in the treatment of subjects afflicted with a disease in which causes clotting imbalances such as but not limited to: a liver disease, uremia, a cancer, a bone marrow disorder, an exposure to snake venom, a vitamin K deficiency, an anticoagulation therapy, an accidental ingestion of the anticoagulant warfarin, multiple blood transfusions (stored units of blood lose some of their clotting factors), or a combination thereof. In another embodiment, disclosed herein is a method of treating deep vein thrombosis in a subject comprising administering a CTP-modified coagulation factor disclosed herein. In another embodiment, disclosed herein is a method of preventing uncontrolled bleeding in a subject with hemophilia comprising administering a CTP-modified coagulation factor disclosed herein. In another embodiment, disclosed herein is a method of preventing bleeding episodes in a subject with hemophilia comprising administering a CTP-modified coagulation factor disclosed herein. In another embodiment, disclosed herein is a method of controlling bleeding episodes in a subject with hemophilia B (congenital factor IX deficiency).

In another embodiment, the compositions and methods disclosed herein are for the treatment of bleeding episodes in hemophilia A or B patients with inhibitors to FVIII or FIX and in patients with acquired hemophilia; prevention of bleeding in surgical interventions or invasive procedures in hemophilia A or B patients with inhibitors to FVIII or FIX and in patients with acquired hemophilia; treatment of bleeding episodes in patients with congenital FVII deficiency and prevention of bleeding in surgical interventions or invasive procedures in patients with congenital FVII deficiency. In another embodiment, the compositions and methods disclosed herein are for the treatment or prevention of muscle bleeds. In another embodiment, the compositions and methods disclosed herein are for the treatment or prevention of joint bleeds. In another embodiment, the compositions and methods disclosed herein manufacture therapeutic or prophylactic treatment of epistaxis and gum bleeding, mucous membrane bleeding, bleeding into the central nervous system. In another embodiment, the compositions and methods disclosed herein manufacture therapeutic or prophylactic treatment of gastrointestinal or cerebral bleeding. In another embodiment, the compositions and methods disclosed herein manufacture therapeutic or prophylactic treatment of low frequency mild bleeds. In another embodiment, the compositions and methods disclosed herein manufacture therapeutic or prophylactic treatment of low frequency moderate bleeds. In another embodiment, the compositions and methods disclosed herein manufacture therapeutic or prophylactic treatment of high frequency mild bleeds. In another embodiment, the compositions and methods disclosed herein manufacture therapeutic or prophylactic treatment of high frequency moderate bleeds.

In one embodiment, the compositions and methods disclosed herein manufacture therapeutic or prophylactic treatment of asymptomatic hemophilia. In another embodiment, the compositions and methods disclosed herein manufacture therapeutic or prophylactic treatment of mild to moderate hemophilia. In another embodiment, the compositions and methods disclosed herein manufacture therapeutic or prophylactic treatment of severe hemophilia.

In one embodiment, the compositions and methods disclosed herein manufacture therapeutic or prophylactic treatment of hemorrhage, which in one embodiment, is uncontrollable hemorrhage, and, in another embodiment, intracerebral hemorrhage. In another embodiment, the compositions and methods disclosed herein manufacture therapeutic or prophylactic treatment of neonatal coagulopathies; severe hepatic disease; high-risk surgical procedures; traumatic blood loss; bone marrow transplantation; thrombocytopenias and platelet function disorders; urgent reversal of oral anticoagulation; congenital deficiencies of factors V, VII, X, and XI; or von Willebrand disease, in one embodiment, von Willebrand disease with inhibitors to von Willebrand factor.

In one embodiment, a CTP-modified coagulation factor disclosed herein is for the treatment of hemophilia or a related disease as described herein in a subject.

In another embodiment, a [(CTP)n>1-coagulation factor] as described herein comprises a full length coagulation factor or an active fragment thereof connected via a peptide bond on its carboxy terminus to at least one CTP unit with no CTPs on its amino terminus. In another embodiment, a [(CTP)n>1-coagulation factor] as described herein comprises a coagulation factor or an active fragment thereof connected via a peptide bond to at least one CTP unit which is connected to an additional CTP unit via a peptide bond with no CTPs on its amino terminus. In another embodiment, one nucleic acid molecule encodes an engineered coagulation factor comprising at least one CTP attached to its C-terminus and no CTPs on its amino terminus.

In another embodiment, disclosed herein is an expression vector comprising a polynucleotide molecule as described herein. In another embodiment, disclosed herein is a n expression vector comprising a polynucleotide encoding a CTP-modified polypeptide consisting of a Factor IX (FIX) polypeptide and three CTPs attached to the carboxy terminus of said FIX polypeptide. In another embodiment, disclosed herein is a n expression vector comprising a polynucleotide encoding a CTP-modified polypeptide consisting of a Factor VIIa (FVIIa) polypeptide and three CTPs attached to the carboxy terminus of said FVIIa polypeptide.

In another embodiment, disclosed herein is a cell comprising the expression vector as described herein. In another embodiment, disclosed herein is a cell comprising an expression vector comprising a polynucleotide encoding a CTP-modified polypeptide consisting of a Factor IX (FIX) polypeptide and three CTPs attached to the carboxy terminus of said FIX polypeptide. In another embodiment, disclosed herein is a cell comprising an expression vector comprising a polynucleotide encoding a CTP-modified polypeptide consisting of a Factor VIIa (FVIIa) polypeptide and three CTPs attached to the carboxy terminus of said FVIIa polypeptide.

In another embodiment, disclosed herein is a composition comprising the expression vector as described herein. In another embodiment, disclosed herein is a composition comprising an expression vector comprising a polynucleotide encoding a CTP-modified polypeptide consisting of a Factor IX (FIX) polypeptide and three CTPs attached to the carboxy terminus of said FIX polypeptide. In another embodiment, disclosed herein is a composition comprising an expression vector comprising a polynucleotide encoding a CTP-modified polypeptide consisting of a Factor VIIa (FVIIa) polypeptide and three CTPs attached to the carboxy terminus of said FVIIa polypeptide.

In another embodiment, disclosed herein is a composition comprising the cell as described herein. In another embodiment, the cell is a eukaryotic cell. In another embodiment, the cell is a prokaryotic cell.

In another embodiment, disclosed herein is a method of producing a CTP-modified coagulation factor, comprising the step of attaching one to ten chorionic CTPs to the carboxy terminus of said coagulation factor, thereby producing a CTP-modified coagulation factor. In another embodiment, disclosed herein is a method of producing a CTP-modified coagulation factor, comprising the step of attaching one to ten polynucleotide sequences encoding a chorionic gonadotropin carboxy terminal peptide (CTP) to the carboxy terminus of a polynucleotide sequence encoding said coagulation factor, thereby producing a CTP-modified coagulation factor. In another embodiment, disclosed herein is a method of producing a CTP-modified Factor IX (FIX) polypeptide, comprising the step of attaching three chorionic CTPs to the carboxy terminus of said FIX polypeptide, thereby producing a CTP-modified FIX polypeptide. In another embodiment, disclosed herein is a method of producing a CTP-modified Factor VIIa (FVIIa) polypeptide, comprising the step of attaching three chorionic CTPs to the carboxy terminus of said FVIIa polypeptide, thereby producing a CTP-modified FVIIa polypeptide.

In another embodiment, the engineered coagulation factors disclosed herein are synthesized using a polynucleotide molecule encoding a polypeptide disclosed herein. In some embodiments, the polynucleotide molecule encoding the engineered coagulation factors disclosed herein is ligated into an expression vector, comprising a transcriptional control of a cis-regulatory sequence (e.g., promoter sequence). In some embodiments, the cis-regulatory sequence is suitable for directing constitutive expression of an engineered coagulation factor disclosed herein. In some embodiments, the cis-regulatory sequence is suitable for directing tissue-specific expression of the engineered coagulation factors disclosed herein. In some embodiments, the cis-regulatory sequence is suitable for directing inducible expression of the engineered coagulation factors disclosed herein.

In one embodiment, disclosed herein are long-acting dual GLP-1/glucagon receptor agonists and methods of producing and using same. In another embodiment, long-acting GLP-1/glucagon receptor agonists comprise a carboxy terminal peptide (CTP, also referred to as CTP unit). In another embodiment, long-acting polypeptides which comprise a GLP-1/glucagon receptor agonist further comprise a carboxy terminal peptide (CTP) of human Chorionic Gonadotropin (hCG). In another embodiment, CTP acts as a protectant against the degradation of a GLP-1/glucagon receptor agonist. In another embodiment, CTP extends the Cmax of a GLP-1/glucagon receptor agonist. In another embodiment, CTP extends the Tmax of a GLP-1/glucagon receptor agonist. In another embodiment, CTP extends the circulatory half-life of a GLP-1/glucagon receptor agonist. In some embodiments, CTP enhances the potency of a GLP-1/glucagon receptor agonist.

In another embodiment, disclosed herein is a method of extending the biological half-life of a GLP-1/glucagon receptor agonist, comprising the step of attaching one to ten CTPs to the carboxy terminus of the GLP-1/glucagon receptor agonist, thereby extending the biological half-life of the GLP-1/glucagon receptor agonist. In another embodiment, disclosed herein is a method of extending the biological half-life of a GLP-1/glucagon receptor agonist, comprising the step of attaching one to five CTPs to the carboxy terminus of the GLP-1/glucagon receptor agonist, thereby extending the biological half-life of the GLP-1/glucagon receptor agonist. In another embodiment, disclosed herein is a method for extending the circulatory half-life of a GLP-1/glucagon receptor agonist. In another embodiment, disclosed herein is a method for increasing the half-life of a GLP-1/glucagon receptor agonist. In another embodiment, disclosed herein is a method for extending the half-life of a GLP-1/glucagon receptor agonist.

In one embodiment, disclosed herein is a CTP-modified polypeptide comprising a dual GLP-1/Glucagon receptor agonist and at least one chorionic gonadotropin carboxy terminal peptide (CTP) attached to the amino terminus or carboxy terminus of said agonist.

In another embodiment, the agonist is a protein, a polypeptide, or a peptide. In another embodiment, the peptide is an oxyntomodulin (OXM).

In another embodiment, the CTP-modified polypeptide comprises a peptide that comprises fewer than 50 amino acids and at least one chorionic gonadotropin carboxy terminal peptide, attached to an amino or a carboxy terminus of the peptide. In another embodiment, the peptide is OXM.

Oxyntomodulin peptide is useful for the treatment of metabolic disorders such as diabetes and obesity. However, due to the short half-life of the peptide and its low stability in-vivo, repeated daily administrations of supraphysiological doses are required in order to achieve pharmacological effect in humans. As demonstrated herein below (see Examples), all OXM-CTP variants disclosed herein demonstrated superior pharmacokinetic profile in rats as compared to native OXM, with a substantial increase in the exposure and elongated half-life. Surprisingly, the OXM-CTP-CTP variant demonstrated superior PK parameters as compared to the CTP-OXM-CTP-CTP variant which is fused to 3 copies of CTP, one in the N-terminus and two in tandem in the C-terminus.

Fusion of 2 and 3 CTPs to the C-terminus of OXM resulted in similar fold of increase compared to the native peptide half-life (21.6 and 21 respectively), following SC administration (see Examples below). Therefore, it was expected that fusion of four and five CTP to the C-terminus of OXM would not significantly elongate the half-life above 20-fold. However, a surprisingly marked increase in OXM half-life (i.e. 50-fold) was demonstrated for the OXM-4CTP variants and the OXM-5CTP variant with 30-fold increase in the exposure as reflected by the area under the curve (AUC) parameter (see Examples herein).

Hence, in one embodiment, disclosed herein is a CTP-modified polypeptide comprising an OXM peptide and at least one chorionic gonadotropin carboxy terminal peptide (CTP) attached to the amino terminus or carboxy terminus of the oxyntomodulin peptide.

In one embodiment, a CTP-modified OXM as described herein comprises a full length OXM or an active fragment thereof connected via a peptide bond on its amino or carboxy terminus to at least one CTP unit with no CTPs on its amino terminus. In another embodiment, a CTP-modified OXM as described herein comprises a full length OXM or an active fragment thereof connected via a peptide bond on its carboxy terminus to at least one CTP unit with no CTPs on its amino terminus. In another embodiment, a CTP-modified OXM as described herein comprises a full length OXM or an active fragment thereof connected via a peptide bond on its amino terminus to at least one CTP unit with no CTPs on its carboxy terminus. In another embodiment, disclosed herein is a nucleic acid molecule encoding an engineered OXM as described herein above, which in one embodiment, comprises at least one CTP attached to its carboxy terminus or its amino terminus.

InIn another embodiment, the CTP-modified polypeptide comprises a peptide that comprises fewer than 50 amino acids and at least one chorionic gonadotropin carboxy terminal peptide, attached to an amino or a carboxy terminus of the peptide. In one embodiment, the CTP-modified polypeptide disclosed herein comprises a peptide that comprises fewer than 40 amino acids and at least one chorionic gonadotropin carboxy terminal peptide, attached to an amino or a carboxy terminus of the peptide. In another embodiment, the CTP-modified polypeptide disclosed herein comprises a peptide that comprises fewer than 30, 20, or 10 amino acids. In one embodiment, the peptide comprising fewer than 50 amino acids is a dual GLP-1/Glucagon receptor agonist. In another embodiment, the peptide comprising fewer than 50 amino acids is OXM.

In another embodiment, OXM comprises the following amino acid (AA) sequence: HSQGTFTSDYSKYLDSRRAQDFVQWLMNTKRNRNNIA (SEQ ID NO: 57). In another embodiment, OXM consists of the amino acid sequence of SEQ ID NO: 57.

In another embodiment, OXM is human OXM or any mammal OXM. A skilled artisan would appreciate that OXM may also be termed glucagon-37 or bioactive enteroglucagon. In another embodiment, OXM is a dual GLP-1/Glucagon receptor agonist. A skilled artisan would appreciate that the term OXM may encompass a biologically active fragment of OXM. In another embodiment, biologically active OXM extends from amino acid 30 to amino acid 37 of SEQ ID NO: 57. In another embodiment, biologically active OXM extends from amino acid 19 to amino acid 37 of SEQ ID NO: 57. In another embodiment, OXM disclosed herein corresponds to an octapeptide from which the two C-terminal amino acids are deleted. In another embodiment, OXM disclosed herein corresponds to any fragment of SEQ ID NO: 57 which retains OXM activity as described herein.

In one embodiment, two chorionic gonadotropin carboxy terminal peptides are attached to OXM, one CTP on the carboxy terminus and one CTP on the amino terminus of the OXM peptide. In another embodiment, two chorionic gonadotropin carboxy terminal peptides are attached to OXM on the carboxy terminus of the OXM peptide. In another embodiment, two chorionic gonadotropin carboxy terminal peptides are attached to OXM, both on the amino terminus of the OXM peptide. In another embodiment, three chorionic gonadotropin carboxy terminal peptides are attached to OXM, one CTP on the amino terminus and two CTPs on the carboxy terminus of the OXM peptide. In another embodiment, three chorionic gonadotropin carboxy terminal peptides are attached to the carboxy terminus of the OXM peptide. In another embodiment, four chorionic gonadotropin carboxy terminal peptides are attached to the carboxy terminus of the OXM peptide. In another embodiment, five chorionic gonadotropin carboxy terminal peptides are attached to the carboxy terminus of the OXM peptide. In another embodiment, 1-10 CTP are attached to the amino or carboxy terminus of OXM. In another embodiment, 1-10 CTPs are attached to the amino terminus of OXM. In another embodiment, 1-10 CTPs are attached to the carboxy terminus of OXM.

In another embodiment, disclosed herein is a method of producing a CTP-modified polypeptide comprising an OXM peptide and at least one CTP attached to the amino terminus or carboxy terminus of the OXM peptide, the method comprising the step of attaching at least one chorionic gonadotropin carboxy terminal peptide attached to the amino terminus or carboxy terminus of the OXM peptide.

In one embodiment, disclosed herein is a method of extending the biological half-life of a peptide comprising fewer than 50 amino acids, comprising the step of attaching at least one chorionic gonadotropin carboxy terminal peptides to an amino or a carboxy terminus of the agonist, thereby improving the biological half-life of the agonist.

In one embodiment, disclosed herein is a method of extending the biological half-life of a dual GLP-1/Glucagon receptor agonist, comprising the step of attaching at least one chorionic gonadotropin carboxy terminal peptides to an amino or a carboxy terminus of the agonist, thereby improving the biological half-life of the agonist.

In another embodiment, disclosed herein is a method of extending the biological half-life of OXM, comprising the step of attaching at least one CTP to an amino or a carboxy terminus of the OXM, thereby improving the biological half-life of OXM.

In one embodiment, disclosed herein is a method of improving the AUC of a peptide comprising fewer than 50 amino acids, comprising the step of attaching at least one CTP to a carboxy terminus of the agonist, thereby improving the AUC of the agonist.

In one embodiment, disclosed herein is a method of improving the AUC of a dual GLP-1/Glucagon receptor agonist, comprising the step of attaching at least one CTP to a carboxy terminus of the agonist, thereby improving the AUC of the agonist.

In another embodiment, disclosed herein is a method of improving the AUC of OXM, comprising the step of attaching at least one CTP to a carboxy terminus of the OXM, thereby improving the AUC of OXM.

In one embodiment, the term OXM further includes a homologue of a known OXM. In one embodiment, the homologue is a functional homologue. A skilled artisan would appreciate that the term "functional" may encompass the ability of an OXM homologue, peptide or protein disclosed herein to suppress appetite. In addition, the skilled artisan would appreciate the ability an OXM homologue, peptide or protein disclosed herein has to extend another protein's or peptide's biological half-life. A skilled artisan would appreciate that the biological half-life (T½) of a OXM protein, peptide or homologue disclosed would encompass the time it takes for half of the amount of the protein, peptide or homologue to be degraded or to not be present in a biological medium in a subject. In another embodiment, the biological medium is serum, cerebospinal fluid, tissue, mucosa, and the like.

In one embodiment, OXM binds to a receptor and mediates appetite suppression. In another embodiment, the receptor is a dual GLP-1/Glucagon receptor. In another embodiment, the receptor is a GLP-1 receptor. In another embodiment, the receptor is a glucagon receptor. In yet another embodiment, the receptor is any receptor known in the art to bind to OXM.

In another embodiment, disclosed herein is a homologue of an OXM. A skilled artisan would appreciate that an OXM homologue may encompass a peptide homologue of the peptide of SEQ ID NO: 57.

In another embodiment, disclosed herein is a homologue of an OXM having an appetite suppressing activity. In another embodiment, disclosed herein is a homologue of an OXM having functional binding. In another embodiment, disclosed herein is homologues of an OXM as described herein having an appetite suppression and activity. In another embodiment, disclosed herein is homologues of an OXM as described herein having functional binding. In another embodiment, homologues comprise polypeptides which are at least 50%, at least 55%, at least 60%, at least 65%, at least 70%, at least 75%, at least 80%, at least 85%, at least 87%, at least 89%, at least 91%, at least 93%, at least 95%, at least 96%, at least 97%, at least 98%, or at least 99% homologous to an OXM as determined using BlastP software of the National Center of Biotechnology Information (NCBI) using default parameters.

In one embodiment, disclosed herein is a pharmaceutical composition comprising the CTP-modified polypeptide disclosed herein.

In one embodiment, disclosed herein is a polypeptide comprising a GLP-1/Glucagon receptor agonist and two CTPs attached to the carboxy terminus of the GLP-1/Glucagon receptor agonist. In another embodiment, disclosed herein is a polypeptide comprising a GLP-1/Glucagon receptor agonist and two to three CTPs, two to four , two to five CTPs , two to six CTPs , two to seven CTPs , two to eight CTPs , two to nine CTPs , or two to ten CTPs attached to the carboxy terminus of the GLP-1/Glucagon receptor agonist.

In one embodiment, disclosed herein is a polypeptide comprising an OXM and two CTPs attached to the carboxy terminus of the OXM. In another embodiment, disclosed herein is a polypeptide comprising an OXM and two to three CTPs, two to four CTPs , two to five CTPs , two to six CTPs , two to seven CTPs , two to eight CTPs two to nine CTPs , or two to ten CTPs attached to the carboxy terminus of the OXM.

In one embodiment, disclosed herein is a polypeptide comprising a GLP-1/Glucagon receptor agonist and three CTPs attached to the carboxy terminus of the GLP-1/Glucagon receptor agonist. In another embodiment, disclosed herein is a polypeptide comprising a GLP-1/Glucagon receptor agonist and three to four CTPs , three to five CTPs , three to six CTPs , three to seven CTPs , three to eight CTPs , three to nine CTPs , or three to ten CTPs attached to the carboxy terminus of the GLP-1/Glucagon receptor agonist.

In one embodiment, disclosed herein is a polypeptide comprising an OXM and three CTPs attached to the carboxy terminus of the OXM. In another embodiment, disclosed herein is a polypeptide comprising an OXM and three to four CTPs, three to five CTPs , three to six CTPs , three to seven CTPs , three to eight CTPs , three to nine CTPs , or three to ten CTPs attached to the carboxy terminus of the OXM.

In one embodiment, disclosed herein is a polypeptide comprising a GLP-1/Glucagon receptor agonist and four CTPs attached to the carboxy terminus of the GLP-1/Glucagon receptor agonist. In another embodiment, disclosed herein is a polypeptide comprising a GLP-1/Glucagon receptor agonist and four to five CTPs, four to six , four to seven CTPs , four to eight CTPs , four to nine CTPs , or four to ten CTPs attached to the carboxy terminus of the GLP-1/Glucagon receptor agonist.

In one embodiment, disclosed herein is a polypeptide comprising an OXM and four CTPs attached to the carboxy terminus of the OXM. In another embodiment, disclosed herein is a polypeptide comprising an OXM and four to five CTPs , four to six CTPs , four to seven CTPs , four to eight CTPs , four to nine CTPs , or four to ten CTPs attached to the carboxy terminus of the OXM.

In one embodiment, disclosed herein is a polypeptide comprising a GLP-1/Glucagon receptor agonist and five CTPs attached to the carboxy terminus of the GLP-1/Glucagon receptor agonist. In another embodiment, disclosed herein is a polypeptide comprising a GLP-1/Glucagon receptor agonist and five to six CTPs , five to seven five to eight CTPs , five to nine CTPs , or five to ten CTPs attached to the carboxy terminus of the GLP-1/Glucagon receptor agonist.

In one embodiment, disclosed herein is a polypeptide comprising an OXM and five CTPs attached to the carboxy terminus of the OXM. In another embodiment, disclosed herein is a polypeptide comprising an OXM and five to six CTPs , five to seven CTPs ,five to eight CTPs , five to nine CTPs , or five to ten CTPs attached to the carboxy terminus of the OXM.

In one embodiment, disclosed herein is a polypeptide consisting of a GLP-1/Glucagon receptor agonist and two CTPs attached to the carboxy terminus of the GLP-1/Glucagon receptor agonist. In another embodiment, disclosed herein is a polypeptide consisting of a GLP-1/Glucagon receptor agonist and two to three CTPs , two to four , two to five CTPs , two to six CTPs , two to seven CTPs , two to eight CTPs , two to nine CTPs , or two to ten CTPs attached to the carboxy terminus of the GLP-1/Glucagon receptor agonist.

In one embodiment, disclosed herein is a polypeptide consisting of an OXM and two CTPs attached to the carboxy terminus of the OXM. In another embodiment, disclosed herein is a polypeptide consisting of an OXM and two to three CTPs , two to four CTPs , two to five CTPs , two to six CTPs , two to seven CTPs two to eight CTPs attached to the carboxy terminus of the OXM. In another embodiment, disclosed herein is a polypeptide consisting of an OXM and two to nine CTPs attached to the carboxy terminus of the OXM. In another embodiment, disclosed herein is a polypeptide consisting of an OXM and two to ten CTPs attached to the carboxy terminus of the OXM.

In one embodiment, disclosed herein is a polypeptide consisting of a GLP-1/Glucagon receptor agonist and three CTPs attached to the carboxy terminus of the GLP-1/Glucagon receptor agonist. In another embodiment, disclosed herein is a polypeptide consisting of a GLP-1/Glucagon receptor agonist and three to four CTPs , three to five CTPs , three to six CTPs , three to seven CTPs , three to eight CTPs , three to nine CTPs , or three to ten CTPs attached to the carboxy terminus of the GLP-1/Glucagon receptor agonist.

In one embodiment, disclosed herein is a polypeptide consisting of an OXM and three CTPs attached to the carboxy terminus of the OXM. In another embodiment, disclosed herein is a polypeptide consisting of an OXM and three to four CTPs , three to five CTPs , three to six CTPs , three to seven CTPs , three to eight CTPs three to nine CTPs , or three to ten CTPs attached to the carboxy terminus of the OXM.

In one embodiment, disclosed herein is a polypeptide consisting of a GLP-1/Glucagon receptor agonist and four CTPs attached to the carboxy terminus of the GLP-1/Glucagon receptor agonist. In another embodiment, disclosed herein is a polypeptide consisting of a GLP-1/Glucagon receptor agonist and four to five CTPs , four to six CTPs , four to seven CTPs , four to eight CTPs , four to nine CTPs , or four to ten CTPs attached to the carboxy terminus of the GLP-1/Glucagon receptor agonist.

In one embodiment, disclosed herein is a polypeptide consisting of an OXM and four CTPs attached to the carboxy terminus of the OXM. In another embodiment, disclosed herein is a polypeptide consisting of an OXM and four to five CTPs , four to six CTPs , four to seven CTPs , four to eight CTPs , four to nine CTPs , or four to ten CTPs attached to the carboxy terminus of the OXM.

In one embodiment, disclosed herein is a polypeptide consisting of a GLP-1/Glucagon receptor agonist and five CTPs attached to the carboxy terminus of the GLP-1/Glucagon receptor agonist. In another embodiment, disclosed herein is a polypeptide consisting of a GLP-1/Glucagon receptor agonist and five to six CTPs , five to seven CTPs , five to eight CTPs , five to nine CTPs , or five to ten CTPs attached to the carboxy terminus of the GLP-1/Glucagon receptor agonist.

In one embodiment, disclosed herein is a polypeptide consisting of an OXM and five CTPs attached to the carboxy terminus of the OXM. In another embodiment, disclosed herein is a polypeptide consisting of an OXM and five to six CTPs , five to seven CTPs , five to eight CTPs , five to nine CTPs , or five to ten CTPs attached to the carboxy terminus of the OXM.

In one embodiment, disclosed herein is a polypeptide consisting essentially of a GLP-1/Glucagon receptor agonist and two CTPs attached to the carboxy terminus of the GLP-1/Glucagon receptor agonist. In another embodiment, disclosed herein is a polypeptide consisting essentially of a GLP-1/Glucagon receptor agonist and two to three CTPs , two to four CTPs , two to five CTPs ,two to six CTPs , two to seven CTPs , two to eight CTPs , two to nine CTPs , or two to ten CTPs attached to the carboxy terminus of the GLP-1/Glucagon receptor agonist.

In one embodiment, disclosed herein is a polypeptide consisting essentially of an OXM and two CTPs attached to the carboxy terminus of the OXM. In another embodiment, disclosed herein is a polypeptide consisting essentially of an OXM and two to three CTPs , two to four CTPs , two to five CTPs two to six CTPs , two to seven CTPs , two to eight CTPs , two to nine CTPs , or two to ten CTPs attached to the carboxy terminus of the OXM.

In one embodiment, disclosed herein is a polypeptide consisting essentially of a GLP-1/Glucagon receptor agonist and three CTPs attached to the carboxy terminus of the GLP-1/Glucagon receptor agonist. In another embodiment, disclosed herein is a polypeptide consisting essentially of a GLP-1/Glucagon receptor agonist and three to four CTPs , three to five CTPs , three to six CTPs , three to seven CTPs , three to eight CTPs , three to nine CTPs , or three to ten CTPs attached to the carboxy terminus of the GLP-1/Glucagon receptor agonist.

In one embodiment, disclosed herein is a polypeptide consisting essentially of an OXM and three CTPs attached to the carboxy terminus of the OXM. In another embodiment, disclosed herein is a polypeptide consisting essentially of an OXM and three to four CTPs three to five CTPs , three to six CTPs , three to seven CTPs , three to eight CTPs , three to nine CTPs , three to ten CTPs attached to the carboxy terminus of the OXM.

In one embodiment, disclosed herein is a polypeptide consisting essentially of a GLP-1/Glucagon receptor agonist and four CTPs attached to the carboxy terminus of the GLP-1/Glucagon receptor agonist. In another embodiment, disclosed herein is a polypeptide consisting essentially of a GLP-1/Glucagon receptor agonist and four to five CTPs , four to six CTPs , four to seven CTPs , four to eight CTPs , four to nine CTPs , or four to ten CTPs attached to the carboxy terminus of the GLP-1/Glucagon receptor agonist.

In one embodiment, disclosed herein is a polypeptide consisting essentially of an OXM and four CTPs attached to the carboxy terminus of the OXM. In another embodiment, disclosed herein is a polypeptide consisting essentially of an OXM and four to five CTPs , four to six CTPs , four to seven CTPs four to eight CTPs , four to nine CTPs , or four to ten CTPs attached to the carboxy terminus of the OXM.

In one embodiment, disclosed herein is a polypeptide consisting essentially of a GLP-1/Glucagon receptor agonist and five CTPs attached to the carboxy terminus of the GLP-1/Glucagon receptor agonist. In another embodiment, disclosed herein is a polypeptide consisting essentially of a GLP-1/Glucagon receptor agonist and five to six CTPs , five to seven CTPs , five to eight, five to nine CTPs , or five to ten CTPs attached to the carboxy terminus of the GLP-1/Glucagon receptor agonist.

In one embodiment, disclosed herein is a polypeptide consisting essentially of an OXM and five CTPs attached to the carboxy terminus of the OXM. In another embodiment, disclosed herein is a polypeptide consisting essentially of an OXM and five to six CTPs ,five to seven CTPs , five to eight CTPs , five to nine CTPs , or five to ten CTPs attached to the carboxy terminus of the OXM.

In another embodiment, disclosed herein is a polypeptide comprising, consisting essentially of, or consisting of a GLP-1/Glucagon receptor agonist having no CTPs on its amino terminus. In another embodiment, disclosed herein is a polypeptide comprising, consisting essentially of, or consisting of a GLP-1/Glucagon receptor agonist lacking a CTP on its amino terminus. In another embodiment, disclosed herein is a polypeptide comprising, consisting essentially of, or consisting of a GLP-1/Glucagon receptor agonist having at least one CTP on its carboxy terminus and no CTPs on its amino terminus. In another embodiment, disclosed herein is a polypeptide comprising, consisting essentially of, or consisting of a GLP-1/Glucagon receptor agonist having the number of CTPs on its carboxy terminus as described herein and no CTPs on its amino terminus.

In another embodiment, disclosed herein is a polypeptide comprising, consisting essentially of, or consisting of an OXM having no CTPs on its amino terminus. In another embodiment, disclosed herein is a polypeptide comprising, consisting essentially of, or consisting of an OXM lacking a CTP on its amino terminus. In another embodiment, disclosed herein is a polypeptide comprising, consisting essentially of, or consisting of an OXM having at least one CTP on its carboxy terminus and no CTPs on its amino terminus. In another embodiment, disclosed herein is a polypeptide comprising, consisting essentially of, or consisting of an OXM having the number of CTPs on its carboxy terminus as described herein and no CTPs on its amino terminus.

In one embodiment, the amino acid sequence of the CTP-modified polypeptide comprises SEQ ID NO: 58, 59, 60, 61, 62, 63, or 64, further disclosed herein. In another embodiment, the amino acid sequence of the CTP-modified polypeptide is selected from the group consisting of: SEQ ID NO: 58, 59, 60, 61, 62, 63, or 64, further disclosed herein.

In one embodiment, following expression a mature CTP-modified OXM polypeptide is secreted, the signal peptide having been cleaved from the precursor protein resulting in a mature protein. For example, in the precursor CTP-modified OXM polypeptide set forth in SEQ ID NO: 62, amino acids 1-26: MAT G S R T S L L L A F G L L C L P W L Q E G S A (SEQ ID NO: 115) represent the signal peptide of the precursor CTP-modified OXM polypeptide, and amino acids H S Q G T F T S D Y S K Y L D S R R A Q D F V Q W L M N T K R N R N N I A S S S S K A P P P S L P S P S R L P G P S D T P I L P Q S S S S K A P P P S L P S P S R L P G P S D T P I L P Q S S S S K A P P P S L P S P S R L P G P S D T P I L P Q (SEQ ID NO: 114) represent the mature engineered CTP-modified OXM polypeptide lacking the signal peptide. In one embodiment, the amino acid sequence of CTP-modified OXM without the signal peptide is set forth in SEQ ID NO: 114. In another embodiment, the signal peptide of CTP-modified OXM is set forth in SEQ ID NO: 115.

In another embodiment, disclosed herein is a polynucleotide encoding a polypeptide as described herein. In another embodiment, the polynucleotide comprises SEQ ID NO: 65, 66, 67, 68, 69, 70 or 71, further disclosed herein. In another embodiment, the polynucleotide is selected from the group consisting of SEQ ID NO: 65, 66, 67, 68, 69, 70 or 71, further disclosed herein.

In one embodiment, disclosed herein is a method of reducing cholesterol in a subject, comprising the step of attaching at least one CTP to an amino or a carboxy terminus of the OXM, thereby reducing cholesterol in a subject.

In another embodiment, disclosed herein is a method of reducing glycerol in a subject, comprising the step of attaching at least one CTP to an amino or a carboxy terminus of the OXM, thereby reducing glycerol in a subject.

In one embodiment, disclosed herein is a long-acting OXM. In one embodiment, a long-acting OXM disclosed herein maintains the biological activity of unmodified OXM. In another embodiment, the long-acting OXM disclosed herein comprises OXM biological activity. In another embodiment, the biological activity of a long-acting OXM disclosed herein comprises reducing digestive secretions. In another embodiment, the biological activity of a long-acting OXM disclosed herein comprises reducing and delaying gastric emptying. In another embodiment, the biological activity of a long-acting OXM disclosed herein comprises the inhibition of the fed motility pattern in the small intestine. In another embodiment, the biological activity of a long-acting OXM disclosed herein comprises the inhibition of acid secretion stimulated by pentagastrin. In another embodiment, the biological activity of a long-acting OXM disclosed herein comprises an increase of gastric somatostatin release. In another embodiment, the biological activity of a long-acting OXM disclosed herein comprises potentiating the effects of peptide YY. In another embodiment, the biological activity of a long-acting OXM disclosed herein comprises the inhibition of ghrelin release. In another embodiment, the biological activity of a long-acting OXM disclosed herein comprises the stimulation of aminopyrine accumulation and cAMP production. In another embodiment, the biological activity of a long-acting OXM disclosed herein comprises binding the GLP-1 receptor. In another embodiment, the biological activity of a long-acting OXM disclosed herein comprises stimulating H+ production by activating adenylate cyclase. In another embodiment, the biological activity of a long-acting OXM disclosed herein comprises inhibiting histamine-stimulated gastric acid secretion. In another embodiment, the biological activity of a long-acting OXM disclosed herein comprises inhibiting food intake. In another embodiment, the biological activity of a long-acting OXM disclosed herein comprises stimulating insulin release. In another embodiment, the biological activity of a long-acting OXM disclosed herein comprises inhibiting exocrine pancreatic secretion.

In another embodiment, the biological activity of a long-acting OXM disclosed herein comprises inhibiting pancreatic secretion through a vagal neural indirect mechanism. In another embodiment, the biological activity of a long-acting OXM disclosed herein comprises reducing hydromineral transport through the small intestine. In another embodiment, the biological activity of a long-acting OXM disclosed herein comprises stimulating glucose uptake. In another embodiment, the biological activity of a long-acting OXM disclosed herein comprises controlling/stimulating somatostatin secretion. In another embodiment, the biological activity of a long-acting OXM disclosed herein comprises reduction in both food intake and body weight gain. In another embodiment, the biological activity of a long-acting OXM disclosed herein comprises reduction in adiposity. In another embodiment, the biological activity of a long-acting OXM disclosed herein comprises appetite suppression. In another embodiment, the biological activity of a long-acting OXM disclosed herein comprises induction of anorexia. In another embodiment, the biological activity of a long-acting OXM disclosed herein comprises reducing body weight in overweight and obese subjects. In another embodiment, the biological activity of a long-acting OXM disclosed herein comprises inducing changes in the levels of the adipose hormones leptin and adiponectin. In another embodiment, the biological activity of a long-acting OXM disclosed herein comprises increasing energy expenditure in addition to decreasing energy intake in overweight and obese subjects.

Thus, in one embodiment, disclosed herein is a method of effecting any of the above-mentioned biological activities of OXM by administering a CTP-modified OXM disclosed herein. In another embodiment, disclosed herein is a method for reducing food intake, reducing body weight, or both in a subject, comprising the step of administering to the subject a CTP-modified OXM peptide. In another embodiment, the subject has diabetes. In another embodiment, the subject is overweight. In another embodiment, the subject is obese.

A skilled artisan would appreciate that the terms "reducing, reduction, lowering, etc." when used in relation to the methods disclosed herein may encompass 100% reduction from a previously measured or determined level or from a normal level. In another embodiment, the reduction is by 89-99% , is by 79-88% , is by 69-78% , is by 59-68% , is by 49-58% , is by 39-48% , is by 29-38% , is by 19-28% , is by 9-18% , is by 5-8% , or is by 1-4% from a previously determined level.

The *in-vivo* biological activity of OXM-CTP variants was assessed in two animal models, IPGTT in mice, which evaluate the ability of OXM to induce glucose tolerance following glucose administration, and food intake inhibition in lean rats, which assess the ability of OXM to inhibit animal's food consumption. It was demonstrated that OXM-CTP variants: CTP-OXM-CTP-CTP, OXM-CTP-CTP, OXM-CTP-CTP-CTP, OXM-CTP-CTP-CTP-CTP and OXM-CTP-CTP-CTP-CTP-CTP induced glucose tolerance as reflected by reduction of 20-30% of blood glucose AUC compared to vehicle group (see Example 4, herein). These results indicate that the biological activity of OXM-CTP variants was not inhibited in-vivo due to potential steric interference of CTP fusion to the binding of OXM to its receptor. The marked reduction in glucose induced by these variants correlated with their improved PK profiles. Therefore, in one embodiment, disclosed herein is a method of inducing glucose tolerance in a subject, the method comprising the step of administering to the subject an effective dose of a composition comprising a CTP-modified polypeptide comprising an OXM peptide and at least one CTP attached to the amino terminus or caboxy terminus of the OXM peptide, thereby inducing glucose tolerance in a subject.

In another embodiment, disclosed herein is a method of inducing glucose tolerance in a subject, the method comprising the step of administering to the subject an effective dose of a composition comprising a CTP-modified polypeptide comprising a GLP-1/Glucagon receptor agonist and at least one CTP attached to the amino terminus or caboxy terminus of the GLP-1/Glucagon receptor agonist peptide, thereby inducing glucose tolerance in a subject.

In another embodiment, disclosed herein is a use of a CTP-modified polypeptide comprising a dual GLP-1/Glucagon receptor agonist and at least one CTP attached to the amino terminus or caboxy terminus of said agonist for inducing glucose tolerance in a subject. In one embodiment, said dual GLP-1/Glucagon receptor agonist is OXM.

In another embodiment, disclosed herein is a use of a CTP-modified polypeptide comprising a dual GLP-1/Glucagon receptor agonist and at least one CTP attached to the amino terminus or caboxy terminus of said agonist in the preparation of a medicament for inducing glucose tolerance in a subject. In one embodiment, said dual GLP-1/Glucagon receptor agonist is OXM.

In one embodiment, disclosed herein is a method of increasing insulin sensitivity in a subject, the method comprising the step of administering to the subject an effective dose of a composition comprising a CTP-modified polypeptide comprising a GLP-1/Glucagon receptor agonist and at least one CTP attached to the amino terminus or caboxy terminus of the GLP-1/Glucagon receptor agonist peptide, thereby increasing insulin sensitivity in a subject.

In one embodiment, disclosed herein is a method of reducing insulin resistance in a subject, the method comprising the step of administering to the subject an effective dose of a composition comprising a CTP-modified polypeptide comprising a GLP-1/Glucagon receptor agonist and at least one CTP attached to the amino terminus or caboxy terminus of the GLP-1/Glucagon receptor agonist peptide, thereby reducing insulin resistance in a subject.

In one embodiment, disclosed herein is a method of increasing insulin sensitivity and reducing insulin resistance in a subject, the method comprising the step of administering to the subject an effective dose of a composition comprising a CTP-modified polypeptide comprising a GLP-1/Glucagon receptor agonist and at least one CTP attached to the amino terminus or caboxy terminus of the GLP-1/Glucagon receptor agonist peptide, thereby increasing insulin sensitivity and reducing insulin resistance in a subject.

The ability of OXM-CTP variants OXM-3CTP, OXM-4CTP and OXM-5CTP to inhibit food intake was assessed. Native OXM inhibited food intake only in the first hour following administration, however, all variants manifested substantial prolonged improved inhibition in food intake compared to OXM. The cumulative food inhibition of OXM-5CTP was surprisingly sustained for at least 141 hrs emphasizing the elongated half-life of this variant (see Examples herein).

Hence, in one embodiment, disclosed herein is a method of inducing food intake inhibition in a subject, the method comprising the step of administering to the subject an effective dose of a composition comprising a CTP-modified polypeptide comprising an OXM (OXM) peptide and at least one CTP attached to the amino terminus or carboxy terminus of the OXM peptide, thereby inducing food intake inhibition in the subject.

In another embodiment, disclosed herein is a method of inducing food intake inhibition in a subject, the method comprising the step of administering to the subject an effective dose of a composition comprising a CTP-modified polypeptide comprising a GLP-1/Glucagon receptor agonist and at least one CTP attached to the amino terminus or carboxy terminus of the GLP-1/Glucagon receptor agonist peptide, thereby inducing food intake inhibition in the subject.

In another embodiment, disclosed herein is a method of preventing, reducing or suppressing food intake in a subject, the method comprising the step of administering to the subject an effective dose of a composition comprising CTP-modified polypeptide comprising a GLP-1/Glucagon receptor agonist and at least one CTP attached to the amino terminus or carboxy terminus of the GLP-1/Glucagon receptor agonist peptide, thereby preventing reducing or suppressing food intake by a subject. In another embodiment, preventing, reducing or suppressing food intake by a subject reduces the chances of the subject developing undesired weight gain. In another embodiment, preventing reducing or suppressing food intake in a subject reduces the chances of the subject developing obesity.

In another embodiment, disclosed herein is a use of a CTP-modified polypeptide comprising a GLP-1/Glucagon receptor agonist and at least one CTP attached to the amino terminus or carboxy terminus of said agonist for preventing undesired weight gain by a subject. In one embodiment, said GLP-1/Glucagon receptor agonist is OXM.

In another embodiment, disclosed herein is a use of a CTP-modified polypeptide comprising a GLP-1/Glucagon receptor agonist and at least one CTP attached to the amino terminus or carboxy terminus of said agonist in the preparation of a medicament for preventing undesired weight gain by a subject. In one embodiment, said GLP-1/Glucagon receptor agonist is OXM.

In another embodiment, disclosed herein is a method of preventing, reducing or suppressing food intake in a subject, the method comprising the step of administering to the subject an effective dose of a composition comprising CTP-modified polypeptide comprising an OXM (OXM) peptide and at least one CTP attached to the amino terminus or carboxy terminus of the OXM peptide, thereby preventing reducing or suppressing food intake by a subject. In another embodiment, preventing, reducing or suppressing food intake by a subject reduces the chances of the subject developing undesired weight gain. In another embodiment, preventing reducing or suppressing food intake in a subject reduces the chances of the subject developing obesity.

In another embodiment, disclosed herein is a method of preventing undesired weight gain by a subject, the method comprising the step of administering to the subject an effective dose of a composition comprising CTP-modified polypeptide comprising a GLP-1/Glucagon receptor agonist and at least one CTP attached to the amino terminus or carboxy terminus of the GLP-1/Glucagon receptor agonist peptide, thereby preventing weight gain in a subject. In another embodiment, the weight gain leads to or results in obesity of the subject. In another embodiment, the risk of gaining weight gain is due to a psychological condition, or due to a genetic predisposposition to gain weight by the subject. In another embodiment, the psychological condition is depression, anxiety or post-traumatic stress disorder (PTSD).

In another embodiment, disclosed herein is a use of a CTP-modified polypeptide comprising a GLP-1/Glucagon receptor agonist and at least one CTP attached to the amino terminus or carboxy terminus of said agonist for preventing undesired weight gain by a subject. In one embodiment, said GLP-1/Glucagon receptor agonist is OXM.

In another embodiment, disclosed herein is a use of a CTP-modified polypeptide comprising a GLP-1/Glucagon receptor agonist and at least one CTP attached to the amino terminus or carboxy terminus of said agonist in the preparation of a medicament for preventing undesired weight gain by a subject. In one embodiment, said GLP-1/Glucagon receptor agonist is OXM.

In another embodiment, disclosed herein is a method of preventing undesired weight gain by a subject, the method comprising the step of administering to the subject an effective dose of a composition comprising CTP-modified polypeptide comprising an OXM peptide and at least one CTP attached to the amino terminus or carboxy terminus of the OXM peptide, thereby preventing weight gain in a subject. In another embodiment, the weight gain leads to or results in obesity of the subject. In another embodiment, the risk of gaining weight gain is due to a psychological condition, or due to a genetic predisposposition to gain weight by the subject. In another embodiment, the psychological condition is depression, anxiety or post-traumatic stress disorder (PTSD).

In another embodiment, disclosed herein is a method of treating obesity in a subject, the method comprising administering to the subject an effective dose of a composition comprising a CTP-modified polypeptide comprising a GLP-1/Glucagon receptor agonist peptide and at least one chorionic gonadotropin carboxy terminal peptide attached to the amino terminus or carboxy terminus of the GLP-1/Glucagon receptor agonist peptide, thereby treating obesity in the subject. In another embodiment, the subject is genetically predisposed to being obese. In another embodiment, the method of treating obesity results in a reduction of body weight in a subject. In another embodiment, the reduction in body weight is due to body fat reduction.

In another embodiment, disclosed herein is a use of a CTP-modified polypeptide comprising a GLP-1/Glucagon receptor agonist and at least one chorionic gonadotropin carboxy terminal peptide attached to the amino terminus or carboxy terminus of said agonist for treating obesity in a subject. In one embodiment, said GLP-1/Glucagon receptor agonist is OXM.

In another embodiment, disclosed herein is a use of a CTP-modified polypeptide comprising a GLP-1/Glucagon receptor agonist and at least one chorionic gonadotropin carboxy terminal peptide attached to the amino terminus or carboxy terminus of said agonist in the preparation of a medicament for treating obesity in a subject. In one embodiment, said GLP-1/Glucagon receptor agonist is OXM.

In another embodiment, disclosed herein is a method of treating obesity in a subject, the method comprising administering to the subject an effective dose of a composition comprising a CTP-modified polypeptide comprising an OXM peptide and at least one chorionic gonadotropin carboxy terminal peptide attached to the amino terminus or carboxy terminus of the OXM peptide, thereby treating obesity in the subject. In another embodiment, the subject is genetically predisposed to being obese.

In one embodiment, disclosed herein is a method of treating type II diabetes in a subject, the method comprising administering to the subject an effective dose of a composition comprising a CTP-modified polypeptide comprising a GLP-1/Glucagon receptor agonist peptide and at least one chorionic gonadotropin carboxy terminal peptide attached to the amino terminus or carboxy terminus of the GLP-1/Glucagon receptor agonist peptide, thereby treating type II diabetes in the subject.

In another embodiment, disclosed herein is a use of a CTP-modified polypeptide comprising a GLP-1/Glucagon receptor agonist and at least one chorionic gonadotropin carboxy terminal peptide attached to the amino terminus or carboxy terminus of said agonist for treating type II diabetes in a subject. In one embodiment, said GLP-1/Glucagon receptor agonist is OXM.

In another embodiment, disclosed herein is a use of a CTP-modified polypeptide comprising a GLP-1/Glucagon receptor agonist and at least one chorionic gonadotropin carboxy terminal peptide attached to the amino terminus or carboxy terminus of said agonist in the preparation of a medicament for treating type II diabetes in a subject. In one embodiment, said GLP-1/Glucagon receptor agonist is OXM.

In one embodiment, disclosed herein is a method of treating type II diabetes in a subject, the method comprising administering to the subject an effective dose of a composition comprising a CTP-modified polypeptide comprising an OXM peptide and at least one chorionic gonadotropin carboxy terminal peptide attached to the amino terminus or carboxy terminus of the OXM peptide, thereby treating type II diabetes in the subject.

In another embodiment, disclosed herein is a method of treating a metabolic disorder in a subject, the method comprising administering to the subject an effective dose of a composition comprising a CTP-modified polypeptide comprising a GLP-1/Glucagon receptor agonist and at least one chorionic gonadotropin carboxy terminal peptide attached to the amino terminus or carboxy terminus of the GLP-1/Glucagon receptor agonist peptide, thereby treating a metabolic disorder in the subject.

In another embodiment, disclosed herein is a use of a CTP-modified polypeptide comprising a GLP-1/Glucagon receptor agonist and at least one chorionic gonadotropin carboxy terminal peptide attached to the amino terminus or carboxy terminus of said agonist for treating a metabolic disorder in a subject. In one embodiment, said GLP-1/Glucagon receptor agonist is OXM.

In another embodiment, disclosed herein is a use of a CTP-modified polypeptide comprising a GLP-1/Glucagon receptor agonist and at least one chorionic gonadotropin carboxy terminal peptide attached to the amino terminus or carboxy terminus of said agonist in the preparation of a medicament for treating a metabolic disorder in a subject. In one embodiment, said GLP-1/Glucagon receptor agonist is OXM.

In another embodiment, disclosed herein is a method of treating a metabolic disorder in a subject, the method comprising administering to the subject an effective dose of a composition comprising a CTP-modified polypeptide comprising an OXM peptide and at least one chorionic gonadotropin carboxy terminal peptide attached to the amino terminus or carboxy terminus of the OXM peptide, thereby treating a metabolic disorder in the subject.

In another embodiment, the metabolic disorder is diabetic ketoacidosis, or diabetes mellitus or any glucose-related metabolic disorder known in the art. In another embodiment, the metabolic disorder results from a lack of insulin and an overabundance of glucose in a subject.

In another embodiment, the engineered GLP-1/Glucagon receptor agonist variants disclosed herein are synthesized using a polynucleotide molecule encoding a polypeptide disclosed herein. In another embodiment, the polynucleotide molecule encoding the engineered GLP-1/Glucagon receptor agonist disclosed herein is ligated into an expression vector, comprising a transcriptional control of a cis-regulatory sequence (e.g., promoter sequence). In another embodiment, the cis-regulatory sequence is suitable for directing constitutive expression of an engineered GLP-1/Glucagon receptor agonist disclosed herein. In another embodiment, the cis-regulatory sequence is suitable for directing tissue-specific expression of the engineered GLP-1/Glucagon receptor agonist peptides disclosed herein. In another embodiment, the cis-regulatory sequence is suitable for directing inducible expression of the engineered GLP-1/Glucagon receptor agonist variants disclosed herein.

In another embodiment, disclosed herein is a cell comprising the expression vector as described herein. In another embodiment, disclosed herein is a cell comprising an expression vector comprising a polynucleotide encoding a CTP-modified polypeptide comprising or consisting of a GLP-1/Glucagon receptor agonist and at least one CTPs attached to the amino or carboxy terminus of the agonist. In another embodiment, disclosed herein is a cell comprising an expression vector comprising a polynucleotide encoding a CTP-modified polypeptide consisting of a GLP-1/Glucagon receptor agonist and at least one CTPs attached to the amino or carboxy terminus of the agonist.

In another embodiment, disclosed herein is a composition comprising the expression vector as described herein. In another embodiment, disclosed herein is a composition comprising an expression vector comprising a polynucleotide encoding a CTP-modified polypeptide comprising or consisting of a GLP-1/Glucagon receptor agonist and at least one CTPs attached to the amino or carboxy terminus of the agonist. In another embodiment, disclosed herein is a cell comprising an expression vector comprising a polynucleotide encoding a CTP-modified polypeptide consisting of the agonist and at least one CTPs attached to the amino or carboxy terminus of the agonist. In another embodiment, the agonist is a polypeptide, or a peptide. In another embodiment, the peptide is OXM.

In another embodiment, disclosed herein is a composition comprising the cell as described herein. In another embodiment, the cell is a eukaryotic cell. In another embodiment, the cell is a prokaryotic cell.

In one embodiment, disclosed herein is a dual GLP-1/Glucagon receptor agonist. In another embodiment, disclosed herein is a recombinant OXM as described hereinabove. In one embodiment, disclosed herein is a n engineered OXM as described hereinabove. In one embodiment, the engineered OXM as described hereinabove is referred to as a CTP-modified OXM.

In one embodiment, the CTPs that are attached to the carboxy terminus of the OXM are attached in tandem to the carboxy terminus. In one embodiment, the CTPs that are attached to the amino terminus of the OXM are attached in tandem to the amino terminus.

In another embodiment, disclosed herein is a pharmaceutical composition comprising a CTP-modified polypeptide consisting of a GLP-1/Glucagon receptor agonist and three CTPs attached to the carboxy terminus of the OXM.

In another embodiment, disclosed herein is a pharmaceutical composition comprising a CTP-modified polypeptide consisting of an OXM and three CTPs attached to the carboxy terminus of the OXM.

In one embodiment, an engineered GLP-1/Glucagon receptor agonist as described herein has equivalent or improved biological activity compared to the non-CTP-modified GLP-1/Glucagon receptor agonist. In another embodiment, an engineered GLP-1/Glucagon receptor agonist as disclosed herein has equivalent or improved pharmacological measurements compared to the non-CTP-modified GLP-1/Glucagon receptor agonist. In another embodiment, an engineered GLP-1/Glucagon receptor agonist as disclosed herein has equivalent or improved pharmacokinetics compared to the non-CTP-modified GLP-1/Glucagon receptor agonist. In another embodiment, an engineered GLP-1/Glucagon receptor agonist as disclosed herein has equivalent or improved pharmacodynamics compared to the non-CTP-modified GLP-1/Glucagon receptor agonist.

A skilled artisan would appreciate that while particular embodiments of CTP-modified polypeptides have been disclosed above, a polypeptide or peptide of interest may encompass any polypeptide or peptide of interest having a functional activity. In another embodiment the polypeptide sequence-of-interest is insulin, is enkephalin, is an ACTH, is a glucagon, is an insulin-like growth factor, is an epidermal growth factor, is an acidic or basic fibroblast growth factor, is a platelet-derived growth factor, is a granulocyte-CSF, is a macrophage-CSF, is an IL-2, is an IL-3, is a tumor necrosis factor, is an LHRH, is an LHRH analog, is a somatostatin, is a growth hormone releasing factor, is an endorphin, is an alveolar surfactant protein, is a natriuretic factor, is an adhesin, is an angiostatin, is an endostatin,is a cytokine, is a receptor peptide, is a receptor binding ligand, is an antibody, is an antibody fragment, or is a peptide or a protein including any modified form.

In another embodiment, the peptide disclosed herein comprises a peptide of interest having additionally at least one CTP amino acid peptide on the N-terminus and one CTP amino acid peptide on the C-terminus. In another embodiment, the peptide of interest having additionally at least one CTP amino acid peptide on the N-terminus and one CTP amino acid peptide on the C-terminus comprises a protein selected from the following list: insulin, Albutein/albumin, Activase altiplase/tPA, adenosine deaminase, immune globulin, glucocerebrosidase, Leukine-sargramostim/GM-CSF, G-CSF, Venoglobulin-S/IgG, Proleukin aldesleukin, DNase, factor VIII, Helixate, L-asparaginase, WinRho SDF Rh I, Retavase retaplase/tPA, Factor IX, FSH, globulin, fibrin, interleukin-11, becaplermin/PDGF, lepirudin/herudin, TNF, Thymoglobulin, factor VIIa, interferon alpha-2a, interferon alfa n-1, interferon alfa-N3, interferon beta-1b, interferon garnma-1b, Interleukin-2, or monoclonal antibodies.

In another embodiment, the methods disclosed herein manufacture insulin having additionally at least one CTP amino acid peptide on the N-terminus and one CTP amino acid peptide on the C-terminus for the treatment of diabetes.

In another embodiment, the methods disclosed herein manufacture albumin having additionally at least one CTP amino acid peptide on the N-terminus and one CTP amino acid peptide on the C-terminus for the treatment of hypovolemic shock, hemodialysis or cardiopulmonary bypass.

In another embodiment, the methods disclosed herein manufacture Activase-altiplase/tPA having additionally at least one CTP amino acid peptide on the N-terminus and one CTP amino acid peptide on the C-terminus for the treatment of acute myocardial infarction, acute massive pulmonary embolism, or (change throughout) ischemic stroke.

In another embodiment, the methods disclosed herein manufacture adenosine deaminase having additionally at least one CTP amino acid peptide on the N-terminus and one CTP amino acid peptide on the C-terminus for the treatment of severe combined immunodeficiency disease.

In another embodiment, the methods disclosed herein manufacture immune globulin having additionally at least one CTP amino acid peptide on the N-terminus and one CTP amino acid peptide on the C-terminus for the treatment of transplant recipients.

In another embodiment, the methods disclosed herein manufacture immune globulin is a CMV immune globulin. In another embodiment, the methods disclosed herein manufacture glucocerebrosidase having additionally at least one CTP amino acid peptide on the N-terminus and one CTP amino acid peptide on the C-terminus for the treatment of Gaucher disease.

In another embodiment, the methods disclosed herein manufacture Leukine-sargramostim/GM-CSF having additionally at least one CTP amino acid peptide on the N-terminus and one CTP amino acid peptide on the C-terminus for the Stimulation of hematopoietic progenitor cells.

In another embodiment, the methods disclosed herein manufacture G-CSF having additionally at least one CTP amino acid peptide on the N-terminus and one CTP amino acid peptide on the C-terminus for the treatment of Neutropenia. In another embodiment, the methods disclosed herein manufacture Venoglobulin-S/IgG having additionally at least one CTP amino acid peptide on the N-terminus and one CTP amino acid peptide on the C-terminus for the treatment of Immunodeficiency diseases.

In another embodiment, the methods disclosed herein manufacture Proleukin-aldesleukin having additionally at least one CTP amino acid peptide on the N-terminus and one CTP amino acid peptide on the C-terminus for the treatment of renal carcinoma or metastatic melanoma.

In another embodiment, the methods disclosed herein manufacture DNase having additionally at least one CTP amino acid peptide on the N-terminus and one CTP amino acid peptide on the C-terminus for the treatment of Cystic fibrosis.

In another embodiment, the methods disclosed herein manufacture factor VIII having additionally at least one CTP amino acid peptide on the N-terminus and one CTP amino acid peptide on the C-terminus for the treatment of Hemophilia A.

In another embodiment, the methods disclosed herein manufacture Helixate having additionally at least one CTP amino acid peptide on the N-terminus and one CTP amino acid peptide on the C-terminus for the treatment of Hemophilia A.

In another embodiment, the methods disclosed herein manufacture L-asparaginase having additionally at least one CTP amino acid peptide on the N-terminus and one CTP amino acid peptide on the C-terminus for the treatment of acute lymphoblastic leukemia.

In another embodiment, the methods disclosed herein manufacture WinRho SDF Rh IgG having additionally at least one CTP amino acid peptide on the N-terminus and one CTP amino acid peptide on the C-terminus for the treatment of Rh isoimmunization and immune thrombocytopenic purpura.

In another embodiment, the methods disclosed herein manufacture Retavase retaplase/tPA having additionally at least one CTP amino acid peptide on the N-terminus and one CTP amino acid peptide on the C-terminus for the treatment of acute myocardial infarction.

In another embodiment, the methods disclosed herein manufacture FSH having additionally at least one CTP amino acid peptide on the N-terminus and at least one CTP amino acid peptide on the C-terminus for stimulation of ovulation during assisted reproduction.

In another embodiment, the methods disclosed herein manufacture globulin having additionally at least one CTP amino acid peptide on the N-terminus and at least one CTP amino acid peptide on the C-terminus for the prevention of respiratory syncytial virus disease.

In another embodiment, the methods disclosed herein manufacture fibrin having additionally at least one CTP amino acid peptide on the N-terminus and at least one CTP amino acid peptide on the C-terminus for wound management and hemostasis. In another embodiment, the methods disclosed herein manufacture interleukin-11 having additionally at least one CTP amino acid peptide on the N-terminus and at least one CTP amino acid peptide on the C-terminus for chemotherapy-induced thrombocytopenia.

In another embodiment, the methods disclosed herein manufacture becaplermin/PDGF having additionally at least one CTP amino acid peptide on the N-terminus and at least one CTP amino acid peptide on the C-terminus for the treatment of diabetic foot ulcers.

In another embodiment, the methods disclosed herein manufacture lepirudin/herudin having additionally at least one CTP amino acid peptide on the N-terminus and at least one CTP amino acid peptide on the C-terminus for anticoagulation in heparin-induced thrombocytopenia.

In another embodiment, the methods disclosed herein manufacture soluble TNF having additionally at least one CTP amino acid peptide on the N-terminus and at least one CTP amino acid peptide on the C-terminus for the treatment of rheumatoid arthritis.

In another embodiment, the methods disclosed herein manufacture Thymoglobulin having additionally at least one CTP amino acid peptide on the N-terminus and at least one CTP amino acid peptide on the C-terminus for the treatment of organ transplant rejection disease.

In another embodiment, the methods disclosed herein manufacture Interleukin-2 having additionally at least one CTP amino acid peptide on the N-terminus and at least one CTP amino acid peptide on the C-terminus for the treatment of renal carcinoma and metastatic melanoma.

In another embodiment, the methods disclosed herein manufacture an OKT3 monoclonal antibody having additionally at least one CTP amino acid peptide on the N-terminus and at least one CTP amino acid peptide on the C-terminus for organ transplant.

In another embodiment, the methods disclosed herein manufacture a Reo monoclonal antibody having additionally at least one CTP amino acid peptide on the N-terminus and at least one CTP amino acid peptide on the C-terminus for prevention of complications from coronary intervention and angioplasty.

In another embodiment, the methods disclosed herein manufacture a monoclonal antibody having additionally at least one CTP amino acid peptide on the N-terminus and at least one CTP amino acid peptide on the C-terminus for treating colorectal cancer, Non-Hodgkin's lymphoma, kidney transplant rejection, metastatic breast cancer, or the prevention of respiratory syncytial virus disease.

In some embodiments, the CTP sequences modification is advantageous in permitting lower dosages to be used. In some embodiments, modifications include, but are not limited to N terminus modification, C terminus modification, polypeptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992), which is incorporated by reference as if fully set forth herein. Further details in this respect are disclosed hereinunder.

In some embodiments, polypeptide bonds (-CO-NH-) within the polypeptide are substituted. In some embodiments, the polypeptide bonds are substituted by N-methylated bonds (-N(CH3)-CO-). In some embodiments, the polypeptide bonds are substituted by ester bonds (-C(R)H-C-O-O-C(R)-N-). In some embodiments, the polypeptide bonds are substituted by ketomethylen bonds (-CO-CH2-). In some embodiments, the polypeptide bonds are substituted by α-aza bonds (-NH-N(R)-CO-), wherein R is any alkyl, e.g., methyl, carba bonds (-CH2-NH-). In some embodiments, the polypeptide bonds are substituted by hydroxyethylene bonds (-CH(OH)-CH2-). In some embodiments, the polypeptide bonds are substituted by thioamide bonds (-CS-NH-). In some embodiments, the polypeptide bonds are substituted by olefinic double bonds (-CH=CH-). In some embodiments, the polypeptide bonds are substituted by retro amide bonds (-NH-CO-). In some embodiments, the polypeptide bonds are substituted by polypeptide derivatives (-N(R)-CH2-CO-), wherein R is the "normal" side chain, naturally presented on the carbon atom. In some embodiments, these modifications occur at any of the bonds along the polypeptide chain and even at several (2-3 bonds) at the same time.

In some embodiments, natural aromatic amino acids of the polypeptide such as Trp, Tyr and Phe, are substituted for synthetic non-natural acid such as Phenylglycine, TIC, naphthylelanine (Nol), ring-methylated derivatives of Phe, halogenated derivatives of Phe or o-methyl-Tyr. In some embodiments, the polypeptides disclosed herein include one or more modified amino acid or one or more non-amino acid monomers (e.g. fatty acid, complex carbohydrates etc).

In one embodiment, "amino acid" or "amino acid" is understood to include the 20 naturally occurring amino acid; those amino acid often modified post-translationally *in vivo,* including, for example, hydroxyproline, phosphoserine and phosphothreonine; and other unusual amino acid including, but not limited to, 2-aminoadipic acid, hydroxylysine, isodesmosine, norvaline, nor-leucine and ornithine. In one embodiment, "amino acid" includes both D- and L-amino acid.

In some embodiments, the polypeptides disclosed herein are utilized in therapeutics which require the polypeptides to be in a soluble form. In some embodiments, the polypeptides disclosed herein include one or more non-natural or natural polar amino acid, including but not limited to serine and threonine which are capable of increasing polypeptide solubility due to their hydroxyl-containing side chain.

In some embodiments, the polypeptides disclosed herein is are utilized in a linear form, although it will be appreciated by one skilled in the art that in cases where cyclicization does not severely interfere with polypeptide characteristics, cyclic forms of the polypeptide can also be utilized.

In some embodiments, the polypeptides disclosed herein are biochemically synthesized such as by using standard solid phase techniques. In some embodiments, these biochemical methods include exclusive solid phase synthesis, partial solid phase synthesis, fragment condensation, or classical solution synthesis. In some embodiments, these methods are used when the polypeptide is relatively short (about 5-15kDa) and/or when it cannot be produced by recombinant techniques (i.e., not encoded by a nucleic acid sequence) and therefore involves different chemistry.

In some embodiments, solid phase polypeptide synthesis procedures are well known to one skilled in the art and further described by John Morrow Stewart and Janis Dillaha Young, Solid Phase Polypeptide Syntheses (2nd Ed., Pierce Chemical Company, 1984). In some embodiments, synthetic polypeptides are purified by preparative high performance liquid chromatography [Creighton T. (1983) Proteins, structures and molecular principles. WH Freeman and Co. N.Y.] and the composition of which can be confirmed via amino acid sequencing by methods known to one skilled in the art.

In some embodiments, recombinant protein techniques are used to generate the polypeptides disclosed herein. In some embodiments, recombinant protein techniques are used for generation of relatively long polypeptides (e.g., longer than 18-25 amino acid). In some embodiments, recombinant protein techniques are used for the generation of large amounts of the polypeptide disclosed herein. In some embodiments, recombinant techniques are described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

In one embodiment, a polypeptide disclosed herein is synthesized using a polynucleotide encoding a polypeptide disclosed herein. In some embodiments, the polynucleotide encoding a polypeptide disclosed herein is ligated into an expression vector, comprising a transcriptional control of a cis-regulatory sequence (e.g., promoter sequence). In some embodiments, the cis-regulatory sequence is suitable for directing constitutive expression of the polypeptide disclosed herein. In some embodiments, the cis-regulatory sequence is suitable for directing tissue specific expression of the polypeptide disclosed herein. In some embodiments, the cis-regulatory sequence is suitable for directing inducible expression of the polypeptide disclosed herein.

In some embodiments, the polypeptides of interest disclosed herein are utilized in a linear form, although it will be appreciated by one skilled in the art that in cases where cyclicization does not severely interfere with cytokines characteristics, cyclic forms of the cytokines can also be utilized.

In some embodiments, the polypeptide of interests disclosed herein is biochemically synthesized such as by using standard solid phase techniques. In some embodiments, these biochemical methods include exclusive solid phase synthesis, partial solid phase synthesis, fragment condensation, or classical solution synthesis. In some embodiments, these methods are used when the polypeptide of interests are relatively short (about 5-15kDa) and/or when it cannot be produced by recombinant techniques (i.e., not encoded by a nucleic acid sequence) and therefore involves different chemistry.

In some embodiments, solid phase polypeptide of interests synthesis procedures are well known to one skilled in the art and further described by John Morrow Stewart and Janis Dillaha Young, Solid Phase Polypeptide Syntheses (2nd Ed., Pierce Chemical Company, 1984). In some embodiments, synthetic polypeptides are purified by preparative high performance liquid chromatography [Creighton T. (1983) Proteins, structures and molecular principles. WH Freeman and Co. N.Y.] and the composition of which can be confirmed via amino acid sequencing by methods known to one skilled in the art.

In some embodiments, recombinant protein techniques are used to generate the polypeptide of interests disclosed herein. In some embodiments, recombinant protein techniques are used for generation of relatively long polypeptides (e.g., longer than 18-25 amino acid). In some embodiments, recombinant protein techniques are used for the generation of large amounts of the polypeptide of interests disclosed herein. In some embodiments, recombinant techniques are described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al., (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

In another embodiment, polypeptides of interests disclosed herein are synthesized using a polynucleotide encoding a polypeptide disclosed herein. In some embodiments, the polynucleotide encoding polypeptide of interests disclosed herein is ligated into an expression vector, comprising a transcriptional control of a cis-regulatory sequence (e.g., promoter sequence). In some embodiments, the cis-regulatory sequence is suitable for directing constitutive expression of the polypeptide of interests disclosed herein. In some embodiments, the cis-regulatory sequence is suitable for directing tissue specific expression of the polypeptide of interests disclosed herein. In some embodiments, the cis-regulatory sequence is suitable for directing inducible expression of the polypeptide of interests disclosed herein.

In some embodiment, tissue-specific promoters suitable for use with a nucleotide sequence disclosed herein include sequences which are functional in specific cell population, example include, but are not limited to promoters such as albumin that is liver specific [Pinkert et al., (1987) Genes Dev. 1:268-277], lymphoid specific promoters [Calame et al., (1988) Adv. Immunol. 43:235-275]; in particular promoters of T-cell receptors [Winoto et al., (1989) EMBO J. 8:729-733] and immunoglobulins; [Banerji et al. (1983) Cell 33729-740], neuron-specific promoters such as the neurofilament promoter [Byrne et al. (1989) Proc. Natl. Acad. Sci. USA 86:5473-5477], pancreas-specific promoters [Edlunch et al. (1985) Science 230:912-916] or mammary gland-specific promoters such as the milk whey promoter (U.S. Pat. No. 4,873,316 and European Application Publication No. 264,166). Inducible promoters suitable for use with a nucleotide sequence disclosed herein include for example the tetracycline-inducible promoter (Srour, M.A., et al., 2003. Thromb. Haemost. 90: 398-405).

A skilled artisan would appreciate that the phrase "a polynucleotide" may encmpass a single or double stranded nucleic acid sequence which be isolated and provided in the form of an RNA sequence, a complementary polynucleotide sequence (cDNA), a genomic polynucleotide sequence and/or a composite polynucleotide sequences (e.g., a combination of the above).

A skilled artisan would appreciate that the term "complementary polynucleotide sequence" may encompass a sequence, which results from reverse transcription of messenger RNA using a reverse transcriptase or any other RNA dependent DNA polymerase. In one embodiment, the sequence can be subsequently amplified *in vivo* or *in vitro* using a DNA polymerase.

A skilled artisan would appreciate that the term "genomic polynucleotide sequence" may encompass a sequence derived (isolated) from a chromosome and thus it represents a contiguous portion of a chromosome.

A skilled artisan would appreciate that the term "composite polynucleotide sequence" may encompass a sequence, which is at least partially complementary and at least partially genomic. In one embodiment, a composite sequence can include some exonal sequences required to encode the polypeptide disclosed herein, as well as some intronic sequences interposing there between. In one embodiment, the intronic sequences can be of any source, including of other genes, and typically will include conserved splicing signal sequences. In one embodiment, intronic sequences include cis acting expression regulatory elements.

In some embodiments, polynucleotides disclosed herein are prepared using PCR techniques or any other technique known to one skilled in the art. In some embodiments, the procedure involves the ligation of two different DNA sequences (See, for example, "Current Protocols in Molecular Biology", eds. Ausubel et al., John Wiley & Sons, 1992).

In one embodiment, the polypeptides disclosed herein can be provided to the individual *per se.* In one embodiment, the polypeptides disclosed herein can be provided to the individual as part of a pharmaceutical composition where it is mixed with a pharmaceutically acceptable carrier.

In one embodiment, the oral dosage form comprises predefined release profile. In one embodiment, the oral dosage form disclosed herein comprises an extended release tablets, capsules, lozenges or chewable tablets. In one embodiment, the oral dosage form disclosed herein comprises a slow release tablets, capsules, lozenges or chewable tablets. In one embodiment, the oral dosage form disclosed herein comprises an immediate release tablets, capsules, lozenges or chewable tablets. In one embodiment, the oral dosage form is formulated according to the desired release profile of the pharmaceutical active ingredient as known to one skilled in the art.

Peroral compositions, in some embodiments, comprise liquid solutions, emulsions, suspensions, and the like. In some embodiments, pharmaceutically-acceptable carriers suitable for preparation of such compositions are well known in the art. In some embodiments, liquid oral compositions comprise from about 0.012% to about 0.933% of the desired compound or compounds, or in another embodiment, from about 0.033% to about 0.7%.

In some embodiments, compositions for use in the methods disclosed herein comprise solutions or emulsions, which in some embodiments are aqueous solutions or emulsions comprising a safe and effective amount of the compounds disclosed herein and optionally, other compounds, intended for topical intranasal administration. In some embodiments, h compositions comprise from about 0.01% to about 10.0% w/v of a subject compound, more preferably from about 0.1% to about 2.0, which is used for systemic delivery of the compounds by the intranasal route.

In another embodiment, the pharmaceutical compositions are administered by intravenous, intra-arterial, or intramuscular injection of a liquid preparation. In some embodiments, liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are administered intra-arterially, and are thus formulated in a form suitable for intra-arterial administration. In another embodiment, the pharmaceutical compositions are administered intramuscularly, and are thus formulated in a form suitable for intramuscular administration.

Further, in another embodiment, the pharmaceutical compositions are administered topically to body surfaces, and are thus formulated in a form suitable for topical administration. Suitable topical formulations include gels, ointments, creams, lotions, drops and the like. For topical administration, the compounds disclosed herein are combined with an additional appropriate therapeutic agent or agents, prepared and applied as solutions, suspensions, or emulsions in a physiologically acceptable diluent with or without a pharmaceutical carrier.

In one embodiment, pharmaceutical compositions disclosed herein are manufactured by processes well known in the art, e.g., by means of conventional mixing, dissolving, granulating, dragee-making, levigating, emulsifying, encapsulating, entrapping or lyophilizing processes.

In one embodiment, pharmaceutical compositions for use in accordance with a nucleotide sequence disclosed herein is formulated in conventional manner using one or more physiologically acceptable carriers comprising excipients and auxiliaries, which facilitate processing of the active ingredients into preparations which, can be used pharmaceutically. In one embodiment, formulation is dependent upon the route of administration chosen.

In one embodiment, injectables, disclosed herein are formulated in aqueous solutions. In one embodiment, injectables, disclosed herein are formulated in physiologically compatible buffers such as Hank's solution, Ringer's solution, or physiological salt buffer. In some embodiments, for transmucosal administration, penetrants appropriate to the barrier to be permeated are used in the formulation. Such penetrants are generally known in the art.

In one embodiment, the preparations described herein are formulated for parenteral administration, e.g., by bolus injection or continuous infusion. In some embodiments, formulations for injection are presented in unit dosage form, e.g., in ampoules or in multidose containers with optionally, an added preservative. In some embodiments, compositions are suspensions, solutions or emulsions in oily or aqueous vehicles, and contain formulatory agents such as suspending, stabilizing and/or dispersing agents.

The compositions also comprise, in some embodiments, preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjustors, such as polymers, including cellulose and derivatives thereof; and polyvinyl alcohol and acid and bases to adjust the pH of these aqueous compositions as needed. The compositions also comprise, in some embodiments, local anesthetics or other actives. The compositions can be used as sprays, mists, drops, and the like.

In some embodiments, pharmaceutical compositions for parenteral administration include aqueous solutions of the active preparation in water-soluble form. Additionally, suspensions of the active ingredients, in some embodiments, are prepared as appropriate oily or water based injection suspensions. Suitable lipophilic solvents or vehicles include, in some embodiments, fatty oils such as sesame oil, or synthetic fatty acid esters such as ethyl oleate, triglycerides or liposomes. Aqueous injection suspensions contain, in some embodiments, substances, which increase the viscosity of the suspension, such as sodium carboxymethyl cellulose, sorbitol or dextran. In another embodiment,, the suspension also contain suitable stabilizers or agents which increase the solubility of the active ingredients to allow for the preparation of highly concentrated solutions.

In another embodiment, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

In another embodiment, the pharmaceutical composition delivered in a controlled release system is formulated for intravenous infusion, implantable osmotic pump, transdermal patch, liposomes, or other modes of administration. In one embodiment, a pump is used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

In some embodiments, the active ingredient is in powder form for constitution with a suitable vehicle, e.g., sterile, pyrogen-free water based solution, before use. Compositions are formulated, in some embodiments, for atomization and inhalation administration. In another embodiment, compositions are contained in a container with attached atomizing means.

In one embodiment, the preparation disclosed herein is formulated in rectal compositions such as suppositories or retention enemas, using, e.g., conventional suppository bases such as cocoa butter or other glycerides.

In some embodiments, pharmaceutical compositions suitable for use in context disclosed herein include compositions wherein the active ingredients are contained in an amount effective to achieve the intended purpose. In some embodiments, a therapeutically effective amount means an amount of active ingredients effective to prevent, alleviate or ameliorate symptoms of disease or prolong the survival of the subject being treated.

In one embodiment, determination of a therapeutically effective amount is well within the capability of those skilled in the art.

The compositions also comprise preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfote and others; aromatic agents; viscosity adjustors, such as polymers, including cellulose and derivatives thereof; and polyvinyl alcohol and acid and bases to adjust the pH of these aqueous compositions as needed. The compositions also comprise local anesthetics or other actives. The compositions can be used as sprays, mists, drops, and the like.

Some examples of substances which can serve as pharmaceutically-acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the Tween^{™} brand emulsifiers; wetting agents, such sodium lauryl sulfate; coloring agents; flavoring agents; tableting agents, stabilizers; antioxidants; preservatives; pyrogen-free water; isotonic saline; and phosphate buffer solutions. The choice of a pharmaceutically-acceptable carrier to be used in conjunction with the compound is basically determined by the way the compound is to be administered. If the subject compound is to be injected, in one embodiment, the pharmaceutically-acceptable carrier is sterile, physiological saline, with a blood-compatible suspending agent, the pH of which has been adjusted to about 7.4.

In addition, the compositions further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hyroxypropylmethyl cellulose), viscosity increasing agents(e.g. carbomer, colloidal silicon dioxide, ethyl cellulose, guar gum), sweeteners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, cellulose (e.g. Avicel^{™}, RC-591), tragacanth and sodium alginate; typical wetting agents include lecithin and polyethylene oxide sorbitan (e.g. polysorbate 80). Typical preservatives include methyl paraben and sodium benzoate. In another embodiment, peroral liquid compositions also contain one or more components such as sweeteners, flavoring agents and colorants disclosed above.

The compositions also include incorporation of the active material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions will influence the physical state, solubility, stability, rate of *in vivo* release, and rate of *in vivo* clearance.

Also comprehended herein are particulate compositions coated with polymers (e.g. poloxamers or poloxamines) and the compound coupled to antibodies directed against tissue-specific receptors, ligands or antigens or coupled to ligands of tissue-specific receptors.

In some embodiments, compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. In another embodiment, the modified compounds exhibit substantially longer half-lives in blood following intravenous injection than do the corresponding unmodified compounds. In one embodiment, modifications also increase the compound's solubility in aqueous solution, eliminate aggregation, enhance the physical and chemical stability of the compound, and greatly reduce the immunogenicity and reactivity of the compound. In another embodiment, the desired *in vivo* biological activity is achieved by the administration of such polymer-compound abducts less frequently or in lower doses than with the unmodified compound.

In some embodiments, preparation of effective amount or dose can be estimated initially from in vitro assays. In one embodiment, a dose can be formulated in animal models and such information can be used to more accurately determine useful doses in humans.

In one embodiment, toxicity and therapeutic efficacy of the active ingredients described herein can be determined by standard pharmaceutical procedures *in vitro,* in cell cultures or experimental animals. In one embodiment, the data obtained from these *in vitro* and cell culture assays and animal studies can be used in formulating a range of dosage for use in human. In one embodiment, the dosages vary depending upon the dosage form employed and the route of administration utilized. In one embodiment, the exact formulation, route of administration and dosage can be chosen by the individual physician in view of the patient's condition. [See e.g., Fingl, et al., (1975) "The Pharmacological Basis of Therapeutics", Ch. 1 p.1].

In one embodiment, depending on the severity and responsiveness of the condition to be treated, dosing can be of a single or a plurality of administrations, with course of treatment lasting from several days to several weeks or until cure is effected or diminution of the disease state is achieved.

In one embodiment, the amount of a composition to be administered will, of course, be dependent on the subject being treated, the severity of the affliction, the manner of administration, the judgment of the prescribing physician, etc.

In one embodiment, compositions including the preparation disclosed herein formulated in a compatible pharmaceutical carrier are also be prepared, placed in an appropriate container, and labeled for treatment of an indicated condition.

In one embodiment, compositions disclosed herein are presented in a pack or dispenser device, such as an FDA approved kit, which contain one or more unit dosage forms containing the active ingredient. In one embodiment, the pack, for example, comprise metal or plastic foil, such as a blister pack. In one embodiment, the pack or dispenser device is accompanied by instructions for administration. In one embodiment, the pack or dispenser is accommodated by a notice associated with the container in a form prescribed by a governmental agency regulating the manufacture, use or sale of pharmaceuticals, which notice is reflective of approval by the agency of the form of the compositions or human or veterinary administration. Such notice, in one embodiment, is labeling approved by the U.S. Food and Drug Administration for prescription drugs or of an approved product insert.

In one embodiment, it will be appreciated that the polypeptides disclosed herein can be provided to the individual with additional active agents to achieve an improved therapeutic effect as compared to treatment with each agent by itself. In another embodiment, measures (e.g., dosing and selection of the complementary agent) are taken to adverse side effects which are associated with combination therapies.

In some embodiments, "polypeptide" as used herein encompasses native polypeptides (either degradation products, synthetically synthesized polypeptides or recombinant polypeptides) and peptidomimetics (typically, synthetically synthesized polypeptides), as well as peptoids and semipeptoids which are polypeptide analogs, which have, in some embodiments, modifications rendering the polypeptides comprising a polypeptide of interest even more stable while in a body or more capable of penetrating into cells.

### Manufacturing

In one embodiment, disclosed herein is a method of manufacturing a human chorionic gonadotropin peptide (CTP)-modified polypeptide of interest polypeptide, the method comprising the steps of: (a) stably transfecting a predetermined number of cells with an expression vector comprising a coding portion encoding said CTP-modified polypeptide of interest, wherein said transfected cell expresses and optionally secretes said CTP-modified polypeptide of interest; (b) obtaining cell clones that overexpress said CTP-modified polypeptide of interest; (c) expanding said clones in solution to a predetermined scale; (d) harvesting said solution containing said clones; (e) filtering said solution containing said clones to obtain a clarified harvest solution; and, (f) purifying said clarified harvest solution to obtain a purified protein solution having a desired concentration of a CTP-modified polypeptide of interest, thereby manufacturing a human chorionic gonadotropin peptide (CTP)-modified polypeptide of interest polypeptide. In another embodiment, the CTP-modified polypeptide of interest is secreted. In another embodiment, the CTP-modified polypeptide of interest is not secreted. In the manufacturing of CTP-modified polypeptide of interest transfection is an early step. Once the final high expressing clone is selected, each production includes thawing of a working cell bank (WCB), expansion, harvest and purification. (See Example 12, steps 1-7 describing clone expansion through harvest; steps 8-16 describing purification)

In one embodiment, polynucleotides disclosed herein are inserted into expression vectors (i.e., a nucleic acid construct) to enable expression of the recombinant polypeptide. In one embodiment, the expression vector disclosed herein includes additional sequences which render this vector suitable for replication and integration in prokaryotes. In one embodiment, the expression vector disclosed herein includes additional sequences which render this vector suitable for replication and integration in eukaryotes. In one embodiment, the expression vector disclosed herein includes a shuttle vector which renders this vector suitable for replication and integration in both prokaryotes and eukaryotes. In some embodiments, cloning vectors comprise transcription and translation initiation sequences (e.g., promoters, enhancer) and transcription and translation terminators (e.g., polyadenylation signals).

In one embodiment, a method of manufacture of a CTP-modified polypeptide, for example, a CTP-modified polypeptide of interest, comprises a step comprising the use of an expression vector, wherein said expression vector comprises a promoter, a coding sequence for a CTP-modified polypeptide, and a polyadenylation sequence. In another embodiment, the polypeptide is polypeptide of interest. In another embodiment the polyadenylation sequence is a simian virus (SV) 40 polyadenylation sequence.

In one embodiment, a variety of prokaryotic or eukaryotic cells can be used as host-expression systems to express the polypeptides disclosed herein. In some embodiments, these include, but are not limited to, microorganisms, such as bacteria transformed with a recombinant bacteriophage DNA, plasmid DNA or cosmid DNA expression vector containing the polypeptide coding sequence; yeast transformed with recombinant yeast expression vectors containing the polypeptide coding sequence; plant cell systems infected with recombinant virus expression vectors (e.g., cauliflower mosaic virus, CaMV; tobacco mosaic virus, TMV) or transformed with recombinant plasmid expression vectors, such as Ti plasmid, containing the polypeptide coding sequence.

In one embodiment, non-bacterial expression systems are used (e.g. mammalian expression systems such as CHO cells or cells derived from CHO cells) to express the polypeptide disclosed herein. In one embodiment, the expression vector used to express polynucleotides disclosed herein in mammalian cells is pCI-DHFR vector comprising a CMV promoter and a neomycin resistance gene.

In one embodiment, the expression vector disclosed herein can further include additional polynucleotide sequences that allow, for example, the translation of several proteins from a single mRNA such as an internal ribosome entry site (IRES) and sequences for genomic integration of the promoter-chimeric polypeptide.

In some embodiments, mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

In some embodiments, expression vectors containing regulatory elements from eukaryotic viruses such as retroviruses are used by methods disclosed herein. SV40 vectors include pSVT7 and pMT2. In some embodiments, vectors derived from bovine papilloma virus include pBV-1MTHA, and vectors derived from Epstein Bar virus include pHEBO, and p2O5. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

In one embodiment, various methods can be used to introduce the expression vector encoding the CTP-modified polypeptide of interest disclosed herein into cells. "Transfection" of eukaryotic host cells with a polynucleotide or expression vector, resulting in genetically modified cells or transgenic cells, can be performed by any method well known in the art and described, e.g., Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, and electroporation. In addition, see U.S. Patent Nos. 5,464,764 and 5,487,992 for positive-negative selection methods. Transfection methods further include but are not limited to liposome-mediated transfection, calcium phosphate co-precipitation, electroporation, polycation (such as DEAE-dextran)-mediated transfection, protoplast fusion, viral infections (including recombinant viral infections) and microinjection. Preferably, the transfection is a stable transfection. The transfection method that provides optimal transfection frequency and expression of the heterologous genes encoding the peptide of interest disclosed herein in the particular host cell line and type is favored. Suitable methods can be determined by routine procedures. For stable transfectants the constructs are either integrated into the host cell's genome or an artificial chromosome/mini-chromosome or located episomally so as to be stably maintained within the host cell.

The practice disclosed herein will employ, unless otherwise indicated, conventional techniques of cell biology, molecular biology, cell culture, immunology and the like which are in the skill of one in the art. These techniques are fully disclosed in the current literature. See e.g. Sambrook et al., Molecular Cloning: A Laboratory Manual, 2nd Ed., Cold Spring Harbor Laboratory Press, Cold Spring Harbor, N.Y. (1989); Ausubel et al., Current Protocols in Molecular Biology (1987, updated); Brown ed., Essential Molecular Biology, IRL Press (1991); Goeddel ed., Gene Expression Technology, Academic Press (1991); Bothwell et al. eds., Methods for Cloning and Analysis of Eukaryotic Genes, Bartlett Publ. (1990); Wu et al., eds., Recombinant DNA Methodology, Academic Press (1989); Kriegler, Gene Transfer and Expression, Stockton Press (1990); McPherson et al., PCR: A Practical Approach, IRL Press at Oxford University Press (1991); Gait ed., Oligonucleotide Synthesis (1984); Miller & Calos eds., Gene Transfer Vectors for Mammalian Cells (1987); Butler ed., Mammalian Cell Biotechnology (1991); Pollard et al., eds., Animal Cell Culture, Humana Press (1990); Freshney et al., eds., Culture of Animal Cells, Alan R. Liss (1987); Studzinski, ed., Cell Growth and Apoptosis, A Practical Approach, IRL Press at Oxford University Press (1995); Melamed et al., eds., Flow Cytometry and Sorting, Wiley-Liss (1990); Current Protocols in Cytometry, John Wiley & Sons, Inc. (updated); Wirth & Hauser, Genetic Engineering of Animals Cells, in: Biotechnology Vol. 2, Pühler ed., VCH, Weinheim 663-744; the series Methods of Enzymology (Academic Press, Inc.), and Harlow et al., eds., Antibodies: A Laboratory Manual (1987).

A heterologous gene of interest encoding the CTP-modified polypeptide of interest may be introduced into the cell disclosed herein by various methods, for example by viral transformation, transfection or microinjection. The heterologous gene of interest may be introduced into the cell as linear DNA or as part of an expression vector. A number of eukaryotic expression vectors are known which allow multiple cloning sites for the insertion of one or more heterologous genes and their expression. Commercial suppliers include among others companies such as Stratagene, La Jolla, Calif., USA; Invitrogen, Carlsbad, Calif., USA; Promega, Madison, Wis., USA or BD Biosciences Clontech, Palo Alto, Calif., USA. The transfection of the cells with a DNA or an expression vector which code(s) for one or more genes of interest is carried out by conventional methods as described for example in Sambrook et al., 1989 or Ausubel et al., 1994. Suitable methods of transfection include for example liposome-mediated transfection, calcium phosphate co-precipitation, electroporation, polycation- (e.g. DEAE dextran)-mediated transfection, protoplast fusion, microinjection and viral infections. Preferably, stable transfection is carried out in which the DNA molecules are either integrated into the genome of the host cell or an artificial chromosome/minichromosome, or are episomally contained in stable manner in the host cell. The transfection method which gives the optimum transfection frequency and expression of one or more heterologous genes of interest in the host cell in question is preferred.

In some embodiments, introduction of nucleic acid by viral infection offers several advantages over other methods such as lipofection and electroporation, since higher transfection efficiency can be obtained due to the infectious nature of viruses.

In one embodiment, it will be appreciated that the polypeptides disclosed herein can also be expressed from a nucleic acid construct administered to the individual employing any suitable mode of administration, described hereinabove (i.e., in-vivo gene therapy). In one embodiment, the nucleic acid construct is introduced into a suitable cell via an appropriate gene delivery vehicle/method (transfection, transduction, homologous recombination, etc.) and an expression system as needed and then the modified cells are expanded in culture and returned to the individual (i.e., ex-vivo gene therapy).

The heterologous gene of interest is usually functionally linked to a promoter which enables the transcription of the gene of interest, and to other regulatory elements which allow transcription and translation (expression) of the gene of interest or increase its efficiency.

A skilled artisan would appreciate that the term "promoter" may encompass a polynucleotide sequence which enables and controls transcription of the genes or sequences functionally linked to it. A promoter contains recognition sequences for binding RNA polymerase and the initiation site for transcription (transcription initiation site). In order to express a desired sequence in a certain cell type or a host cell a suitable functional promoter must be chosen. The skilled man will be familiar with a variety of promoters from various sources, including constitutive, inducible and repressible promoters. They are deposited in databanks such as GenBank, for example, and may be obtained as separate elements or elements cloned within polynucleotide sequences from commercial or individual sources. In inducible promoters the activity of the promoter may be reduced or increased in response to a signal. One example of an inducible promoter is the tetracycline (tet) promoter. This contains tetracycline operator sequences (tetO) which can be induced by a tetracycline-regulated transactivator protein (tTA). In the presence of tetracycline the binding of tTA to tetO is inhibited. Examples of other inducible promoters are the jun, fos, metallothionein and heat shock promoter (see also Sambrook, J., Fritsch, E. F. & Maniatis, T., Molecular Cloning: A Laboratory Manual Cold Spring Harbor Laboratory, Cold Spring Harbor, N.Y., 1989; Gossen, M. et al., Curr Opi Biotech 1994, 5, 516-520). Of the promoters which are particularly suitable for high expression in eukaryotes, there are for example the ubiquitin/S27a promoter of the hamster (WO 97/15664), SV 40 early promoter, adenovirus major late promoter, mouse metallothionein-1 promoter, the long terminal repeat region of Rous Sarcoma Virus and the early promoter of human Cytomegalovirus. Examples of other heterologous mammalian promoters are the actin, immunoglobulin or heat shock promoter(s).

For example, the promoter may be functionally linked to enhancer sequences in order to increase the transcriptional activity. For this, one or more enhancers and/or several copies of an enhancer sequence may be used, e.g. a CMV or SV40 enhancer. For example, the promoter may be functionally linked to enhancer sequences in order to increase the transcriptional activity. For this, one or more enhancers and/or several copies of an enhancer sequence may be used, e.g. a CMV or SV40 enhancer.

A skilled artisan would appreciate that the term enhancer may encompass a polynucleotide sequence which in the cis location acts on the activity of a promoter and thus stimulates the transcription of a gene functionally connected to this promoter. Unlike promoters the effect of enhancers is independent of position and orientation and they can therefore be positioned in front of or behind a transcription unit, within an intron or even within the coding region. The enhancer may be located both in the immediate vicinity of the transcription unit and at a considerable distance from the promoter. It is also possible to have a physical and functional overlap with the promoter. The skilled artisan will be aware of a number of enhancers from various sources (and deposited in databanks such as GenBank, e.g. SV40 enhancers, CMV enhancers, polyoma enhancers, adenovirus enhancers) which are available as independent elements or elements cloned within polynucleotide sequences (e.g. deposited at the ATCC or from commercial and individual sources). A number of promoter sequences also contain enhancer sequences such as the frequently used CMV promoter. The human CMV enhancer is one of the strongest enhancers identified hitherto. One example of an inducible enhancer is the metallothionein enhancer, which can be stimulated by glucocorticoids or heavy metals.

Basically, the regulatory elements include promoters, enhancers, termination and polyadenylation signals and other expression control elements. Both inducible and constitutively regulatory sequences are known for the various cell types. "Transcription-regulatory elements" generally comprise a promoter upstream of the gene sequence to be expressed, transcription initiation and termination sites and a polyadenylation signal.

A skilled artisan would appreciate that the term "transcription initiation site" may encompass a nucleic acid in the construct which corresponds to the first nucleic acid which is incorporated in the primary transcript, i.e. the mRNA precursor. The transcription initiation site may overlap with the promoter sequences.

A skilled artisan would appreciate that the term "transcription termination site" may encompass a nucleotide sequence which is normally at the 3' end of the gene of interest or of the gene section which is to be transcribed, and which brings about the termination of transcription by RNA polymerase.

A skilled artisan would appreciate that the term "polyadenylation signal" may encompass a signal sequence which causes cleavage at a specific site at the 3' end of the eukaryotic mRNA and posttranscriptional incorporation of a sequence of about 100-200 adenine nucleotides (polyA tail) at the cleaved 3'-end. The polyadenylation signal comprises the sequence AATAAA about 10-30 nucleotides upstream of the cleavage site and a sequence located downstream. Various polyadenylation elements are known such as tk polyA, SV40 late and early polyA or BGH polyA (described for example in U.S. Pat. No. 5,122,458).

"Translation regulatory elements" comprise a translation initiation site (AUG), a stop codon and a polyA signal for each polypeptide to be expressed. For optimum expression it may be advisable to remove, add or change 5'- and/or 3'-untranslated regions of the nucleic acid sequence which is to be expressed, in order to eliminate any potentially unsuitable additional translation initiation codons or other sequences which might affect expression at the transcription or expression level. In order to promote expression, ribosomal consensus binding sites may alternatively be inserted immediately upstream of the start codon. In order to produce a secreted polypeptide the gene of interest usually contains a signal sequence which codes for a signal precursor peptide which transports the synthesized polypeptide to and through the ER membrane. The signal sequence is often but not always located at the amino terminus of the secreted protein and is cleaved by signal peptidases after the protein has been filtered through the ER membrane. The gene sequence will usually but not necessarily contain its own signal sequence. If the native signal sequence is not present a heterologous signal sequence may be introduced in known manner. Numerous signal sequences of this kind are known to the skilled artisan and deposited in sequence databanks such as GenBank and EMBL.

A skilled artisan would appreciate that the terms "polypeptides", "polypeptide" or grammatical equivalents thereof, may be used interchangeably to encompass amino acid sequences or proteins and may encompass polymers of amino acids of any length. This term also includes proteins which have been modified post-translationally by reactions such as glycosylation, phosphorylation, acetylation or protein processing. The structure of the polypeptide may be modified, for example, by substitutions, deletions or insertions of amino acids and fusion with other proteins while retaining its biological activity.

In order to produce one or more gene products of interest in the cells, the cells may be grown in a serum-free culture medium and in suspension culture under conditions which allow expression of the gene of interest. If for example the gene of interest is under the control of a constitutive promoter, there is no need to add special inducers. If the expression of the gene of interest is under the control of an inducible promoter, for example, a corresponding inducer must be added to the cell culture medium in a sufficient but non-toxic concentration. The cells can be expanded as desired by multiple subpassaging and transferred into suitable cell culture vessels. The gene product(s) is or are produced as either a cellular, membrane-bound or secretory product.

In one embodiment, a step of manufacturing a CTP-modified polypeptide of interest comprises stably transfecting a predetermined number of cells with an expression vector comprising a coding portion encoding said CTP-modified polypeptide of interest. In another embodiment, a step of manufacturing a CTP-modified polypeptide of interest comprises stably transfecting cells with an expression vector comprising a coding portion encoding said CTP-modified polypeptide of interest. In one embodiment, cells are CHO cells. In another embodiment, cells are DG44 cells. In another embodiment, cells are any cells known in the art suitable for expression and secretion of CTP-modified polypeptide of interest.

In another embodiment, the transfected cells express CTP-modified polypeptide of interest. In another embodiment, the CTP-modified polypeptide of interest being manufactured and expressed consists of two CTP attached to the carboxy terminus of said polypeptide of interest, and one chorionic gonadotropin carboxy terminal peptide attached to the amino terminus of said polypeptide of interest. In another embodiment, the CTP-modified polypeptide of interest being manufactured and expressed consists of one chorionic gonadotropin carboxy terminal peptide attached to the carboxy terminus of said polypeptide of interest. In other embodiment, the expression of CTP-modified polypeptide of interest is at a high expression level. In another embodiment, said CTP-modified polypeptide of interest is highly glycosylated. In another embodiment, said CTP-modified polypeptide of interest is highly sialated. As described above in detail, CTP-modified polypeptide of interest may have different glycosylation content and patterns. A CTP-modified polypeptide of interest manufactured by the methods disclosed herein may include any of the glycosylation patterns and content as disclosed above. In general, method of manufacture presented here, provide a CTP-modified polypeptide of interest having a high glycosylation content and a high percentage of glycosylation sites glycosylated.

In one embodiment, a step of manufacturing a CTP-modified polypeptide of interest comprises obtaining cell clones that overexpress the CTP-modified polypeptide of interest. In another embodiment, expression of CTP-modified polypeptide of interest is optimal. In another embodiment, the level of expression is between 30-1500 mg/L. In another embodiment, the level of expression is at least 30 mg/L. In another embodiment, the level of expression is at least 40 mg/L. In another embodiment, the level of expression is at least 50 mg/L.. In another embodiment, the level of expression is at least 60 mg/L. In another embodiment, the level of expression is at least 70 mg/L. In another embodiment, the level of expression is between 50-70 mg/L. In another embodiment, the level of expression is at least 200 mg/L. In another embodiment, the level of expression is at least 300 mg/L. In another embodiment, the level of expression is at least 400 mg/L. In another embodiment, the level of expression is at least 500 mg/L. In another embodiment, the level of expression is at least 600 mg/L. In another embodiment, the level of expression is at least 700 mg/L. In another embodiment, the level of expression is at least 800 mg/L. In another embodiment, the level of expression is at least 900 mg/L. In another embodiment, the level of expression is at least 1000 mg/L. In another embodiment, the level of expression is at least 1100 mg/L. In another embodiment, the level of expression is at least 1200 mg/L. In another embodiment, the level of expression is at least 1300 mg/L. In another embodiment, the level of expression is at least 1400 mg/L. In another embodiment, the level of expression is at least 1500 mg/L. In another embodiment, the clones in step (b) are propagated in medium to form a master cell bank (MCB) and a working cell bank (WCB). In one embodiment, clones at step (c) are obtained from a MCB. In another embodiment, clones at step (c) are obtained from a WCB.

The product of interest is obtained from the cell culture medium as a secreted gene product. If a protein or polypeptide is expressed without a secretion signal, however, the gene product may also be isolated from cell lysates. In order to obtain a pure homogeneous product which is substantially free from other recombinant proteins and host cell proteins, conventional purification procedures are carried out. First of all, cells and cell debris are frequently removed from the culture medium or lysate. The desired gene product can then be freed from contaminating soluble proteins, polypeptides and nucleic acids, e.g. by fractionation on immunoaffinity and ion exchange columns, ethanol precipitation, reversed phase HPLC or chromatography on Sephadex, hydroxyapatite, silica or cation exchange resins such as DEAE (see Examples herein). Methods known in the art and which result in the purification of a heterologous protein expressed by recombinant host cells are known to the skilled man and described in the literature, e.g. by Harris et al. (Harris et al., Protein Purification: A Practical Approach, Pickwood and Hames, eds., IRL Press, Oxford, 1995) and Scopes (Scopes, R., Protein Purification, Springer Verlag, 1988). These methods may be employed in whole or in part in the methods disclosed herein.

In another embodiment, disclosed herein is a method of preparing one or more products in mammalian cells under serum-free conditions, characterized in that (i) mammalian cells contain a gene of interest which codes for a peptide of interest disclosed herein; (ii) the mammalian cells are grown under serum-free conditions which allow replication of the mammalian cells; (iii) in each case at least one (1) of these mammalian cell(s) are deposited in a cell culture vessel under serum-free conditions; (iv) the suitably deposited mammalian cells are replicated under serum-free conditions; (v) the replicated cells are cultivated under serum-free conditions in which the gene of interest is expressed; and (vi) the gene product is then isolated from the cells or culture supernatant and purified. In another embodiment of this process the mammalian cell is a transfected mammalian cell into which the gene of interest has been introduced. Accordingly, methods disclosed herein also relates to a method of preparing recombinant gene products, characterized in that before step (i) of the process described above the mammalian cells are transfected with a nucleic acid which at least codes for a gene of interest. Stable transfection of the corresponding mammalian cell is preferred.

Examples of serum-free, protein-free or chemically defined media include for example the commercially obtainable media Ham's F12 (Sigma, Deisenhofen, DE), RPMI 1640 (Sigma), Dulbecco's Modified Eagle's medium (DMEM; Sigma), Minimal Essential medium (MEM; Sigma), Iscove's Modified Dulbecco's medium (IMDM; Sigma), CDCHO (Invitrogen, Carlsbad, Calif., USA), CHO-S-SFMII (Invitrogen), serum-free CHO medium (Sigma), CD-PowerCHO2 medium (Lonza) and protein-free CHO medium (Sigma). Each of these media can if desired be supplemented with various compounds such as hormones and/or other growth factors (e.g. insulin, transferrin, epidermal growth factor, insulin-like growth factor), salts (e.g. sodium chloride, calcium, magnesium, phosphate), buffers (e.g. HEPES), nucleosides (e.g. adenosine, thymidine), glutamine, glucose or other equivalent nutrients, antibiotics and/or trace elements or commercially available Feed such as Power Feed A (Lonza) If the replicable cells are recombinant cells which express one or more selectable markers, one or more suitable selection agents such as antibiotics may also be added to the medium.

It will be appreciated that other than containing the necessary elements for the transcription and translation of the inserted coding sequence (encoding the polypeptide), the expression construct disclosed herein can also include sequences engineered to optimize stability, production, purification, yield or activity of the expressed polypeptide.

In some embodiments, transformed cells are cultured under effective conditions, which allow for the expression of high amounts of recombinant polypeptide. In some embodiments, effective culture conditions include, but are not limited to, effective media, bioreactor, temperature, pH and oxygen conditions that permit protein production. A skilled artisan would appreciate that the term "an effective medium" may encompass any medium in which a cell is cultured to produce the recombinant polypeptide disclosed herein. In some embodiments, a medium typically includes an aqueous solution having assimilable carbon, nitrogen and phosphate sources, and appropriate salts, minerals, metals and other nutrients, such as vitamins. In some embodiments, cells disclosed herein can be cultured in conventional fermentation bioreactors, shake flasks, test tubes, microtiter dishes and petri plates. In some embodiments, culturing is carried out at a temperature, pH and oxygen content appropriate for a recombinant cell. In some embodiments, culturing conditions are within the expertise of one of ordinary skill in the art.

In one embodiment, culture conditions comprised dissolved oxygen (DO) content at about 20-80%. In another embodiment, DO content is at about 20-30%. In another embodiment, DO content is at about 30-40%. In another embodiment, DO content is at about 40-50%. In another embodiment, DO content is at about 50-60%. In another embodiment, DO content is at about 60-70%. In another embodiment, DO content is at about 70-80%.

In one embodiment, culture conditions comprise pH starting at one temperature and shifting to another during the manufacture. In another embodiment, pH starts at about 7.3 and shifts to about 6.7 during bioreactor incubation. In another embodiment, pH starts at about 7.3, about 7.2 or about 7.1 and shifts to about 6.7, about 6.8, about 6.9 or about 7.0 during bioreactor incubation. Each possibility represents an embodiment disclosed herein.

In some embodiments, depending on the vector and host system used for production, resultant polypeptides disclosed herein either remain within the recombinant cell, or are secreted into the medium.

In one embodiment, following a predetermined time in culture, recovery of the recombinant polypeptide is effected.

A skilled artisan would appreciate that the phrase "recovering the recombinant polypeptide" used herein may encompass collecting the whole medium containing the polypeptide and can imply additional steps of separation or purification.

In one embodiment, polypeptides disclosed herein are purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, hydroxyapatite chromatography, chromatofocusing and differential solubilization.

In one embodiment, each column can be run under controlled or non-controlled temperature.

In one embodiment, to facilitate recovery, the expressed coding sequence can be engineered to encode the polypeptide disclosed herein and fused to a cleavable moiety. In one embodiment, a fusion protein can be designed so that the polypeptide can be readily isolated by affinity chromatography; e.g., by immobilization on a column specific for the cleavable moiety. In one embodiment, a cleavage site is engineered between the polypeptide and the cleavable moiety and the polypeptide can be released from the chromatographic column by treatment with an appropriate enzyme or agent that specifically cleaves the fusion protein at this site [e.g., see Booth et al., Immunol. Lett. 19:65-70 (1988); and Gardella et al., J. Biol. Chem. 265:15854-15859 (1990)].

In one embodiment, the polypeptide disclosed herein is retrieved in "substantially pure" form. A skilled artisan would appreciate that the phrase "substantially pure" may encompass a purity that allows for the effective use of the protein in the applications described herein. Such a form may also include highly glycosylated and highly sialylated forms as also disclosed herein.

In one embodiment, a subject disclosed herein, is a human subject. In another embodiment, the subject is a domesticated animal. In another embodiment, the subject is a pet. In another embodiment, the subject is a mammal. In another embodiment, the subject is a farm animal. In another embodiment, the subject is a monkey. In another embodiment, the subject is a horse. In another embodiment, the subject is a cow. In another embodiment, the subject is a mouse. In another embodiment, the subject is a rat. In another embodiment, the subject is canine. In another embodiment, the subject is feline. In another embodiment, the subject is bovine, ovine, porcine, equine, murine, or cervine. In one embodiment, the subject is male. In another embodiment, the subject is female. In one embodiment, the subject is a child, in another embodiment, an adolescent, in another embodiment, an adult or, in another embodiment, an elderly subject. In another embodiment, the subject is a pediatric subject, in another embodiment, a geriatric subject.

In one embodiment, the polypeptide disclosed herein is synthesized using *in vitro* expression systems. In one embodiment, *in vitro* synthesis methods are well known in the art and the components of the system are commercially available.

In one embodiment, production of a polypeptide of interest modified by CTPs using recombinant DNA technology is performed.

In some embodiments, the recombinant polypeptides are synthesized and purified; their therapeutic efficacy can be assayed either *in vivo* or *in vitro.* In one embodiment, the binding activities of the recombinant a polypeptide of interest modified by CTPs disclosed herein can be ascertained using various assays.

In one embodiment, a method of manufacturing CTP-modified polypeptide of interest comprises a step for obtaining clones that optimally express said CTP-modified polypeptide of interest from said WCB, and expanding said clones. In another embodiment, a method of manufacturing CTP-modified polypeptide of interest comprises a step for obtaining clones that optimally express said CTP-modified polypeptide of interest from said MCB, and expanding said clones. In another embodiment, the cell clones are expanded in solution through a series of subcultivation steps up to production bioreactor level. In another embodiment, the solution containing said sub-cultivated clones is seeded in a bioreactor. In another embodiment, the bioreactor is a disposable bioreactor. In another embodiment, the bioreactor comprises a stainless steel bioreactor, a rocking motion bioreactor such as Wave system from GE, a perfusion bioreactor, or any other bioreactor system known in the art. In one embodiment, removal of cells from a bioreactor is accomplished by use of a disposable filter system. If a large scale manufacture is performed, continuous centrifugation could be used prior to use of a filtering system.

In one embodiment, the cell clones are expanded further or up-scaled by serially culturing said cells in increasing sizes of the bioreactor until a desired scale is reached. In another embodiment, a bioreactor is run in a fed-batch mode. In another embodiment, a bioreactor is run in a batch mode. In another embodiment, a bioreactor is run in a repeated-batch mode. In another embodiment, a bioreactor is run in a perfusion mode. Each possibility described above, is another embodiment.

Peak viable cell densities differ depending on the type of bioreactor employed. In one embodiment, the peak viable cell density of a bioreactor used in methods of manufacturing disclosed herein is about 0.2 x 10⁶ - 1.4 x 10⁶ cells/ml. In another embodiment, the peak viable cell density of a bioreactor used in methods of manufacturing disclosed herein is about 0.05 × 10⁶ - 100 × 10⁶. In another embodiment, the peak viable cell density of a bioreactor is about 0.05 × 10⁶ - 0.5 × 10⁶. In another embodiment, the peak viable cell density of a bioreactor is about 0.5 × 10⁶ - 5 × 10⁶. In another embodiment, the peak viable cell density of a bioreactor is about 5.0 × 10⁶ - 50 × 10⁶. In another embodiment, the peak viable cell density of a bioreactor is about 50 × 10⁶ - 100 × 10⁶.

Feed schemes for bioreactor use could be different, e.g. repeated daily feeding from a certain day, or fixed in several days, in addition % of feed added could be different from few % up to even 50% or more. Each possibility is an embodiment disclosed herein.

DMSO may be added to a bioreactor at different concentrations as is known in the art. In one embodiment, 0.1-3% DMSO is added to a bioreactor during its use. In another embodiment, 0.1-0.5% DMSO is added. In another embodiment, 0.5-1.0 % DMSO is added. In another embodiment, 1.0-1.5 % DMSO is added. In another embodiment, 1.5-2.0 % DMSO is added. In another embodiment, 2.0-2.5 % DMSO is added. In another embodiment, 2.5-3.0 % DMSO is added.

In one embodiment, a method of manufacturing a CTP-modified polypeptide of interest comprises the step of purifying a clarified harvest solution in order to obtain a purified protein solution. In another embodiment, a purified protein solution manufactured using methods presented herein, comprises at least 5-95% CTP-modified polypeptide of interest. In another embodiment, a purified protein solution manufactured using methods presented herein, comprises at least 5% CTP-modified polypeptide of interest. In another embodiment, a purified protein solution manufactured using methods presented herein, comprises at least 10% CTP-modified polypeptide of interest. In another embodiment, a purified protein solution manufactured using methods presented herein, comprises at least 20% CTP-modified polypeptide of interest. In another embodiment, a purified protein solution manufactured using methods presented In another embodiment, a purified protein solution manufactured using methods presented herein, comprises at least 30% CTP-modified polypeptide of interest., comprises at least 40% CTP-modified polypeptide of interest. In another embodiment, a purified protein solution comprises at least 50% CTP-modified polypeptide of interest. In another embodiment, a purified protein solution comprises at least 60% CTP-modified polypeptide of interest. In another embodiment, a purified protein solution comprises at least 70% CTP-modified polypeptide of interest. In another embodiment, a purified protein solution comprises at least 80% CTP-modified polypeptide of interest. In another embodiment, a purified protein solution comprises at least 90% CTP-modified polypeptide of interest. In another embodiment, a purified protein solution manufactured using methods presented herein, comprises at least 95% CTP-modified polypeptide of interest.

In one embodiment, a clarified harvest is held up to 24 hours at 2-25°C. In another embodiment, the clarified harvest is stored at least 5°C for up to one month.

In one embodiment, the clarified harvest obtained in step (e) is tested for bioburden, bacterial endotoxin, specific protein content, residual DNA, viruses, virus-like particles, and/or Mycoplasma, or any combination thereof.

In one embodiment, the purification of the clarified harvest in step (f) is accomplished by sequentially performing the steps comprising: (g) concentrating, diafiltering and purifying said clarified harvest solution, wherein said concentration, diafiltration and purifying is accomplished by hollow fiber cassette or tangential flow cassette sequentially passing said clarified harvest solution through an anion exchange column and a hydrophobic interaction column; (h) obtaining said clarified harvest obtained following step; (i) and inactivating viruses present in said clarified harvest by incubating in a solution toxic to said viruses; (j) obtaining said clarified harvest solution from (h) and concentrating, diafiltering and purifying said clarified harvest solution, wherein said concentration, diafiltration and purification is followed by sequentially passing said clarified harvest solution through a Hydroxyapatite Mixed-Mode column and a cation exchange column; (j) obtaining said clarified harvest solution following step (i) and physically removing said clarified harvest solution from viruses by nanofiltration; (k) obtaining said clarified harvest solution following step (j) and concentrating, diafiltering and purifying said clarified harvest solution to arrive at a maximally purified clarified harvest containing said a highly glycosylated form of CTP-modified polypeptides.

In one embodiment, ultrafiltration and diafiltration to concentrate and filter a clarified harvest may be performed using a hollow fiber cartridge, or equivalent TFF based UFDF step. The cartridge nominal molecular weight cutoff size is 10,000 kDa. In another embodiment, a membrane cartridge could comply PES/PS/RC membranes with a cut-off of 3 kDa to 30 kDa.

In another embodiment, the anion exchange column of step (g) is a DEAE-Sepharose Fast Flow column. In another embodiment, the DEAE column purifies the highly glycosylated form of said CTP-modified polypeptide of interest. In one embodiment, the higher the glycosylation the better the pharmacodynamics of the CTP-modified polypeptide of interest. In another embodiment, an anion exchange column may comprise other anion exchange columns known in the art, for example a Capto DEAE anion exchange column or other resins such as Eshmuno Q.

In one embodiment, the hydrophobic column of step (g) is a Phenyl Hydrophobic Interaction Chromatography (HIC) column. The number of cycles of use for phenyl HIC may range between about 1-10. In one embodiment, 1-3 cycles are performed. In another embodiment, 1-5 cycles are performed. In another embodiment, 1-6 cycles are performed. In another embodiment, 1-7 cycles are performed. In another embodiment, 1-8 cycles are performed. In another embodiment, 1-9 cycles are performed. In another embodiment, 1-10 cycles are performed. In another embodiment, buffers known in the art are used for washing and elution. In one embodiment, an elution buffer comprises Ammonium Sulfate with propylene glycol. In one embodiment, an elution buffer comprises Ammonium Sulfate with ethylene glycol.

In one embodiment, a Hydroxyapatite Mixed-Mode column comprises a ceramic hydroxyapatite Mixed-Mode column (CHT). The number of cycles of use for CHT may range between about 1-10. In one embodiment, 1-3 cycles are performed. In another embodiment, 1-5 cycles are performed. In another embodiment, 1-6 cycles are performed. In another embodiment, 1-7 cycles are performed. In another embodiment, 1-8 cycles are performed. In another embodiment, 1-9 cycles are performed. In another embodiment, 1-10 cycles are performed. Elution from a CHT column may be performed with between about 3-10 column volumes (CV). In one embodiment, elution is performed with about 3 CV. In another embodiment, elution is performed with about 4 CV. In another embodiment, elution is performed with about 5 CV. In another embodiment, elution is performed with about 6 CV. In another embodiment, elution is performed with about 7 CV. In another embodiment, elution is performed with about 8 CV. In another embodiment, elution is performed with about 9 CV. In another embodiment, elution is performed with about 10 CV.

In one embodiment, viruses that could be present in the clarified harvest due to contamination are inactivated in the clarified harvest. In another embodiment, the viruses are inactivated using a 1% Triton-X 100 solution. In another embodiment, the viruses are inactivated using a 0.2 to 2% Triton-X 100 solution. In another embodiment, the viruses are inactivated using 0.5% Triton-X 100 solution. In another embodiment, the viruses are inactivated using a 1-4% Triton-X 100 solution. In another embodiment, the viruses are inactivated using a 0.2-0.5% Triton-X 100 solution. In another embodiment, the viruses are inactivated using a 0.5-1.0% Triton-X 100 solution. In another embodiment, the viruses are inactivated using a 2% Triton-X 100 solution. In another embodiment, the viruses are inactivated using a 3% Triton-X 100 solution. In another embodiment, the viruses are inactivated using a 4% Triton-X 100 solution. In another embodiment, the viruses are inactivated using a 5-10% Triton-X 100 solution. In another embodiment, viral inactivation in a Triton-X 100 solution is for about 0.5 to 24 hours. In another embodiment, viral inactivation in a Triton-X solution is for about 0.5 to 1 hours. In another embodiment, viral inactivation in a Triton-X solution is for about 1 to 2 hours. In another embodiment, viral inactivation in a Triton-X solution is for about 2 to 3 hours. In another embodiment, viral inactivation in a Triton-X solution is for about 3 to 4 hours. In another embodiment, viral inactivation in a Triton-X solution is for about 4 to 6 hours. In another embodiment, viral inactivation in a Triton-X solution is for about 6 to 8 hours. In another embodiment, viral inactivation in a Triton-X solution is for about 8 to 10 hours. In another embodiment, viral inactivation in a Triton-X solution is for about 10 to 12 hours. In another embodiment, viral inactivation in a Triton-X solution is for about 12 to 24 hours.

It will be appreciated by the skilled artisan that other concentrations or other solutions available in the art and that are toxic to these viruses, including but not limited to, sodium cholate and Tween 80 may be used in methods disclosed herein. In another embodiment, a mixture of Tri-n-butyl phosphate (TNBP) and Polysorbate 80 (Tween 80) is used to inactivate the virus in step (h).

In one embodiment, viruses are physically removed by using nanofiltration. It will be appreciated by the skilled artisan that any filter known in the art for removing viruses may be applied in methods disclosed herein. In another embodiment, nanofiltration is carried out using a Planova or Planova type filter cartridge (1-60 mm²). Such methods are followed by confirmation of viral clearance from the clarified harvest using methods known in the art.

In one embodiment, a cation exchange column of step (i) herein is a SP-Sepharose Fast Flow column. In another embodiment, the cation exchange column comprises a CM sepharose Capto S. In another embodiment, the cation exchange column comprises any known in the art for the purpose herein.

In one embodiment, the methods disclosed herein achieve at least a 20% recovery rate of highly glycosylated CTP-modified polypeptide of interest. In another embodiment, the methods achieve a recovery rate of at least 5%, at least 10%, at least 15%, 20%, at least 25%, at least 30%, at least 40%, at least 50%, at least 60%, at least 70%, at least 80%, at least 90%, at least 95%, at least 96%, at least 97%, at least 98%, at least 99%, at least 99.9% recovery rate of highly glycosylated CTP-modified polypeptide of interest.

In one embodiment, following purification of highly glycosylated CTP-modified polypeptide of interest, the methods disclosed herein further comprise characterizing said CTP-modified polypeptide. In another embodiment, the purity of the CTP-modified polypeptide of interest is determined. In another embodiment, glycosylation content is determined. In another embodiment, glycosylation site occupancy is determined. In one embodiment, purity, glycosylation content and glycosylation site occupancy are determined in the manufactured CTP-modified polypeptide of interest.

In another embodiment, the cell clones utilized in methods disclosed herein are stored in a frozen cell bank. In another embodiment, the cell clones are stored in a lyophilized cell bank.

In another embodiment, the cell bank of methods and compositions disclosed herein is a master cell bank. In another embodiment, the cell bank is a working cell bank. In another embodiment, the cell bank is Good Manufacturing Practice (GMP) cell bank. In another embodiment, the cell bank is intended for production of clinical-grade material. In another embodiment, the cell bank conforms to regulatory practices for human use. In another embodiment, the cell bank is any other type of cell bank known in the art.

"Good Manufacturing Practices" are defined, in another embodiment, by (21 CFR 210-211) of the United States Code of Federal Regulations. In another embodiment, "Good Manufacturing Practices" are defined by other standards for production of clinical-grade material or for human consumption; e.g. standards of a country other than the United States. Each possibility represents a separate embodiment disclosed herein.

In another embodiment, the medium used for propagating cells contains methotrexate (MXT). In another embodiment, the medium is methotrexate-free medium. In another embodiment, the concentration of MXT present in a medium is between about 0.1-2 uM. In another embodiment, the concentration of MXT present in the medium is about 0.1-0.5 uM. In another embodiment, the concentration of MXT present in the medium is about 0.5-1.0 uM. In another embodiment, the concentration of MXT present in the medium is about 1.0-1.5 uM. In another embodiment, the concentration of MXT present in the medium is about 1.5-2.0 uM. It will be well appreciated that the term "medium" may encompass a liquid or gel or powder that is suitable for growth or culture of the cells comprising the CTP-modified polypeptide of interest disclosed herein. Such medium may be alternatively referred to as "growth medium" or "culture medium" and may include but is not limited to, nutrient media, enriched media, minimal media, differential media, transport media, or selective media. In a further aspect, selective medium may be suitable for selecting a particular group of cells during the manufacturing process.

In one embodiment, the purified protein solution contains at least 5-95% CTP-modified polypeptide of interest. In another embodiment, the purified protein solution contains at least 5 % CTP-modified polypeptide of interest. In another embodiment, the purified protein solution contains at least 10 % CTP-modified polypeptide of interest. In another embodiment, the purified protein solution contains at least 15 % CTP-modified polypeptide of interest. In another embodiment, a purified protein solution contains at least 20% CTP-modified polypeptide of interest. In another embodiment, the purified protein solution contains at least 30% CTP-modified polypeptide of interest. In another embodiment, the purified protein solution contains at least 40% CTP-modified polypeptide of interest. In another embodiment, the purified protein solution contains at least 50% CTP-modified polypeptide of interest. In another embodiment, the purified protein solution contains at least 60% CTP-modified polypeptide of interest. In another embodiment, the purified protein solution contains at least 70% CTP-modified polypeptide of interest. In another embodiment, the purified protein solution contains at least 80% CTP-modified polypeptide of interest. In another embodiment, the purified solution contains at least 90-95% CTP-modified polypeptide of interest. In another embodiment, the purified solution contains 95.1-99.9% CTP-modified polypeptide of interest. In another embodiment, the purified solution contains 100% CTP-modified polypeptide of interest.

In one embodiment, the CTP-modified polypeptide of interest is highly glycosylated. It will be well appreciated by the skilled artisan that the term "highly glycosylated" when in reference to a CTP-modified polypeptide of interest, may encompass a glycosylation level of about 70-80% of total CTP-modified polypeptide of interest polypeptide. In another embodiment, highly glycosylated CTP-modified polypeptide of interest has a glycosylation level of at least 70%. In another embodiment, highly glycosylated CTP-modified polypeptide of interest has a glycosylation level of at least 80%. In another embodiment, the term may encompass a glycosylation level of about 81-90% of the total CTP-modified polypeptide of interest polypeptide. In another embodiment, highly glycosylated CTP-modified polypeptide of interest has a glycosylation level of at least 90%. In another embodiment, the term may encompass a glycosylation level of about 91-95% of the total CTP-modified polypeptide of interest polypeptide. In another embodiment, the term may encompass a glycosylation level of about 95.1-99% of the total CTP-modified polypeptide of interest polypeptide. In another embodiment, the term may encompass a glycosylation level of 100% of the total CTP-modified polypeptide of interest polypeptide. Highly glycosylated CTP-modified polypeptide of interest polypeptides may have beneficial properties in methods of use for a long-acting polypeptide of interest, supporting reduced frequency of administration. The high glycosylation levels contribute to the significant increased hydrodynamic volume of a CTP-modified polypeptide of interest, for example a CTP-modified polypeptide of interest, as compared to recombinant polypeptide of interest. This may result in an elongated circulating time of CTP-modified polypeptide of interest.

In one embodiment, the number of O-glycans per CTP is at least 4-6. In another embodiment, the number of O-glycans per CTP is between 4-6. In another embodiment, the number of O-glycans per CTP is at least 4-8. In another embodiment, the number of O-glycans per CTP is between 4-8. In one embodiment, the number of O-glycans per CTP is at least 6-8. In one embodiment, the number of O-glycans per CTP is between 6-8. In another embodiment, the number of O-glycans per CTP is at least 4. In another embodiment, the number of O-glycans per CTP is at least 5. In another embodiment, the number of O-glycans per CTP is at least 6. In another embodiment, the number of O-glycans per CTP is at least 7. In another embodiment, the number of O-glycans per CTP is 8.

In one embodiment, the number of O-glycans per CTP-modified polypeptide of interest polypeptide having one CTP attached is at least 4-6. In another embodiment, the number of O-glycans per CTP-modified polypeptide of interest polypeptide having one CTP attached is at least 6-8. In another embodiment, the number of O-glycans per CTP-modified polypeptide of interest polypeptide having one CTP attached is at least 4-8. In another embodiment, the number of O-glycans per CTP-modified polypeptide of interest polypeptide having two CTP units attached is at least 8-12. In another embodiment, the number of O-glycans per CTP-modified polypeptide of interest polypeptide having two CTP units attached is at least 12-16. In another embodiment, the number of O-glycans per CTP-modified polypeptide of interest polypeptide having two CTP units attached is at least 8-16. In another embodiment, the number of O-glycans per CTP-modified polypeptide of interest polypeptide having three CTP units attached is at least 12-18. In another embodiment, the number of O-glycans per CTP-modified polypeptide of interest polypeptide having three CTP units attached is at least 18-24. In another embodiment, the number of O-glycans per CTP-modified polypeptide of interest polypeptide having three CTP units attached is at least 12-24. In another embodiment, the number of O-glycans per CTP-modified polypeptide of interest polypeptide having four CTP units attached is at least 16-24. In another embodiment, the number of O-glycans per CTP-modified polypeptide of interest polypeptide having four CTP units attached is at least 24-32. In another embodiment, the number of O-glycans per CTP-modified polypeptide of interest polypeptide having four CTP units attached is at least 16-32. In another embodiment, the number of O-glycans per CTP-modified polypeptide of interest polypeptide having five CTP units attached is at least 20-30. In another embodiment, the number of O-glycans per CTP-modified polypeptide of interest polypeptide having five CTP units attached is at least 30-40. In another embodiment, the number of O-glycans per CTP-modified polypeptide of interest polypeptide having five CTP units attached is at least 20-40. In another embodiment, the number of O-glycans per CTP-modified polypeptide of interest polypeptide having six CTP units attached is at least 24-36. In another embodiment, the number of O-glycans per CTP-modified polypeptide of interest polypeptide having six CTP units attached is at least 36-48. In another embodiment, the number of O-glycans per CTP-modified polypeptide of interest polypeptide having six CTP units attached is at least 24-48. In another embodiment, the number of O-glycans per CTP-modified polypeptide of interest polypeptide having seven CTP units attached is at least 28-35. In another embodiment, the number of O-glycans per CTP-modified polypeptide of interest polypeptide having seven CTP units attached is at least 42-56. In another embodiment, the number of O-glycans per CTP-modified polypeptide of interest polypeptide having seven CTP units attached is at least 28-56. In another embodiment, the number of O-glycans per CTP-modified polypeptide of interest polypeptide having eight CTP units attached is at least 32-48. In another embodiment, the number of O-glycans per CTP-modified polypeptide of interest polypeptide having eight CTP units attached is at least 48-64. In another embodiment, the number of O-glycans per CTP-modified polypeptide of interest polypeptide having eight CTP units attached is at least 32-64. In another embodiment, the number of O-glycans per CTP-modified polypeptide of interest polypeptide having nine CTP units attached is at least 36-54. In another embodiment, the number of O-glycans per CTP-modified polypeptide of interest polypeptide having nine CTP units attached is at least 54-72. In another embodiment, the number of O-glycans per CTP-modified polypeptide of interest polypeptide having nine CTP units attached is at least 36-72. In another embodiment, the number of O-glycans per CTP-modified polypeptide of interest polypeptide having ten CTP units attached is at least 40-60. In another embodiment, the number of O-glycans per CTP-modified polypeptide of interest polypeptide having ten CTP units attached is at least 60-80. In another embodiment, the number of O-glycans per CTP-modified polypeptide of interest polypeptide having five CTP units attached is at least 40-80.

In one embodiment, O-glycan occupancy per CTP is at least 70%. In another embodiment, O-glycan occupancy per CTP is at least 80%. In another embodiment, O-glycan occupancy per CTP is at least 90%. In another embodiment, O-glycan occupancy per CTP is 100%.

In one embodiment, the CTP-modified polypeptide of interest is highly sialylated. It will be appreciated by the skilled artisan that the term "highly sialylated" when in reference to a CTP-modified polypeptide of interest, may encompass a sialylation level of about 70-80% of total CTP-modified polypeptide of interest polypeptide. In another embodiment, the term may encompass a sialylation level of about 80-90% of the total CTP-modified polypeptide of interest polypeptide. In another embodiment, the term may encompass a sialylation level of about 90-95% of the total CTP-modified polypeptide of interest polypeptide. In another embodiment, the term may encompass a sialylation level of about 95.1-99% of the total CTP-modified polypeptide of interest polypeptide. In another embodiment, the term may encompass a sialylation level of 100% of the total CTP-modified polypeptide of interest polypeptide. In another embodiment, an O-glycan structure in a CTP-modified polypeptide of interest comprises a mono-sialylated core 1.

In one embodiment, the CTP-modified polypeptide of interest polypeptide consists of two CTP attached to the carboxy terminus of said polypeptide of interest, and one chorionic gonadotropin carboxy terminal peptide attached to the amino terminus of said polypeptide of interest. In another embodiment, the CTP-modified polypeptide of interest polypeptide consists of one chorionic gonadotropin carboxy terminal peptide attached to the carboxy terminus of said polypeptide of interest.

In one embodiment, the expression vector comprising a coding portion encoding said CTP-modified polypeptide of interest also comprises a promoter, a coding sequence for said CTP-modified polypeptide, and a polyadenylation sequence. In one embodiment, the polyadenylation sequence is a simian virus (SV) 40 polyadenylation sequence.

In one embodiment, the CTP-modified polypeptide of interest is expressed at a level of between 30 - 1500 mg/L. In another embodiment, the CTP-modified polypeptide of interest is expressed at a level of at least 30 mg/L. In another embodiment, the CTP-modified polypeptide of interest is expressed at a level of at least 40 mg/L. In another embodiment, the CTP-modified polypeptide of interest is expressed at a level of at least 50 mg/L. In another embodiment, the CTP-modified polypeptide of interest is expressed at a level of at least 60 mg/L. In another embodiment, the CTP-modified polypeptide of interest is expressed at a level of at least 70 mg/L. In another embodiment, the CTP-modified polypeptide of interest is expressed at a level of at least 50-70 mg/L. In another embodiment, the CTP-modified polypeptide of interest is expressed at a level of at least 80 mg/L. In another embodiment, the CTP-modified polypeptide of interest is expressed at a level of at least 90 mg/L. In another embodiment, the CTP-modified polypeptide of interest is expressed at a level of at least 70-100 mg/L. In another embodiment, the CTP-modified polypeptide of interest is expressed at a level of at least 100 mg/L. In another embodiment, the CTP-modified polypeptide of interest is expressed at a level of at least 200 mg/L. In another embodiment, the CTP-modified polypeptide of interest is expressed at a level of at least 100-200 mg/L. In another embodiment, the CTP-modified polypeptide of interest is expressed at a level of at least 300 mg/L. In another embodiment, the CTP-modified polypeptide of interest is expressed at a level of at least 200-300 mg/L. In another embodiment, the CTP-modified polypeptide of interest is expressed at a level of at least 400 mg/L. In another embodiment, the CTP-modified polypeptide of interest is expressed at a level of at least 300-400 mg/L. In another embodiment, the CTP-modified polypeptide of interest is expressed at a level of at least 500 mg/L. In another embodiment, the CTP-modified polypeptide of interest is expressed at a level of at least 500-600 mg/L. In another embodiment, the CTP-modified polypeptide of interest is expressed at a level of at least 600 mg/L. In another embodiment, the CTP-modified polypeptide of interest is expressed at a level of at least 600-700 mg/L. In another embodiment, the CTP-modified polypeptide of interest is expressed at a level of at least 700 mg/L. In another embodiment, the CTP-modified polypeptide of interest is expressed at a level of at least 701-800 mg/L. In another embodiment, the CTP-modified polypeptide of interest is expressed at a level of at least 800 mg/L. In another embodiment, the CTP-modified polypeptide of interest is expressed at a level of at least 801-900 mg/L. In another embodiment, the CTP-modified polypeptide of interest is expressed at a level of at least 900 mg/L. In another embodiment, the CTP-modified polypeptide of interest is expressed at a level of at least 901-1000 mg/L. In another embodiment, the CTP-modified polypeptide of interest is expressed at a level of at least 1000 mg/L. In another embodiment, the CTP-modified polypeptide of interest is expressed at a level of at least 1001-1100 mg/L. In another embodiment, the CTP-modified polypeptide of interest is expressed at a level of at least 1100 mg/L. In another embodiment, the CTP-modified polypeptide of interest is expressed at a level of at least 1101-1200 mg/L. In another embodiment, the CTP-modified polypeptide of interest is expressed at a level of at least 1200 mg/L. In another embodiment, the CTP-modified polypeptide of interest is expressed at a level of at least 1201-1300 mg/L. In another embodiment, the CTP-modified polypeptide of interest is expressed at a level of at least 1300 mg/L. In another embodiment, the CTP-modified polypeptide of interest is expressed at a level of at least 1301-1400 mg/L. In another embodiment, the CTP-modified polypeptide of interest is expressed at a level of at least 1400 mg/L. In another embodiment, the CTP-modified polypeptide of interest is expressed at a level of at least 1401-1500 mg/L. In another embodiment, the CTP-modified polypeptide of interest is expressed at a level of at least 1500 mg/L.

It will be appreciated by the skilled artisan that the term "expression" may encompass transcription and/or translation of a heterologous nucleic acid sequence within a host cell. The level of expression of a desired product/protein of interest in a host cell may be determined on the basis of either the amount of corresponding mRNA or cDNA that is present in the cell, or the amount of the desired polypeptide/protein of interest encoded by the selected sequence as in the present examples. For example, mRNA transcribed from a selected sequence can be quantitated by Northern blot hybridization, ribonuclease RNA protection, in situ hybridization to cellular RNA or by PCR (see Sambrook et al., 1989; Ausubel et al., 1987 updated). Proteins encoded by a selected sequence can be quantitated by various methods, e.g. by ELISA, by Western blotting, by radioimmunoassays, by immunoprecipitation, by assaying for the biological activity of the protein, by immunostaining of the protein followed by FACS analysis (see Sambrook et al., 1989; Ausubel et al., 1987 updated) or by homogeneous time-resolved fluorescence (HTRF) assays. In one embodiment, quantitation of the CTP-modified polypeptide of interest comprises use of a reverse phase high performance liquid chromatography (RP-HPLC). In another embodiment, the RP-HPLC comprises a C-18 column. In another embodiment, the RP-HPLC comprises a C-8 column. In another embodiment, methods disclosed herein use an RP-HPLC to quantitate a CTP-modified polypeptide of interest in the harvest (See Example steps 3 to 8). In another embodiment, methods disclosed herein use an RP-HPLC to quantitate a CTP-modified polypeptide of interest in the first steps of purification (See Example steps 9-12.

In another embodiment, a cell bank, or frozen stock disclosed herein exhibits viability upon thawing of greater than 90%. In another embodiment, the storage is for an indefinite amount of time.

In another embodiment, the storage is for 2 weeks. In another embodiment, the storage is for 3 weeks. In another embodiment, the storage is for 1 month. In another embodiment, the storage is for 2 months. In another embodiment, the storage is for 3 months. In another embodiment, the storage is for 5 months. In another embodiment, the storage is for 6 months. In another embodiment, the storage is for 9 months. In another embodiment, the storage is for 1 year.

In another embodiment, a cell bank, or frozen stock disclosed herein is cryopreserved by a method that comprises growing a culture of the cells in a defined media disclosed herein, freezing the culture in a solution comprising glycerol, and storing the cell clones at below -20 degrees Celsius. In another embodiment, the temperature is about -70 degrees Celsius. In another embodiment, the temperature is about ⁻70-⁻80 degrees Celsius. In another embodiment, any defined media disclosed herein may be used in this method. Each defined media represents a separate embodiment disclosed herein.

In another embodiment of methods and compositions disclosed herein, the culture is inoculated from a cell bank. In another embodiment, the culture is inoculated from a frozen stock. In another embodiment, the culture is inoculated from a starter culture. In another embodiment, the culture is inoculated from a colony. In another embodiment, the culture is inoculated at mid-log growth phase. In another embodiment, the culture is inoculated at approximately mid-log growth phase. In another embodiment, the culture is inoculated at another growth phase.

In another embodiment of methods and compositions disclosed herein, the solution used for freezing comprises DMSO in an amount of 2-20%. In another embodiment, the amount is 2%. In another embodiment, the amount is 20%. In another embodiment, the amount is 1%. In another embodiment, the amount is 1.5%. In another embodiment, the amount is 3%. In another embodiment, the amount is 4%. In another embodiment, the amount is 5%. In another embodiment, the amount is 2%. In another embodiment, the amount is 2%. In another embodiment, the amount is 7%. In another embodiment, the amount is 7.5%. In another embodiment, the amount is 9%. In another embodiment, the amount is 10%. In another embodiment, the amount is 12%. In another embodiment, the amount is 14%. In another embodiment, the amount is 16%. In another embodiment, the amount is 18%. In another embodiment, the amount is 22%. In another embodiment, the amount is 25%. In another embodiment, the amount is 30%. In another embodiment, the amount is 35%. In another embodiment, the amount is 40%.

In another embodiment, the additive is sucrose. In another embodiment, the additive is any other colligative additive or additive with anti-freeze properties that is known in the art. Each possibility represents a separate embodiment disclosed herein.

In one embodiment, a freezing solution used in the methods and for the compositions disclosed herein comprises conditioned media and DMSO. In one embodiment, a freezing solution used in the methods and for the compositions disclosed herein comprises about 46.255% conditioned media and 7.5% DMSO.

In one embodiment, the cell culture is grown by techniques routine in the art. In another embodiment, a constant pH is maintained during growth of the cell culture. In another embodiment, the pH is maintained at about 7.0. In another embodiment, the pH is about 6. In another embodiment, the pH is about 6.5. In another embodiment, the pH is about 7.5. In another embodiment, the pH is about 8. In another embodiment, the pH is 6.5-7.5. In another embodiment, the pH is 6-8. In another embodiment, the pH is 6-7. In another embodiment, the pH is 7-8.

In another embodiment, a constant temperature is maintained during growth of the culture. In another embodiment, the temperature is maintained at about 37°C. In another embodiment, the temperature is 37°C. In another embodiment, the temperature is 25°C. In another embodiment, the temperature is 27°C. In another embodiment, the temperature is 28°C. In another embodiment, the temperature is 30°C. In another embodiment, the temperature is 32°C. In another embodiment, the temperature is 34°C. In another embodiment, the temperature is 35°C. In another embodiment, the temperature is 36°C. In another embodiment, the temperature is 38°C. In another embodiment, the temperature is 39°C.

In another embodiment, a constant dissolved oxygen concentration is maintained during growth of the culture. In another embodiment, the dissolved oxygen concentration is maintained at 20% of saturation. In another embodiment, the concentration is 15% of saturation. In another embodiment, the concentration is 16% of saturation. In another embodiment, the concentration is 18% of saturation. In another embodiment, the concentration is 22% of saturation. In another embodiment, the concentration is 25% of saturation. In another embodiment, the concentration is 30% of saturation. In another embodiment, the concentration is 35% of saturation. In another embodiment, the concentration is 40% of saturation. In another embodiment, the concentration is 45% of saturation. In another embodiment, the concentration is 50% of saturation. In another embodiment, the concentration is 55% of saturation. In another embodiment, the concentration is 60% of saturation. In another embodiment, the concentration is 65% of saturation. In another embodiment, the concentration is 70% of saturation. In another embodiment, the concentration is 75% of saturation. In another embodiment, the concentration is 80% of saturation. In another embodiment, the concentration is 85% of saturation. In another embodiment, the concentration is 90% of saturation. In another embodiment, the concentration is 95% of saturation. In another embodiment, the concentration is 100% of saturation. In another embodiment, the concentration is near 100% of saturation.

In another embodiment of methods and compositions disclosed herein, the culture is grown in media having a maximum volume of 2 liters (L) per vessel. In another embodiment, the media has a maximum volume of 200 ml per vessel. In another embodiment, the media has a maximum volume of 300 ml per vessel. In another embodiment, the media has a maximum volume of 500 ml per vessel. In another embodiment, the media has a maximum volume of 750 ml per vessel. In another embodiment, the media has a maximum volume of 1 L per vessel. In another embodiment, the media has a maximum volume of 1.5 L per vessel. In another embodiment, the media has a maximum volume of 2.5 L per vessel. In another embodiment, the media has a volume of 3 L per vessel. In another embodiment, the media has a volume of 5 L per vessel. In another embodiment, the media has a volume of at least 5 L per vessel. In another embodiment, the media has a volume of at least 10 L per vessel.

In another embodiment, the media has a minimum volume of 2 L per vessel. In another embodiment, the media has a minimum volume of 500 ml per vessel. In another embodiment, the media has a minimum volume of 750 ml per vessel. In another embodiment, the media has a minimum volume of 1 L per vessel. In another embodiment, the media has a minimum volume of 1.5 L per vessel. In another embodiment, the media has a minimum volume of 2.5 L per vessel. In another embodiment, the media has a minimum volume of 3 L per vessel. In another embodiment, the media has a minimum volume of 4 L per vessel. In another embodiment, the media has a minimum volume of 5 L per vessel. In another embodiment, the media has a minimum volume of 6 L per vessel. In another embodiment, the media has a minimum volume of 8 L per vessel. In another embodiment, the media has a minimum volume of 10 L per vessel.

In another embodiment, the step of freezing is performed when the culture has a density of 1 × 10⁶ viable cells (VC)/ml. In another embodiment, the biomass is 1.5 × 10⁶ VC/ml. In another embodiment, the biomass is 1.5 × 10⁶ VC/ml. In another embodiment, the biomass is 2 × 10⁶ VC/ml. In another embodiment, the biomass is 3 x 10⁶ VC/ml. In another embodiment, the biomass is 4 × 10⁶ VC/ml. In another embodiment, the biomass is 5 x 10⁶ VC/ml. In another embodiment, the biomass is 7 × 10⁶ VC/ml. In another embodiment, the biomass is 9 × 10⁶ VC/ml. In another embodiment, the biomass is 10 × 10⁶ VC/ml. In another embodiment, the biomass is 12 × 10⁶ VC/ml. In another embodiment, the biomass is 15 × 10⁶ VC/ml. In another embodiment, the biomass is 20 × 10⁷ VC/ml. In another embodiment, the biomass is 25 × 10⁶ VC/ml. In another embodiment, the biomass is 30 × 10⁷ VC/ml. In another embodiment, the biomass is 33 × 10⁶ VC/ml. In another embodiment, the biomass is 40 × 10⁶ VC/ml. In another embodiment, the biomass is 50 × 10⁶ VC/ml. In another embodiment, the biomass is more than 50 × 10⁶ VC/ml.

In another embodiment of methods and compositions disclosed herein, the cell culture is flash-frozen in liquid nitrogen, followed by storage at the final freezing temperature. In another embodiment, the culture is frozen in a more gradual manner; e.g. by placing in a vial of the culture in the final storage temperature. In another embodiment, the culture is frozen by any other method known in the art for freezing cell cultures.

It will be understood by the skilled artisan that the terms "cell culture" and "tissue culture" may be used interchangeably and denote the maintenance of cells in vitro, in suspension culture in a liquid medium or on surface such as glass, plastic or agar provided with liquid medium. In general, "cell culture" necessitates a medium that is buffered to maintain a constant suitable pH. Media used in cell culture are generally formulated to include an adequate supply of necessary nutrients and can be osmotically tailored to the particular cells being maintained, with temperature and gas phase also being controlled within suitable limits. Cell culture techniques are well known in the art. See, e.g., Morgan et al. 1993 Animal Cell Culture, BIOS Scientific Publishers, Oxford, UK; and Adams, R. L. P. 1990 Cell Culture for Biochemists, Second Edition, Elsevier.

It will be appreciated by the skilled artisan that the term "passage" may encompass the act of subculturing a cell population. A skilled artisan would appreciate that the term "subculture" may encompass a cell culture established by the inoculation of sterile medium, which in one embodiment is a fresh sterile medium, with a sample from a previous culture.

It will also be appreciated by the skilled artisan that the term "cell strain" may encompass a population of cells derived from a primary culture using subcultivation techniques. Thus, a primary culture can be subcultured into two or more new cultures and the subculturing repeated at periodic intervals for several months to maintain the cell strain. Subculturing can be carried out using established cell culture techniques.

In one embodiment, passaged cell strains, and immortalized cell lines can be characterized by their expression of specific functional markers such as keratins, hormonal and growth factor receptors and the like.

In some aspects, cultures may be carried out in serum-free defined media with added growth factors. In other aspects the media contains serum with or without added growth factors. Such modifications may be empirically determined by the skilled artisan so as to optimize cell proliferation.

It will be appreciated by a skilled artisan that the term "cell line" can encompass a population of cells derived from a single explant which are characterized as having the potential for unlimited proliferation *in vitro.* A cell line can be isolated from a primary culture based on its ability to survive and continue to grow in culture. Cell lines which have been derived originally from tumor tissue may have been transformed *in vivo,* although not all neoplastic cell populations have the capacity to grow indefinitely *in vitro.* Further, cell lines generally retain their differentiated character through many rounds of division.

Suitable cell culture substrates are generally a container that can be sterilized, does not leach toxic factors and does not distort microscopy images. Thus plates formed from glass and plastic are suitable substrates herein. Plastic containers may further be treated to encourage cell attachment using techniques known in the art (Ramsey et al. 1984 In vitro 20:802). Suitable tissue culture media generally consist of an isotonic, buffered, basal nutrient medium which provides an energy source, coupled with inorganic salts, amino acids, vitamins and various supplements. Supplements may include serum (e.g., fetal calf serum, or the like) various antibiotics to prevent contamination or to provide selective conditions, attachment and growth factors, or the like. A number of media formulations are known in the art, such as, but not limited to, minimal essential medium (MEM), Rosewell Park Memorial Institute (RPMI) 1640 or Dulbecco's modified Eagle's medium (DMEM). Suitable tissue culture conditions are also known in the art. See, e.g., Morgan et al. 1993 Animal Cell Culture, BIOS Scientific Publishers Ltd., Oxford, UK, and Adams, R. L. P. 1990 Cell Culture for Biochemists, Second Edition, Elsevier. In another embodiment disclosed herein, methods of manufacture of CTP-modified polypeptide of interest is a serum-free process. In another embodiment disclosed herein, methods of manufacture of CTP-modified polypeptide of interest is an animal derived-free process.

In another embodiment of methods and compositions disclosed herein, the storage temperature of the culture is between ⁻20 and ⁻80 degrees Celsius (°C). In another embodiment, the temperature is significantly below ⁻20 °C. In another embodiment, the temperature is not warmer than ⁻70 °C. In another embodiment, the temperature is ⁻70 °C. In another embodiment, the temperature is about ⁻70 °C. In another embodiment, the temperature is ⁻20 °C. In another embodiment, the temperature is about ⁻20 °C. In another embodiment, the temperature is ⁻30 °C. In another embodiment, the temperature is 40 °C. In another embodiment, the temperature is⁻50 °C. In another embodiment, the temperature is ⁻60 °C. In another embodiment, the temperature is ⁻80 °C. In another embodiment, the temperature is ⁻30 - ⁻70 °C. In another embodiment, the temperature is ⁻40 - ⁻70 °C. In another embodiment, the temperature is ⁻50 - ⁻70 °C. In another embodiment, the temperature is ⁻60 - ⁻70 °C. In another embodiment, the temperature is ⁻30 - ⁻80 °C. In another embodiment, the temperature is ⁻40 - ⁻80 °C. In another embodiment, the temperature is ⁻50 - ⁻80 °C. In another embodiment, the temperature is ⁻60 - ⁻80 °C. In another embodiment, the temperature is ⁻70 - ⁻80 °C. In another embodiment, the temperature is colder than ⁻70 °C. In another embodiment, the temperature is colder than ⁻80 °C.

In another embodiment, for cryopreservation, the cells are frozen slowly until they reach a temperature below -70°C in medium that includes a cryoprotectant and vials are then transferred to a liquid-nitrogen freezer to maintain them at temperatures below -130°C.

In another embodiment of methods and compositions disclosed herein, the cryopreservation, or frozen storage, is for a maximum of 24 hours. In another embodiment, the cryopreservation, or frozen storage is for maximum of 2 days, is for maximum of 3 days, is for maximum of 4 days, is for maximum of 1 week, is for maximum of 2 weeks, is for maximum of 3 weeks, is for maximum of 1 month, is for maximum of 2 months, is for maximum of 3 months, is for maximum of 5 months, is for maximum of 6 months,is for maximum of 9 months, or is for maximum of 1 year. Each possibility listed above is an embodiment disclosed herein.

In another embodiment, the cryopreservation, or frozen storage is for a minimum of 1 week, is for minimum of 2 weeks, is for minimum of 3 weeks, is for minimum of 1 month, is for minimum of 2 months, is for minimum of 3 months, is for minimum of 5 months, is for minimum of 6 months, is for minimum of 9 months, is for minimum of 1 year, is for minimum of 1.5 years, is for minimum of 2 years, is for minimum of 3 years, is for minimum of 5 years, is for minimum of 7 years, is for minimum of 10 years, or is for longer than 10 years. Each possibility listed above is an embodiment disclosed herein.

In another embodiment of methods and compositions disclosed herein, the cells exhibit growth after thawing following an extended period of cryopreservation or frozen storage. In another embodiment, the cells exhibit growth within about 15-22 hours after inoculating fresh media with cells from the cell bank or starter culture. In another embodiment, the cells exhibit growth within about 12-20 hours after inoculating fresh media with cells from the cell bank or starter culture. In one embodiment, to ensure viability, genetic stability, and phenotypic stability, cell lines need to be maintained in the exponential growth phase (via subculturing on a regular basis).

An "extended period" of cryopreservation, or frozen storage, is, in another embodiment, 1 month. In another embodiment, the period is 2 months. In another embodiment, the period is 3 months. In another embodiment, the period is 5 months. In another embodiment, the period is 6 months. In another embodiment, the period is 9 months. In another embodiment, the period is 1 year. In another embodiment, the period is 1.5 years. In another embodiment, the period is 2 years. In another embodiment, the period is 2-7 years. In another embodiment, the period is for at least 7 years. In another embodiment, the period is for at least 10 years.

In another embodiment, the cells of the methods and compositions disclosed herein retain a viability of over 90% after thawing following cryopreservation. In another embodiment, the viability upon thawing is close to 100% following the period of cryopreservation. In another embodiment, the viability upon thawing is close to 90%. In another embodiment, the viability upon thawing is at least 90%. In another embodiment, the viability upon thawing is over 80%.

In another embodiment, a cell bank, frozen stock, or batch of vaccine doses disclosed herein is grown in a defined cell culture media. Such media are known in the art and may include, but not limited to Dulbecco's Modified Eagle's Medium (DMEM) (ATCC^{®} No. 30-2002), Iscove's Modified Dulbecco's Medium (IMDM) (ATCC^{®} No. 30-2005), Hybri-Care Medium (ATCC^{®} No. 46-X), McCoy's 5A and RPMI-1640 (ATCC^{®} No. 30-2007), Ham's Nutrient Mixtures (ATCC^{®} CCL-61^{™}), PowerCHO^{™} Chemically Defined, Serum-free CHO Medium (Lonza Cat. No. 12-771Q); or any other media known in the art. In another embodiment these media may be supplemented in antibiotics or animal sera, as will be empirically determined by the skilled artisan.

In one embodiment, disclosed herein are bioreactors and methods, which allow the cultivation of mammalian cells in large scale volumes. Furthermore, and in another embodiment, said bioreactors and methods, allow the cultivation of mammalian cells under optimal conditions, even if grown in large scale volumes and therefore allow a process performance and product quality independent of the size of the bioreactor. The duration of time of incubation within the bioreactor can vary, just by changing the scale and bioreactor system, for example the duration may be between 8-9, or it may be between 15-16 days. In another embodiment, the duration of incubation in a bioreactor is about 7 days, about 8 days, about 9 days, about 10 days, about 11 days, about 12 days, about 13 days, about 14 days, about 15 days, about 16 days, about 17 days, about 18 days, about 19 days, about 20 days or more. In another embodiment, when a perfusion bioreactor is used, the duration of incubation may be up to 7-120 days.

In another embodiment, disclosed herein are large-scale bioreactors which allow the cultivation of mammalian cells in a homogenous environment with respect to process parameters such as pH, dissolved oxygen tension (DOT) and temperature, maintaining a well-mixed cell suspension and blending nutrient feeds within the bioreactor. In another embodiment, the bioreactor is a disposable bioreactor.

Methods disclosed herein solves the technical problems underlying methods disclosed herein by the provision of bioreactors, bioreactor systems and methods for the cultivation of eukaryotic cells, especially of mammalian cells, according to the claims.

In one embodiment, the bioreactor has a volume of at least 250 liters (L). In another embodiment, the bioreactor has a volume of at least 500 L. In another embodiment the volume is at least 1000 L, at least 2000 L, at least 5,000 L, at least 10,000L at least 12,000L or at least 15,000 L.

In another embodiment, the cells are subcultivated in increasing volumes of bioreactors (see Example 12).

### Compositions

In some embodiment, polypeptide of interest polypeptides manufactured using the methods disclosed herein can be used to treat a subject, with conditions related to growth and weight, such as a growth deficiency disorder, AIDS wasting, aging, impaired immune function of HIV-infected subjects, a catabolic illness, surgical recovery, a congestive cardiomyopathy, liver transplantation, liver regeneration after hepatectomy, chronic renal failure, renal osteodystrophy, osteoporosis, achondroplasia/hypochondroplasia, skeletal dysplasia, a chronic inflammatory or nutritional disorder such as Crohn's disease, short bowel syndrome, juvenile chronic arthritis, cystic fibrosis, male infertility, X-linked hypophosphatemic rickets, Down's syndrome, Spina bifida, Noonan Syndrome, obesity, impaired muscle strength and fibromyalgia. In one embodiment, the polypeptides disclosed herein can be provided to the individual *per se.* In one embodiment, the polypeptides disclosed herein can be provided to the individual as part of a pharmaceutical composition where it is mixed with a pharmaceutically acceptable carrier.

A skilled artisan would appreciate that the term a "pharmaceutical composition" may encompass a preparation of one or more of the active ingredients described herein with other chemical components such as physiologically suitable carriers and excipients. The purpose of a pharmaceutical composition is to facilitate administration of a compound to an organism.

The modified peptides disclosed herein can be formulated into suitable pharmaceutical preparations such as capsules and injections in admixture with carriers, diluents, etc. known per se, which can be orally or parenterally administered to mammals (e.g. cows, horses, pigs, sheep, humans).

A skilled artisan would appreciate that the term "active ingredient" may encompass a polypeptide sequence of interest, which is accountable for the biological effect.

In some embodiments, any of the compositions disclosed herein will comprise at least two CTP sequences bound to a protein of interest, in any form. In one embodiment, disclosed herein are combined preparations. In one embodiment, "a combined preparation" defines especially a "kit of parts" in the sense that the combination partners as defined above can be dosed independently or by use of different fixed combinations with distinguished amounts of the combination partners i.e., simultaneously, concurrently, separately or sequentially. In some embodiments, the parts of the kit of parts can then, e.g., be administered simultaneously or chronologically staggered, that is at different time points and with equal or different time intervals for any part of the kit of parts. The ratio of the total amounts of the combination partners, in some embodiments, can be administered in the combined preparation. In one embodiment, the combined preparation can be varied, e.g., in order to cope with the needs of a patient subpopulation to be treated or the needs of the single patient which different needs can be due to a particular disease, severity of a disease, age, sex, or body weight as can be readily made by a person skilled in the art.

A skilled artisan would appreciate that the phrases "physiologically acceptable carrier" and "pharmaceutically acceptable carrier" which can be used interchangeably used refer to a carrier or a diluent that does not cause significant irritation to an organism and does not abrogate the biological activity and properties of the administered compound. An adjuvant is included under these phrases. In one embodiment, one of the ingredients included in the pharmaceutically acceptable carrier can be for example polyethylene glycol (PEG), a biocompatible polymer with a wide range of solubility in both organic and aqueous media (Mutter et al. (1979).

A skilled artisan would appreciate that the term "excipient" may encompass an inert substance added to a pharmaceutical composition to further facilitate administration of an active ingredient. In one embodiment, excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition, which is incorporated herein by reference.

In one embodiment, suitable routes of administration, for example, include oral, rectal, transmucosal, transnasal, intestinal or parenteral delivery, including intramuscular, subcutaneous and intramedullary injections as well as intrathecal, direct intraventricular, intravenous, intraperitoneal, intranasal, or intraocular injections.

In one embodiment, the preparation is administered in a local rather than systemic manner, for example, via injection of the preparation directly into a specific region of a patient's body.

In another embodiment, polypeptides of interest disclosed herein comprising a polypeptide of interest are administered in a dose of 1-90 micrograms in 0.1-5 ml solution. In another embodiment, polypeptides disclosed herein comprising a polypeptide of interest are administered in a dose of 1-50 micrograms in 0.1-5 ml solution. In another embodiment, polypeptides disclosed herein comprising a polypeptide of interest are administered in a dose of 1-25 micrograms in 0.1-5 ml solution. In another embodiment, polypeptides disclosed herein comprising a polypeptide of interest are administered in a dose of 50-90 micrograms in 0.1-5 ml solution. In another embodiment, polypeptides disclosed herein comprising a polypeptide of interest are administered in a dose of 10-50 micrograms in 0.1-5 ml solution.

In another embodiment, polypeptides of interest disclosed herein comprising a polypeptide of interest are administered in a dose of 1-90 micrograms in 0.1-5 ml solution by intramuscular (IM) injection, subcutaneous (SC) injection, or intravenous (IV) injection once a week. In another embodiment, polypeptides disclosed herein comprising a polypeptide of interest are administered in a dose of 1-90 micrograms in 0.1-5 ml solution by intramuscular (IM) injection, subcutaneous (SC) injection, or intravenous (IV) injection twice a week. In another embodiment, polypeptides disclosed herein comprising a polypeptide of interest are administered in a dose of 1-90 micrograms in 0.1-5 ml solution by intramuscular (IM) injection, subcutaneous (SC) injection, or intravenous (IV) injection three times a week. In another embodiment, polypeptides disclosed herein comprising a polypeptide of interest are administered in a dose of 1-90 micrograms in 0.1-5 ml solution by intramuscular (IM) injection, subcutaneous (SC) injection, or intravenous (IV) injection once every two weeks. In another embodiment, polypeptides disclosed herein comprising a polypeptide of interest are administered in a dose of 1-90 micrograms in 0.1-5 ml solution by intramuscular (IM) injection, subcutaneous (SC) injection, or intravenous (IV) injection once every 17 days. In another embodiment, polypeptides disclosed herein comprising a polypeptide of interest are administered in a dose of 1-90 micrograms in 0.1-5 ml solution by intramuscular (IM) injection, subcutaneous (SC) injection, or intravenous (IV) injection once every 19 days weeks.

In another embodiment, protein drugs of molecular weight lower than 50,000 daltons, such as interferons, are in general short-lived species *in vivo,* having short circulatory half-lives of several hours. In another embodiment, the subcutaneous route of administration in general provides slower release into the circulation. In another embodiment, the CTP modified polypeptide disclosed herein prolongs the half-live of protein drugs of molecular weight lower than 50,000 daltons, such as interferons. In another embodiment, the CTP modified polypeptide disclosed herein enable interferons to exert their beneficial effects for a longer period of time.

In another embodiment, the immunogenicity of a CTP modified polypeptide comprising a polypeptide of interest is equal to an isolated polypeptide of interest. In another embodiment, the immunogenicity of a CTP modified polypeptide comprising a polypeptide of interest is comparable to an isolated polypeptide of interest. In another embodiment, modifying a polypeptide of interest as described herein with CTP peptides reduces the immunogenicity of the polypeptide of interest. In another embodiment, the CTP modified polypeptide comprising a polypeptide of interest is as active as an isolated polypeptide of interest protein. In another embodiment, the CTP modified polypeptide comprising a polypeptide of interest is more active than an isolated polypeptide of interest. In another embodiment, the CTP modified polypeptide comprising a polypeptide of interest maximizes the polypeptide of interest's protective ability against degradation while minimizing reductions in bioactivity.

In another embodiment, the polypeptide of interest disclosed herein can be provided to the individual *per se.* In one embodiment, the polypeptide of interest disclosed herein can be provided to the individual as part of a pharmaceutical composition where it is mixed with a pharmaceutically acceptable carrier.

Various embodiments of dosage ranges are contemplated herein. The dosage of the polypeptide disclosed herein, in one embodiment, is in the range of 0.05-80 mg/day. In another embodiment, the dosage is in the range of 0.05-50 mg/day. In another embodiment, the dosage is in the range of 0.1-20 mg/day. In another embodiment, the dosage is in the range of 0.1-10 mg/day. In another embodiment, the dosage is in the range of 0.1-5 mg/day. In another embodiment, the dosage is in the range of 0.5-5 mg/day. In another embodiment, the dosage is in the range of 0.5-50 mg/day. In another embodiment, the dosage is in the range of 5-80 mg/day. In another embodiment, the dosage is in the range of 35-65 mg/day. In another embodiment, the dosage is in the range of 35-65 mg/day. In another embodiment, the dosage is in the range of 20-60 mg/day. In another embodiment, the dosage is in the range of 40-60 mg/day. In another embodiment, the dosage is in a range of 45-60 mg/day. In another embodiment, the dosage is in the range of 40-60 mg/day. In another embodiment, the dosage is in a range of 60-120 mg/day. In another embodiment, the dosage is in the range of 120-240 mg/day. In another embodiment, the dosage is in the range of 40-60 mg/day. In another embodiment, the dosage is in a range of 240-400 mg/day. In another embodiment, the dosage is in a range of 45-60 mg/day. In another embodiment, the dosage is in the range of 15-25 mg/day. In another embodiment, the dosage is in the range of 5-10 mg/day. In another embodiment, the dosage is in the range of 55-65 mg/day.

In one embodiment, the dosage is 20 mg/day. In another embodiment, the dosage is 30 mg/day. In another embodiment, the dosage is 40 mg/day. In another embodiment, the dosage is 50 mg/day. In another embodiment, the dosage is 60 mg/day. In another embodiment, the dosage is 70 mg/day. In another embodiment, the dosage is 80 mg/day. In another embodiment, the dosage is 90 mg/day. In another embodiment, the dosage is 100 mg/day.

Various embodiments of dosage ranges are contemplated herein. The dosage of the cytokine disclosed herein, in one embodiment, is in the range of 0.005-100 mg/day. In another embodiment, the dosage is in the range of 0.005-5 mg/day. In another embodiment, the dosage is in the range of 0.01-50 mg/day. In another embodiment, the dosage is in the range of 0.1-20 mg/day. In another embodiment, the dosage is in the range of 0.1-10 mg/day. In another embodiment, the dosage is in the range of 0.01-5 mg/day. In another embodiment, the dosage is in the range of 0.001-0.01 mg/day. In another embodiment, the dosage is in the range of 0.001-0.1 mg/day. In another embodiment, the dosage is in the range of 0.1-5 mg/day. In another embodiment, the dosage is in the range of 0.5-50 mg/day. In another embodiment, the dosage is in the range of 0.2-15mg/day. In another embodiment, the dosage is in the range of 0.8-65 mg/day. In another embodiment, the dosage is in the range of 1-50 mg/day. In another embodiment, the dosage is in the range of 5-10 mg/day. In another embodiment, the dosage is in the range of 8-15 mg/day. In another embodiment, the dosage is in a range of 10-20mg/day. In another embodiment, the dosage is in the range of 20-40 mg/day. In another embodiment, the dosage is in a range of 60-120 mg/day. In another embodiment, the dosage is in the range of 12-40 mg/day. In another embodiment, the dosage is in the range of 40-60 mg/day. In another embodiment, the dosage is in a range of 50-100mg/day. In another embodiment, the dosage is in a range of 1-60 mg/day. In another embodiment, the dosage is in the range of 15-25 mg/day. In another embodiment, the dosage is in the range of 5-10 mg/day. In another embodiment, the dosage is in the range of 55-65 mg/day.

In another embodiment, a polypeptide comprising a polypeptide of interest and CTP units is formulated in an intranasal dosage form. In another embodiment, a polypeptide comprising a polypeptide of interest and CTP units is formulated in an injectable dosage form. In another embodiment, a polypeptide comprising a polypeptide of interest and CTP units is administered to a subject in a dose ranging from 0.0001 mg to 0.6 mg. In another embodiment, a polypeptide comprising a polypeptide of interest and CTP units is administered to a subject in a dose ranging from 0.001 mg to 0.005 mg. In another embodiment, a polypeptide comprising a polypeptide of interest and CTP units is administered to a subject in a dose ranging from 0.005 mg to 0.01 mg. In another embodiment, a polypeptide comprising a polypeptide of interest and CTP units is administered to a subject in a dose ranging from 0.01 mg to 0.3 mg. In another embodiment, a polypeptide comprising a polypeptide of interest and CTP units is administered to a subject in a dose in a dose ranging from 0.2 mg to 0.6 mg.

In another embodiment, a polypeptide comprising a polypeptide of interest and CTP units is administered to a subject in a dose ranging from 1-100 micrograms. In another embodiment, a polypeptide comprising a polypeptide of interest and CTP units is administered to a subject in a dose ranging from 10-80 micrograms. In another embodiment, a polypeptide comprising a polypeptide of interest and CTP units is administered to a subject in a dose ranging from 20-60 micrograms. In another embodiment, a polypeptide comprising a polypeptide of interest and CTP units is administered to a subject in a dose ranging from 10-50 micrograms. In another embodiment, a polypeptide comprising a polypeptide of interest and CTP units is administered to a subject in a dose ranging from 40-80 micrograms. In another embodiment, a polypeptide comprising a polypeptide of interest and CTP units is administered to a subject in a dose ranging from 10-30 micrograms. In another embodiment, a polypeptide comprising a polypeptide of interest and CTP units is administered to a subject in a dose ranging from 30-60 micrograms.

In another embodiment, a polypeptide comprising a polypeptide of interest and CTP units is administered to a subject in a dose ranging from 0.2 mg to 2 mg. In another embodiment, a polypeptide comprising a polypeptide of interest and CTP units is administered to a subject in a dose ranging from 2 mg to 6 mg. In another embodiment, a polypeptide comprising a polypeptide of interest and CTP units is administered to a subject in a dose ranging from 4 mg to 10 mg. In another embodiment, a polypeptide comprising a polypeptide of interest and CTP units is administered to a subject in a dose ranging from 5 mg and 15 mg.

In another embodiment, a polypeptide comprising a polypeptide of interest and CTP units is injected into the muscle (intramuscular injection). In another embodiment, a polypeptide comprising a polypeptide of interest and CTP units is injected below the skin (subcutaneous injection). In another embodiment, a polypeptide comprising an IFN protein and CTP units is injected into the muscle. In another embodiment, a polypeptide comprising an IFN protein and CTP units is injected below the skin.

In another embodiment, the methods disclosed herein include increasing the compliance in the use of polypeptide of interest therapy, comprising providing to a subject in need thereof, a polypeptide comprising a polypeptide of interest, one CTP attached to an amino terminus of the polypeptide of interest, and two CTP attached to a carboxy terminus of the polypeptide of interest, thereby increasing compliance in the use of polypeptide of interest therapy.

In another embodiment, the methods disclosed herein include increasing the compliance of patients afflicted with chronic illnesses that are in need of a polypeptide of interest therapy. In another embodiment, the methods disclosed herein enable reduction in the dosing frequency of a polypeptide of interest by modifying the polypeptide of interest with CTPs as described hereinabove. In another embodiment, the term compliance comprises adherence. In another embodiment, the methods disclosed herein include increasing the compliance of patients in need of a polypeptide of interest therapy by reducing the frequency of administration of the polypeptide of interest. In another embodiment, reduction in the frequency of administration of the polypeptide of interest is achieved due to the CTP modifications which render the CTP-modified polypeptide of interest more stable. In another embodiment, reduction in the frequency of administration of the polypeptide of interest is achieved as a result of increasing T1/2 of the polypeptide of interest. In another embodiment, reduction in the frequency of administration of the polypeptide of interest is achieved as a result of increasing the clearance time of the polypeptide of interest. In another embodiment, reduction in the frequency of administration of the polypeptide of interest is achieved as a result of increasing the AUC measure of the polypeptide of interest.

In another embodiment, a polypeptide comprising a polypeptide of interest and CTP units is administered to a subject once a day. In another embodiment, a polypeptide comprising a polypeptide of interest and CTP units is administered to a subject once every two days. In another embodiment, a polypeptide comprising a polypeptide of interest and CTP units is administered to a subject once every three days. In another embodiment, a polypeptide comprising a polypeptide of interest and CTP units is administered to a subject once every four days. In another embodiment, a polypeptide comprising a polypeptide of interest and CTP units is administered to a subject once every five days. In another embodiment, a polypeptide comprising a polypeptide of interest and CTP units is administered to a subject once every six days. In another embodiment, a polypeptide comprising a polypeptide of interest and CTP units is administered to a subject once every week. In another embodiment, a polypeptide comprising a polypeptide of interest and CTP units is administered to a subject once every 7-14 days. In another embodiment, a polypeptide comprising a polypeptide of interest and CTP units is administered to a subject once every 10-20 days. In another embodiment, a polypeptide comprising a polypeptide of interest and CTP units is administered to a subject once every 5-15 days. In another embodiment, a polypeptide comprising a polypeptide of interest and CTP units is administered to a subject once every 15-30 days.

In another embodiment, the dosage is in a range of 50-500 mg/day. In another embodiment, the dosage is in a range of 50-150 mg/day. In another embodiment, the dosage is in a range of 100-200 mg/day. In another embodiment, the dosage is in a range of 150-250 mg/day. In another embodiment, the dosage is in a range of 200-300 mg/day. In another embodiment, the dosage is in a range of 250-400 mg/day. In another embodiment, the dosage is in a range of 300-500 mg/day. In another embodiment, the dosage is in a range of 350-500 mg/day.

In one embodiment, the dosage is 20 mg/day. In one embodiment, the dosage is 30 mg/day. In one embodiment, the dosage is 40 mg/day. In one embodiment, the dosage is 50 mg/day. In one embodiment, the dosage is 0.01 mg/day. In another embodiment, the dosage is 0.1 mg/day. In another embodiment, the dosage is 1 mg/day. In another embodiment, the dosage is 0.530 mg/day. In another embodiment, the dosage is 0.05 mg/day. In another embodiment, the dosage is 50 mg/day. In another embodiment, the dosage is 10 mg/day. In another embodiment, the dosage is 20-70 mg/day. In another embodiment, the dosage is 5 mg/day.

In another embodiment, the dosage is 1-90 mg/day. In another embodiment, the dosage is 1-90 mg/2 days. In another embodiment, the dosage is 1-90 mg/3 days. In another embodiment, the dosage is 1-90 mg/4 days. In another embodiment, the dosage is 1-90 mg/5 days. In another embodiment, the dosage is 1-90 mg/6 days. In another embodiment, the dosage is 1-90 mg/week. In another embodiment, the dosage is 1-90 mg/9 days. In another embodiment, the dosage is 1-90 mg/11 days. In another embodiment, the dosage is 1-90 mg/14 days.

In another embodiment, the polypeptide of interest dosage is 10-50 mg/day. In another embodiment, the dosage is 10-50 mg/2 days. In another embodiment, the dosage is 10-50 mg/3 days. In another embodiment, the dosage is 10-50 mg/4 days. In another embodiment, the dosage is 10-50 micrograms mg/5 days. In another embodiment, the dosage is 10-50 mg/6 days. In another embodiment, the dosage is 10-50 mg/week. In another embodiment, the dosage is 10-50 mg/9 days. In another embodiment, the dosage is 10-50 mg/11 days. In another embodiment, the dosage is 10-50 mg/14 days.

Oral administration, in one embodiment, comprises a unit dosage form comprising tablets, capsules, lozenges, chewable tablets, suspensions, emulsions and the like. Such unit dosage forms comprise a safe and effective amount of the desired polypeptide of interest disclosed herein, each of which is in one embodiment, from about 0.7 or 3.5 mg to about 280 mg/70 kg, or in another embodiment, about 0.5 or 10 mg to about 210 mg/70 kg. The pharmaceutically-acceptable carriers suitable for the preparation of unit dosage forms for peroral administration are well-known in the art. In some embodiments, tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. In one embodiment, glidants such as silicon dioxide can be used to improve flow characteristics of the powder-mixture. In one embodiment, coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. In some embodiments, the selection of carrier components depends on secondary considerations like taste, cost, and shelf stability, which are not critical for the purposes disclosed herein, and can be readily made by a person skilled in the art.

In another embodiment, disclosed herein is a method of reducing the dosing frequency of a polypeptide of interest, comprising the step of attaching at least one CTP to the polypeptide of interest as disclosed hereinabove in detail.

In another embodiment, disclosed herein is a method of increasing compliance in the use of therapy administering a polypeptide of interest, comprising providing to a subject in need thereof, a CTP-modified polypeptide of interest disclosed herein, wherein said CTP-modified polypeptide is manufactured as disclosed herein.

In one embodiment, determination of a therapeutically effective amount is well within the capability of those skilled in the art.

In one embodiment, compositions including the preparation disclosed herein formulated in a compatible pharmaceutical carrier are also be prepared, placed in an appropriate container, and labeled for treatment of an indicated condition.

In another embodiment, a polypeptide of interest, for example EPO, IFN, a cytokine, a coagulation factor, FVII, FVIIa, FIX, dual GLP-1/Glucagon receptor agonist, GLP-1, or OXM - as described herein, is lyophilized (i.e., freeze-dried) preparation in combination with complex organic excipients and stabilizers such as nonionic surface active agents (i.e., surfactants), various sugars, organic polyols and/or human serum albumin. In another embodiment, a pharmaceutical composition comprises a lyophilized polypeptide of interest, for example EPO, IFN, a cytokine, a coagulation factor, FVII, FVIIa, FIX, GLP-1/Glucagon receptor agonist, GLP-1, or OXM - as described herein as described in sterile water for injection. In another embodiment, a pharmaceutical composition comprises a lyophilized coagulation factor as described in sterile PBS for injection. In another embodiment, a pharmaceutical composition comprises a lyophilized polypeptide of interest, for example EPO, IFN, a cytokine, a coagulation factor, FVII, FVIIa, FIX, GLP-1/Glucagon receptor agonist, GLP-1, or OXM - as described herein as described in sterile 0.9% NaCl for injection.

In another embodiment, the pharmaceutical composition comprises a polypeptide of interest, for example EPO, IFN, a cytokine, a coagulation factor, FVII, FVIIa, FIX, GLP-1/Glucagon receptor agonist, GLP-1, or OXM - as described herein and complex carriers such as human serum albumin, polyols, sugars, and anionic surface active stabilizing agents. In another embodiment, the pharmaceutical composition comprises a coagulation factor as described herei a polypeptide of interest, for example EPO, IFN, a cytokine, a coagulation factor, FVII, FVIIa, FIX, GLP-1/Glucagon receptor agonist, GLP-1, or OXM - as described herein n and lactobionic acid and an acetate/glycine buffer. In another embodiment, the pharmaceutical composition comprises a polypeptide of interest, for example EPO, IFN, a cytokine, a coagulation factor, FVII, FVIIa, FIX, GLP-1/Glucagon receptor agonist, GLP-1, or OXM - as described herein and amino acids, such as arginine or glutamate that increase the solubility of interferon compositions in water. In another embodiment, the pharmaceutical composition comprises a lyophilized polypeptide of interest, for example EPO, IFN, a cytokine, a coagulation factor, FVII, FVIIa, FIX, GLP-1/Glucagon receptor agonist, GLP-1, or OXM - as described herein as described herein and glycine or human serum albumin (HSA), a buffer (e g. acetate) and an isotonic agent (e.g NaCl). In another embodiment, the pharmaceutical composition comprises a lyophilized polypeptide of interest, for example EPO, IFN, a cytokine, a coagulation factor, FVII, FVIIa, FIX, GLP-1/Glucagon receptor agonist, GLP-1, or OXM - as described herein as described herein and phosphate buffer, glycine and HSA.

In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein is stabilized when placed in buffered solutions having a pH between about 4 and 7.2. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein is in a buffered solution having a pH between about 4 and 8.5. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein is in a buffered solution having a pH between about 6 and 7. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein is in a buffered solution having a pH of about 6.5. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein is stabilized with an amino acid as a stabilizing agent and in some cases a salt (if the amino acid does not contain a charged side chain).

In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein is a liquid composition comprising a stabilizing agent at between about 0.3% and 5% by weight which is an amino acid.

In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein provides dosing accuracy and product safety. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein provides a biologically active, stable liquid formulation for use in injectable applications. In another embodiment, the pharmaceutical composition comprises a non-lyophilized polypeptide of interest, for example EPO, IFN, a cytokine, a coagulation factor, FVII, FVIIa, FIX, GLP-1/Glucagon receptor agonist, GLP-1, or OXM - as described herein as described herein.

In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein provides a liquid formulation permitting storage for a long period of time in a liquid state facilitating storage and shipping prior to administration.

In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises solid lipids as matrix material. In another embodiment, the injectable pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises solid lipids as matrix material. In another embodiment, the production of lipid microparticles by spray congealing was described by Speiser (Speiser and al., Pharm. Res. 8 (1991) 47-54) followed by lipid nanopellets for peroral administration (Speiser EP 0167825 (1990)). In another embodiment, lipids, which are used, are well tolerated by the body (e. g. glycerides composed of fatty acids which are present in the emulsions for parenteral nutrition).

In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises polymeric microparticles. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises nanoparticles. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises liposomes. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises lipid emulsion. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises microspheres. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises lipid nanoparticles. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises lipid nanoparticles comprising amphiphilic lipids. In another embodiment, the pharmaceutical composition comprising a polypeptide of interest, for example EPO, IFN, a cytokine, a coagulation factor, FVII, FVIIa, FIX, GLP-1/Glucagon receptor agonist, GLP-1, or OXM - as described herein as described herein comprises lipid nanoparticles comprising a drug, a lipid matrix and a surfactant. In another embodiment, the lipid matrix has a monoglyceride content which is at least 50% w/w.

In one embodiment, compositions disclosed herein are presented in a pack or dispenser device, such as an FDA approved kit, which contain one or more unit dosage forms containing the active ingredient. In one embodiment, the pack, for example, comprise metal or plastic foil, such as a blister pack. In one embodiment, the pack or dispenser device is accompanied by instructions for administration. In one embodiment, the pack or dispenser is accommodated by a notice associated with the container in a form prescribed by a governmental agency regulating the manufacture, use or sale of pharmaceuticals, which notice is reflective of approval by the agency of the form of the compositions or human or veterinary administration. Such notice, in one embodiment, is labeling approved by the U.S. Food and Drug Administration for prescription drugs or of an approved product insert.

In one embodiment, it will be appreciated that the polypeptide of interest, for example EPO, IFN, a cytokine, a coagulation factor, FVII, FVIIa, FIX, GLP-1/Glucagon receptor agonist, GLP-1, or OXM - as described hereins disclosed herein can be provided to the individual with additional active agents to achieve an improved therapeutic effect as compared to treatment with each agent by itself. In another embodiment, measures (e.g., dosing and selection of the complementary agent) are taken to avoid adverse side effects which are associated with combination therapies.

In one embodiment, disclosed herein is a cell comprising the expression vector. In another embodiment, disclosed herein is a composition comprising the expression vector.

As is generally known in the art, the modified peptides and proteins disclosed herein may be coupled to labels, drugs, targeting agents, carriers, solid supports, and the like, depending on the desired application. The labeled forms of the modified biologicals may be used to track their metabolic fate; suitable labels for this purpose include, especially, radioisotope labels such as iodine 131, technetium 99, indium 111, and the like. The labels may also be used to mediate detection of the modified proteins or peptides in assay systems; in this instance, radioisotopes may also be used as well as enzyme labels, fluorescent labels, chromogenic labels, and the like. The use of such labels is particularly helpful if the peptide or protein is itself a targeting agent such as an antibody or a receptor ligand.

Similar linking techniques, along with others, may be employed to couple the modified peptides and proteins disclosed herein to solid supports. When coupled, these modified peptides and proteins can then be used as affinity reagents for the separation of desired components with which specific reaction is exhibited.

Finally, the modified peptides and proteins disclosed herein may be used to generate antibodies specifically immunoreactive with these new compounds. These antibodies are useful in a variety of diagnostic and therapeutic applications, depending on the nature of the biological activity of the unmodified peptide or protein. It is to be understood that disclosed herein is antibodies that are immunoreactive with CTP-modified polypeptide as described herein. In one embodiment, such antibodies may be used to distinguish or identify CTP-modified polypeptides of interest, for example EPO, IFN, a cytokine, a coagulation factor, FVII, FVIIa, FIX, GLP-1/Glucagon receptor agonist, GLP-1, or OXM - as described herein that were administered from endogenous polypeptides of interest, for example EPO, IFN, a cytokine, a coagulation factor, FVII, FVIIa, FIX, GLP-1/Glucagon receptor agonist, GLP-1, or OXM - as described herein, respectively. In another embodiment, the antibodies may be used to localize administered CTP-modified polypeptides of interest, for example EPO, IFN, a cytokine, a coagulation factor, FVII, FVIIa, FIX, GLP-1/Glucagon receptor agonist, GLP-1, or OXM - as described herein.

It is to be understood that the compositions and methods disclosed herein comprising the elements or steps as described herein may, in another embodiment, consist of those elements or steps, or in another embodiment, consist essentially of those elements or steps. A skilled artisan would appreciate that the term "comprise" may encompass inclusion of the indicated active agent, such as the CTP-modified polypeptide of interest, as well as inclusion of other active agents, and pharmaceutically acceptable carriers, excipients, emollients, stabilizers, etc., as are known in the pharmaceutical industry. A skilled artisan would appreciate that the term "consisting essentially of" may encompass a composition, whose only active ingredient is the indicated active ingredient, however, other compounds may be included which are for stabilizing, preserving, etc. the formulation, but are not involved directly in the therapeutic effect of the indicated active ingredient. A skilled artisan would appreciate that the term "consisting essentially of" may encompass components which facilitate the release of the active ingredient. A skilled artisan would appreciate that the the term "consisting" may encompass a composition, which contains the active ingredient and a pharmaceutically acceptable carrier or excipient.

Additional objects, advantages, and novel features disclosed herein will become apparent to one ordinarily skilled in the art upon examination of the following examples, which are not intended to be limiting. Additionally, each of the various embodiments and aspects disclosed herein as delineated hereinabove and as claimed in the claims section below finds experimental support in the following examples.

### EXAMPLES

### EXAMPLE 1 - Generation of EPO constructs

### MATERIALS AND METHODS:

***Construction of expression vector pCI-dhfr:*** pCI-neo mammalian expression vector was purchased from Promega (catalog no.E1841). The vector contains a CMV IE enhancer/promoter and neomycin phosphotransferase gene. The pSV2-dhfr clone was purchased from ATCC (Catalog No.37146). The plasmid contains the murine dhfr gene. The construction of pCI-dhfr vector was performed as follows:

The pSV2-dhfr plasmid was digested with restriction enzyme BglII (3' end of the dhfr gene). DNA polymerase I, Large (Klenow) Fragment was used to fill-in the 5' overhangs to form blunt ends. The DNA was then digested with restriction enzyme AvrII (5' end of the dhfr gene). The dhfr gene (AvrII -blunt end) fragment was isolated.

The pCI-neo vector was digested with restriction enzyme BstXI (3' end of the neo gene). DNA polymerase I, Large (Klenow) Fragment was used to remove the 3' overhangs to form blunt ends. The DNA was then digested with restriction enzyme AvrII (5' end of the neo gene). The expression vector (AvrII -blunt end) was isolated.

The dhfr gene was ligated into pCI vector to form an expression vector containing the dhfr gene (pCI-dhfr).

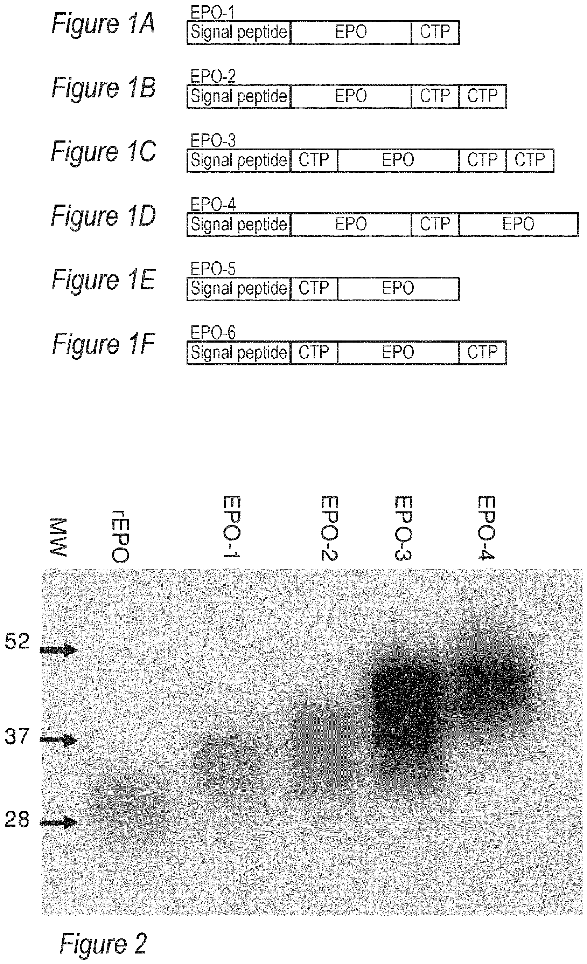

***Construction of hEPO-CTP variants:*** A cassette gene containing the C-Terminal peptide (CTP) of the beta subunit of hCG was fused to the coding sequence of human EPO (NP_000790.2) at different locations. Four EPO-CTP variants were constructed as illustrated in Figures 1A-D. The proEPO signal peptide was used for the construction of the secreted EPO-CTP variants. XbaI - NotI fragments containing Epo sequences were ligated into the pCI-dhfr expression vector disclosed herein.

**Table 2** hereinbelow summarizes the primer sequences used for constructing the CTP - containing polypeptides disclosed herein.

**Table 2**

| Primer number | SEQ ID NO | sequence | Restriction site (underlined in sequence) |
|---|---|---|---|
| 1 | 72 | 5' AATCTAGAGGTCATCATGGGGGTGC 3' | XbaI |
| 2 | 73 | 5'ATTGCGGCCGCGGATCCAGAAGACCTTTATTG 3' | NotI |
| 17^{R} | 74 | 5' TAAATATTGGGGTGTCCGAGGGCCC 3' | SspI |
| 10 | 75 | 5' CCAATATTACCACAAGCCCCACCACGCCTCAT 3' | SspI |
| 11^{R} | 76 | 5'TGCGGCCGCGGATCCTTATCTGTCCCCTGTCCTGC 3' | NotI |
| 15 | 77 | 5' GCCCTGCTGTCGGAAGC 3' | |
| 2^{R} | 78 | 5' ATTGCGGCCGCGGATCCAGAAGACCTTTATTG | NotI |
| 23^{R} | 79 | 5'CTTTGAGGAAGAGGAGCCCAGGACTGGGAGGC3' | |
| 24 | 80 | 5' CCTGGGCTCCTCTTCCTCAAAGGC 3' | |
| 38^{R} | 81 | 5' GCTTCCGACAGCAGGGC 3' | |

***EPO-1 701-1-p17-6 (Epo-1*** - ***SEQ ID NO: 1):*** The XbaI-NotI 702 bp fragment was constructed by PCR using the above primers (SEQ ID NOs: 72-81). Then the XbaI-NotI PCR fragment containing Epo-ctp sequence was ligated into pCI-dhfr expression vector.

***EPO-2 701-2-p24-2 (Epo-2- SEQ ID NO: 2):*** The XbaI/ApaT fragment (hGH-ctp) of pCI-dhfr-401-2-p21-2 (hGH-ctpx2) was replaced by the XbaI/ApaI fragment (EPO-ctp) of 701-1-p17-6 to create an Epo-ctpx2.

***EPO-4-701-4-p42-1(Epo-4* - *SEQ ID NO: 4):*** Firstly, a fragment from pCI-dhfr- EPO-ctp (701-1-p17-6) was constructed by PCR using primers 1 and 17 followed by XbaI/SspI digestion. This resulted in a fragment containing EPO and partial 5' CTP.

Secondly, a new fragment was constructed by overlapping PCR, on pGT123-hEpo as a template, using primer 10 and primer 11. SspI/NotI digestion resulted in fragment containing 3' partial CTP and Epo.

The two fragments were ligated into pCI-dhfr to construct the p701-4-p42-1 clone.

***EPO-3-p56-6 (Epo-3 SEQ ID NO; 3):*** Primers were purchased from Sigma-Genosys. PCR reaction was performed using primer 15 (SEQ ID NO: 77) and primer 2^{R} (SEQ ID NO: 78) and plasmid DNA of pCI-dhfr- EPO-ctp x2 (701-2-p24-2) as a template. As a result of the PCR amplification, a 486 bp product was formed and ligated into TA cloning vector (Invitrogen, catalog K2000-01). Stu I -NotI fragment containing *Epo-ctp x2 sequence was isolated (209 bp).

Three sequential PCR reactions were performed. The first reaction was conducted with primer 1 (SEQ ID NO: 72) and primer 23^{R} (SEQ ID NO: 79) and plasmid DNA of pGT123-epo-ctp as a template; as a result of the PCR amplification, an 80 bp product was formed (signal peptide).

The second reaction was conducted with primer 24 (SEQ ID NO: 80) and primer 11^{R} (SEQ ID NO: 76) and plasmid DNA of 701-4-p42-1 as a template; as a result of the PCR amplification, a 610 bp product was formed.

The last reaction was conducted with primers 1 (SEQ ID NO: 72) and 11^{R} (SEQ ID NO: 76) and a mixture of the products of the previous two reactions as a template; as a result of the PCR amplification, a 700 bp product was formed and the XbaI-StuI fragment was isolated.

The two fragments (XbaI-StuI and StuI -NotI) were inserted into the eukaryotic expression vector pCI-dhfr (triple ligation) to yield the 701-3-p56-6 clone.

***EPO-5-p91-4 (Epo-5 SEQ ID NO; 5*** - ***(ctp- Epo):*** Primers were ordered from Sigma-Genosys. A PCR reaction was performed using primer 1 (SEQ ID NO: 72) and primer 11^{R} (SEQ ID NO: 76) and plasmid DNA of pCI-dhfr- ctp-EPO-ctp x2 (701-3-p56-6) as a template; as a result of the PCR amplification, a 670 bp product was formed and ligated into TA cloning vector (Invitrogen, catalog K2000-01). XbaI -NotI fragment containing ctp-Epo sequence was ligated into our eukaryotic expression vector pCI-dhfr to yield the 701-5-p91-4 clone.

***EPO-6-p90-1 (Epo-6 SEQ ID NO: 6* - *(ctp- Epo-ctp):*** Three PCR reactions were performed. The first reaction was conducted with primer 1 (SEQ ID NO: 72) and primer 38^{R} (SEQ ID NO: 81) and plasmid DNA of 701-3-p56-6 as a template; as a result of the PCR amplification, a 400 bp product was formed.

The second reaction was conducted with primer 15 (SEQ ID NO: 77) and primer 2^{R} (SEQ ID NO: 78) and plasmid DNA of 701-1-p17-6 as a template; as a result of the PCR amplification, a 390 bp product was formed.

The last reaction was conducted with primers 1 (SEQ ID NO: 72) and 2^{R} (SEQ ID NO: 78) and a mixture of the products of the previous two reactions as a template; as a result of the PCR amplification, a 787 bp product was formed and ligated into TA cloning vector (Invitrogen, catalog K2000-01). The XbaI -NotI fragment containing ctp-Epo-ctp sequence was ligated into the eukaryotic expression vector pCI-dhfr to yield the 701-6-p90-1 clone.

### EXAMPLE 2 - Expression and Isolation of EPO-CTP polypeptides

### MATERIALS AND METHODS

***DNA transfection and clone selection:*** DG44 cells were transfected with pCI-DHFR expression vectors containing EPO-CTP variants using FuGENE6 Reagent (FuGENE Transfection Reagent - Roche Cat.11 815 091 001). 48 hr following transfection, cells were diluted and seeded at least 50-200 cells per well in a selective medium (CD DG44 Medium w/o HT (Gibco: Scotland part: #07990111A) Sku num.:ME060027 supplemented with 8 mM L-Glutamine Biological Industries: Cat: 03-020-1A) and 18 mL/L of 10 % Pluronic F-68 solution (Gibco: Cat: 240040-032). Selected clones were screened for highest protein production using commercial ELISA. 3-5 producing clones per each variant were frozen for a backup cell bank. A selected clone for each variant was adapted to growth in larger scale cultures up to 1L flasks on an orbital shaker platform. Supernatants were collected and analyzed by ELISA, SDS-PAGE and western blot. Following the withdrawal of aliquots, the protein-containing supernatants were kept frozen until further use.

***Cell culture:*** DG44 cells were maintained in DG44 medium with HT (cat# 12610-010, Gibco) supplemented with 8 mM L-Glutamine (Biological Industries: Cat: 03-020-1A) and 18 mL/L of 10 % Pluronic F-68 solution (Gibco: Cat: 240040-032), at 37 ⁰C in humidified 8 % CO₂ incubator. Transfected clones were maintained in DG44 basal medium without HT supplement, hypoxanthine and thymidine, with pluronic acid and L-glutamine.

***Sample preparation:*** Supernatants were collected, filtrated and analyzed by ELISA to determine protein concentration. SDS-PAGE and western blot were used to determine purity and identity. Following ELISA, sample concentrations were defined and the solution was dialyzed against PBS. Following the withdrawal of aliquots, the protein-contained supernatants were kept frozen at -20 ⁰C until further use.

***Western Blotting:*** Samples were electrophoresed on nondenaturing 15 % SDS-polyacrylamide gels. Gels were allowed to equilibrate for 10 min in 25 mM Tris and 192 mM glycine in 20 % (vol/vol) methanol). Proteins were transferred to a 0.2 µm pore size nitrocellulose membrane (Sigma, Saint Louis, MO) at 250 mA for 3h, using a Mini Trans-Blot electrophoresis cell (Biorad Laboratories, Richmond, CA). The nitrocellulose membrane was incubated in 5 % non-fat dry milk for 2 h at room temperature. The membrane was incubated with EPO anti-serum (1:1000 titer) overnight at least 4 °C followed by three consecutive washes in PBS containing 0.1 % Tween (10 min/wash). The membrane was incubated with secondary antibody conjugated to Horse Radish Peroxidase (HRP) (Zymed, San Francisco, CA) for 2 h at room temperature, followed by three washes. Finally, the nitrocellulose paper was reacted with enhanced chemiluminescent substrate (ECL) (Pierce, Rockford, IL) for 5 min, dried with a Whatman sheet, and exposed to X-ray film.

### RESULTS

**Table 3** hereinbelow shows the concentrations of the various CTP-modified EPO forms obtained from 5 selected clones and their preparation for further testing.

**Table 3**

| ***#Version*** | ***# Clone*** | ***Stock Titer IU*/*ml¹*** | ***Post dilution in Mock sup. according to Epo3 titer IU*/*ml²*** | ***Post ultrafiltration IU*/*ml³*** |
|---|---|---|---|---|
| Epo0 SEQ ID NO: 8 | 17 | 3093 | 102 | 335 |
| Epo1 SEQ ID NO: 1 | 47 | 1049 | 104 | 291 |
| Epo2 SEQ ID NO: 2 | 67 | 2160 | 110 | 303 |
| Epo3 SEQ ID NO: 3 | 85 | 105 | 119 | 392 |
| Epo4 SEQ ID NO: 4 | 112 | 6100 | ND | 342 |

| | | | | |
|---|---|---|---|---|
| 1. EPO variants stock concentration were determined by ELISA (Quantikine IVD Epo ELISA, DEP00, R&D Systems) 2. Samples EPO-0, 1, 2 and 4 were diluted to 105 IU/ml in mock sup (Adjusted to Epo3 titer). Epo0 = wild type EPO expressed in the same system as the CTP modified EPOs 3. All samples were concentrated and dialyzed by ultrafiltration against PBS to a final concentration of 180 IU/ml. | | | | |

All proteins were detected by Western blot as illustrated in **Figure 2****.**

### EXAMPLE 3 - Generation and Utilization of Coagulation Factor IX

### Cloning and expression of recombinant FIX molecule:

Factor IX clones were constructed in our eukaryotic expression vector pCI-neo (Promega, catalog no. E1841). ORF Clone of Homo sapiens coagulation factor IX was ordered from "OriGene" (RC219065). Primers were ordered from Sigma-Genosys.

### Construction of 301-1-pCI-neo-p200-11 (Factor IX-ctp x2):

Primer 101: 5' GTTTAGTGAACCGTCAGAAT 3' (SEQ ID NO: 82)

Primer 103^{R:} 5' TTGAGGAAGATGTTCGTGTA 3' (contains the SspI site of factor IX) (SEQ ID NO: 83)

A PCR reaction was conducted with primer 101 and primer 103^{R} and plasmid DNA, cDNA clone of Factor IX (OriGene" RC219065) as a template; as a result of the PCR amplification, a ~ 1085 bp (per 10) product was formed and purified from the gel (the fragment containing the amino terminus of Factor IX sequence).

Primer 98: 5' ATTACAGTTGTCGCAGGTGA 3' (SEQ ID NO: 84)

Primer 99^{R} : 5' GCTGGAGCTAGTGAGCTTTGTTTTTTCCTT 3' (SEQ ID NO: 85)

Primer 100: 5' GCTCACTAGCTCCAGCAGCAAGGCC 3' (SEQ ID NO: 107)

Primer 27^{R}: 5' TTTTCACTGCATTCTAGTTGTGG 3' (SEQ ID NO: 86)

Three PCR reactions were performed. The first reaction was conducted with primer 98 and primer 99^{R} and plasmid DNA, cDNA clone of Factor IX (OriGene, RC219065) as a template; as a result of the PCR amplification, a ~ 540 bp product was formed.

The second reaction was conducted with primer 100 and primer 27^{R} and plasmid DNA of 402-2-p72-3 (hGH-CTP-CTP) as a template; as a result of the PCR amplification, a ~ 258 bp product was formed.

The last reaction (per 3) was conducted with primers 98 and 27^{R} and a mixture of the products of the previous two reactions as a template; as a result of the PCR amplification, a ∼ 790 bp product was formed and ligated into TA cloning vector (Invitrogen, catalog K2000-01). SspI - EcoRI fragment was isolated (TA 3-3).

Another PCR reaction was conducted (per 12) with primer 101 and primer 27^{R} and a mixture of the products of per 10 and SspI-EcoRI fragment from per 3 as a template; as a result of the PCR amplification, a ∼ 1700 bp product was formed (Factor IX-ctp-ctp) and ligated into TA cloning vector (Invitrogen, catalog K2000-01) (lig 180).

A mistake was found in the Factor IX sequence so fragments were replaced in order to form an insert of Factor IX-ctp-ctp with the correct DNA sequence.

TA- per 3-3 was digested with SspI and Xbal and the large fragment was isolated (vector). TA 180-4 was digested with SspI and Xbal and the small fragment (insert) was isolated and ligated to the isolated large fragment of TA-pcr-3-3digested with SspI and Xbal. The new plasmid TA-183-2 was digated with Sal I and NotI, and the Factor IX-CTP-CTP insert was isolated (~1575 bp). This fragment was inserted into eukaryotic expression vector pCI-neo (digested with Sal I and Not I) to yield the 301-2-p200-11 clone.

**pCI-dhfr -Factor 9- ctpx2 (p223-4) construction:** Vector pCI-dhfr (p6-1) was digested with SmaI and NotI. Factor IX-CTP-CTP (p200-11) was digested with ASisI F.I. and NotI. The two fragments were ligated.

**pCI-dhfr Factor 9-ctp x3 (p225-7) construction:** Vector pCI-dhfr OXM-CTP×3 (p216-4) was digested with Xbal and ApaI. Factor IX-CTP-CTP (223-4) was digested with XbaI and ApaI. The two fragments were ligated.

**pCI-dhfr Factor 9-ctp x3 T148A (p243-2) construction:** Plasmid p225-7 contained Threonine at position 148, since the more common version of FIX contains Alanine at this position, Thr was replaced to Ala using site directed mutagenesis method.

Primer 75: ctcccagttcaattacagct (SEQ ID NO: 87)

Primer 122r: ggaaaaactgcctcagcacgggtgagc (SEQ ID NO: 88)

Primer 123: gtgctgaggcagtttttcctgatgtggactat (SEQ ID NO: 89)

Primer 124r: caacacagtgggcagcag (SEQ ID NO: 90)

Three PCR reactions were performed. The first reaction was conducted with primer 75 and primer 122r and plasmid DNA p225-7 as a template; as a result of the PCR amplification, a ∼ 692 bp product was formed and purified from the gel. A second PCR reaction was conducted with primer 123 and primer 124r and plasmid DNA p225-7 as a template; as a result of the PCR amplification, a -237 bp product was formed and purified from the gel. The third - overlap PCR reaction reaction was conducted with primers 75 and 124r, and a mixture of the products of the previous two reactions as a template; as a result of the PCR amplification, a ∼ 910 bp product was formed. This overlap PCR product was digested with Xbal and NsiI and re ligated into p225-7 plasmid (digested with Xbal and NsiI) to yield Factor IX-ctpx3 T148A designated p243-2.

**FIX-4CTP (p259-4) construction:** 3.5CTP fragment was isolated from oxym-4CTP (p254-3) by restriction enzymes Apa1 and Xbal. FIX+0.5CTP fragment was isolated from FIX-3CTP (p243-2) with restriction enzymes Apa1 and Xbal. The two fragments were ligated.

**FIX-5CTP (p260-18) construction:** 4.5CTP fragment was isolated from oxym-5CTP (255-1) by restriction enzymes Apa1 and Xbal. FIX+0.5CTP fragment was isolated from FIX-3CTP (p243-2) using enzymes Apa1 and Xbal. The two fragments were ligated.

Dg44 cells were plated in 100mm tissue culture dishes and grown to 50-60% confluence. A total of 2 µg (microgram) of FIX cDNA was used for the transfection of one 100mm plate using the FuGene reagent (Roche) in protein-free medium (Invitrogene CD Dg44). The media was removed 48 hours after transfection and replaced with a protein-free medium (Invitrogene CD Dg44) without nucleosides and in the presence of 800 µg/ml of G418 (Neomycin). After 14 days, the transfected cell population was transferred into T25 tissue culture flasks, and selection continued for an additional 10-14 days until the cells began to grow as stable clones. High expressing clones were selected. Approximately 2×10⁷ cells were used to inoculate 300 ml of growth medium in a 1700 cm² roller bottle (Corning, Corning NY) supplemented with 5 ng/ml of Vitamin K3 (menadione sodium bisulfate; Sigma). The production medium (harvest) was collected after a rapid decrease in cell viability to about 70%. The production medium was first clarified and then concentrated approximately 20-fold and dialyzed with PBS using flow filtration cassette (10KDa MWCO; Millipore Corp.).

**Determination of FIX antigen level:** FIX-CTP harvest antigen levels were determined using AssayMax Human FIX ELISA kit (AssayPro-EF1009-1). The calculated protein concentration is the average of three different dilutions in two independent runs ( **Table 4**).

**Table 4: Calculated protein concentration**

| | FIX-CTP | FIX-CTP-CTP |
|---|---|---|
| FIX Ag level (µg/ml) | 41.9 | 19.2 |
| SD | 8.76 | 3.67 |
| %CV | 20.92 | 19.15 |

**FIX SDS-PAGE - immune blot:** FIX-CTP harvests or purified rhFIX (American Diagnostics), 100 ng of protein, were loaded on 12% Tris-Glycine gel using Precision Plus Dual Color Protein Marker (Bio-Rad). The SDS-PAGE analysis was performed by Western immunoblot using anti-human FIX polyclonal antibody and anti-human gamma carboxylation monoclonal antibody (American Diagnostics). As previously reported, rhFIX migrated at least 55KDa, while FIX fused to two CTPs migrated at 75KDa. Both variants of FIX-CTP proteins were shown to be gamma carboxylated, an essential post-tranaslation modification for FIX activity and function.

**Determination of FIX chromogenic activity:** A comparative assessment of the *in vitro* potency of FIX-CTP harvests versus rhFIX protein (American Diagnostics) was performed using the commercially available chromogenic activity test kit, BIOPHEN (Hyphen BioMed 221802). In the presence of thrombin, phospholipids, calcium, excess amounts of FXIa activates sampled FIX into FIXa. FIXa forms an enzymatic complex with thrombin, activated FVIII:C (supplied in an excess amounts), phospholipids, and calcium and activates Factor X, present in the assay system, into FXa. The activity directly correlates with the amount of FIX, which is the limiting factor. The generated FXa is then measured by its specific activity on FXa chromogenic substrate (pNA). The amount of pNA generated is directly proportional to FIXa activity. rhFIX and FIX-CTP harvests were serially diluted, and the potency was assessed by comparing a dose-response curve of the FIX harvests to a reference preparation consisting of rhFIX or human plasma. The average EC50 of FIX was 21 ng/ml, while the FIX-(CTP)₂ harvest calculated EC50 was 382 ng/ml, and the FIX-CTP harvest calculated EC50 was 1644 ng/ml. An approximately 15-fold decrease in the enzymatic activity of the FIX-(CTP)₂ harvest was observed.

**FIX Clotting activity (aPTT):** The activated partial thromboplastin time (aPTT) is a measure of the integrity of the intrinsic and common pathways of the coagulation cascade. The aPTT is the time, in seconds, for plasma to clot following the addition of an intrinsic pathway activator, phospholipid and calcium. The aPTT reagent is called a partial thromboplastin because tissue factor is not included with the phospholipid as it is with the protime (PT) reagent. The activator initiates the system and then the remaining steps of the intrinsic pathway take place in the presence of phospholipid. Reference aPTT range varies from laboratory to laboratory, but is usually in the range of 27-34 seconds.

The principal of the assay was to quantitate the ability of FIX-CTP harvests to restore the clotting activity of FIX-depleted human plasma by the addition of rhFIX. 300 µl of FIX-deficient human plasma was mixed with 100µl of rhFIX or FIX-CTP harvests and serially diluted. Following a 60 second incubation at 37°C, thromboplastin, CaCl₂, and phospholipids were added to the mixture, and clotting time in seconds was determined (performed by American Medical Laboratories). The potency was assessed by comparing a dose-response curve of the FIX harvests to a reference preparation consisting of rhFIX or human plasma. One unit of FIX activity corresponds to the FIX concentration that equals the activity of one ml normal human plasma. The presented aPTT results indicate that FIX-(CTP)₂ exhibit a 5.7-fold reduction in its specific coagulation activity compared to rhFIX **(Table 5**). Moreover, the aPTT results together with the chromogenic activity *in vitro* assay suggest that FIX-(CTP)₂ harvest has an improved enzymatic activity vs. FIX-CTP harvest **(Table 5**). An improved activity of FIX-CTP proteins can be obtained following optimization of the expression system (i.e. co-transfection with Furin and optimization of Vitamin K3 medium concentration), which was strengthened following super-transfection with Furin (data not shown).

**Table 5: FIX clotting activity**

| rhFIX(AD) (µg/ml) | PTT(Sec) | FIX-CTP (µg/ml) | PTT (Sec) | FIX-CTP-CTP (µg/ml) | PTT (Sec) |
|---|---|---|---|---|---|
| 5 | 31.3 | 9 | 45.2 | 4 | 47.5 |
| 1.25 | 35.7 | 2.25 | 53.3 | 1 | 55.9 |
| 0.3125 | 43 | 0.5625 | 64.1 | 0.25 | 67 |
| 0.078125 | 52.1 | 0.140625 | 76.3 | 0.0625 | 77.4 |

**Pharmacokinetic study:** rhFIX (American Diagnostic) and FIX-CTP harvests were administered in a single intravenous injection to Sprague-Dawley rats (six rats per substance) at a dose of 75 µg/kg body weight **(Table 6).**

**Table 6: PK study plan of operation**

| Treated Groups | Test Article | No. of animals/ group | Dose Route | Gender | Dose Level (µg/kg) | Dose Level (µg per animal) | Injected Vol. (µl) | Con. (µg/ml) | *Time-Points (hours post-dose) |
|---|---|---|---|---|---|---|---|---|---|
| *1* | rFIX | *6* | IV | M | 75 | 15 | 500 | 30 | 0 (Pre-dose) 0.083, 0.5, 1.5, 4, 8, 24, 48, 72. |
| *2* | rFIX-CTP | *6* | IV | M | 75 | 15 | 500 | 30 | 0 (Pre-dose) 0.083, 0.5, 1.5, 4, 8, 24, 48, 72. |
| *3* | rFIX-CTP-CTP | *6* | IV | M | 75 | 15 | 1000 | 15 | 0 (Pre-dose) 0.083, 0.5, 1.5, 4, 8, 24, 48, 72. |

Blood samples were drawn retro-orbitally from 3 rats alternately at 0.083, 0.5 1.5, 4, 8, 24, 48, and 72 hours post-dosing. Plasma was prepared immediately after sampling and stored at - 20°C until analysis. FIX concentration was quantitated by FIX ELISA-specific assay (AssayPro). A pharmacokinetic profile was calculated for each protein and represents the mean of 3 animals at each time point (data not shown). The terminal half-lives were calculated using PK solutions 2.0 software. **Table 7** summarizes the observed FIX concentrations at the different sampling time points.

**Table 7: Observed FIX concentrations**

| Time (Hr) | FIX-AD (ng/ml) | FIX-CTP (ng/ml) | FIX-CTP-CTP (ng/ml) |
|---|---|---|---|
| 0.083 | 1506.7 | 1477.5 | 1914.8 |
| 0.5 | 1949.8 | 1150.1 | 1830.1 |
| 1.5 | 2189.4 | 1009.0 | 1264.3 |
| 4 | 733.90 | 709.33 | 1000.00 |
| 8 | 319.80 | 167.20 | 1234.67 |
| 24 | BLQ | 54.625 | 230 |
| 48 | BLQ | BLQ | 120.9 |

The PK profile and summary of the terminal half-lives are summarized in Table 8. FIX-CTP harvests exhibit an improved T½_{β} values compared to rhFIX (2- and 5-fold increases, respectively). Since in FIX dosing collection, animal serum concentrations of FIX at 24hr were below limit of quantitation (BLQ), additional PK parameters were not calculated.

**Table 8: Summary of PK parameters**

| **Product** | **Terminal half-life- (hr)** | **Ratio (FIX-(CTP)ₓ/rhFIX)** |
|---|---|---|
| rhFIX (American Diagnostics) | 2.62 | - |
| FIX-CTP | 5.55 | 2.11 |
| FIX-CTP (FIX-CTP-CTP) | 12.9 | 4.92 |

In this study, a novel approach was described for prolonging FIX half-life while retaining the therapeutic potency. Adding a CTP peptide to an active protein has a harmful potential in interfering with the protein's activity. Therefore, the generation of an active recombinant FIX-CTP by adding a CTP sequence at the C-terminus of the FIX is unexpected.

### Characterization of an immunoaffinity purified FIX-CTP-CTP

### FIX-CTP-CTP purification

In order to evaluate a protein at high grade content with increased activity whose PK profile mimics and can be extrapolated to a clinical setting, FIX-CTP-CTP is a FIX modified with 2 CTP units in tandem in its carboxy-terminal. FIX-CTP-CTP was purified using matrix-bound monoclonal antibody against γ carboxyglutamyl (Gla) residues present in the N-terminal region of FIX (American Diagnostics Cat. # 3570MX). The monoclonal antibody was bound to Sepharose CL-4B. The FIX-CTP-CTP harvest at a concentration of 88 µg/ml was dialyzed against 20mM Tris, 150Mm NaCl and 10mM EDTA at PH =7.4. The loading rate was 0.5 ml/min, elution was performed using 20Mm Tris-HCl, 350 mM NaCl and 50 mM CaCl, and the unbound fraction was recycled five times. Finally, the elution fraction was dialyzed with PBS, pulled and concentrated.

**Determination of FIX antigen level:** FIX-CTP harvests, FIX-(CTP)₂ harvests, and FIX-(CTP)₂ purified protein levels were determined using the Human FIX ELISA kit (Affinity Biologicals; Cat. #FIX-AG RUO). The calculated protein concentration (µg/ml) is the average of two independent runs (Data not shown, Table 9).

**Table 9: Calculated protein concentration**

| | **FIX-CTP** | **FIX-CTP-CTP** | **FIX-CTP-CTP (purified)** |
|---|---|---|---|
| **FIX Ag level (µg/ml)** | 125.78 | 88.53 | 172.9 |
| SD | 17.28 | 21.31 | 2.63 |
| %CV | 13.74 | 24.08 | 1.52 |

Additionally, FIX-CTP-CTP was quantitated by Bradford assay. The calculated concentration was 202 µg/ml, which is similar to the concentration obtained by human FIX ELISA.

**SDS-PAGE blots:** FIX-CTP-CTP harvest, unbound fraction and purified protein, were loaded on a 12% Tris-Glycine gel using Precision Plus Dual Color Protein Marker (Bio-Rad). The SDS-PAGE Coomassie analysis was performed by staining the gel with Coommasie blue reagent (800ng of protein). A Western immunoblot was performed with 100 ng of protein, anti-human FIX polyclonal antibody (Ab), and anti-human gamma carboxylation monoclonal Ab (American Diagnostics Cat #499 and #3570). The immunoaffinity purification procedure significantly enriched the FIX-CTP-CTP portion while reduced impurity (data not shown).

**N-terminal sequencing:** FIX-CTP-CTP purified protein was separated by 12% Tris-Glycine SDS-PAGE and subsequently electro-blotted to PVDF membrane. The band of interest was cut out and put on a purified Biobrene treated glass fiber filter. The N-terminal sequence analysis was carried out by Edmann degradation using a pulsed liquid protein sequencer equipped with a 140 C HPLC micro-gradient system. N-terminal sequencing revealed that FIX-CTP-CTP is a mixture of incomplete and complete pro-peptide cleaved proteins. Inadequate pro-peptide cleavage was shown to reduce FIX coagulation activity. By co-transfection with Furin, the pro-peptide cleavage process can be an improved.

**Determination of FIX chromogenic activity:** A comparative assessment of the *in vitro* potency of FIX-CTP-CTP purified protein versus rhFIX (American Diagnostics) and a pool of human normal plasma was performed using the commercially available chromogenic activity test kit, BIOPHEN (Hyphen BioMed 221802). In the presence of thrombin, phospholipids and calcium, excess amounts of FXIa activates FIX into FIXa. FIXa forms an enzymatic complex with thrombin (supplied in excess amounts), phospholipids and calcium activates Factor X, present in the assay system, into FXa. The activity directly correlates with the amount of FIX, which is the limiting factor. The generated FXa was measured by its specific activity on FXa chromogenic substrate (pNA). The amount of pNA generated was directly proportional to FIXa activity. rhFIX, human plasma and FIX-CTP-CTP were serially diluted, and potency was assessed by comparing a dose-response curve (Data not shown). The average EC₅₀ of rhFIX was 68.74 ng/ml while FIX-CTP-CTP calculated EC₅₀ was 505 ng/ml. An approximately 7-fold decrease in the enzymatic activity of FIX-CTP-CTP was observed vs. recombinant FIX and a 16.5-fold decrease versus normal human pulled plasma. This reduced activity could be explained by inadequate cleavage of N-terminal pro-peptide, which was identified by N-terminal analysis.

**FIX Clotting activity (aPTT):** The activated partial thromboplastin time (aPTT) is a measure of the integrity of the intrinsic and common pathways of the coagulation cascade. The aPTT is the time (measured in seconds) it takes plasma to clot following the addition of an intrinsic pathway activator, phospholipid and calcium.

The assay quantitated the ability of the FIX-CTP-CTP protein to restore the clotting activity of FIX depleted human plasma by the addition of rhFIX. 300 µl of FIX-deficient human plasma was mixed with 100 µl of rhFIX, FIX-CTP-CTP (FIX-CTP-CTP (the CTP are in tandem at the C-terminal)), or normal pool human plasma which was further diluted. Following a 60 second incubation at 37°C, Tissue Factor (TF), CaCl₂, and phospholipids were added to the mixture. Clotting time in seconds was determined. Potency was assessed by comparing a dose-response curve of FIX-CTP-CTP to a reference preparation of rhFIX or human plasma. One unit of FIX was defined as the amount of FIX which equals to the activity of 1 ml human normal plasma.

The aPTT results indicate that FIX-CTP-CTP coagulation activity is only 1.4 less then normal pool human plasma and similar to the rhFIX. The aPTT results together with the chromogenic activity *in vitro* assay suggest that FIX-CTP-CTP purification did not damage its activity.

**Pharmacokinetic activity of FIX-CTP-CTP:** Purified FIX-CTP-CTP, rhFIX (American Diagnostic) and harvests containing FIX-CTP-CTP and FIX-CTP were administered in a single intravenous injection to Sprague-Dawley rats (eight rats per substance) in a dose of 100µg/kg body weight (Table 10).

**Table 10: PK study outline**

| Treated Groups | Test Article | No.of animals/ group/ time point | Dose Level (pg/kg) | Dose Level (µg per animal) | Injected Vol. (µl) | Con. (µg/ml) | Time-Points (hours post-dose) |
|---|---|---|---|---|---|---|---|
| A | rFIX | 8 | 100 | 20 | 500 | 40 | 0 (Pre-dose) 0.083, 0.5, 1, 2, 4, 7, 10, 24, 48, 72. |
| B | rFIX-CTP (harvest) | 8 | 100 | 20 | 500 | 40 | 0 (Pre-dose) 0.083, 0.5, 1, 2, 4, 7, 10, 24, 48, 72. |
| C | rFIX-CTP-CTP(harvest) | 6 | 100 | 20 | 500 | 40 | 0 (Pre-dose) 0.083, 0.5, 1, 2, 4, 7, 10, 24, 48, 72. |
| D | rFIX-CTP-CTP (purified) | 4 | 100 | 20 | 500 | 40 | 0.083, 0.5 1, 2, 4, 7, 10, 24, 4, 8, 72. |

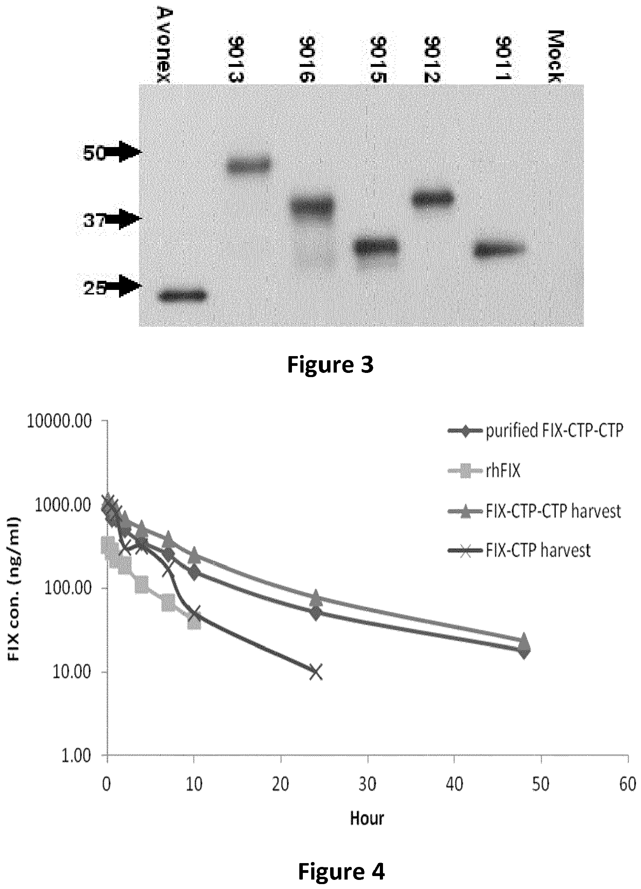

Blood samples were drawn retro-orbitally from 4 rats alternately at 0.083, 0.5, 2, 4, 7 10, 24,48, and 72 hours post-dosing. Citrated plasma (0.32%) was prepared immediately after sampling and stored at -20°C until analysis. FIX concentration was quantitated using a human FIX ELISA kit (Affinity Biologicals). The pharmacokinetic profile was calculated for each protein as the mean of 4 animals at each time point **(****Figure 4****).** The terminal half-life was calculated using PK Solutions 2.0 Software. **Table 11** summarizes the observed FIX concentrations at different sampling time points.

**Table 11: Observed FIX concentrations**

| **Time (hr)** | **FIX-CTP harvest ng/ml** | **FIX-(CTP)₂ harvest ng/ml** | **rhFIX ng/ml** | **Purified FIX-CTP-CTP ng/ml** |
|---|---|---|---|---|
| **0.085** | 1038.97 | 1123.62 | 325.05 | 886.48 |
| **0.5** | 939.12 | 956.80 | 274.58 | 670.92 |
| **1** | 791.97 | 843.85 | 222.90 | 674.17 |
| **2** | 304.98 | 673.31 | 186.00 | 503.91 |
| **4** | 315.37 | 525.50 | 109.69 | 357.36 |
| **7** | 171.45 | 384.36 | 67.62 | 257.02 |
| **10** | 50.34 | 250.73 | 40.20 | 158.66 |
| **24** | 10.07 | 78.50 | BLQ | 52.13 |
| **48** | BLQ | 23.40 | BLQ | 18.07 |

A summary of the PK parameters are presented in **Table 12.**

**Table 12: Summary of PK parameters**

| | **T½ (hr)** | **AUC ng-hr/ml** | **MRT (hr)** | **Vd ml/Kg** | **CL Ml/hr/Kg** |
|---|---|---|---|---|---|
| **FIX-CTP harvest** | 4.17 | 3622 | 4.5 | 155.1 | 27.6 |
| **FIX-(CTP)₂ harvest** | 10.44 | 9105.7 | 12 | 165.4 | 10.9 |
| **rhFIX** | 3.72 | 1416.8 | 5.1 | 373.8 | 70.183 |
| **Purified FIX-CTP-CTP** | 11.14 | 6314.2 | 12.3 | 254.5 | 15.83 |

The FIX-CTP-CTP harvest demonstrated an improved PK profile compared to FIX-CTP harvest. Furthermore, purified FIX-CTP-CTP exhibited a 3-fold increase in T½_{β} value and a 4.5-fold increase in AUC compared to rhFIX.

The reduced amount of secreted FIX fused to tandem CTP molecules versus fusion of a single CTP appears to be due to the addition of an extra CTP and not to reduced detection by ELISA, because the Bradford-purified FIX-CTP-CTP calculated concentration was similar to the ELISA-calculated concentration.

FIX-CTP-CTP clotting activity was similar to pooled human plasma; however, its *in vitro* chromogenic activity was significantly lower when compared to rhFIX or pooled human plasma. The chromogenic activity assay was reported as a very sensitive assay compared to the coagulation assay. The reason for reduced activity of FIX-CTP-CTP may vary. Addition of CTP may decrease the affinity of FIX to FXIa or reduce post-transcriptional modifications (e.g. 12-10 GLA residues and pro-peptide cleavage). N-terminal analysis revealed that the proteolytic cleavage of the FIX-CTP-CTP pro-peptide was not fully completed prior to secretion. Since this post-transcriptional modification is crucial for the normal enzymatic activity of the protein, co-transfection with Furine-PACE plasmid is favorable and may improve FIX-CTP-CTP activity.

Finally, FIX-CTP-CTP comparative PK study in rats demonstrated that fusion of two tandem CTPs to the C-terminal of FIX generated a FIX with an extended half-life.

**FIX depleted mouse model:** In order to assess the *in vivo* activity, FIX knockout mice are obtained, and a breeding colony is established. 10µg of either commercial recombinant hFIX (BeneFIX^{®}) or rFIX-(CTP)₂ (FIX-CTP-CTP) are injected into the tail vein of an anaesthetized FIX knockout mouse (22-28g). The amount of injected protein equals to the required concentration of FIX in normal plasma (5µg/ml). Blood samples are taken from the clipped tail into heparinized capillary tubes at specific time points. Plasma samples are assessed for FIX levels by ELISA and efficacy is measured by aPTT coagulation assay.

**Increasing FIX Propeptide cleavage efficacy:** CTP peptide cDNA was fused to the 3' end of human FIX cDNA. The corresponding rFIX and Furin expressing constructs were co-transfected into Dg44 cells; a human rFIX cDNA was also co-transfected with the Furin plasmid as a control. Secretion of high level of FIX leads to secretion of a mixture of pro-factor and a mature factor FIX, due to limited amount of the Furin protease in the cell. Co-transfection of a Furin expressing vector with a pro-factor expressing vector increases the recovery and result in the secretion of fully processed FIX in to the medium.

Following FIX-(CTP)₂ and Furin co-transfection, stable clones are generated and harvest is collected for pro-peptide cleavage evaluation. 100 ng of protein, are loaded on 12% Tris-Glycine gel using Precision Plus Dual Color Protein Marker (Bio-Rad). The SDS-PAGE analysis is performed by Western immunoblot using anti-human FIX polyclonal Ab (American Diagnostics) and anti-pro-peptide polyclonal antibody. As previously reported, rhFIX migrated at least 55KDa, while FIX fused to two CTPs migrated at 75 kDa. Both variants of FIX proteins are shown to undergo a proper, full pro-peptide cleavage.

To determine whether proper pro-peptide cleavage improves FIX-(CTP)₂ enzymatic activity, a comparative assessment of chromogenic and coagulation activity of FIX-(CTP)₂ harvest co transfecated with Furin is performed. A significant improvement in FIX-(CTP)₂ specific activity is observed, which is similar to rhFIX.

In conclusion, the results described herein suggest that FIX-CTP-CTP can be used efficiently for treating Hemophilia B patients. FIX fused to CTP constructs benefit from improved *in vivo* pharmacologic performance that overcomes the drawback in certain *in vitro* measures. This proposed treatment is advantageous over previous treatments as the rate of infusions and the amount of required doses are reduced.

It is important to notice that when an albumin-fused molecule strategy was used to improve the FIX half-life, the recombinant FIX became inactive. The present novel approach lead to the design and purification of a novel recombinant FIX-fused protein that presents an improved long-lasting activity. Since mere size modifications did not improve the pharmacokinetics of injected FIX, the finding that CTP fused to FIX facilitates pharmacokinetic parameters was unexpected. The presence of highly glycosylated peptide-sialic acid residues stabilized the protein and protected it from interactions with vascular receptors without abrogating key determinants of FIX function.

FIX-CTP has a similar therapeutic efficacy to rFIX in hemophilia B patients and required less frequent dosing. A single injection of FIX-CTP is sufficient to control bleeding episodes and reduce the number of injections that are needed during surgical intervention in hemophilia B patients.

The CTP technology was utilized for the development of a long-acting FIX. Specifically, extending the half-life of recombinant rFIX molecule was performed by fusion of at least one human CTP to FIX. The recombinant FIX-CTP was expressed in mammalian cells and characterized *in vitro* and *in vivo.* It was demonstrated that the *in vitro* activity of rFIX-CTP was comparable to rFIX. Pharmacokinetics and efficacy studies in rats demonstrated improved properties of the rFIX-CTP. The results of this study demonstrate that it is feasible to develop a half-life extended rFIX molecule having similar haemostatic properties to the wild type enzyme.

### EXAMPLE 4 - Comparative Assessment of Purified FIX-CTP₃ vs. FIX-CTP₄ and FIX CTPs

### 2.1 Study objective

A comparative assessment of the pharmacokinetic parameters of FIX-CTP₄ and FIX-CTPs versus FIX-CTPs following a partial purification process.

### 2.2 Production of FIX-CTP₄ and FIX-CTPs harvests

FIX cDNA (OriGene RC219065) fused at the C-terminal to four or five tandem CTP sequences was expressed in Dg44 cells using Excellgene expression system in the presence of 10 ng/L of vitamin K3 (Sigma, Mennadion). The harvests were collected (300ml), filtered and frozen.

### 2.3 Production of FIX-CTP₃ harvest

FIX-CTPs was expressed in-house in CHO cells using pCI-DHFR vector, clone 196, BR-9 in the presence of 25 ng/L of vitamin K3 (Sigma). The harvests were collected and filtered.

All FIX-CTP samples (3, 4 and 5 CTP) were purified only by Jacalin column because of a lack of material.

### 2.4 Determination of FIX antigen level

FIX antigen level was determined using Human FIX ELISA kit (Affinity Biologicals; Cat. # FIX-AG RUO). The calculated protein concentration is the average of four independent runs. FIX-CTPs concentration was slightly higher as compared to the two additional versions (Table 13).

**Table 13: FIX antigen level**

| | **3 CTP Final Jacalin40** | **4 CTP Final Jacalin40** | **5 CTP Final Jacalin40** |
|---|---|---|---|
| Av. (ng/ml) | 1016.69 | 4644.11 | 1686.82 |
| SD | 225.41 | 925.63 | 160.07 |
| %CV | 22.17 | 19.93 | 9.49 |

### 2.5 FIX-CTP Coomassie stain and immune-blot

FIX-CTP₃, FIX-CTP₄, and FIX-CTPs harvests were loaded on 12% Tris-Glycine gel using Precision Plus Dual Color Protein Marker (Bio-Rad). The SDS-PAGE analysis was performed by Western immuno-blot using anti-CTP polyclonal Ab (Adar Biotech Production) or anti-Gla Ab (American Diagnostica).

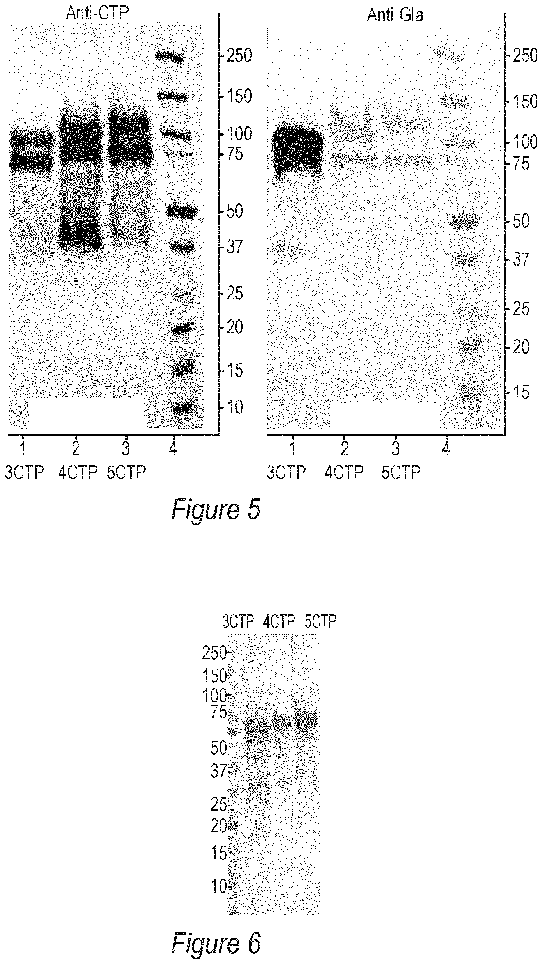

As previously reported, FIX fused to three CTPs migrated at 80 kDa while FIX fused to four or five CTPs migrated at 85 KDa or 90 KDa, respectively. As expected, FIX-CTP₄ and FIX-CTPs harvests from Excellgene showed very low levels of gamma carboxylation compared to FIX-CTP₃ harvest, which was produced at Prolor **(****Figure 5****).**

After a purification process utilizing Jacalin column (immunoaffinity purification of glycosylated proteins), FIX-CTP₃, FIX-CTP₄, and FIX-CTPs were loaded on 12% Tris-Glycine gel using Precision Plus Dual Color Protein Marker (Bio-Rad). The SDS-PAGE was stained by Coomassie blue Dye for samples detection. All variants showed much cleaner band profiles **(****Figure 6****),** suggesting an improved purity.

### 2.6 Determination of FIX chromogenic activity

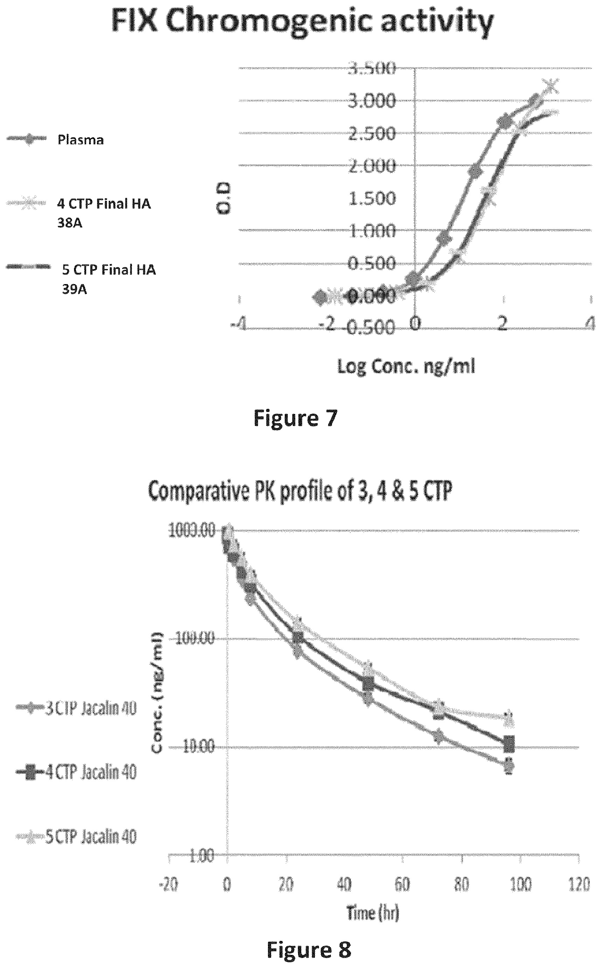

A comparative assessment of the *in vitro* potency of fully purified (HA column) FIX-CTP₃, FIX-CTP₄, and FIX-CTPs versus human pool normal plasma was performed using a commercially available chromogenic activity test kit, BIOPHEN (Hyphen BioMed 221802). All samples were serially diluted, and the potency was assessed by comparing a dose-response curve to a reference preparation of normal human plasma. The reduced chromogenic activity of FIX-CTP₄ and FIX-CTPs **(****Figure 7****)** as compared to plasma can be a consequence of improper post-transcriptional modifications of FIX proteins, e.g. inappropriate gamma carboxylation and pro-peptide cleavage or, alternatively, due to the addition of CTP cassettes. The fluctuation in the FIX-CTP₄ and FIX-CTPs activity **(Table 14)** might be caused by inappropriate quantitation capabilities of the FIX ELISA due to CTP masking of the antigen site.

**Table 14: Sample/plasma EC50 ratio**

| **Sample** | **Sample/plasma EC50 ratio** |
|---|---|
| Plasma | 1 |
| 3 CTP Final HA | 2 |
| 4 CTP Final HA | 5.35 |
| 5 CTP Final HA | 2.73 |

### 2.7 Pharmacokinetic study

Jacalin-purified FIX-CTP₃, FIX-CTP₄, and FIX-CTPs (Group A, B and C, respectively) were administered in a single intravenous injection to Sprague-Dawley rats (six rats per treatment group) at a dose of 250 µg/kg body weight. Blood samples were drawn retro-orbitally from 3 rats alternately at 0.083, 0.5 2, 5, 8, 24, 48, 72 and 96 hours post-dosing **(Table 15).** Citrated plasma (0.38%) was prepared immediately after sampling and stored at -20°C until analysis.

**Table 15: PK study plan of operation**

| Treatment Group | Treatment | No. of animals/ group | Dose Route | Dose Level (µg per animal) | Injected Vol. (µl) | Cone. (µg/ml) | Time-Points (hr post-dose) |
|---|---|---|---|---|---|---|---|
| A | FIX-CTP*3 Jacalin 40 | 6 | IV | 50 | 200 | 250 | 0.083, 0.5, 2, 5, 8, 24, 48, 72, 96 |
| B | FIX-CTP*4 Jacalin 40 | 6 | IV | 50 | 200 | 250 | 0.083, 0.5, 2, 5, 8, 24, 48, 72, 96 |
| C | FIX-CTP*5 Jacalin 40 | 6 | IV | 50 | 200 | 250 | 0.083, 0.5, 2, 5, 8, 24, 48, 72, 96 |

FIX concentration in plasma samples were quantified using human FIX ELISA kits (Affinity Biologicals). The pharmacokinetic profile was calculated and is the mean of 3 animals at each time point. Terminal half-lives were calculated using PK Solutions 2.0 Software. **Table 16** below summarizes the calculated FIX concentrations at the different sampling time points.

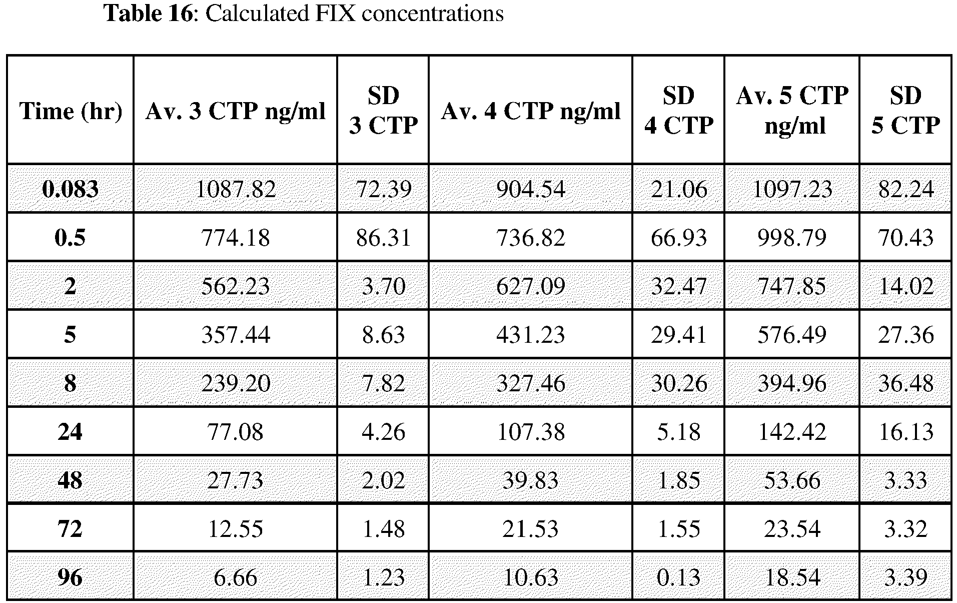

The PK profile and a summary of the PK parameters are presented in **Table 17** below and in **Figure 8****.** A full PK analysis profile at all time points suggested that addition of 4 or 5 CTP cassettes to FIX did not increase its half-life as compared to FIX-CTP₃. The AUC following FIX-CTPs administration increased by 1.4- to 1.6-fold versus FIX-CTP₃, which was not statistically significant.

**Table 17: PK profile and a summary of the PK parameters**

| **24- 96hr** | **3 CTP** | **4 CTP** | **5 CTP** |
|---|---|---|---|
| **Half-life (hr)** | 20.43 | 22.02 | 23.96 |
| **AUC (ng-hr/ml)** | 8218.38 | 10504.49 | 13329.41 |
| **Vd (ml/kg)** | 700.76 | 586.02 | 494.89 |
| **CL (ml/hr/kg)** | 23.77 | 18.45 | 14.32 |

Since 96 hr post-dosing samples were shown to have very low FIX concentrations, which were at the lower limit of quantification of the assay, the terminal half-life was recalculated providing a more precise and scientifically appropriate calculation **(Table 18).** According to this calculation, even smaller differences were obtained between the half-life of FIX-CTP₃, FIX-CTP₄, and FIX-CTPs.

**Table 18: Recalculated terminal half-life**

| **8-72 hr** | **3 CTP** | **4 CTP** | **5 CTP** |
|---|---|---|---|
| **Half-life (hr)** | 15.38 | 16.63 | 16.04 |

### 2.8 Conclusions:

In this study, the pharmacokinetic parameters and potential clotting activity of FIX-CTP₃, FIX-CTP₄, and FIX-CTPs were assessed. Fusion of 4 and 5 CTPs to FIX did not provide a superior or improved half-life extension, as compared to FIX-CTP₃, and reduced chromogenic activity was observed. **Table 19** below summarizes the percent improvement of half-life for the different FIX-CTP fused variants (1 to 5 CTPs). Fusion of CTP to FIX improved its pharmacokinetic behavior, but, unpredictably, this improvement was limited. Surprisingly, following fusion of 3, 4 or 5 CTPs in tandem to FIX, a similar half-life value was calculated.

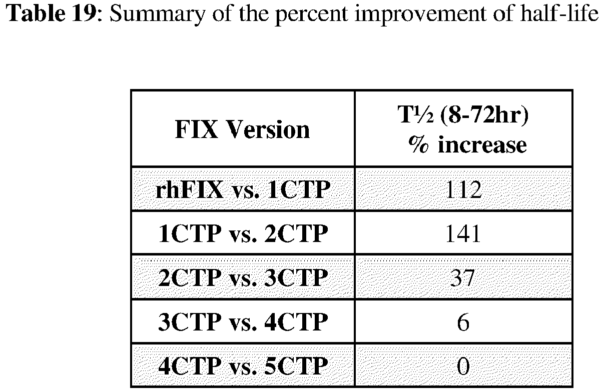

These data suggest that fusion of 3 CTPs to FIX produces a maximal improvement in protein half-life, confirming that FIX-CTP₃ is the optimal variant in terms of half-life, structure and potential clotting activity for further clinical development.

### EXAMPLE 5 - Generation and utilization of Coagulation Factor FVII

A long-acting version of activated Factor VII (FVIIa) coagulation factor will be useful for the treatment of patients with hemophilia A and B. FVIIa-CTP₃ recombinant protein has the clinical potential to improve the treatment of hemophilia patients by reducing the frequency of infusions and even by reducing the drug load, enabling a prophylactic treatment approach which can significantly improves a patient's quality of life, avoid spontaneous bleeding episodes and accumulated damage to the joint and other organs.

The generation of a recombinant FVIIa-CTP molecule with an extended half-life based on fusion of FVII to a human CTP is described herein. The recombinant FVIIa-CTP was expressed in mammalian cells and characterized *in vitro* and *in vivo.* It was demonstrated that rFVII-CTP activity was comparable to rFVII. Pharmacokinetic and efficacy studies in rats demonstrated improved properties of rFVII-CTP. The results of this study demonstrated that it is feasible to develop a half-life extended rFVIIa molecule with very similar haemostatic properties to the wild-type enzyme.

**Cloning and expression of recombinant FVII molecule:** Several Factor VII clones were constructed in our eukaryotic expression vector (pCI-dhfrr) **(****Figure 9****).** Human MGC verified FL cDNA clone (IRCM) containing the sequence of *homo sapiens* coagulation Factor VII was ordered from "Open Biosystems" (OB-MHS4426). The following primers were synthesized by Sigma-Genosys in the following sequence: Primer 67: 5'CTCGAGGACATGGTCTCCCAGGCCC3' (contains the 5' end of Factor VII DNA and the restriction site of XhoI) (SEQ ID NO: 91); Primer 68^{R}: 5' TCTAGAATAGGTATTTTTCCACATG3' (contains the restriction site of Xbal) (SEQ ID NO: 92); Primer 69: 5' TCTAGAAAAAAGAAATGCCAGC3' (contains the restriction site of Xbal) (SEQ ID NO: 93); and Primer 70^{R}: 5'GCGGCCGCATCCTCAGGGAAATGGGGCTCGCA3' (contains the 3' end of Factor VII DNA and the restriction site of NotI) (SEQ ID NO: 94).

Cloning was performed in two sets of PCR reactions. The first reaction was conducted with primer 67 and primer 68^{R} using a cDNA plasmid with the Factor VII sequence (OB-MHS4426) as a template; as a result of the PCR amplification, a ~534 bp product was formed, isolated and ligated into a TA cloning vector (Invitrogen, Catalog No: K2000-01). A XhoI -Xbal fragment containing the amino terminus of the Factor VII sequence was isolated. The second reaction was conducted with primer 69 and primer 70^{R} and again, a cDNA plasmid with the Factor VII sequence (OB-MHS4426) was used as a template. As a result of the PCR amplification, a ~813 bp product was formed and ligated into TA cloning vector (Invitrogen, Catalog No: K2000-01). A XbaI-NotI fragment containing the carboxy terminus of Factor VII sequence was isolated. The two fragments were inserted into our eukaryotic expression vector pCI-dhfr (triple ligation) to yield the 501-0-p136-1 clone.

Plasmid 501-p136-1 (Factor VII in pCI-dhfr vector) was digested with restriction enzymes XhoI and KpnI. A fragment of ~1186 bp was isolated. A partial Factor VII clone (1180 bp-1322 bp) followed by a CTP sequence, termination sequence and NotI sequence that was synthesized by GeneArt (0721543) was digested with restriction enzymes KpnI and NotI. A fragment of ~253 bp was isolated. The two fragments were inserted into our eukaryotic expression vector pCI-dhfr (triple ligation) to yield the 501-1-p137-2 clone. pCI-dhfr-701-2-p24-2 was digested with restriction enzymes XhoI and ApaI, and the large fragment (vector) was isolated.

pCI-dhfr-501-2-p137-2 (Factor VII-ctp x1) was digested with restriction enzymes XhoI and ApaI, and a ~1200 bp insert was isolated. The vector and insert were ligated to yield 501-2-p139-2. Dg44 cells were plated in 100 mm tissue culture dishes and grown to confluence of 50-60%. A total of 2 µg of DNA was used for transfection of one 100 mm plate using the FuGene reagent (Roche) in protein-free medium (Invitrogen CD Dg44). The medium was removed 48 hours post-transfection and replaced with a protein-free medium (Invitrogen CD Dg44) without nucleosides. After 14 days, the transfected cell population was transferred into T25 tissue culture flasks, and the selection was continued for 10-14 days until the cells began to grow well as a stable clone. High-expressing clones were selected and approximately 2×10⁷ cells were used to inoculate 300 ml of growth medium in a 1700cm² roller bottle (Corning, Corning NY) supplemented with 5 ng/ml of Vitamin K3 (menadione sodium bisulfate; Sigma). The production medium (harvest) was collected after a rapid decrease in the cell viability to around 70%. The production medium was first clarified and then concentrated approximately 20-fold and dialyzed to PBS using flow filtration cassette (10KDaMWCO; Millipore Corp, Billerica, MA).

### Determination of FVII antigen level

The cDNA coding the CTP peptide was fused to the 3' end of the cDNA coding human FVII. The corresponding rFVII construct was transfected into Dg44 cells. As a control, a human rFVII cDNA was utilized. The production medium (harvest) was collected, concentrated and the secreted recombinant FVII was further evaluated. rFVII, rFVII-CTP and rFVII-CTP-CTP antigen levels were determined by AssayMax Human FVII ELISA kit (AssayPro) **(****Figure 10A****)**. There was no significant difference in the secretion level of rFVII-CTP and rFVII-(CTP)₂ compared to native rFVII.

### SDS-PAGE blots

SDS-PAGE analysis was done by loading 50 ng of either harvest, purified or activated rFVII protein. Samples were loaded on 12% Tris-Glycine gel using Precision Plus Dual Color Protein Marker (Bio-Rad). The SDS-PAGE analysis was done by performing a Western immunoblot using an anti-human FVII monoclonal antibody (Ab) (R&D systems) or anti-CTP polyclonal antibody generated in Rabbit.

The level of rFVII antigen correlated with the detected protein level in a SDS-PAGE immunoblotted with anti-FVII Ab. rFVII-CTP migrated as a single band, while the corresponding molecular weight of the FVII control was approximately 52 KDa (data not shown). Both proteins reacted with antibodies specific for FVII on immunoblots. The rFVII-CTP also reacted with antibodies specific for CTP. rFVII was secreted in its zymogene form with no trace of activated protein.

### FVII Chromogenic activity:

rFVII, rFVII-CTP and rFVII-(CTP)₂ harvest activities were determined using a commercially available chromogenic test kit (AssaySense Human FVII Chromogenic Activity Assay Kit (AssayPro). For functional characterization of the rFVII-CTP and its ability to be further activated (FVIIa), concentrated rFVII-CTP (harvests) were placed in a commercially available chromogenic test kit that measure the ability of TF/FVIIa to activate Factor X to Factor Xa that in the presence of FXa specific substrate releases a quantitated signal (AssayPro). The addition of the CTP peptide at the C-terminal of the rFVII protein did not impair the FVII serine protease activity (Figure 10B, 10C).

### FVII clotting activity:

Prothrombin time (PT) measures the extrinsic pathway of coagulation. The PT is the time (measured in seconds) it takes plasma to clot following the addition of an extrinsic pathway activator, phospholipid and calcium. It is used to determine the clotting tendency of blood, specifically in the measure of warfarin dosage, liver damage, and vitamin K status. The reference range for prothrombin time is usually around 12-15 seconds. Specifically, the assay quantitated the ability of FVII-CTP and FVII-(CTP)₂ harvest to restore the clotting activity of FVII-depleted human plasma by the addition of rhFVII. 300 µl of FVII-deficient human plasma was mixed with 100 µl of FVII, FVII-CTP and FVII-(CTP)₂ harvests at specific concentrations, or normal pool human plasma and were further diluted. Following a 60 second incubation at 37°C, Tissue Factor (TF), CaCl₂, and phospholipids were added to the mixture. The clotting time in seconds was determined. Potency was assessed by comparing a dose-response curve of FVII-CTP and FVII-(CTP)₂ harvests to a reference preparation consisting of rhFVII or human pool plasma. One unit of active FVII was defined as the amount of FVII which equals to the activity of one ml human normal plasma. The PT Clotting activity of rFVII and rFVII-CTP was measured on a coagulometer (Instrumentation Laboratory).

As previously shown, the addition of a CTP peptide at the C-terminal of the rFVII protein did not damage its serine protease activity and lead to the initiation and activation of a native Factor X and Factor IX in human plasma. Following the insertion of an additional CTP at the C terminal, there was a three-fold reduction in the serine protease activity (data not shown).

### Pharmacokinetics study:

rFVII, rFVII-CTP, and rFVII-(CTP)₂ harvests were administered intravenously to Sprague-Dawley rats (six rats per substance) with a dose of 100µg/kg body weight. For all of the *in vivo* experiments, the amount of the respective protein was determined on the basis of FVII ELISA kit. For each FVII test substance, the injected amount was calculated by taking into account the differences in the molecular weight of rFVII versus rFVII-CTP, which leads to a different molar concentration.

Blood samples were drawn retro-orbitally using an altering sampling scheme to minimize interference of the sampling procedure levels to be quantified: from 3 rats at 30 and 90 min and at 2, 6, and 48 hrs, and from the remaining three rats at 15 and 60 min and at 1.5, 4, and 24 hrs alternately. Plasma was prepared immediately after sampling and stored at -20°C until analysis. FVII concentration was quantified by FVII ELISA specific assay. Half-life and AUC were calculated using a linear trapezoidal rule. Comparison of these clearance parameters revealed that the *in vivo* half-life and rFVII-(CTP)₂ AUC are significantly higher than those of rFVII **(Table 20).**

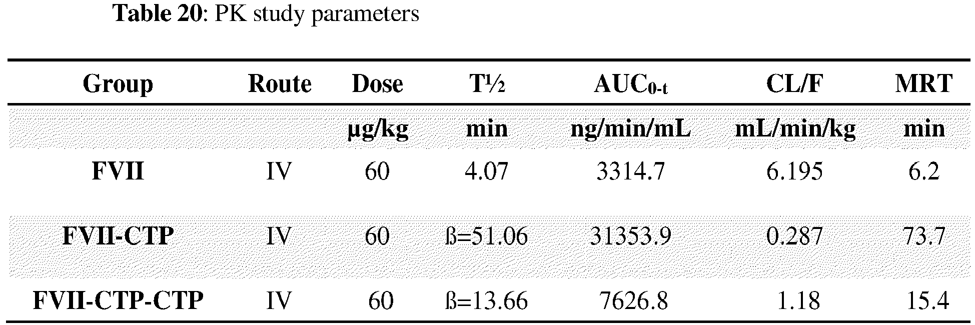

### Characterization of recombinant FVIIa-CTP:

During activation, FVII is cleaved at R152 resulting in heavy and light chain domains that are held together by a single disulfide bridge. rFVIIa-(CTP)₂ is purified and activated by an ion exchange column purification process. In order to fully evaluate rFVIIa-(CTP)₂, the protein is loaded on SDS-PAGE under reducing conditions to commercial FVIIa (NovoSeven^{®}). The heavy and the light chain domains are separated and migrate as separated bands of molecular weights 55 and 25 KDa. Both proteins react with antibodies specific for FVII, but the heavy chain of the rFVIIa-CTP specifically reacts with anti-CTP-specific antibodies, indicating that this band represents the FVII heavy chain fused to CTP. The light chain reacts specifically with anti-gamma carboxylase Ab. The FVIIa protein concentration is determined by FVIIa-specific ELISA kit.

### FVIIa N-terminal sequencing:

rFVII-CTP-CTP in activated or zymogene purified proteins is separated by SDS-PAGE (on 12% Tris-Glycine) and subsequently electroblotted to a PVDF membrane. The bands of interest are cut out and put on a purified Biobrene-treated glass fiber filter. The N-terminal sequence analysis is carried out by Edmann degradation using a pulsed liquid protein sequencer equipped with a 140C HPLC microgradient system. The identity of the recombinant protein and proper pro-peptide cleavage is further verified by N-terminal sequencing.

### FVIIa clotting activity:

In order to evaluate FVII-(CTP)₂ coagulation activity, activated partial thromboplastin time assay (aPTT) is performed. FVII-deficient plasma sample is substituted with rFVIIa (NovoSeven^{®}) or rFVIIa-(CTP)z. 300 µl of FVII deficient human plasma is mixed with 100 µl of FVIIa or rFVIIa-(CTP)₂ at specific concentrations, or normal pooled human plasma which is further diluted. Following 60 seconds incubation at 37°C. Tissue Factor (TF), CaCl₂, and phospholipids are added to the mixture. Clotting time in seconds is determined. Potency is assessed by comparing a dose-response curve of rFVIIa-(CTP)z to a reference preparation consisting of rhFVIIa or human pool normal plasma. One unit of FVIIa is defined as the amount of FVIIa which equals to the activity of 1 ml human normal plasma. The aPTT clotting activity of rFVII and rFVIIa-(CTP)₂ is measured on a coagulometer (Instrumentation Laboratory). The aPTT clotting activity of rFVIIa and rFVIIa-(CTP)₂ is similar.

### Pharmacokinetics studies in rats:

In order to characterize the influence of the CTP addition to the rFVIIa on its longevity potential, a comparative pharmacokinetic study in rats is performed. NovoSeven^{®} (rFVIIa) and rFVIIa-(CTP)₂ in TBS are injected IV to 6 SD rats. The levels of FVIIa over time are detected using a FVIIa ELISA kit. The half-life and AUC are calculated for each protein. Comparison of these clearance parameters reveals that the *in vivo* measures of half-life, recovery, and AUC of the rFVIIa-(CTP)₂ are superior to those of NovoSeven^{®}.

### FVIIa-CTP in vivo efficacy model (FVIII-deficient mouse model of hemophilia):

In order to assess the *in vivo* activity model, FVIII knockout mice are obtained, and a breeding colony is established. 10 µg of either commercial recombinant hFVIIa (NovoSeven^{®}) or rFVIIa-(CTP)₂ are injected into the tail vein of an anaesthetized FVIII knockout mouse (22-28g). The amount of injected protein equals to the required concentration of FVIII in normal plasma (5µg/ml). Blood samples are taken from the clipped tail into heparinized capillary tubes at specific time points. Plasma samples are assessed for FVIIa levels by ELISA, and efficacy is measured by a PTT coagulation assay.

In this study, a fusion construct of FVII with CTP is generated. This recombinant protein is the basis for a treatment that provides a prolonged half-life and retention of therapeutic potency.

These results suggest that rFVIIa-(CTP)₂ has a similar therapeutic efficacy to rFVIIa in hemophilia patients. Moreover, this technology requires less frequent dosing. It appears that a single injection of rFVIIa-(CTP)₂ is sufficient to control bleeding episodes and reduce the number of injections that are needed during surgical intervention. This recombinant protein may be used as a long term prophylactic treatment.

### EXAMPLE 6 - Comparative assessment of Purified FVII-CTP₃, FVII-CTP₄, and FVII-CTP₅

### 5.1 Study objective

Comparative assessment of pharmacokinetic parameters and clotting activity of FVII-CTP₄ and FVII-CTP₅ versus FVII-CTP₃.

### 5.2 Production of FVII-CTP₄ and FVII-CTP₅ harvests

FVII cDNA fused at the C-terminal to four or five tandem CTP sequences was expressed in Dg44 cells using the Excellgene expressing system in the presence of 20 µg/L of vitamin K3 (Sigma, Mennadion). The harvest was collected (300 ml), filtered and frozen.

### 5.3 Production of FVII-CTP₃ harvest

FVII-CTP₃ was expressed in-house in mammalian expressing system, CHO cells, using pCI-DHFR vector. Stable transfected pool #71 was grown in shake flasks, in the presence of 25 ng/L of vitamin K3 (Sigma). The harvests were collected and filtered.

All FVII-CTP harvests (3, 4 and 5 CTPs) were concentrated and dialyzed against TBS (50 mM Tris, 150mM NaCl, pH 7.4) using Pellicon XL MWCO 10kDa. Auto-activation of the FVII was induced by incubation of purified FVII-CTP₃ in the presence of CaCl₂ overnight at 2-8°C. Activation is also conducted by using a Q resin column.

### 5.4 Determination of FVII antigen level

FVII antigen level was determined using Human FVII ELISA kit (Zymotest HyPhen) **(Table 21).** The calculated protein concentration is the average of two independent runs.

**Table 21: FVII antigen level**

| | **FVII-CTP₃** | **FVII-CTP₄** | **FVII-CTP₅** |
|---|---|---|---|
| **Av. (ng/ml)** | 224357.3 | 87884.1 | 589423 |
| **SD** | 44789.5 | 3248.7 | 5309 |
| **%CV** | 20.0 | 3.7 | 9 |

### 5.5 FVII-CTP immune-blot

FVII-CTP₃, FVII-CTP₄, and FVII-CTP₅ harvests were loaded on 12% Tris-Glycine gel (*expedeon*) using Precision plus dual color protein marker (Bio-Rad). The SDS-PAGE analysis was performed by Western immune-blot using anti-CTP polyclonal Ab (Adar Biotech Production) or anti-Gla Ab (American Diagnostica).

FVII fused to three, four and five CTP migrated at 80, 90 and 100kDa, respectively. As expected, FVII-CTP₄ and FVII-CTP₅ harvests from Excellgene contain low gamma carboxylation content as compared to FVII-CTP₃ harvest which was produced at Prolor since the production process wasn't optimized (Data not shown).

### 5.6 Comparative assessment of FVII in vitro potency

A comparative assessment of the *in vitro* potency of HA purified (highly gamma carboxylated fraction) FVII-CTP₃, FVII-CTP₄, and FVII-CTP₅ versus normal human pool plasma was performed using a commercially available chromogenic activity test kit, BIOPHEN (Hyphen BioMed 221304). All samples were serially diluted, and the potency was assessed by comparing a dose-response curve to a reference preparation consisting of normal human plasma. FVII-CTP₃ and FVII-CTP₅ demonstrated chromogenic activity lower than pooled normal plasma (Data not shown). FVII-CTP₄ demonstrated higher activity as reflected by EC50 ratios, compared to FVII-CTP₃ and FVII-CTP₅ **(Table 22)**.

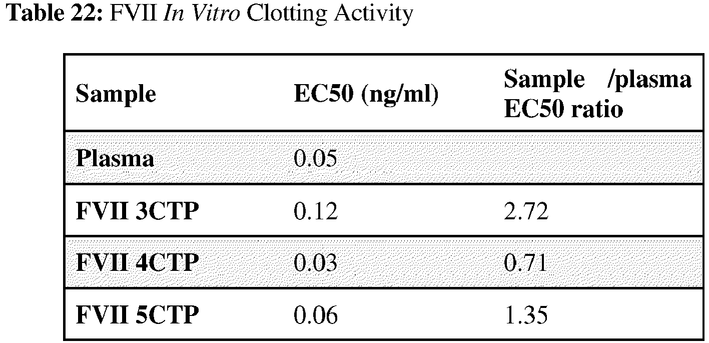

### 5.7 FVII In Vitro Clotting Activity:

Factor VII (FVII) activity assay, which was performed in Sheba Medical Center, the Israel National Coagulation Center, is a prothrombin (PT)-based assay using immuno-adsorbed plasma deficient in Factor VII (Siemens). The PT reagent is innovin, and the assay is performed in the Sysmex^{®} CA 1500 instrument. FVII normal range is within 55-145%.

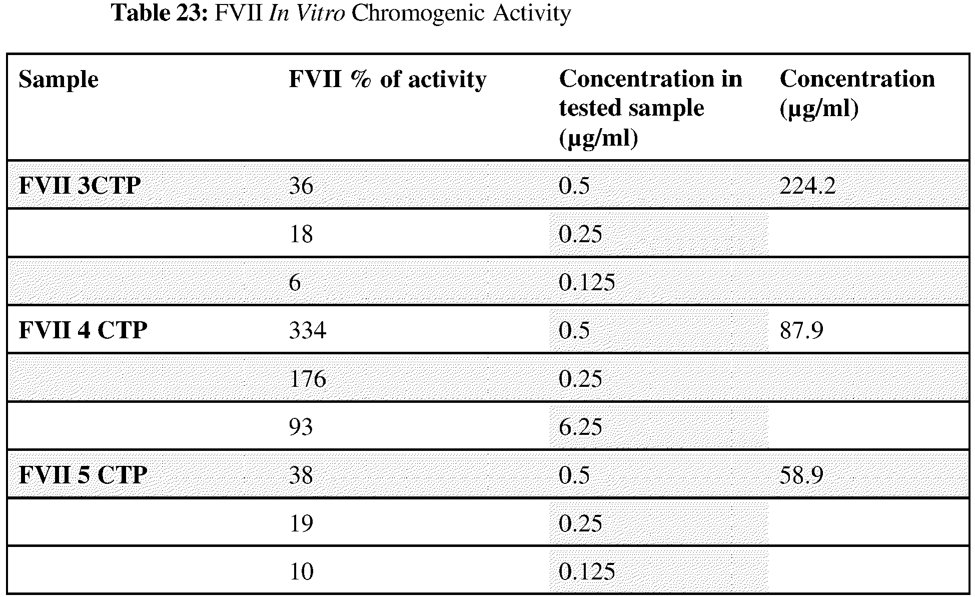

Since the normal level of circulating FVII in the body is around 0.5 µg/ml, FVII-CTP₃ and FVII-CTP₅ harvests exhibit 3-fold reductions in their coagulation activity versus normal human pool plasma; this result correlates with the obtained chromogenic activity **(Table 23).**

The FVII-CTP₄ harvest exhibits a 3-fold increase in its potential coagulation activity versus normal human pool plasma as observed in the chromogenic activity assay **(Table 23).** The activity percentage of FVII-CTP₄ is much higher compared to activity percentage of FVII-CTP₃ and FVII-CTP₅. Methodological limitations of the ELISA method may limit the accuracy of Ag level calculations of FVII-CTP₄.

### 5.8 Pharmacokinetic study

Two pharmacokinetic studies were performed in order to determine the FVII-CTP₃, FVII-CTP₄, and FVII-CTP₅ pharmacokinetics (PK) parameters. During the first study, FVII-CTP₃, FVII-CTP₄, and FVII-CTP₅ (Group A, B and C, respectively) were administered in a single intravenous injection to Sprague Dawley rats (six rats per treatment) in a dose of 250 µg/kg body weight. Blood samples were drawn retro-orbitally from 3 rats alternately at 0.083, 0.5 2, 5, 8, 24, 48, 72 and 96 hours post-dosing (Table 24). Citrated plasma (0.38%) was prepared immediately after sampling and stored at -20°C until analysis.

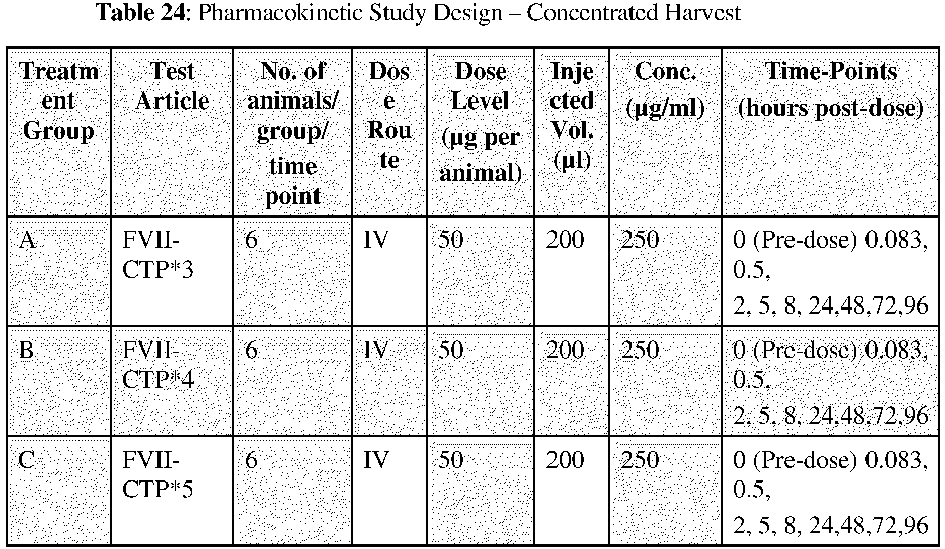

FVII concentration in plasma samples were quantified using human FVII Elisa kits (Zymutest FVII-Biophen). The pharmacokinetic profile was calculated and is the mean of 3 animals at each time point. Terminal half-life values were calculated using PK Solutions 2.0 Software. **Table 25** below summarizes the calculated FVII concentrations at the different sampling time points. The PK profile (Figures 23-24) and a summary of the PK parameters **(Table 26)** are also presented below. FVII-CTP₅ demonstrated a superior profile as compared to FVII-CTP₃ and FVII-CTP₄ **(Table 26).**

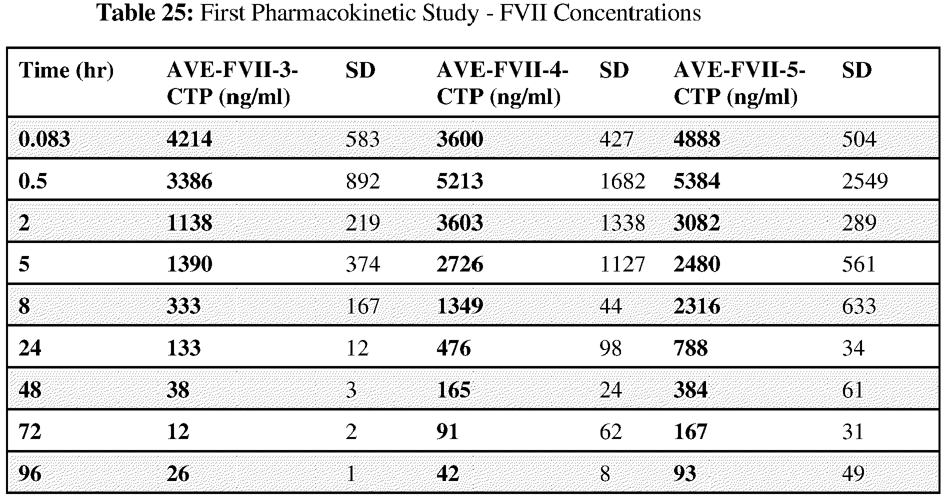

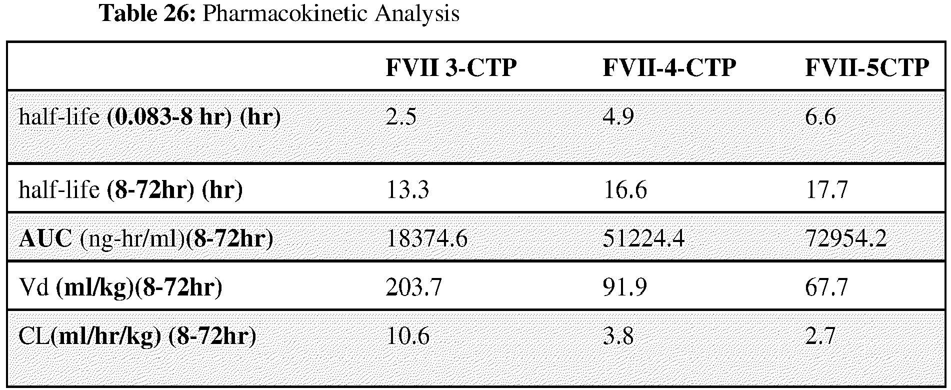

The addition of four or five CTPs significantly elongated FVII half-life as compared to 3 CTPs by 2- and 3-fold, respectively **(Table 26).** This superiority was more significant in the initial part of the study (0.083-8 hr), suggesting a potential improved protein recovery and reduced extra vascular clearance. AUC following FVII-CTP₄ and FVII-CTP₅ administration increased by 3- and 4-fold, respectively, versus FVII-CTP₃. Clearance was also reduced while adding 4 and 5 CTPs to FVII **(Table 26).**

As observed in the study, the addition of four and five CTPs significantly elongated FVII half-life as compared to 3 CTPs, both in the initial and terminal half-life. The half-life values in the first and second study are different due to a different analysis approach which was effected by the dose and study duration, nevertheless the overall trend was maintained. The AUC following FVII-CTP₄ and FVII-CTP₅ administration increased by 2.5- and 7-fold, respectively, versus FVII-CTP₃.

### 5.9 Conclusions:

In this study, the PK parameters and potential clotting activity of FVII-CTP₃, FVII-CTP₄, and FVII-CTP₅ were assessed. Fusion of 4 and 5 CTPs to FVII provided a superior and improved half-life, exposure and reduced clearance as compared to FVII-CTP₃ while maintaining a similar chromogenic and *in vitro* clotting activity. These results were observed at different concentrations of protein and were consistent for both harvest and purified protein. While evaluating the overall effect of fusion of CTP at the C terminus to FVII, fusion of 1-5 CTPs considerably increased the half-life and AUC of FVII in a CTP proportional manner, suggesting that as the CTP portion of the molecule increases, FVII longevity and stability is significantly improved while maintaining its initial *in vitro* clotting activity, as summarized in Table 27 hereinbelow.

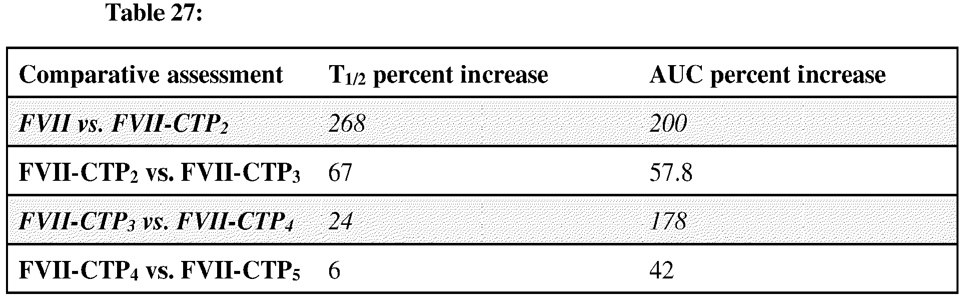

As previously reported, FVII half-life correlates with the half-life of the activated form of FVII (FVIIa) both in humans and animals. Therefore, it is anticipated that a similar improvement in half-life will be obtained for the activated versions following CTP fusion.

### EXAMPLE 7: EXPRESSION AND ISOLATION OF IFN-CTP POLYPEPTIDES MATERIALS AND METHODS

**DNA transfection and clone selection:** DG44 cells were transfected with pCI-DHFR expression vectors containing IFNβ-CTP variants using FuGENE6 Reagent (FuGENE Transfection Reagent - Roche Cat.11 815 091 001). 48 hr following transfection, cells were diluted and seeded at least 50-200 cells per well in a selective medium (CD DG44 Medium w/o HT (Gibco: Scotland part: #07990111A) Sku num.:ME060027 supplemented with 8 mM L-Glutamine Biological Industries: Cat: 03-020-1A) and 18 mL/L of 10 % Pluronic F-68 solution (Gibco: Cat: 240040-032). Selected clones were screened for highest protein production using commercial ELISA. 3-5 producing clones per each variant were frozen for a backup cell bank. A selected clone for each variant was adapted to growth in larger scale cultures up to 1L flasks on an orbital shaker platform. Supernatants were collected and analyzed by ELISA, SDS-PAGE and western blot. Following the withdrawal of aliquots, the protein-containing supernatants were kept frozen until further use.

**Cell culture:** DG44 cells were maintained in DG44 medium with HT (cat# 12610-010, Gibco) supplemented with 8 mM L-Glutamine (Biological Industries: Cat: 03-020-1A) and 18 mL/L of 10 % Pluronic F-68 solution (Gibco: Cat: 240040-032), at 37 °C in humidified 8 % CO₂ incubator. Transfected clones were maintained in DG44 basal medium without HT supplement, hypoxanthine and thymidine, with pluronic acid and L-glutamine.

**Sample preparation:** Supernatants were collected, filtrated and analyzed by ELISA to determine protein concentration. SDS-PAGE and western blot were used to determine purity and identity. Following ELISA, sample concentrations were defined and the solution was dialyzed against PBS. Following the withdrawal of aliquots, the protein-contained supernatants were kept frozen at -20⁰C until further use.

**Western Blotting:** Samples were electrophoresed on nondenaturing 15 % SDS-polyacrylamide gels. Gels were allowed to equilibrate for 10 min in 25 mM Tris and 192 mM glycine in 20 % (vol/vol) methanol). Proteins were transferred to a 0.2 µm pore size nitrocellulose membrane (Sigma, Saint Louis, MO) at 250 mA for 3h, using a Mini Trans-Blot electrophoresis cell (Biorad Laboratories, Richmond, CA). The nitrocellulose membrane was incubated in 5 % non-fat dry milk for 2 h at room temperature. The membrane was incubated with IFN anti-serum (1:1000 titer) overnight at least 4 °C followed by three consecutive washes in PBS containing 0.1 % Tween (10 min/wash). The membrane was incubated with secondary antibody conjugated to Horse Radish Peroxidase (HRP) (Zymed, San Francisco, CA) for 2 h at room temperature, followed by three washes. Finally, the nitrocellulose paper was reacted with enhanced chemiluminescent substrate (ECL) (Pierce, Rockford, IL) for 5 min, dried with a Whatman sheet, and exposed to X-ray film.

**Figure 3** indicates that MOD-901X-variants are recognized by anti IFN-β1a antibodies. The SDS PAGE gel was stained using Coomassie blue (a) or (B). blotted and stained using monoclonal anti-IFN-β1a antibodies

### Materials and Methods (Examples 8-11):

### Plasmid Construction

Seven OXM plasmids were constructed in eukaryotic expression vector (pCI-dhfrr) based on the OXM amino acid sequence (accession # NP_002045) and the CTP amino acid sequence (accession # NP_149032).

A schematic presentation of these plasmids is shown in **Table 28.** A detailed description of plasmids construction follows.

**Table 28: Schematic description of plasmids**

| | Plasmid variant description | Nucleic Acid Sequence |
|---|---|---|
| 1 | OXM-CTP-CTP | SEQ ID NO: 67 |
| 2 | CTP-OXM-CTP | SEQ ID NO: 65 |
| 3 | CTP-CTP-OXM | SEQ ID NO: 68 |
| 4 | CTP-OXM-CTP-CTP | SEQ ID NO: 66 |
| 5 | OXM-CTP-CTP-CTP | SEQ ID NO: 69 |
| 6 | OXM-CTP-CTP-CTP-CTP | SEQ ID NO: 70 |
| 7 | OXM-CTP-CTP-CTP-CTP-CTP | SEQ ID NO: 71 |

A nucleic acid sequence of the OXM variant CTP-OXM-CTP (0.5C CTP-OXM-CTP, GenArt, GA # 0804377) was synthesized after a codon usage optimization for DG44 expression system. An XbaI-NotI fragment containing 0.5C CTP-OXM-CTP sequence was isolated. The fragment was inserted into the eukaryotic expression vector pCI-dhfr to yield the 601-0-p142-1 clone.

### Construction of CTP-OXM-CTP

The following primers were used in order to synthesize 601-6-p149-1 (CTP-OXM-CTP).
**Primer 25** 5' CTCTAGAGGACATGGCCAC 3' (SEQ ID NO: 95).
**Primer 85R**5' CTGGCTGTGCTGGGGCAGAATGGGTGT 3' (SEQ ID NO: 96).
**Primer 86** 5' CCCCAGCACAGCCAGGG 3' (SEQ ID NO: 97).
**Primer 74R** 5' GCGGCCGCATCCAGACCT 3' (SEQ ID NO: 98).

Three PCR reactions were performed. The first reaction was conducted with primer 25 and primer 85R and plasmid DNA of 402-3-p81-4 (CTP-hGH-CTP-CTP) as a template; as a result of the PCR amplification, a -181 bp product was formed. The second reaction was conducted with primer 86 and primer 74R and plasmid DNA of 601-0-p142-1 (0.5C CTP-OXM-CTP) as a template; as a result of the PCR amplification, a -224 bp product was formed. The last reaction was conducted with primers 25 and 74R and a mixture of the products of the previous two reactions as a template; as a result of the PCR amplification, a ~ 391 bp product was formed and ligated into TA cloning vector (Invitrogen, catalog K2000-01). An XbaI-NotI fragment containing CTP-OXM-CTP sequence was isolated. The fragment was inserted into our eukaryotic expression vector pCI-dhfr to yield 601-6- p 149-1 clone.

The nucleic acid sequence encoding CTP-OXM-CTP is as follows:

tctagaggacatggccaccggcagcaggaccagcctgctgctggccttcggcctgctgtgcctgccatggctgcaggaggg
cagcgccagctcttcttctaaggctccacccccatctctgcccagccccagcagactgccgggccccagcgacacacccattctgccccagc
acagccagggcaccttcaccagcgactacagcaagtacctggacagcagaagggcccaggacttcgtccagtggctgatgaacaccaaga
ggaacaggaacaacatcgcttcctctagctccaaggcccctccaccctctctgcctagcccctctcggctgcctggcccatccgacacaccaa
tcctgccacagtgatgaaggtctggatgcggccgc (SEQ ID NO: 65).

The amino acid sequence encoding CTP-OXM-CTP is as follows:

MATGSRTSLLLAFGLLCLPWLQEGSASSSSKAPPPSLPSPSRLPGPSDTPILPQHS
QGTFTSDYSKYLDSRRAQDFVQWLMNTKRNRNNIASSSSKAPPPSLPSPSRLPGPSDTPIL
PQ (SEQ ID NO: 58).

### Construction of CTP-OXM-CTP-CTP

The following primers were used in order to synthesize 601-3-p158-2 (CTP-OXM-CTP-CTP):
**Primer 25** 5' CTCTAGAGGACATGGCCAC 3' (contains the restriction site of XbaI ) (SEQ ID NO: 99).
**Primer 87^{R}** 5' GCTGGAGCTAGCGATGTTGTTCCTGTTCC 3' (contains the 3' end of OXM and the 5' end of CTP) (SEQ ID NO: 100).
**Primer 88** 5' ACATCGCTAGCTCCAGCAGCAAGGCC 3' (contains the 3' end of OXM and the 5' end of CTP) (SEQ ID NO: 101).
**Primer 74^{R}** 5' GCGGCCGCATCCAGACCT 3' (contains the restriction site of NotI) (SEQ ID NO: 102).

Three PCR reactions were performed. The first reaction was conducted with primer 25 and primer 87R and plasmid DNA of 601-6-p149-1 (CTP-OXM-CTP-CTP) as a template; as a result of the PCR amplification, a ∼ 290 bp product was formed. The second reaction was conducted with primer 88 and primer 74R and plasmid DNA of 402-3-p81-4 (CTP-hGH-CTP-CTP) as a template; as a result of the PCR amplification, a ~200 bp product was formed. The last reaction was conducted with primers 25 and 74R and a mixture of the products of the previous two reactions as a template; as a result of the PCR amplification, a ~ 450 bp product was formed and ligated into TA cloning vector (Invitrogen, catalog K2000-01). Xbal -NotI fragment containing CTP-OXM-CTP-CTP sequence was isolated. The fragment was inserted in to our eukaryotic expression vector pCI-dhfr to yield 601-3-p158-2 clone.

The nucleic acid sequence encoding CTP-OXM-CTP-CTP is as follows:

tctagaggacatggccaccggcagcaggaccagcctgctgctggccttcggcctgctgtgcctgccatggctgcaggaggg
cagcgccagctcttcttctaaggctccacccccatctctgcccagccccagcagactgccgggccccagcgacacacccattctgccccagc
acagccagggcaccttcaccagcgactacagcaagtacctggacagcagaagggcccaggacttcgtccagtggctgatgaacaccaaga
ggaacaggaacaacatcgctagctccagcagcaaggcccctcccccgagcctgccctccccaagcaggctgcctgggccctccgacaca
ccaatcctgccacagagcagctcctctaaggcccctcctccatccctgccatccccctcccggctgcctggcccctctgacacccctatcctgc
ctcagtgatgaaggtctggatgcggccgc (SEQ ID NO: 66).

The amino acid sequence encoding CTP-OXM-CTP-CTP is as follows:

MATGSRTSLLLAFGLLCLPWLQEGSASSSSKAPPPSLPSPSRLPGPSDTPILPQHS
QGTFTSDYSKYLDSRRAQDFVQWLMNTKRNRNNIASSSSKAPPPSLPSPSRLPGPSDTPIL
PQSSSSKAPPPSLPSPSRLPGPSDTPILPQ (SEQ ID NO: 59).

### Construction of OXM-CTP-CTP

The following primers were used in order to synthesize 601-2-p160-2 (OXM-CTP-CTP):
**Primer 75** 5' CTCCCAGTTCAATTACAGCT 3' (contains sequence of pCI-dhfr before Xbal restriction site) (SEQ ID NO: 103).
**Primer 89^{R}** 5' GCTGTGAGCGCTGCCCTCCTGCAG 3' (contains 5' end of OXM) (SEQ ID NO: 104).
**Primer 90** 5' GCGCTCACAGCCAGGGCACCTTC 3' (contains the 5' end of OXM) (SEQ ID NO: 105).
**Primer 74^{R}** 5' GCGGCCGCATCCAGACCT 3' (contains the restriction site of NotI) (SEQ ID NO: 106).

Three PCR reactions were performed. The first reaction was conducted with primer 75 and primer 89R and plasmid DNA of 601-0-p142-1 (0.5C CTP-OXM-CTP) as a template; as a result of the PCR amplification, a ~175 bp product was formed. The second reaction was conducted with primer 90 and primer 74R and plasmid DNA of 601-3-p158-2 (CTP-OXM-CTP-CTP) as a template; as a result of the PCR amplification, a ∼ 200 bp product was formed. The last reaction was conducted with primers 25 and 74R and a mixture of the products of the previous two reactions as a template; as a result of the PCR amplification, a ~ 391 bp product was formed and ligated into TA cloning vector (Invitrogen, catalog K2000-01). XbaI -NotI fragment containing OXM-CTP-CTP sequence was isolated. The fragment was inserted into our eukaryotic expression vector pCI-dhfr to yield the 601-2-p160-2 clone.

The nucleic acid sequence encoding OXM-CTP-CTP is as follows:

tctagaggacatggccacagggagcaggaccagcctgctgctggctttcggcctgctgtgtctgccatggctgcaggaggg
cagcgctcacagccagggcaccttcaccagcgactacagcaagtacctggacagcagaagggcccaggacttcgtccagtggctgatgaa
caccaagaggaacaggaacaacatcgctagctccagcagcaaggcccctcccccgagcctgccctccccaagcaggctgcctgggccct
ccgacacaccaatcctgccacagagcagctcctctaaggcccctcctccatccctgccatccccctcccggctgcctggcccctctgacacc
cctatcctgcctcagtgatgaaggtctggatgcggccgc (SEQ ID NO: 67)

The amino acid sequence encoding OXM-CTP-CTP is as follows:

LEDMATGSRTSLLLAFGLLCLPWLQEGSAHSQGTFTSDYSKYLDSRRAQDFV
QWLMNTKRNRNNIASSSSKAPPPSLPSPSRLPGPSDTPILPQSSSSKAPPPSLPSPSRLPGPS
DTPILPQ (SEQ ID NO: 60)

### Construction of CTP- CTP-OXM

A nucleic acid sequence of OXM variant (CTPx2-OXM, GenArt, GA # 1067101) was synthesized after a codon usage optimization for DG44 expression system. The fragment was inserted in to Excellgene eukaryotic expression vector to yield p162 clone.

The nucleic acid sequence encoding CTPx2-OXM is as follows:

ctatagggcgaattgaaggaaggccgtcaaggccgcatgagctctctagaggacatggccaccggcagcaggaccagcct
gctgctggccttcggcctgctgtgcctgccatggctgcaggagggcagcgccagctccagcagcaaggcccctcccccgagcctgccctc
cccaagcaggctgcctgggccctccgacacaccaatcctgccacagagcagctcctctaaggcccctcctccatccctgccatccccctccc
ggctgcctggcccctctgacacccctatcctgcctcagcacagccagggcaccttcaccagcgactacagcaagtacctggacagcagaag
ggcccaggacttcgtccagtggctgatgaacaccaagaggaacaggaacaacatcgcttgatgaaggtctggatgcggccgcggtaccctg
ggcctcatgggccttcctttcactgcccgctttccag (SEQ ID NO: 68).

The amino acid sequence encoding CTPx2-OXM is as follows:





 (SEQ ID NO: 61).

### Construction of OXM-CTPx3:

A nucleic acid sequence of OXM variant (OXM-CTPx3, GenArt, GA # 1017864) was synthesized after a codon usage optimization for DG44 expression system. XbaI -NotI fragment containing OXM-CTPx3 sequence was isolated. The fragment was inserted in to our eukaryotic expression vector pCI-dhfr to yield pCI-dhfr-OXM-ctpx3-p216-4 clone.

The nucleic acid sequence encoding OXM-CTPx3 is as follows:

tctagactcgagcgatcgccatggccaccggctctaggacctccctgctgctggccttcggcctgctgtgcctgccctggctg
caggaaggcagcgctcactcccagggcaccttcacctccgactactccaagtacctggactctcggagagcccaggacttcgtgcagtggct
gatgaacaccaagcggaaccggaacaatatcgcctcctcaagctccaaggcacctccaccttccctgcctagcccttccagactccctgggc
ccagtgacacccctatcctgcctcagtccagctccagcaaggccccaccccctagcctgccttctccttctcggctgcctggccccagcgata
ctccaattctgccccagtcctccagcagtaaggctccccctccatctctgccatcccccagcagactgccaggcccttctgatacacccatcctc
ccacagtgatgaggatccgcggccgc (SEQ ID NO: 69).

The amino acid sequence encoding OXM-CTPx3 is as follows:

M A T G S R T S L L L A F G L L C L P W L Q E G S A H S Q G T
F T S D Y S K Y L D S R R A Q D F V Q W L M N T K R N R N N I A S S
S S K A P P P S L P S P S R L P G P S D T P I L P Q S S S S K A P P P S L
P S P S R L P G P S D T P I L P Q S S S S K A P P P S L P S P S R L P G P
S D T P I L P Q (SEQ ID NO: 62).

### Construction of OXM-CTPx4:

A nucleic acid sequence of OXM variant (OXM-CTPx4, GenArt, GA # 1115769) was synthesized after a codon usage optimization for DG44 expression system. XbaI -NotI fragment containing OXM-CTPx4 sequence was isolated. The fragment was inserted in to the eukaryotic expression vector pCI-dhfr to yield pCI-dhfr-OXM-ctpx4 -p254-3 clone.

The nucleic acid sequence encoding OXM-CTPx4 is as follows:

tctagactcgagcgatcgccatggctaccggctccagaacctctctgctgctggccttcggcctgctgtgtctgccttggctgc
aagagggcagcgctcattcccagggcaccttcacctccgactactccaagtacctggactctcgcagagcccaggacttcgtgcagtggctg
atgaacaccaagcggaaccggaacaatatcgcctcctccagctccaaggcccctcctccatctctgccatcccccagtagactgcctgggcc
ctctgacacccctatcctgcctcagtccagctcctctaaggccccaccaccttccctgcctagcccttcaagactgccaggccctagcgataca
ccaattctgccccagtcctccagcagcaaggctcccccacctagcctgccttctccatcaaggctgcctggcccatccgataccccaattttgc
ctcagagcagctctagcaaggcacctccccccagtctgccctctccaagcagactccctggcccttcagacactcccattctgccacagtgat
gaggatccgcggccgc (SEQ ID NO: 70)

The amino acid sequence encoding OXM-CTPx4 is as follows:

M A T G S R T S L L L A F G L L C L P W L Q E G S A H S Q G T
F T S D Y S K Y L D S R R A Q D F V Q W L M N T K R N R N N I A S S
S S K A P P P S L P S P S R L P G P S D T P I L P Q S S S S K A P P P S L
P S P S R L P G P S D T P I L P Q S S S S K A P P P S L P S P S R L P G P
S D T P I L P Q S S S S K A P P P S L P S P S R L P G P S D T P I L P Q
(SEQ ID NO: 63).

### Construction of OXM-CTPx5:

A nucleic acid sequence of OXM variant (OXM-CTPx5, GenArt, GA # 1115770) was synthesized after a codon usage optimization for DG44 expression system. XbaI -NotI fragment containing OXM-CTPx5 sequence was isolated. The fragment was inserted in to the eukaryotic expression vector pCI-dhfr to yield pCI-dhfr-OXM-ctpx5 -p255-1 clone.

The nucleic acid sequence encoding OXM-CTPx5 is as follows:

ctctagactcgagcgatcgccatggctaccggctccagaacctctctgctgctggccttcggcctgctgtgtctgccttggctg
caagagggcagcgctcattcccagggcaccttcacctccgactactccaagtacctggactctcgcagagcacaggacttcgtgcagtggct
gatgaacaccaagcggaaccggaacaatatcgcctcctccagctccaaggcccctcctccatctctgccatcccccagtagactgcctgggc
cctctgacacccctatcctgcctcagtccagctcctctaaggctccaccaccttccctgcctagcccttcaagactgccaggccctagcgatac
accaattctgccccagtcctccagcagcaaggctcccccacctagcctgccttctccatcaaggctgcctggcccatccgataccccaattttg
cctcagagcagctctagcaaggcacctccccccagtctgccctctccaagcagactccctggcccttcagacactccaatcctcccacagtcc
tctagctctaaagctccacctcccagcctgcccagccctagtagactccccggaccttctgatacccccatcttgccccagtgatgaggatccg
cggccgc (SEQ ID NO: 71).

The amino acid sequence encoding OXM-CTPx5 is as follows:

M A T G S R T S L L L A F G L L C L P W L Q E G S A H S Q G T
F T S D Y S K Y L D S R R A Q D F V Q W L M N T K R N R N N I A S S
S S K A P P P S L P S P S R L P G P S D T P I L P Q S S S S K A P P P S L
P S P S R L P G P S D T P I L P Q S S S S K A P P P S L P S P S R L P G P
S D T P I L P Q S S S S K A P P P S L P S P S R L P G P S D T P I L P Q S
S S S K A P P P S L P S P S R L P G P S D T P I L P Q  (SEQ ID NO: 64).

### Expression, purification and characterization of OXM-CTP variants

All seven variants of OXM-CTPs were transiently expressed and produced in XLG's CHO-Express cell line (Excellgene Company, Switzerland). Concentration levels of OXM-CTPs harvests were determined using an OXM ELISA kit (Bachem Cat# S-1393.0001). The harvest was purified using a DEAE column, followed by a Jacalin column as a second purification step. The final fractions were dialyzed against 10mM buffer citrate, 147 mM NaCl, pH 6. The concentration of purified variants was determined by absorbance at 280 nm using extinction coefficient of 1.9=1mg/ml. 1.9 is the extinction coefficient that theoretically was calculated for the OXM peptide. Since CTP peptide does not absorb at 280 nm, the same extinction coefficient was applied for OXM-CTP variants. OXM native peptide was chemically synthesized (Almac Company, Ireland), and its peptide content was determined by amino acid analysis.

### Pharmacokinetic (PK) profile of OXM-CTP variants

The pharmacokinetic profiles of the OXM peptide and OXM-CTP variants were assessed as follows. Male Sprague-Dawley (SD)-1 rats were administered subcutaneously (SC) or intravenously (IV) with a single dose of native OXM (n=6, 230µg/kg), of variants CTP-OXM-CTP, CTP-OXM-CTP-CTP, OXM-CTP-CTP and CTP-CTP-OXM (n=6, 230µg/kg) or variants # OXM-CTP-CTP-CTP, OXM-CTP-CTP-CTP-CTP, OXM-CTP-CTP-CTP-CTP-CTP (n=6, 153 µg/kg). Cohorts of 3 animals per group were bled at alternating time points. OXM serum concentration was analyzed using commercial ELISA kit (Bachem, Cat# S-1393.0001). The study design is summarized in **Table 29.** The study was divided into 3 sequential experiments, Experiment #1 (Groups 1-4), Experiment #2 (Groups 5-9) and Experiment #3 (Groups 10-13).

**Table 29: Summary of the PK study design. IPGTT study in C57BL/6 mice**

| Group ^{#} | Test Article Variant # | No. of animals/ group/ timepoint | Dose Route | Gender | Dose Level of OXM (µg/rat) | Dose Level of OXM (µg/kg) | Time-Points (hours post-dose) |
|---|---|---|---|---|---|---|---|
| 1 | OXM | 6/3 | IV | Male | 34 | 230 | 0 (Pre-dose), 5min, 15min, 25min, 35min, 45min, 60min, 75min, 1.5h, 2h |
| 2 | CTP-OXM-CTP | 6/3 | IV | Male | 34 | 230 | 0 (Pre-dose), 10min, 30min, 1h, 2h, 3h, 4h, 6h, 8h, 24h, 36h, 48h |
| 3 | CTP-OXM-CTP-CTP | 6/3 | IV | Male | 34 | 230 | 0 (Pre-dose), 10min, 30min, 1h, 2h, 3h, 4h, 6h, 8h, 24h, 36h, 48h |
| 4 | CTP-CTP-OXM | 6/3 | IV | Male | 34 | 230 | 0 (Pre-dose), 10min, 30min, 1h, 2h, 3h, 4h, 6h, 8h, 24h, 36h, 48h |
| 5 | OXM | 6/3 | SC | Male | 34 | 230 | 0 (Pre-dose), 5min, 15min, 25min, 35min, 45min, 60min 75min, 1.5h, 2h |
| 6 | CTP-OXM-CTP | 6/3 | SC | Male | 34 | 230 | 0 (Pre-dose), 10min, 30min, 1h, 2h,4h, 6h, 8h,24h, 36h, 2d, 3d |
| 7 | CTP-OXM-CTP-CTP | 6/3 | SC | Male | 34 | 230 | 0 (Pre-dose), 10min, 30min, 1h, 2h,4h, 6h, 8h, 24h, 36h, 2d, 3d |
| 8 | CTP-CTP-OXM | 6/3 | SC | Male | 34 | 230 | 0 (Pre-dose), 10min, 30min, 1h, 2h,4h, 6h, 8h, 24h, 36h, 2d,3d |
| 9 | OXM-CTP-CTP | 6/3 | SC | Male | 34 | 230 | 0 (Pre-dose), 10min, 30min, 1h, 2h, 4h, 6h, 8h, 24h, 36h, 2d, 3d |
| 10 | OXM | 6/3 | SC | Male | 34 | 230 | 0 (Pre-dose), 5min, 15min, 25min, 35min, 45min, 60min 75min, 1.5h, 2h |
| 11 | OXM-CTPX3 | 6/3 | SC | Male | 23 | 153 | 0 (Pre-dose), 10min, 30min, 1h, 2h, 4h, 6h, 8h, 24h, 36h, 2d, 3d, 4d |
| 12 | OXM-CTPX4 | 6/3 | SC | Male | 23 | 153 | 0 (Pre-dose), 10min, 30min, 1h, 2h,4h, 6h, 8h, 24h, 36h, 2d, 3d, 4d |
| 13 | OXM-CTPX5 | 6/3 | SC | Male | 23 | 153 | 0 (Pre-dose), 10min, 30min, 1h, 2h, 4h, 6h, 8h, 24h, 36h, 2d, 3d, 4d |

Overnight-fasted mice were measured for glucose (pre-dose glucose). Immediately after, they were IP injected with one of the test articles. Fifteen or 120 min post injection, glucose levels were measured (zero time for glucose) followed by glucose administration via IP injection (1.5 g/kg). Additional glucose measurement at 10, 20, 30, 60, 90, 120 and 180 min were performed. Blood glucose level were measured by tail vein sampling using a handheld glucometer.

### Food intake study in C57BL/6 mice

At least one week before injection, mice were weighed and were transferred to a mini cage for acclimatization, (one mouse per cage). During the acclimatization period, they were handled daily and received two injections of vehicle to minimize stress during the study period. The day before the experiment, the mice were fasted. Seventeen hours after fasting, at the early light phase (900-1000h), the mice were weighed again (prior to the IP injection) followed by a single IP injection of 1700 nmol/kg (10 µl/1g mice) of OXM peptide or OXM-CTP variants OXM-CTPX3, OXM-CTPX4 and OXM-CTPX5. After injection, the mice were returned to their home cages (1 mouse per cage), and provided with a pre-weighed amount of chow. Food intake were measured 0, 1, 2, 4, 6, 8, 21, 32 and 44, 55, 68, 80, 93 and 141h post injection by weighing the chow. At the end of experiment, rats were weighed again.

### RESULTS

### EXAMPLE 8: CONSTRUCTION OF CTP-MODIFIED OXM

By genetic engineering, CTP peptide cDNA was fused to human OXM cDNA, generating seven different OXM-CTP variant as detailed in **Table 28.** The nucleotide sequences of the plasmids were verified, and the plasmids were transiently transfected into XLG's CHO-Express cell line (Excellgene Company, Switzerland). The OXM-CTP variants were secreted into the growth medium, harvests were collected and OXM-CTPs levels were determined.

### EXAMPLE 9: EXPRESSION, PURIFICATION AND CHARACTERIZATION OF OXM-CTP VARIANTS

The production media (harvests) were measured for secretion level of OXM-CTP variants. The secretion levels are summarized in **Table 31.** The secretion levels obtained were high considering the standard transient transfection expression levels of recombinant peptides.

**Table 31: Summary of OXM-CTPs secretion level.**

| **Variant** | CTP-OXM-CTP | CTP-OXM-CTP-CTP | OXM-CTP-CTP | CTP-CTP-OXM | OXM-CTPX4 | OXM-CTPX4 | OXM-CTPX5 |
|---|---|---|---|---|---|---|---|
| **Harvest concentration (µg/ml)** | 31.1 | 15.3 | 3.3 | 51.9 | 13.9 | 15.3 | 14.4 |

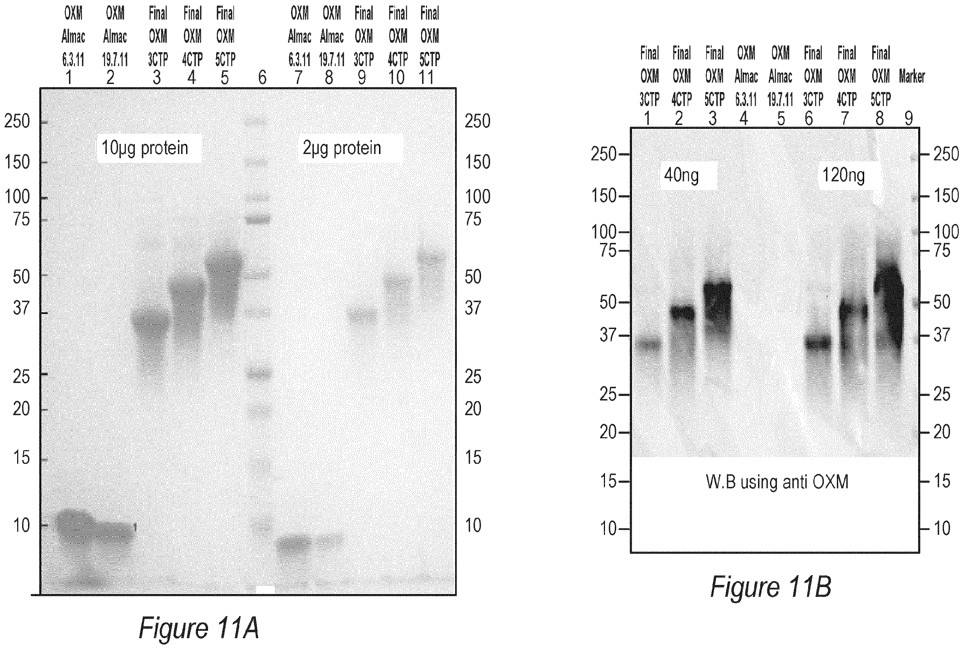

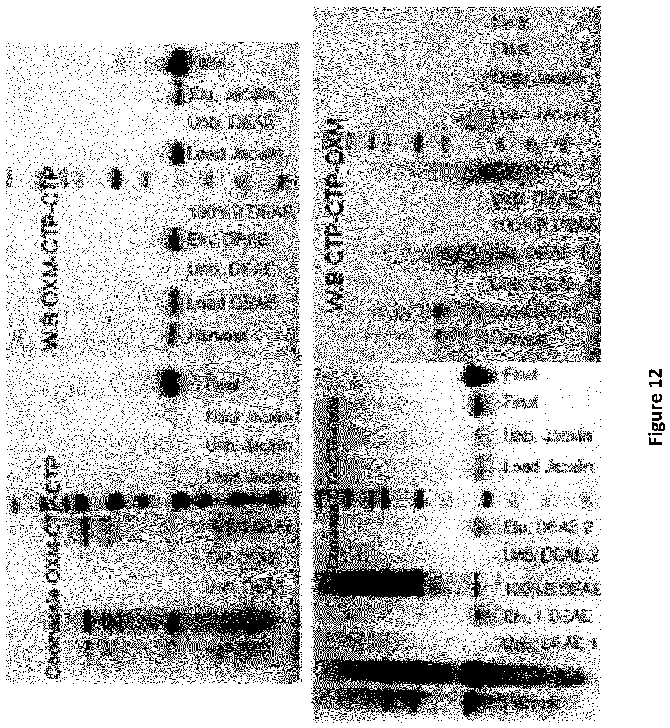

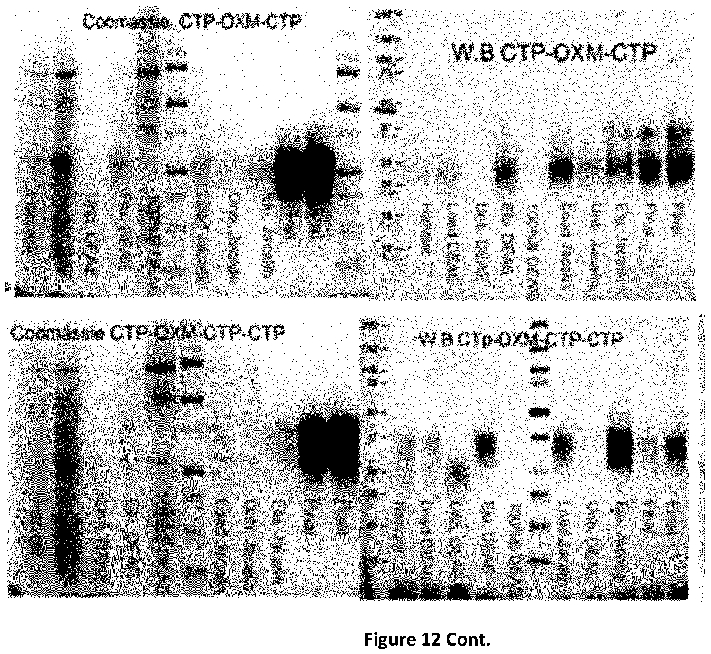

The harvests were purified according to the method described in *Materials and Methods.* The final samples of variants OXM-CTPX3, OXM-CTPX4 and OXM-CTPX5 were analyzed by SDS-PAGE (Coomassie staining **(****Figure 11A****)** and anti-OXM Western Blot Analysis **(****Figure 11B****)).** For variants CTP-OXM-CTP, CTP-OXM-CTP-CTP, OXM-CTP-CTP and CTP-CTP-OXM, samples from the purification process were analyzed by both Coomassie staining and Western Blot analysis **(****Figure 12****).** As expected, OXM-CTP variants showed differences in size correlating to the number of CTP cassettes fused to the peptide. The relatively high molecular weights are an indication that there may be high occupancy of the O-glycan chains on the potential serine sites on the CTP peptide. The OXM peptide did not react with the anti-OXM antibody, probably due to the technical difficulty in transferring a small sized peptide. The Coomassie staining shows that the OXM-CTP variants were highly purified and contained mainly the high form.

### EXAMPLE 10: PHARMACOKINETIC (PK) PROFILE OF OXM-CTP VARIANTS

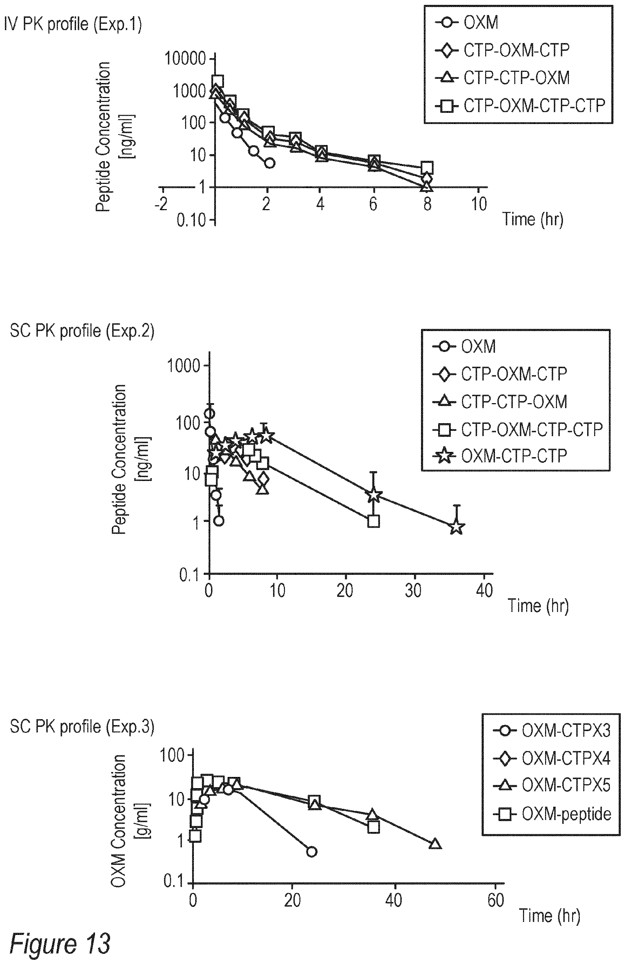

The pharmacokinetic profile of OXM peptide compared to OXM-CTP variants was analyzed in male SD-1 rats. Animals were administered with a single IV (Experiment 1, Figure 13A) or single SC (Experiments 2&3, Figures 13B-13C) injection of native OXM or OXM-CTP variants CTP-OXM-CTP, CTP-OXM-CTP-CTP, OXM-CTP-CTP, CTP-CTP-OXM (230µg/kg peptide) or variants OXM-CTPX3, OXM-CTPX4 and OXM-CTPX5 (153µg/kg peptide). Serum concentration of OXM or OXM-CTP variants at the indicated time points was analyzed using commercial ELISA. The PK profile is shown in **Figure 13****,** and the conventional noncompartmental PK parameters are summarized in Table 32. Addition of CTP peptides to OXM resulted in prolonging the half-life of native OXM, from 0.22hr to 2.7 - 10 h for various OXM-CTP variants (Table 32 SC administration; Experiments 2 &3).

Two copies of CTP were added to OXM peptide in three different ways to produce variants CTP-OXM-CTP, OXM-CTP-CTP and CTP-CTP-OXM. The most significant extension of serum half-life was received when the CTPs were added in tandem to the C-terminus of OXM; variant OXM-CTP-CTP with T½ of 4.76h. Fusion of three CTPs to the OXM C-terminus did not results in an elongated half-life as compared to fusion of two CTPs. Surprisingly, addition of four and five copies of CTPs to OXM c-terminus elongated the T ½ up to 10h **(Table 32).**

Exposure as reflected by the AUC parameter, was most increased by ∼17-fold for variant OXM-CTP-CTP (Experiment 2) and ~30-fold for variants OXM-CTPX4 and OXM-CTPX5 (Experiment 3). These results indicate that there is a superior prolonging effect after the addition of four or five copies of CTP to the OXM peptide.

The bioavailability as calculated from Experiments 1 & 2 was increased for CTP-OXM-CTP-CTP, but no significant improvement was found for CTP-OXM-CTP and CTP-CTP-OXM **(Table 32).**

**Table 32: Conventional noncompartmental PK parameters of OXM peptide and OXM-CTP variants as were determined in three different experiments.**

| | | **T1/2 hr** | **T1/2ß hr** | **Cmax ng/ml** | **Tmax hr** | **AUC(0-t) ng-hr/ml** | **AUC∞ ng-hr/ml** | **CL ml/hr/kg** | **Vd ml/kg** | **MRT hr** | ***Bioavail ability* %** |
|---|---|---|---|---|---|---|---|---|---|---|---|
| **Exp. 1 IV.** | **OXM** | 0.29 | NA | NA | NA | 211.7 | 213.9 | 993.0 | 417.0 | 0.34 | |
| | CTP-OXM-CTP | 0.25 | 1.57 | NA | NA | 592.7 | 598.0 | 355.3 | 806.9 | 0.81 | |
| | CTP-OXM-CTP-CTP | 0.28 | 1.61 | NA | NA | 968.2 | 977.4 | 217.4 | 506.4 | 0.80 | |
| | CTP-CTP-OXM | 0.27 | 1.20 | NA | NA | 488.6 | 490.4 | 433.3 | 748.1 | 0.86 | |
| **Exp. 2 SC.** | **OXM** | 0.22 | | 118.40 | 0.08 | 42.52 | 42.84 | 4960.39 | 1564.40 | 0.37 | 20.09 |
| | CTP-OXM-CTP | 3.28 | | 31.11 | 1.00 | 144.87 | 170.82 | 1243.97 | 5887.75 | 4.73 | 24.44 |
| | CTP-OXM-CTP-CTP | 4.39 | | 30.18 | 6.00 | 313.98 | 320.86 | 662.28 | 4139.25 | 6.62 | 32.43 |
| | OXM-CTP-CTP | 4.76 | | 50.90 | 6.00 | 697.00 | 702.10 | 302.70 | 2078.90 | 8.20 | NA |
| | CTP-CTP-OXM | 2.74 | | 27.77 | 1.00 | 114.15 | 129.85 | 1636.55 | 6459.76 | 4.10 | 23.36 |
| **Exp. 3 SC.** | **OXM** | 0.18 | | 36.4 | 0.08 | 13.1 | 13.3 | 16999 | 4375 | | |
| | OXM-CTPX3 | 3.78 | | 25.1 | 2.0 | 308.9 | 312 | 512.8 | 2797 | | |
| | OXM-CTPX4 | 10.15 | | 21.1 | 2.0 | 400.4 | 431.9 | 355.4 | 5379.6 | | |
| | OXM-CTPX5 | 9.63 | | 21.2 | 8 | 431 | 442 | 346.2 | 4812 | | |

A comparison between the various groups showing the fold increase of T1/2 and AUC∞ demonstrated that OXM-CTPx4 and OX-CTPx5 were superior in these parameters compared to other variants **(Table 33).**

**Table 33: Fold of increase of OXM-CTP variants's PK parameters in compared to OXM peptide as calculated from Experiments 1-3.**

| | Variant | Tl/2 Sample/OXM peptide | AUC∞ Sample/OXM peptide |
|---|---|---|---|
| Exp. 1 IV. | CTP-OXM-CTP | NA | 2.8 |
| | CTP-OXM-CTP-CTP | NA | 5.6 |
| | CTP-CTP-OXM | NA | 2.29 |
| Exp. 2 SC. | CTP-OXM-CTP | 14.9 | 3.98 |
| | CTP-OXM-CTP-CTP | 19.9 | 7.48 |
| | OXM-CTP-CTP | 21.6 | 16.3 |
| | CTP-CTP-OXM | 12.4 | 3.03 |
| Exp. 3 SC. | OXM-CTP-CTP-CTP | 21 | 23.45 |
| | OXM-CTP-CTP-CTP-CTP | 56.4 | 32.478 |
| | OXM-CTP-CTP-CTP-CTP-CTP | 53.5 | 33.23 |

### EXAMPLE 11: CTP-MODIFIED OXM INDUCES GLUCOSE TOLERANCE

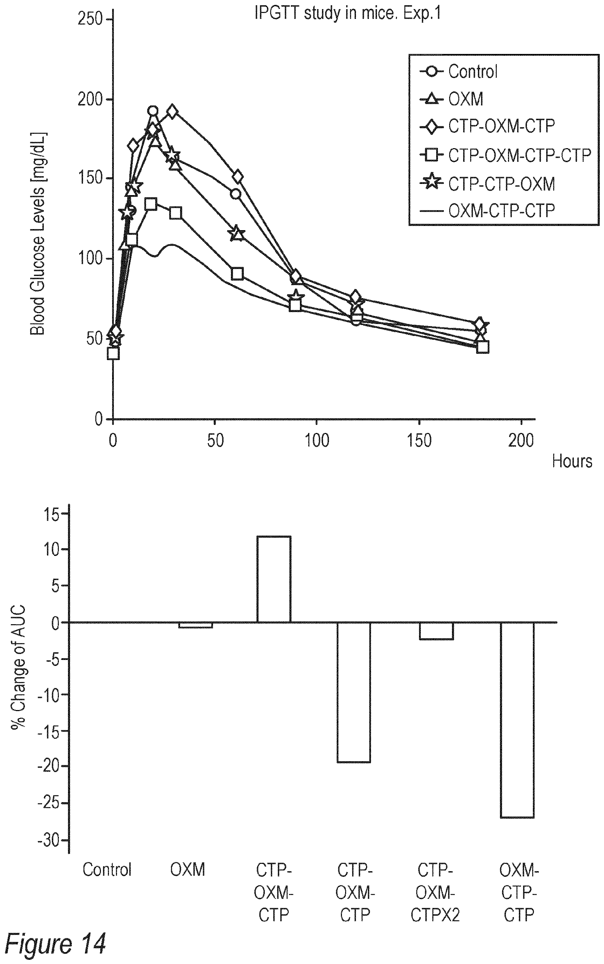

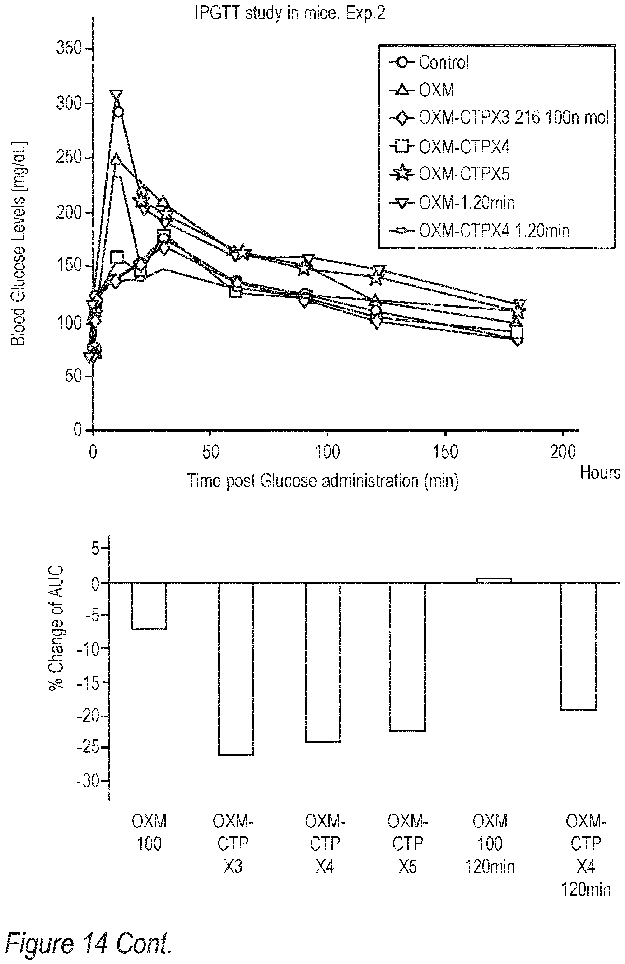

An IPGTT study was carried out in C57BL/6 mice demonstrated that OXM enhanced glucose clearance via stimulation of insulin secretion. The IP glucose tolerance test (IPGTT) evaluates the glucose lowering effect of OXM. In order to evaluate the *in vivo* activity of OXM or OXM-CTP variants, the IPGTT model was applied. Overnight fasted C57BL/6 mice were injected IP with OXM peptide or OXM-CTP variants followed by IP injection of glucose (1.5g/kg) and measurement of blood glucose levels from the tail vein by glucometer **(****Figure 14****).** OXM-CTP variants were assessed in two sequential experiments; Experiment 1 for variants CTP-OXM-CTP, CTP-OXM-CTP-CTP, OXM-CTP-CTP, CTP-CTP-OXM (Figures 14A and 14C) and Experiment 2 for variants OXM-CTP-CTP-CTP, OXM-CTPX4 and OXM-CTPX5 (Figures 14B and 14D). OXM (100 nmol/kg), or OXM-CTP variants (100 nmol/kg) were administered IP 15 min or 2 hrs (OXM peptide and variant OXM-CTPX4) prior to glucose IP injection, and the induction of glucose tolerance was compared to vehicle (buffer) group. A marked effect was measured for OXM-CTP variants CTP-OXM-CTP-CTP, OXM-CTP-CTP, OXM-CTPX3, OXM-CTPX4 and OXM-CTPX5 as reflected by reduction of 20-30% of blood glucose AUC compared to vehicle group as compared to 100nmol/kg of OXM peptide which had low impact on the calculated AUC **(****Figure 14****,** reduction of 1%, Experiment 1 and 6.7%, Experiment 2). Surprisingly, CTP-OXM-CTP resulted in increased glucose levels while CTP-CTP-OXM had a minor impact on glucose tolerance. OXM-CTPX4 induced glucose tolerance activity even when administered 120 min prior to glucose while OXM peptide activity was no longer apparent. This result is aligned with the improved pharmacokinetics profile of this variant.

### EXAMPLE 12: PRODUCTION OF CTP-MODIFIED POLYPEPTIDE OF INTEREST CONSTRUCTS

### Expression of CTP-modified polypeptide of interest

*Cell transfection (nucleofection):* Stable expression is achieved by integration of a gene for a CTP-modified polypeptide of interest into the target cell's chromosome: Initially the gene is introduced into the cell, subsequently into the nucleus and finally it is integrated into chromosomal DNA. After gene transfer, cells are cultivated in medium containing a selection marker such as dihydrofolate reductase (DHFR).

*Clone Selection* - Limiting Dilution of single cell by plating in 96-well plates. The selection ia applied to the single cell culture and may be repeated several times to obtain 100% clonal purity. Highest producing clone, based on growth curve, clone characterization, specific productivity and protein profile, are propagated in 1 L Shake flask prior to inoculation in bioreactor. Briefly:

A polypeptide of interest protein-producing cell line is manufactured by recombinant DNA technology. Animal component-free media is used throughout the derivation of the Master and Working Cell Banks (MCB; WCB). Transfection of mammalian cells, which may include selectable elements, for example dhfr-negative CD DG44 cells (CHO cells), which are adapted to protein-free medium and suspension growth, is carried out using a transfection reagent, for example FuGENE-6 (Roche Applied Science). Stable clones are isolated by limiting dilution steps in cell culture. The highest producing clones are amplified with increasing concentrations of a selectable agent, for example methotrexate (MTX). Based on clone population doubling level (PDL), productivity of CTP-modified polypeptide of interest (picogram per cell per day, PCD), and maximum attained cell density in the selected medium, the highest producing clones are isolated and will be used to prepare the R&D banks followed by manufacturing of a qualified Master Cell Bank (MCB) and Working Cell Bank (WCB).

### Upstream Process

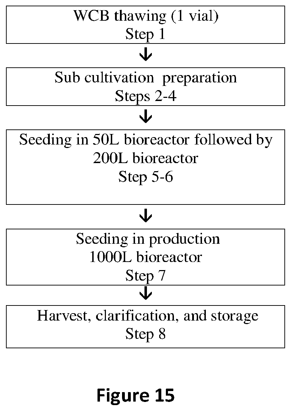

The upstream process for a CTP-modified polypeptide of interest consists of 2 types of medium formulations; growth medium (Medium 1) and production medium (Medium 2). Medium 1 includes an agent for selection, for example methotrexate (MTX), and Medium 2 is identical to Medium 1 excluding the agent for selection, for example MTX. Medium 1 is used for the cell culture propagation **(Step 1-4 of** **Figure 15****)** prior to seeding in a 200 Liter (L) bioreactor, and Medium 2 is used in N-2 steps (two propagations prior to the final product. "N" refers to the final product), a 200 L bioreactor **(Step 5 of** **Figure 15****)** and production bioreactor of 1000 L for up scaling **(Step 6 of** **Figure 15****).**

### Manufacturing Process: Polypeptide of Interest

A selected clone is manufactured at a 1000-L scale in serum-free culture medium. The culture of the given mammalian cell line, for example a CHO cell line, is expanded in several steps from a single vial of the working cell bank (WCB) to the final production culture volume. The production cell culture supernatant is tested for bioburden, bacterial endotoxin, productivity and adventitious virus. The process is performed using 50 L and 200 L bioreactors for seeding bioreactors and a 1000 Liter bioreactor for scaling-up. All product contact surfaces are disposable, while non-disposable product contact equipment are product dedicated. These pieces of equipment are cleaned and sanitized between batches. The culture is expanded to 50 L and 200 L bioreactors prior to inoculation in a 1000 L bioreactor. Final scale-up and fed-batch bioreactor production is performed in disposable bioreactors of 1000 L. Removal of the cells is accomplished using a disposable filter system (Millipore depth filter).

One or two vials of the WCB are thawed at 37°C. The cells are centrifuged at and the cell pellet is resuspended to a target concentration with 10 mL fresh Media low volume shake flask and incubated at 37± 0.5°C, 5±1% COz. After the 2nd subcultivation the cell culture with the higher viability is further expanded; the other cell culture is discarded. Four sub-cultivation steps in shake flask with increasing volumes are conducted with pre-defined step parameters such as seeding concentration and working volume. Volumes followed by two seeding bioreactor steps for a typical 1000 L bioreactor run.

The 50 L and 200 L seed bioreactors are used as seeding bioreactor. Prior to inoculation the 200 L bioreactor is filled with approx. 50 L of an appropriate cell medium, for example PowerCHO 2CD Medium 2 (w/o MTX). The process control is set to the parameters as follows: pH 7, Temperature 37_{°}C, DO 50%. These parameters apply to the medium pre-conditioning and to the seed train cultivation process.

When the process parameters are controlled within their pre-defined ranges the inoculum transfer is started. During cell mass expansion in the 50 L and 200 L seed bioreactors no feed addition is applied to the process. The expected cultivation time in the seed bioreactors is 3 to 4 days before cells are transferred into the 1000 L production bioreactor. Samples for in process control (IPC) are taken on a daily basis.

### Cell propagation in production bioreactor, 1000 Liter (Step 7, Figure 15)

The culture is incubated in the bioreactor for about 11days (dependent on the viability of the cells) at 37°C, 20% dissolved oxygen (DO) and pH 7.2. On Day 3 the pH is shifted to 6.9 until the harvest. On day 4, Feed (Power Feed A without Lipids) is added. Feed volume is equal to 33 % of the final bioreactor volume. On day 6, DMSO is added to the bioreactor. Glucose feed solution is added to the culture in order to maintain a desired concentration and a bolus of 1M of Sodium Bicarbonate is added in order to maintain a desired culture concentration (HCOs). The harvest is performed using predefined criteria. During the first four days, the cell culture is sampled daily for cell count, viability and metabolic analysis. From day 5, the culture is sampled twice-daily for cell count, viability and metabolic analysis and from Day 9 also for specific productivity by Reverse-Phase HPLC. Productivity of the CTP-modified polypeptide of interest is at least 500gr/L with the high glycosylated form consist of at least 70% of the total polypeptide of interest-CTP protein in the harvest.

The example presented herein is manufacturing the CTP-modified polypeptide of interest using a fedbatch mode but one skilled in the art could develop a perfusion mode using in general, similar purification scheme. Alternatively, one skilled in the art could develop a perfusion method wherein duration of incubation could be even up to 7-120 days.

### Cell harvest and storage (Step 7 of Figure 15)

The harvest is performed using a disposable filtration process train. To clarify the harvest a depth filtration and 0.2 µm filtration is performed. The clarification is followed by a 0.45/ 0.2 µm filtration. The depth filters are flushed and the residual liquid is blown out of the system with air. Filtration process is run with a pump speed of ≤ 15 L/min and a maximal defined pressure. Afterwards the filters are washed with Tris-HCl buffer and blown out with pressurized air to increase the product recovery.

The clarified harvest is tested for bioburden, bacterial endotoxin, specific protein content by RP-HPLC, SDS-PAGE, Western Blot, HPC Elisa assay, residual DNA, *in vitro* virus assay, Virus-like particles, S+L- and Mycoplasma.

### Purification Process

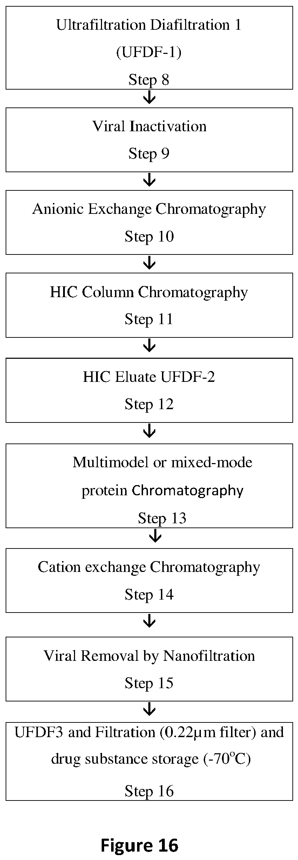

Purification scheme is described in **Figure 16****.**

### Ultrafiltration and Diafiltration 1 - UFDF1 (Step 8)

The Clarified Harvest is concentrated and diafiltered using a hollow fiber cartridge, or equivalent TFF based UFDF step. The cartridge nominal molecular weight cutoff size is 10,000 kDa. The concentrated and diafiltered harvest is tested for specific protein content by RP-HPLC and HCP Elisa.

### Viral Inactivation by Incubation (Step 9)

The material is filtered through a Millipore 1.55 m2 filter followed by a sterile Sartopore 2 XLG 10"filter into a sterile mixing bag (depth filtration step). Next, a solution is added to inactivate viral content, for example Tris / 10% Triton solution is added to the final filtrate volume bringing the Triton concentration to 1% (w/w). After incubation, before loading on an anion exchange column, the product solution is filtered with a 0.2 µm filter unit.

### Anion Exchange-Sepharose Fast Flow Chromatography (Step 10)

An anion exchange column packed with resin is used for this step. The column is packed in a pre-defined bed height. The specific protein in the load is determined prior to the addition of Triton due to the interference caused by the Triton in the assay. The anion exchange column is equilibrated and loaded with the viral inactivated pool and then washed. A second wash is conducted with and the material is eluted and then stored at ambient temperature (18-26°C) for processing the next day or at 2-8°C for longer time. All chromatography steps are conducted in down flow mode. Alternatively, steps could be run in an upflow mode. The eluate is tested for specific protein by RP-HPLC (Target: main peak elutes as single peak), HCP Elisa, SDS-PAGE, Western Blot and residual DNA.

### Hydrophobic Interaction Chromatography (HIC) Column Chromatography (Step 11)

An HIC Resin is used for this step. The column is packed column in a pre-defined bed height. The HIC chromatography is performed in 1 - 5 cycles depending on product quantity. Alternatively, up to 10 cycles could be run. The HIC load is prepared by adjusting the anion exchange column eluate with Ammonium Sulfate. The column is equilibrated and loaded with the adjusted and 0.2 µm filtered anion exchange eluate and then washed and the material is eluted, and then stored at 2-8°C until further processing.

### HIC Eluate Ultrafiltration and Diafiltration 2 (Step 12)

The HIC eluate is concentrated and diafiltered reduce the volume and prepare the material for the CHT Column Step. The HIC eluate is concentrated and then diafiltered. Once the pH and conductivity are determined to be in range, the system is drained and 0.45/0.2 µm filtered to a sterile bag. The final volume of concentrated, diafiltered HIC eluate is stored at ambient room temperature (18-26°C) overnight. The retentate is tested for bioburden, bacterial endotoxin and specific protein by A₂₈₀.

### Multimodel or mixed-mode protein Chromatography (Step 13)

A multimodel or mixed-mode protein chromatography column packed with resin is used for this step. The column is packed column in a pre-defined bed height. The column is equilibrated and loaded with the concentrated and diafiltrated, HIC eluate and washed with 4 column volumes (CV) buffer. The flow-through and wash material are collected and held overnight at ambient temperature (18-26°C) until further processing.

### Cation Exchange-Sepharose Chromatography (Step 14)

A column packed with Cation-Exchange Sepharose Resin is used for this step. The column is packed column in a pre-defined bed height. The load is prepared by adjusting the Multimodal or Mixed-Mode Protein Chromatography flow-through fraction. Following the pH adjustment the solution is loaded onto the column followed by a wash step. The flow-through is collected for further processing. The pH is adjusted and the material is filtered through a 0.45/0.2µm filter. The material is stored at ambient temperature (18-26°C) overnight or at 2-8°C for up to 24 hours. All chromatography steps are done in downflow mode.

### Viral Inactivation by Nanofiltration (Step 15)

The Viral Filtration is performed using an Asahi Planova 20N Virus filter. A Sartopore 2 filter with a 0.45 / 0.2 µm or 0.1 µm membrane is used as prefilter of the nanofilter. The Virus filter is pre-equilibrated and primed with the final formulation made with WFI. The adjusted SP-Flow-Through is passed through the filter train at a continuous pressure and collected in a sterile bioprocess bag. The filter train is flushed with formulation buffer to maximize product recovery. The filter is integrity tested pre and post use per the manufacturer's recommended procedures. The Post use test includes a gold-particle test, also per the manufacturer's procedure.

### UFDF3 and Filtration and Storage of the Drug Substance (Step 16)

The Viral Filtrate is concentrated to a target the final DS concentration (which can vary from 5-100 mg/ml) in preparation for the Bulk Filtration and Fill. A single used or reusable cassette is used for this step with a cut-off of 3-30KDa. The product is concentrated in a first step to 5-25mg/ml and diafiltrated to 10mM Citrate, 147mM NaCl pH 6.4 (≥7 DF volumes). A final product concentration is adjusted and filtered with a Millipak 100 filter.The filtrated product solutions are aliquoted and frozen at a temperature of -70 ± 5 °C.

### Drug Product Manufacturing

The formulation of the drug product (DP) process starts with the thawing of Drug substance (DS). Drug Product is achieved by dilution of the Drug Substance (DS) to the required concentration using the formulation buffer, aseptic filtration and filling in standard 2R vials or other primary packaging such as cartridges or pre-filled syringes. A skilled artisan would appreciate that the term "drug substance" (DS) may encompass or be equivalent to the active pharmaceutical ingredient (API). In one embodiment, a CTP-modified polypeptide of interest, as set forth herein, is a drug substance (DS) comprising the bulk purified drug. The skilled artisan would also appreciate that the term "drug product" (DP) may encompass the finally formulated drug once dispensed into a final container, for example a vial, under aseptic conditions. In one embodiment, a CTP-modified polypeptide of interest, as set forth herein, is a drug product (DP) comprising the finally formulated CTP-modified polypeptide of interest.

### Characterization of CTP-modified polypeptide of interest

CTP-modified polypeptide content in the harvest and the percentage of the high glycosylated forms are determined by a specific RP-HPLC method. Total protein in the harvest is determined by Bradford analysis. The specific protein percentage in the harvest produced by a selected clone is above 70% relative to the total protein in the harvest. In addition, the manufacturing upstream process is developed to enable high percentage of the highly glycosylated CTP-modified protein compared to the low glycosylated form. The highly glycosylated form is the target form, as it results longer extension of the CTP-modified polypeptide half-life.

### O-Glycan Content

Glycoprofiling is performed by releasing glycans followed by glycan labeling with 2-aminobenzamide (2AB), cleaned up and analyzed by NP-HPLC. Briefly, an O-glycan content assay is conducted to calculate the number of O-glycans mol per mol of a CTP-modified polypeptide of interest. The terminal galactose units of the O-glycans are enzymatically cleaved from the protein by β-galactosidase. These free galactose units are separated on a CarboPac PA20-column and detected with pulsed amperometry. Galactose (Gal) is quantified using external calibration with a galactose reference standard. The content of galactose can be directly related to the content of O-glycan structure, Gal-GalNAc. Analysis of drug substance and drug product batches demonstrate a robust batch to batch consistency. This unexpected robust glycosylation content is significant, showing that the number of O-glycans per CTP is improved over that known in the art.

### Intact molecular weight analysis samples

Molecular weight analysis of different DS batches is performed with the aim to obtain information on the number of O-linked glycosylation sites. Intact samples as well as de-sialylated samples using Neuramnidase, and de-O-glycosylated samples using O-glycosidase, are analyzed by on-line LC/ES-MS. The result showing a high % of serine occupancy is unexpected in comparison with levels known in the art (only 4 serines glycosylated compared with up to 6 in the CTP-modified polypeptide of interest manufactured herein).

### O-linked glycosylation site occupancy of CTP-modified protein samples

O-glycosylation site occupancy of 4 different DS batches is performed at M-scan with the aim to obtain information on the number of O-linked glycosylation sites per molecule. Samples are de-sialylated using Neuramnidase followed by tryptic digestion of reduced/carboxymethylaed samples. Finally an on line LC/ES-MS is carried out for the treated samples and interpretation of the MS data is conducted using a designated software. Evaluation of the data obtained from analysis of the tryptic digest mixtures leads to signals allowing 100% of the protein sequence being mapped. O-glycosylation may take place on both the N-terminal and C-terminal CTP region. Sites of occupancy are identified as serine residues following proline as well as two of the four serines in the regions of serine repeats. A total of up to 18 serine residues may serve as attachment sites for O-glycans. No significant differences between the batches are detected.

### Purity

RP-HPLC separates molecules according to their polarity. A mobile phase gradient from a more polar to a less polar solvent is used to elute molecules with a strong polarity earlier than less polar molecules. The related forms are separated from the native protein using UV detection at 220 nm. The relative peak areas (area%) of the related forms and the main peak can be calculated by integrating the corresponding peak areas. The main peak of Drug Substance and Drug Product consists of more than 97% peak area, indicating a highly purified product and an effective purification process.

Size Exclusion HPLC is a chromatographic technique that separates molecules according to size. Within the fractionation range chosen, larger molecules elute earlier than smaller molecules. The separation mechanism is non-adsorptive and molecules are eluted under isocratic conditions. SEC enables monomers to be separated from higher molecular weight forms (such as dimers and polymers) of the target molecule. The SEC method is developed to analyze the content of dimers and polymers in Drug Substance and Drug Product.

### RP-HPLC content method

This method is being used for the content determination of intermediate samples and the determination of %-unglycosylated CTP-modified polypeptide in intermediate samples by reversed phase chromatography. The reversed phase-HPLC separates molecules due to their polarity. Relatively non-polar molecules ligate to the column material while charged and polar molecules are eluted without accomplishing an interaction with the column.

The ligated molecules are eluted with the aid of a gradient from a polar to a less polar solution. Molecules of the strongest polarity eluted first followed by the less polar molecules. The detection is carried out via absorption at 214 nm.

### Viral Clearance

The ability of the manufacturing process to address and mitigate contamination of final drug product with endogenous and adventitious virus has been the subject of a preliminary evaluation. A GLP-compliant study has been conducted according to applicable guidance for investigational products using three model viruses spiked into scaled down segments of the manufacturing process to quantify the ability of these steps to inactivate or clear the spiked virus population. With the amounts of virus expressed as log10 Adjusted Titre, the log10 clearance factor is determined simply by subtracting the value for output from the value for input. As log10 numbers the clearance factors are additive to derive an overall clearance factor for all evaluated steps. A-MuLV is considered to be a model virus representing possible presence of CHO retroviruses, the measures taken to inactivate and remove contaminating A-MuLV virus achieved clearance factor of at least antilog10, e.g. viral log reduction factor (LRF) of about 22, demonstrating that the overall process has an exceptional capacity for viral removal. For the PPV which is a resistant non-enveloped small virus, robust removal by the nanofiltration step is obtained.

While certain features disclosed herein have been illustrated and described herein, many modifications, substitutions, changes, and equivalents will now occur to those of ordinary skill in the art. It is, therefore, to be understood that the appended claims are intended to cover all such modifications and changes as fall within the true spirit disclosed herein.

Embodiments of the invention, corresponding to the original embodiments of PCT/IL2015/051197, published as WO 2016/092550, include the following:
1. A method of manufacturing a human CTP-modified polypeptide of interest, the method comprising the steps of:
   (a) stably transfecting a predetermined number of cells with an expression vector comprising a coding portion encoding said CTP-modified polypeptide of interest;
      (i) wherein said transfected cells express and secrete said CTP-modified polypeptide of interest;
   (b) obtaining cell clones that overexpress said CTP-modified polypeptide of interest;
   (c) expanding said clones in solution to a predetermined scale;
   (d) harvesting said solution containing said clones;
   (e) filtering said solution containing said clones to obtain a clarified harvest solution; and,
   (f) purifying said clarified harvest solution to obtain a purified protein solution having a desired concentration of a CTP-modified polypeptide of interest;
   thereby manufacturing the CTP-modified polypeptide of interest.
2. The method of embodiment 1, wherein said purified protein solution contains at least 70% CTP-modified polypeptide of interest.
3. The method of any one of embodiments 1-2, wherein said CTP-modified polypeptide of interest is highly glycosylated.
4. The method of embodiment 3, wherein the glycosylation pattern of each CTP comprises at least glycosylation at 4 O-linked glycosylation sites.
5. The method of any one of embodiments 1-4, wherein said CTP-modified polypeptide of interest is highly sialylated.
6. The method of any one of embodiments 1-5, wherein when said polypeptide of interest is an erythropoietin (EPO), an interferon (IFN), or a glucagon-like peptide 1 (GLP-1), said CTP-modified polypeptide of interest polypeptide consists of two CTPs attached to the carboxy terminus of said polypeptide of interest, and one CTP attached to the amino terminus of said polypeptide of interest.
7. The method of any one of embodiments 1-5, wherein when said polypeptide of interest is a coagulation factor, a coagulation Factor VII (FVII), an activated coagulation Factor VII (FVIIa) or a coagulation Factor IX (FIX), said CTP-modified polypeptide of interest polypeptide consists of one to five CTPs attached to the carboxy terminus of said polypeptide of interest.
8. The method of embodiment 7, wherein said CTP-modified polypeptide of interest polypeptide consists of three CTPs attached to the carboxy terminal of said polypeptide.
9. The method of any one of embodiments 1-5, wherein when said polypeptide of interest is a dual GLP-1/Glucagon receptor agonist or oxyntomodulin (OXM), said CTP-modified polypeptide of interest polypeptide consists of one to five chorionic CTPs attached to the carboxy terminus of said polypeptide of interest and/or one to five CTPs attached to the amino terminus of said polypeptide of interest.
10. The method of any one of embodiments 1-9, wherein in said step (a)(i) the secreted CTP-modified polypeptide of interest lack a signal peptide.
11. The method of any one of embodiments 1-10, wherein said step (c) comprises expanding clones from a working cell bank (WCB) that optimally expresses and secretes said CTP-modified polypeptide of interest.
12. The method of any one of embodiments 1-10, wherein said step (c) comprises expanding clones obtained from a master cell bank (MCB) that optimally expresses and secretes said CTP-modified polypeptide of interest.
13. The method of any one of embodiments 1-12, wherein said method of manufacturing is an animal-free process.
14. The method of any one of embodiments 1-13, wherein at step (c) said CTP-modified polypeptide of interest is expressed at a level of at least 30 mg/ml.
15. The method of any one of embodiments 1-14, wherein at step (c) expanding said clones in solution comprises expanding said clones in solution through a series of sub-cultivating steps up to production bioreactor level.
16. The method of embodiment 15, wherein said bioreactor comprises a disposable bioreactor, a stainless steel bioreactor, a fed-batch mode bioreactor, a batch mode bioreactor, a repeated batch mode bioreactor, or a perfusion mode bioreactor, or any combination thereof.
17. The method of any one of embodiments 1-16, wherein at step (f) at least 60% of the CTP-modified polypeptides of interest in said clarified harvest comprises a high glycosylation form of said CTP-modified polypeptide.
18. The method of any one of embodiments 1-17, wherein said purification (step f) comprises sequentially performing the following steps comprising:
   (g) concentrating, diafiltering and purifying said clarified harvest solution;
      i.wherein said concentration, diafiltration and purifying is accomplished by sequentially passing said clarified harvest solution through an anion exchange column and a hydrophobic interaction column;
   (h) obtaining said clarified harvest obtained following step (g) and inactivating viruses present in said clarified harvest by incubating in a solution toxic to said viruses;
   (i) obtaining said clarified harvest solution from step (h) and concentrating, diafiltering, and purifying said clarified harvest solution,
      i. wherein said concentration, diafiltration and purifying is followed by sequentially passing said clarified harvest solution through a multimodal or mixed-mode protein chromatography column and a cation exchange column;
   (j) obtaining said clarified harvest solution following step (i) and physically removing said clarified harvest solution from viruses by nanofiltration; and
   (k) obtaining said clarified harvest solution following step (j) and concentrating and diafiltering said clarified harvest solution to arrive at a maximally purified clarified harvest containing said highly glycosylated form of CTP-modified polypeptide of interest.
19. The method of any one of embodiments 1-18, wherein said method achieves at least a 20% recovery rate of highly glycosylated CTP-modified polypeptide of interest.
20. The method of embodiment 18, wherein said viral clearance shows a viral log reduction factor (LRF) of more than 12.
21. The method of any one of embodiments 1-20, further comprising a step of characterizing said CTP-modified polypeptide, said characterizing comprises determining O-glycan content, determining O-glycan occupancy, or determining purity of said CTP-modified polypeptide, or any combination thereof.
22. A CTP-modified polypeptide of interest manufactured by a method comprising the steps of:
   (a) transfecting a predetermined number of cells with an expression vector comprising a coding portion encoding said CTP-modified polypeptide of interest;
      i. wherein said transfected cell expresses and secretes said CTP-modified polypeptide of interest;
   (b) obtaining cell clones that overexpress said CTP-modified polypeptide of interest;
   (c) expanding said clones in solution to a predetermined scale;
   (d) harvesting said solution containing said clones;
   (e) filtering said solution containing said clones to obtain a clarified harvest solution; and,
   (f) purifying said clarified harvest solution to obtain a purified protein solution having a desired concentration of a CTP-modified polypeptide of interest.
23. The CTP-modified polypeptide of interest of embodiment 22, wherein said CTP-modified polypeptide of interest is highly glycosylated.
24. The CTP-modified polypeptide of interest of any one of embodiments 22-23, wherein the glycosylation pattern of each CTP comprises at least glycosylation at 4 O-linked glycosylation sites.
25. The CTP-modified polypeptide of interest of any one of embodiments 22-24, wherein said CTP-modified polypeptide of interest is highly sialylated.
26. The CTP-modified polypeptide of interest of any one of embodiments 22-25, wherein the secreted form lacks a signal peptide.
27. A composition comprising the CTP-modified polypeptide of interest of any one of embodiments 22-26, and a pharmaceutically acceptable carrier.
28. A human chorionic gonadotropin C-terminal peptide (CTP)-modified polypeptide of interest having increased glycosylation occupancy, wherein said glycosylation occupancy comprises more than 4 O-glycans per CTP unit.
29. The human CTP-modified polypeptide of interest of embodiment 28, wherein the purity of the CTP-modified polypeptide of interest is at least 70%.
30. The human CTP-modified polypeptide of interest of any one of embodiments 28-29, wherein said CTP-modified polypeptide of interest is highly sialylated.

## Claims

1. A method of manufacturing a highly glycosylated human chorionic gonadotropin carboxy terminal peptide (CTP)-modified polypeptide of interest, wherein the highly glycosylated CTP-modified polypeptide of interest is glycosylated at between 13 and 18 O-linked glycosylation sites, the method comprising the steps of:
(a) stably transfecting a predetermined number of cells with an expression vector comprising a coding portion encoding said CTP-modified polypeptide of interest;
i. wherein said polypeptide of interest is a coagulation Factor VII (FVII) or an activated coagulation Factor VII (FVIIa),
ii. wherein said CTP-modified polypeptide of interest consists of three CTP units attached to the carboxy terminus of said FVII or said FVIIa, and
iii. wherein said transfected cells express and secrete said CTP-modified polypeptide of interest;
(b) obtaining cell clones that overexpress said CTP-modified polypeptide of interest;
(c) expanding said clones in solution to a predetermined scale and said clones are obtained from a working cell bank (WCB) or master cell bank (MCB) that optimally expresses and secretes said CTP-modified polypeptide of interest;
(d) harvesting said solution containing said clones;
(e) filtering said solution containing said clones to obtain a clarified harvest solution; and,
(f) purifying said clarified harvest solution to obtain a purified protein solution of a CTP-modified polypeptide of interest, wherein at least 70%, of the purified CTP-modified polypeptide of interest is the highly glycosylated form of the CTP-modified polypeptide of interest;
thereby manufacturing a highly glycosylated CTP-modified polypeptide of interest.

2. The method of claim 1, wherein said step (c) comprises expanding clones obtained from a working cell bank (WCB) or master cell bank (MCB) that optimally expresses and secretes said CTP-modified polypeptide of interest.

3. The method of claim 1 or 2, wherein said CTP-modified polypeptide is highly sialylated.

4. The method of any of claims 1-3, wherein in said step (a)(i) the secreted CTP-modified polypeptide of interest lacks a signal peptide.

5. The method of any of claim 1-4, wherein said method of manufacturing is an animal-free process.

6. The method of any of claims 1-5, wherein at step (c) said clones express and secrete the highly glycosylated CTP-modified polypeptide of interest at a level of at least 600 mg/ml.

7. The method of any of claims 1-6, wherein at step (c) expanding said clones in solution comprises expanding said clones in solution through a series of sub-cultivating steps up to production bioreactor level.

8. The method of claim 7, wherein said bioreactor comprises a disposable bioreactor, a stainless steel bioreactor, a fed-batch mode bioreactor, a batch mode bioreactor, a repeated batch mode bioreactor, or a perfusion mode bioreactor, or any combination thereof.

9. The method of any of claims 1-8, wherein said purification (step f) comprises sequentially performing the following steps comprising:
(g) concentrating and diafiltering said clarified harvest solution;
(h) obtaining said clarified harvest obtained following step (g) and inactivating viruses present in said clarified harvest by incubating in a solution toxic to said viruses;
(i) obtaining said clarified harvest solution from step (h) and purifying said clarified harvest solution,
i. wherein said purifying is accomplished by sequentially passing said clarified harvest solution through an anion exchange column and a hydrophobic interaction column, followed by a concentrating and diafiltering step,
ii. wherein said purifying is followed by sequentially passing said clarified harvest solution through a Hydroxyapatite mixed-mode column and a cation exchange column;
(j) obtaining said clarified harvest solution following step (ii) and physically removing said clarified harvest solution from viruses by nanofiltration; and
(k) obtaining said clarified harvest solution following step (j) and concentrating and diafiltering said clarified harvest solution to arrive at a maximally purified clarified harvest containing said CTP-modified polypeptide of interest.

10. The method according to claim 9, wherein:
a. said anion exchange column is a DEAE- Sepharose column;
b. said hydrophobic column is a Phenyl HIC column;
c. said solution toxic to said viruses is a 1% Triton-X 100 solution;
d. said cation exchange column is a SP-Sepharose column; or
e. said viral clearance shows a viral log reduction factor (LRF) of about 22.

11. The method of any of claims 1-10 wherein said method achieves at least a 20% recovery rate of highly glycosylated CTP-modified polypeptide of interest.

12. The method of any of claims 1 to 11, wherein the purity of the highly glycosylated CTP-modified polypeptide of interest protein is at least 90%.

13. The method of any of claims 1 to 12, wherein the glycosylation pattern of said manufactured CTP-modified polypeptide of interest comprises glycosylation of 4 to 6 O-linked glycosylation sites per CTP.

14. The method of any of claims 1-13, further comprising a step of characterizing said CTP-modified polypeptide, said characterizing comprises determining O-glycan content, determining O-glycan occupancy, or determining purity of said CTP-modified polypeptide, or any combination thereof.

15. The method of any of claims 1-14 further comprising a step of formulating said CTP-modified polypeptide of interest with a pharmaceutically acceptable carrier.
